# EUROPEAN PATENT SPECIFICATION

(11) **EP 1 461 079 B1**
(45) Date of publication and mention of the grant of the patent: **10.08.2011**
(21) Application number: 02770472.5
(22) Date of filing: 06.09.2002
(51) Int. Cl.: A61K 39/21, A61K 39/12, C07K 16/00

(54) **HUMAN IMMUNODEFICIENCY VIRUS ENVELOPE CLYCOPROTEIN MUTANTS AND USES THEREOF**
MUTANTEN VON HÜLLGLYKOPROTEINEN DES HUMAN IMMUNODEFICIENCY VIRUS UND DEREN VERWENDUNG
MUTANTS DE GLYCOPROTEINES D'ENVELOPPE DU VIH ET LEURS UTILISATIONS

(30) Priority: 06.09.2001 US 317909 P; 06.09.2001 US 317764 P; 06.09.2001 US 317910 P; 06.09.2001 US 317775 P; 05.04.2002 US 370264 P; 05.04.2002 US 370410 P
(43) Date of publication of application: 29.09.2004
(73) Proprietor: PROGENICS PHARMACEUTICALS, INC., Tarrytown, NY 10591 (US); CORNELL RESEARCH FOUNDATION, INC., Ithaca, NY 14850 (US)
(72) Inventor: MOORE, John P., New York, NY 10021 (US); BINLEY, James, M., San Diego, CA 92122 (US); LU, Min, New York, NY 10021 (US); OLSON, William, C., Ossining, NY 10562 (US); SCHULKE, Norbert, New City, NY 10956 (US); GARDNER, Jason, Ardsley, NY 10591 (US); MADDON, Paul, J., Scarsdale, NY 10583 (US); SANDERS, Rogier, San Diego CA 92101 (US)
(74) Representative: Müller Fottner Steinecke
(86) International application number: PCT/US2002/028331
(87) International publication number: WO 2003/022869

(56) References cited:
- WO-A-01/00648
- US-B1- 6 171 596
- BINLEY J M ET AL: "A recombinant human immunodeficiency virus type 1 envelope glycoprotein complex stabilized by an intermolecular disulfide bond between the gp120 and gp41 subunits is an antigenic mimic of the trimeric virion-associated structure" JOURNAL OF VIROLOGY, THE AMERICAN SOCIETY FOR MICROBIOLOGY, US, vol. 74, no. 2, January 2000 (2000-01), pages 627-643, XP002364345 ISSN: 0022-538X
- SANDERS R W ET AL: "Variable-loop-deleted variants of the Human Immunodeficiency Virus Type 1 envelope glycoprotein can be stabilized by an intermolecular disulfide bond between the gp120 and gp41 subunits" JOURNAL OF VIROLOGY, THE AMERICAN SOCIETY FOR MICROBIOLOGY, US, vol. 74, no. 11, June 2000 (2000-06), pages 5091-5100, XP002210876 ISSN: 0022-538X
- DITZEL H J ET AL: "Mapping the protein surface of human immunodeficiency virus type 1 gp120 using human monoclonal antibodies from phage display libraries" JOURNAL OF MOLECULAR BIOLOGY, LONDON, GB, vol. 267, no. 3, 4 April 1997 (1997-04-04), pages 684-695, XP004462174 ISSN: 0022-2836
- BINLEY JAMES M ET AL: "Enhancing the proteolytic maturation of human immunodeficiency virus type 1 envelope glycoproteins." JOURNAL OF VIROLOGY. MAR 2002, vol. 76, no. 6, March 2002 (2002-03), pages 2606-2616, XP002393503 ISSN: 0022-538X
- SCHÜLKE NORBERT ET AL: "Oligomeric and conformational properties of a proteolytically mature, disulfide-stabilized human immunodeficiency virus type 1 gp140 envelope glycoprotein." JOURNAL OF VIROLOGY. AUG 2002, vol. 76, no. 15, August 2002 (2002-08), pages 7760-7776, XP002393504 ISSN: 0022-538X
- SANDERS ROGIER W ET AL: "Stabilization of the soluble, cleaved, trimeric form of the envelope glycoprotein complex of human immunodeficiency virus type 1." JOURNAL OF VIROLOGY. SEP 2002, vol. 76, no. 17, September 2002 (2002-09), pages 8875-8889, XP002393505 ISSN: 0022-538X

## Description

### Background of the Invention

### I. Viral envelope glycoproteins

The human immunodeficiency virus (HIV) is the agent that causes Acquired Immunodeficiency Syndrome (AIDS), a lethal disease characterized by deterioration of the immune system. The initial phase of the HIV replicative cycle involves the attachment of the virus to susceptible host cells followed by fusion of viral and cellular membranes.

These events are mediated by the exterior viral envelope glycoproteins, which are first synthesized as a fusion-incompetent precursor envelope glycoprotein (env) known as gp160. The gp160 glycoprotein is endoproteolytically processed to the mature envelope glycoproteins gp120 and gp41, which are noncovalently associated with each other in a complex on the surface of the virus. The gp120 surface protein contains the high affinity binding site for human CD4, the primary receptor for HIV, as well as domains that interact with fusion coreceptors, such as the chemokine receptors CCR5 and CXCR4. The gp41 protein spans the viral membrane and contains at its amino-terminus a sequence of amino acids important for the fusion of viral and cellular membranes.

The native, fusion-competent form of the HIV-1 envelope glycoprotein complex is a trimeric structure composed of three gp120 and three gp41 subunits. The receptor-binding (CD4 and co-receptor) sites are located in the gp120 moieties, and the fusion peptides in the gp41 components (Chan, 1997; Kwong, 1998; Kwong, 2000; Poignard, 2001; Tan, 1997; Weissenhorn, 1997; and Wyatt, 1998a).

In the generally accepted model of HIV-1 fusion, the sequential binding of gp120 to CD4 and a co-receptor induces a series of conformational changes in the gp41 subunits, leading to the insertion of the fusion peptides into the host cell membrane in a highly dynamic process (Doms, 2000; Jones, 1998; Melikyan, 2000; Sattentau, 1991; Sullivan, 1998; Trkola, 1996; Wu, 1996; Wyatt, 1998b; and Zhang, 1999). The associations between the six components of the fusion-competent complex are maintained via non-covalent interactions between gp120 and gp41, and between the gp41 subunits (Poignard, 2001; and Wyatt, 1998b). These interactions are relatively weak, making the fusion-competent complex unstable. This instability perhaps facilitates the conformational changes in the various components that are necessary for the fusion reaction to proceed efficiently, but it greatly complicates the task of isolating the native complex in purified form. Put simply, the native complex falls apart before it can be purified, leaving only the dissociated subunits.

Because of their location on the virion surface and central role in mediating viral entry, the HIV envelope glycoproteins provide important targets for HIV vaccine development. Although most HIV-infected individuals mount a robust antibody (Ab) response to the envelope glycoproteins, most anti-gp120 and anti-gp41 antibodies produced during natural infection bind weakly or not at all to virions and are thus functionally ineffective. These antibodies are probably elicited and affinity matured against "viral debris" comprising gp120 monomers or improperly processed oligomers released from virions or infected cells. (Burton, 1997).

Several preventive HIV-1 subunit vaccines have been tested in Phase I and II clinical trials and a multivalent formulation is entering Phase III testing. These vaccines have contained either monomeric gp120 or unprocessed gp160 proteins. In addition, the vaccines mostly have been derived from viruses adapted to grow to high levels in immortalized T cell lines (TCLA viruses). These vaccines have consistently elicited antibodies which neutralize the homologous strain of virus and some additional TCLA viruses. However, the antibodies do not potently neutralize primary HIV-1 isolates (Mascola, 1996). Compared with TCLA strains, the more clinically relevant primary isolates typically possess a different cellular tropism, show a different pattern of coreceptor usage, and have reduced sensitivity to neutralization by soluble CD4 and antibodies. These differences primarily map to the viral envelope glycoproteins (Moore, 1995).

### The importance of oligomerization in envelope glycoprotein structure

There is a growing awareness that current-generation HIV subunit vaccines do not adequately present key neutralization epitopes as they appear on virions (Parren, 1997). There are several ways in which the native structure of virions affects the presentation of antibody epitopes. First, much of the surface area of gp120 and gp41 is occluded by inter-subunit interactions within the trimer. Hence several regions of gp120, especially around the N- and C-termini, that are well exposed (and highly immunogenic) on the monomeric form of the protein, are completely inaccessible on the native trimer (Moore, 1994a). This means that a subset of antibodies raised to gp120 monomers are irrelevant, whether they arise during natural infection (because of the shedding of gp120 monomers from virions or infected cells) or after gp120 subunit vaccination. This provides yet another level of protection for the virus; the immune system is decoyed into making antibodies to shed gp120 that are poorly reactive, and hence ineffective, with virions.

A second, more subtle problem is that the structure of key gp120 epitopes can be affected by oligomerization. A classic example is provided by the epitope for the broadly neutralizing human MAb IgG1b12 (Burton, 1994). This epitope overlaps the CD4-binding site on gp120 and is present on monomeric gp120. However, IgG1b12 reacts far better with native, oligomeric gp120 than might be predicted from its monomer reactivity, which accounts for its unusually potent neutralization activity. Thus, the IgG1b12 epitope is oligomer-dependent, but not oligomer-specific.

The converse situation is more common, unfortunately. Many antibodies that are strongly reactive with CD4-binding site-related epitopes on monomeric gp120 fail to react with the native trimer, and consequently do not neutralize the virus. In some undefined way, oligomerization of gp120 adversely affects the structures recognized by these monoclonal antibodies (Mabs)(Fouts, 1997).

A third example of the problems caused by the native structure of the HIV-1 envelope glycoproteins is provided by gp41 MAbs. Only a single gp41 MAb (2F5) is known to have strong neutralizing activity against primary viruses (Trkola, 1995), and among those tested, 2F5 alone is thought to recognize an intact, gp120-gp41 complex (Sattentau, 1995). All other gp41 MAbs that bind to virions or virus-infected cells probably react with fusion-incompetent gp41 structures from which gp120 has dissociated. Since the most stable form of gp41 is this post-fusion configuration (Weissenhorn, 1997), it can be supposed that most anti-gp41 antibodies are raised (during natural infection or after gp160 vaccination) to an irrelevant gp41 structure that is not present on the pre-fusion form.

Despite these protective mechanisms, most HIV-1 isolates are potently neutralized by a limited subset of broadly reactive human MAbs, so induction of a relevant humoral immune response is not impossible. Mab IgG1b12 blocks gp120-CD4 binding; a second (2G12; Trkola, 1996) acts mostly by steric hindrance of virus-cell attachment; and 2F5 acts by directly compromising the fusion reaction itself. Critical to understanding the neutralization capacity of these MAbs is the recognition that they react preferentially with the fusion-competent, oligomeric forms of the envelope glycoproteins, as found on the surfaces of virions and virus-infected cells (Parren, 1998). This distinguishes them from their less active peers. The limited number of MAbs that are oligomer-reactive explains why so few can neutralize primary viruses. Thus, with rare exceptions, neutralizing anti-HIV antibodies are capable of binding infectious virus while non-neutralizing antibodies are not (Fouts, 1998). Neutralizing antibodies also have the potential to clear infectious virus through effector functions, such as complement-mediated virolysis.

### Modifying the antigenic structure of the HIV envelope glycoproteins

HIV-1 has evolved sophisticated mechanisms to shield key neutralization sites from the humoral immune response, and in principle these mechanisms can be "disabled" in a vaccine. One example is the V3 loop, which for TCLA viruses in particular is an immunodominant epitope that directs the antibody response away from more broadly conserved neutralization epitopes. HIV-1 is also protected from humoral immunity by the extensive glycosylation of gp120. When glycosylation sites were deleted from the V1/V2 loops of SIV gp120, not only was a neutralization-sensitive virus created, but the immunogenicity of the mutant virus was increased so that a better immune response was raised to the wild-type virus (Reitter, 1998). Similarly, removing the V1/V2 loops from HIV-1 gp120 renders the conserved regions underneath more vulnerable to antibodies (Cao, 1997), although it is not yet known whether this will translate into improved immunogenicity.

Of note is that the deletion of the V1, V2 and V3 loops of the envelope glycoproteins of a TCLA virus did not improve the induction of neutralizing antibodies in the context of a DNA vaccine (Lu, 1998). However, the instability of the gp120-gp41 interaction, perhaps exacerbated by variable loop deletions, may have influenced the outcome of this experiment. By increasing the time that the gp120-gp41 complex is presented to the immune system, stabilized envelope proteins expressed in vivo provide a means in principle to significantly improve upon the immune response elicited during natural infection.

### Native and non-native oligomeric forms of the HIV envelope glycoproteins

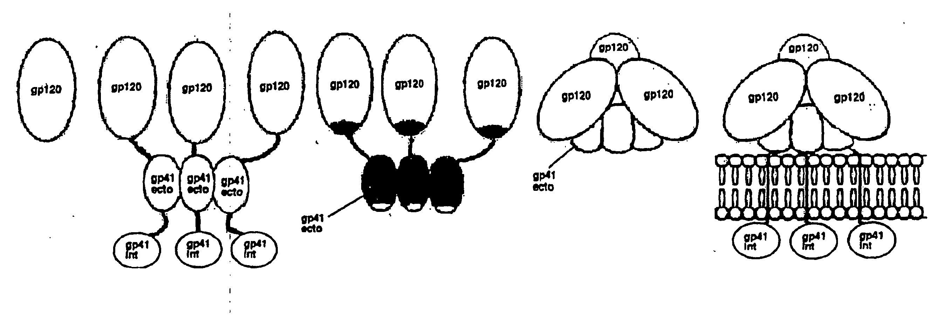

Current data suggest that on the HIV virion three gp120 moieties are non-covalently associated with three, underlying gp41 components in a meta-stable configuration whose fusion potential is triggered by interaction with cell surface receptors. This pre-fusion form may optimally present neutralization epitopes. We refer to this form of the envelope glycoproteins as native gp120-gp41 However, other oligomeric forms are possible, and these are defined in Figure 1.

gp160: The full-length gp160 molecule often aggregates when expressed as a recombinant protein, at least in part because it contains the hydrophobic transmembrane domain. One such molecule is derived from a natural mutation that prevents the processing of the gp160 precursor to gp120/gp41 (VanCott, 1997). The gp160 precursor does not mediate virus-cell fusion and is a poor mimic of fusion-competent gp120/gp41. When evaluated in humans, recombinant gp160 molecules offered no advantages over gp120 monomers (Gorse, 1998).

Uncleaved gp140 (gp140UNC): Stable "oligomers" have been made by eliminating the natural proteolytic site needed for conversion of the gp160 precursor protein into gp120 and gp41 (Berman, 1989; and Earl, 1990). To express these constructs as soluble proteins, a stop codon is inserted within the env gene to truncate the protein immediately prior to the transmembrane-spanning segment of gp41. The protein lacks the transmembrane domain and the long, intracytoplasmic tail of gp41, but retains the regions important for virus entry and the induction of neutralizing antibodies. The secreted protein contains full-length gp120 covalently linked through a peptide bond to the ectodomain of gp41. The protein migrates in SDS-PAGE as a single species with an apparent molecular mass of approximately 140 kilodaltons (kDa) under both reducing and nonreducing conditions. The protein forms higher molecular weight noncovalent oligomers, likely through interactions mediated by the gp41 moieties.

Several lines of evidence suggest that the uncleaved gp140 molecule does not adopt the same conformation as native gp120-gp41. These include observations that uncleaved gp120-gp41 complexes do not avidly bind fusion co-receptors. Furthermore, a gp140 protein was unable to efficiently select for neutralizing MAbs when used to pan a phage-display library, whereas virions were efficient (Parren, 1996). We refer to the uncleaved gp120-gp41 ectodomain material as gp140UNC.

Cleavable but uncleaved gp140 (gp140NON): During biosynthesis, gp160 is cleaved into gp120 and gp41 by a cellular endoprotease of the furin family. Mammalian cells have a finite capacity to cleave gp120 from gp41. Thus, when over-expressed, the envelope glycoproteins can saturate the endogenous furin enzymes and be secreted in precursor form. Since these molecules are potentially cleavable, we refer to them as gp140NON. Like gp140UNC, gp140NON migrates in SDS-PAGE with an apparent molecular mass of approximately 140 kDa under both reducing and nonreducing conditions. gp140NON appears to possess the same non-native topology as gp140UNC.

Cleaved gp140 (gp140CUT): gp140CUT refers to full-length gp120 and ectodomain gp41 fully processed and capable of forming oligomers as found on virions. The noncovalent interactions between gp120 and gp41 are sufficiently long-lived for the virus to bind and initiate fusion with new target cells, a process which is likely completed within minutes during natural infection. The association has, however, to date proven too labile for the production of significant quantities of cleaved gp140s in near homogenous form.

### Stabilization of viral envelope glycoproteins

The metastable pre-fusion conformation of viral envelope proteins such as gp120/gp41 has evolved to be sufficiently stable so as to permit the continued spread of infection yet sufficiently labile to readily allow the conformational changes required for virus-cell fusion. For the HIV isolates examined thus far, the gp120-gp41 interaction has proven too unstable for preparative-scale production of gp140CUT as a secreted protein. Given the enormous genetic diversity of HIV, however, it is conceivable that viruses with superior env stability could be identified using screening methods such as those described herein. Alternatively, viruses with heightened stability could in principle be selected following successive exposure of virus to conditions known to destabilize the gp120-gp41 interaction. Such conditions might include elevated temperatures in the range of 37-60°C and/or low concentrations of detergents or chaotropic agents. The envelope proteins from such viruses could be subcloned into the pPPI4 expression vector and analyzed for stability using our methods as well.

One could also adopt a semi-empirical, engineered approach to stabilizing viral envelope proteins. For example stable heterodimers have been successfully created by introducing complementary "knob" and "hole" mutations in the binding partners (Atwell, 1997). Alternatively or in addition, one could introduce other favorable interactions, such as salt bridges, hydrogen bonds, or hydrophobic interactions. This approach is facilitated by increased understanding of the structures of the surface (SU) and transmembrane (TM) proteins.

SU-TM stabilization can also be achieved by means of one or more introduced disulfide bonds. Among mammalian retroviruses, only the lentiviruses such as HIV have non-covalent associations between the SU and TM glycoproteins. In contrast, the type C and type D retroviruses all have an inter-subunit disulfide bond. The ectodomains of retroviral TM glycoproteins have a broadly common structure, one universal feature being the presence of a small, Cys-Cys bonded loop approximately central in the ectodomain. In the type C and D retroviral TM glycoproteins, an unpaired cysteine residue is found immediately C-terminal to this loop and is almost certainly used in forming the SU-TM disulfide bond (Gallaher, 1995; and Schultz, 1992).

Although gp41 and other lentiviral TM glycoproteins lack the third cysteine, the structural homologies suggest that one could be inserted in the vicinity of the short central loop structure. Thus there is strong mutagenic evidence that the first and last conserved regions of gp120 (C1 and C5 domains) are probable contact sites for gp41.

### II. Particle vaccines

Studies have revealed the advantage that is conferred by converting a soluble protein into a particulate form in the preparation of a vaccine. Precipitated aluminum salts or "alum" remain the only adjuvant utilized in vaccines licensed for human use by the United States Food and Drug Administration. Several other particulate adjuvants have been tested in animals. The major examples include beads prepared from poly(lactic-co-glycolic acid) [PLG] (Cleland, 1994; Hanes, 1997; and Powell, 1994), polystyrene (Kovacsovics-Bankowski, 1995; Raychaudhuri, 1998; Rock, 1996; and Vidard, 1996), liposomes (Alving, 1995), calcium phosphate (He, 2000), and cross-linked or crystallized proteins (Langhein, 1987; and St. Clair, 1999).

In one series of studies, ovalbumin was linked to polystyrene beads (Vidard, 1996). These studies revealed that antigen-specific B cells can bind particulate antigens directly via their surface Ig receptor, enabling them to phagocytose the antigen, process it, and present the resulting peptides to T cells. The optimum size for particulate antigen presentation in this context was found to be 4µm. Other studies with biodegradable PLG microspheres between 1 and 10µm in diameter show that these particles are capable of delivering antigens into the major histocompatibility complex (MHC) class I pathway of macrophages and dendritic cells and are able to stimulate strong cytotoxic T lymphocyte (CTL) responses in vivo (Raychaudhuri, 1998). PLG microspheres containing internalized ovalbumin and other antigens also induced humoral immune responses that were greater than those achieved with soluble antigen alone (Men, 1996; and Partidos, 1996).

The potent, long-lasting immune responses induced after a single immunization with antigen-loaded or antigen-coated microspheres may result from multiple mechanisms: efficient phagocytosis of the small (<10µm) particles, which results in their transport to lymph nodes, antigen processing and presentation to T-helper cells; the gradual release of antigens from the surface or interior of the particles, leading to the stimulation of immune-competent cells; and the sustained presentation of surface antigen (Coombes, 1999; Coombes, 1996; and O'Hagan, 1993). Antigen-presenting cells (APCs) localize to antigen-specific B cells under these conditions, and release cytokines that increase specific antibody production and augment the expansion of these antigen specific B-cell clones. Particulate antigens are also useful for generating mucosal humoral immunity by virtue of their ability to induce secretory IgA responses after mucosal vaccination (O'Hagan, 1993; and Vidard, 1996).

Overall, the use of particulate antigens allows for the simultaneous activation of both the humoral and cell-mediated arms of the immune response by encouraging the production of antigen-specific antibodies that opsonize particulate antigens and by causing the antigens to be phagocytosed and shunted into the MHC Class I antigen presentation pathway (Kovacsovics, 1995; Raychaudhuri, 1998; Rock, 1996; and Vidard, 1996).

Typically, the antigens are attached to the particles by physical adsorption. Antigens have also been incorporated into particles by entrapment, as is commonly performed for PLG-based vaccines (Hanes, 1997). More rarely, the antigens are covalently linked to functional groups on the particles (Langhein, 1987).

**D1 (**WO 01/00648**; Progenics Pharmaceuticals, Inc.) describes stabilized HIV viral envelope proteins including modified gp120 proteins.**

### Summary of the Invention

This invention provides a first stable HIV-1 pre-fusion envelope glycoprotein trimeric complex, wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into the gp41.

This invention also provides a first polypeptide comprising the amino acid sequence of HIV-1 gp120 and HIV-1 gp41, wherein (i) the gp41 sequence has one or more mutations in its N-terminal helix, and (ii) the gp120 and gp41 sequences each have at least one cysteine residue introduced thereinto, permitting the formation of at least one disulfide bond between the gp120 and the gp41 sequences.

A first composition comprising a pharmaceutically acceptable particle and the first trimeric complex operably affixed thereto is disclosed.

A nucleic acid which encodes the above-descrived polypeptides is disclosed A vector comprising the instant nucleic acid and a host cell which comprises the instant vector are disclosed

A method for producing a polypeptide which comprises growing the instant host cell under conditions permitting production of the polypeptide and recovering the polypeptide so produced is also disclosed. This invention further provides a second composition comprising the first trimeric complex or the instant particle composition, and a pharmaceutically acceptable carrier.

This invention further provides a third composition comprising the first trimeric complex or the first particle composition, and an adjuvant.

This invention further provides a method for eliciting an immune response in a subject against HIV-1 or an HIV-1 infected cell comprising administering to the subject a prophylactically or therapeutically effective amount of the first trimeric complex or first particle composition.

Furthermore, a vaccine which comprises a therapeutically effective amount of the instant trimeric complex or the instant particle composition is disclosed.

A first vaccine which comprises a prophylactically effective amount of the first trimeric complex or the first particle composition is disclosed

Moreover a method for preventing a subject from becoming infected with HIV-1 comprising administering to the subject a prophylactically effective amount of the first trimeric complex or the first particle composition, thereby preventing the subject from becoming infected with HIV-1 is disclosed

A method for reducing the likelihood of a subject's becoming infected with HIV-1 comprising administering to the subject a prophylactically effective amount of the first trimeric complex or the first particle composition, thereby reducing the likelihood of the subject's becoming infected with HIV-1 is disclosed

A method for preventing or delaying the onset of, or slowing the rate of progression of, an HIV-1-related disease in an HIV-1-infected subject which comprises administering to the subject a therapeutically effective amount of the first trimeric complex or the first particle composition, thereby preventing or delaying the onset of, or slowing the rate of progression of, the HIV-1-related disease in the subject is disclosed.

In addition, a first method for producing the first particle composition, comprising contacting a pharmaceutically acceptable particle with a stable HIV-1 pre-fusion envelope glycoprotein trimeric complex under conditions permitting the complex to become operably affixed to the particle, wherein (i) each monomeric unit of the complex comprises HIV-1 gp120 and HIV-1 gp41, (ii) the gp41 has one or more mutations in its, N-terminal helix, and (iii) the gp120 and gp41 are bound to each other by at least one disulfide bond between a cysteine residue introduced into the gp120 and a cysteine residue introduced into the gp41 is disclosed.

A second method for producing the first particle composition, comprising contacting (a) a pharmaceutically acceptable particle having operably affixed thereto an agent which binds to a stable HIV-1 pre-fusion envelope glycoprotein trimeric complex and (b) a stable HIV-1 pre-fusion envelope glycoprotein trimeric complex under conditions permitting the complex to bind to the agent, thereby permitting the complex to become operably affixed to the particle, wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into the gp41 is disclosed.

This invention provides a second stable HIV-1 pre-fusion envelope glycoprotein trimeric complex, wherein (i) each monomeric unit of the complex comprises HIV-1 gp120 and HIV-1 gp41, (ii) the gp120 and the gp41 of each monomeric unit are produced by proteolytic cleavage of a polypeptide having therein a mutated furin recognition sequence, (iii) the gp120 and gp41 are bound to each other by at least one disulfide bond between a cysteine residue introduced into the gp120 and a cysteine residue introduced into the gp41, and (iv) the gp120 has deleted from it at least one V-loop present in wild-type HIV-1 gp120.

This invention further provides a second polypeptide comprising the amino acid sequence of HIV-1 gp120 and HIV-1 gp41, wherein (i) the polypeptide has a mutated furin recognition situated so as to permit the production of gp120 and gp41 upon proteolytic cleavage thereof, (ii) the gp120 and gp41 sequences each have at least one cysteine residue introduced thereto, permitting the formation of at least one disulfide bond between the gp120 and the gp41 sequences, and (iv) the gp120 has deleted from it at least one V-loop present in wild-type HIV-1 gap120.

Also, a second particle composition comprising a pharmaceutically acceptable particle and the second trimeric complex operably affixed thereto is disclosed.

A second nucleic acid which encodes the second polypeptide is disclosed

Furthermore, a second vector comprising the second instant nucleic acidic is disclosed

A second host cell which comprises the second vector is disclosed.

Moreover, a second method for producing a polypeptide which comprises growing the second host cell under conditions permitting production of the polypeptide and recovering the polypeptide so produced is disclosed.

A fourth composition comprising the second trimeric complex or the second particle composition, and a pharmaceutically acceptable carrier is disclosed

A fifth composition comprising the second trimeric complex or particle composition, and an adjuvant.

Moreover, a second method for eliciting an immune response in a subject against HIV-1 or an HIV-1 infected cell comprising administering to the subject a prophylactically or therapeutically effective amount of the second trimeric complex or particle composition is disclosed

Disclosed is a vaccine which comprises a therapeutically effective amount of the second trimeric complex or particle composition.

A vaccine which comprises a prophylactically effective amount of the second trimeric complex or particle composition is also disclosed.

Moreover, a method for preventing a subject from becoming infected with HIV-1 comprising administering to the subject a prophylactically effective amount of the second trimeric complex or particle composition, thereby preventing the subject from becoming infected with HIV-1 is described.

A method for reducing the likelihood of a subject's becoming infected with HIV-1 comprising administering to the subject a prophylactically effective amount of the second trimeric complex or particle composition, thereby reducing the likelihood of the subject's becoming infected with HIV-1 is disclosed as well as a method for preventing or delaying the onset of, or slowing the rate of progression of, an HIV-1-related disease in an HIV-1-infected subject which comprises administering to the subject a therapeutically effective amount of the second trimeric complex or particle composition, thereby preventing or delaying the onset of, or slowing the rate of progression of, the HIV-1-related disease in the subject

A first method for producing the second particle composition, comprising contacting a pharmaceutically acceptable particle with a stable HIV-1 pre-fusion envelope glycoprotein trimeric complex under conditions permitting the complex to become operably affixed to the particle, wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into, the gp41 is described as well as finally, a second method for producing the second particle composition, comprising or producing the second particle composition, comprising contacting (a) a pharmaceutically acceptable particle having operably affixed thereto an agent which binds to a stable HIV-1 pre-fusion envelope glycoprotein trimeric complex and (b) a stable HIV-1 pre-fusion envelope glycoprotein trimeric complex under conditions permitting the complex to bind to the agent, thereby permitting the complex to become operably affixed to the particle, wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into the gp41 is described.

### Brief Description of the Figures

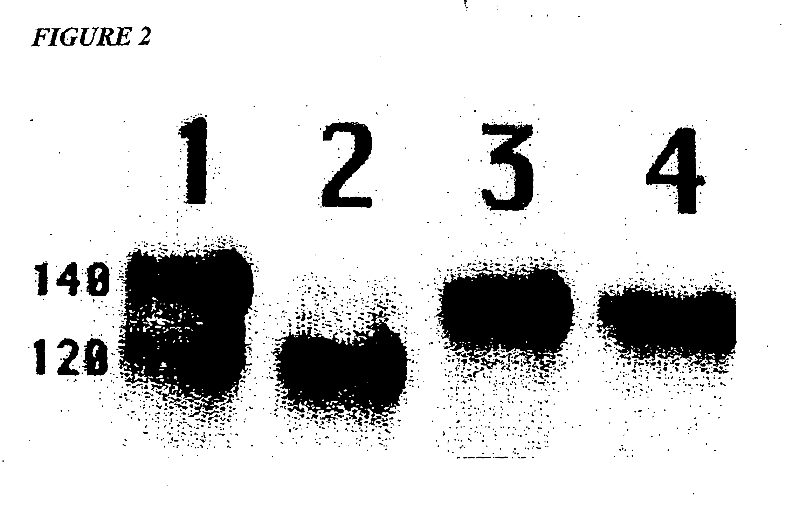

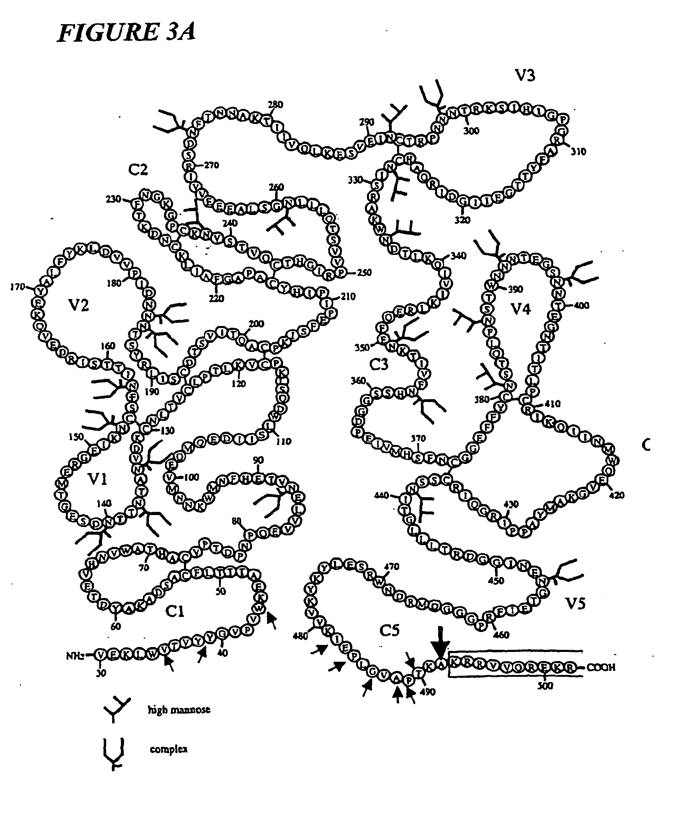

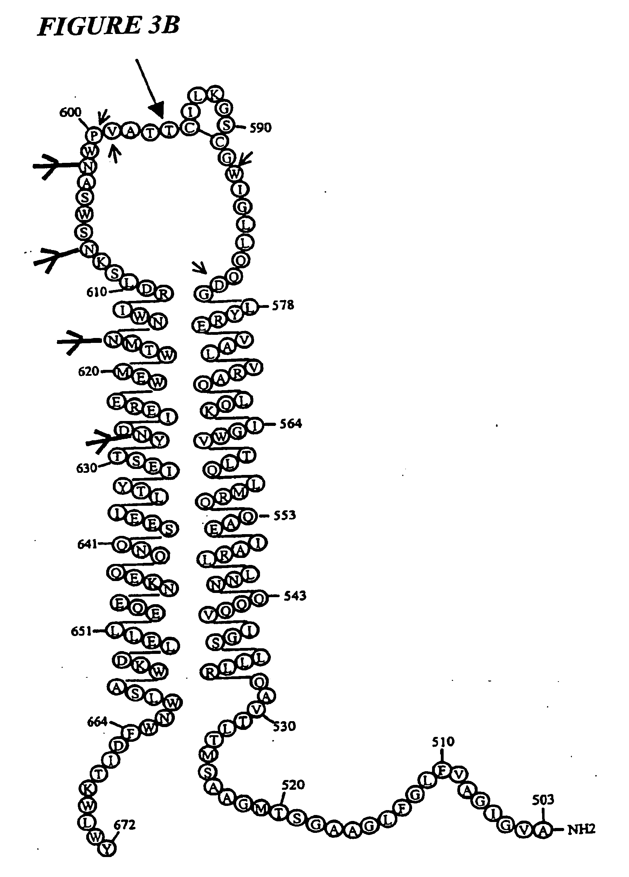

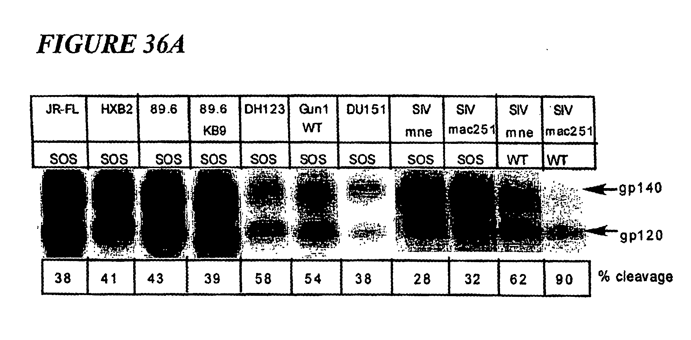

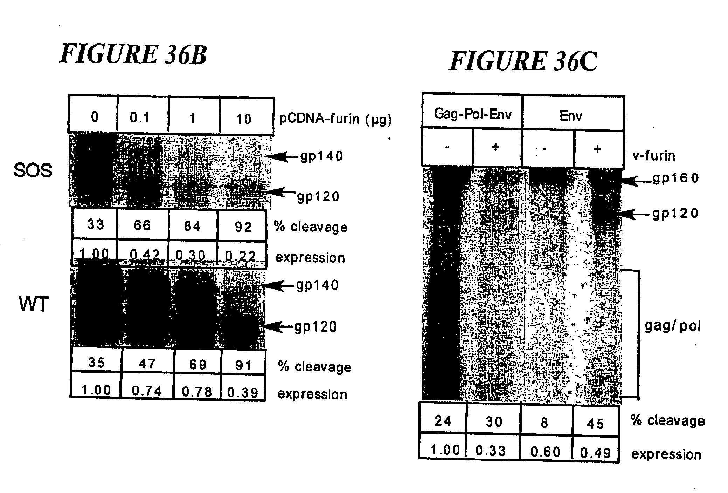

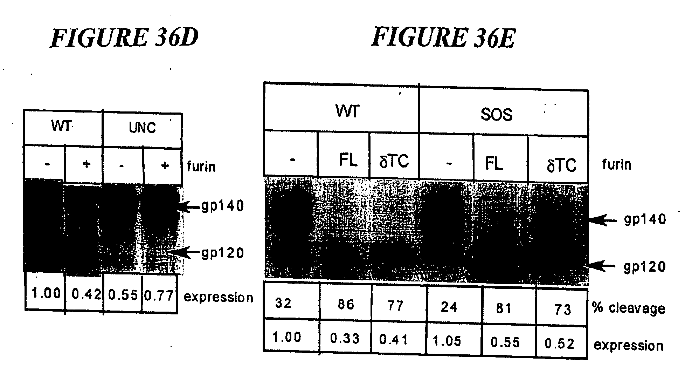

Figure 1
   Different forms of the HIV-1 envelope glycoproteins. The cartoons depict: i) Monomeric gp120; ii) Full-length recombinant gp160; iii) Proteolytically unprocessed gp140 trimer with the peptide bond maintained between gp120 and gp41 (gp140UNC or gp140NON); iv) The SOS gp140 protein, a proteolytically processed gp140 stabilized by an intermolecular disulfide bond; and v) Native, virion-associated gp120-gp41 trimer. The shading of the gp140UNC protein (iii) indicates the major antibody-accessible regions that are poorly, or not, exposed on the SOS gp140 protein or on the native gp120-gp41 trimer.
Figure 2
   Co-transfection of furin increases the efficiency of cleavage of the peptide bond between gp120 and gp41. 293T cells were transfected with DNA expressing HIV-1_{JR-FL} gp140 wild-type (WT) or gp140UNC (gp120-gp41 cleavage site mutant) proteins, in the presence or absence of a co-transfected furin-expressing plasmid. The ³⁵S-labelled envelope glycoproteins secreted from the cells were immunoprecipitated with the anti-gp120 MAb 2G12, then analyzed by SDS-PAGE. Lane 1, gp140WT (gp140/gp120 doublet); Lane 2, gp140WT plus furin (gp120 only); Lane 3, gp140UNC (gp140 only); Lane 4, gp140UNC plus furin (gp140 only). The approximate molecular weights, in kDa, of the major species are indicated on the left.
Figure 3
   Positions of cysteine substitutions in JR-FL gp140. The various residues of the JR-FL gp140WT protein that have been mutated to cysteines in one or more mutants are indicated by closed arrows on the schematics of the gp120 and gp41ECTO subunits. The positions of the alanine-492 and threonine-596 residues that are both mutated to cysteine in the SOS gp140 protein are indicated by the larger, closed arrows. (a) JR-FL gp120. (b) JR-FL gp41. The open boxes at the C-terminus of gp120 and the N-terminus of gp41 indicate the regions that are mutated in the gp140UNC protein to eliminate the cleavage site between gp120 and gp41.
Figure 4
   Immunoprecipitation analysis of selected double cysteine mutants of JR-FL gp140. The ³⁵S-labelled envelope glycoproteins secreted from transfected 293T cells were immunoprecipitated with anti-gp120 and anti-gp41 MAbs, then analyzed by SDS-PAGE. The MAbs used were either 2G12 (anti-gp120 C3-V4 region) or F91 (anti-gp120 CD4 binding site region).
   The positions of the two cysteine substitutions in each protein (one in gp120, the other in gp41ECTO) are noted above the lanes. The gp140WT protein is shown in lane 15. All proteins were expressed in the presence of co-transfected furin, except for the gp140WT protein.
Figure 5
   The efficiency of intermolecular disulfide bond formation is dependent upon the positions of the cysteine substitutions. The ³⁵S-labelled envelope glycoproteins secreted from 293T cells co-transfected with furin and the various gp140 mutants were immunoprecipitated with the anti-gp120 MAb 2G12, then analyzed by SDS-PAGE. For each mutant, the intensities of the 140kDa and 120kDa bands were determined by densitometry and the gp140/gp140+gp120 ratio was calculated and recorded. The extent of shading is proportional to the magnitude of the gp140/gp140+gp120 ratio. The positions of the amino acid substitutions in gp41 and the C1 and C5 domains of gp120 are recorded along the top and down the sides, respectively. N.D. = Not done.
Figure 6
   Confirmation that an intermolecular gp120-gp41 bond forms in the SOS gp140 protein. 293T cells were transfected with plasmids expressing gp140 proteins and, when indicated, a furin-expressing plasmid. The secreted, ³⁵S-labelled glycoproteins were immunoprecipitated with the indicated MAbs and analyzed by SDS-PAGE under reducing (+DTT) or nonreducing conditions. (a) Radioimmunoprecipitations with 2G12 of the SOS gp140, gp140WT and gp140UNC proteins. Immunoprecipitated proteins were resolved by SDS-PAGE under reducing (Lanes 4-6) or non-reducing (Lanes 1-3) conditions. (b) Radioimmunoprecipitations with 2G12 of the SOS gp140 protein and gp140 proteins containing the corresponding single-cysteine mutations. 140kDa protein bands are not observed for either the A492C or the T596C single-cysteine mutant gp140 proteins. (c) Radioimmunoprecipitations with 2G12 of the SOS gp140 proteins produced in the presence or absence of co-transfected furin. Immunoprecipitated proteins were resolved by SDS-PAGE under reducing (Lanes 3-4) or non-reducing (Lanes 1-2) conditions. DTT is shown to reduce the 140kDa SOS protein band produced in the presence but not the absence of exogenous furin.
Figure 7
   Analysis of cysteine mutants of JR-FL gp140. The ³⁵S-labelled envelope glycoproteins secreted from transfected 293T cells were immunoprecipitated with the anti-gp120 MAb 2G12, then analyzed by SDS-PAGE. All gp140s were expressed in the presence of co-transfected furin. Lanes 1-8, gp140s containing the indicated double cysteine mutations. Lanes 9-11, gp140 proteins containing the A492C/T596C double cysteine substitutions together with the indicated lysine to alanine substitutions at residue 491 (Lane 9), residue 493 (Lane 10) or at both residues 491 and 493 (Lane 11). Lanes 12-14, gp140 proteins containing quadruple cysteine substitutions.
Figure 8
   Comparison of the antigenic structures of the SOS gp140, W44C/T596C gp140 mutant, gp140UNC and gp140WT proteins. The ³⁵S-labelled envelope glycoproteins secreted from transfected 293T cells were immunoprecipitated with the indicated anti-gp120 Mabs and anti-gp41 MAbs, then analyzed by SDS-PAGE. Mutant but not wild type gp140s were expressed in the presence of cotransfected furin. (a) Anti-gp120 immunoglobulins that neutralize HIV-1_{JR-FL}. (b) Non-neutralizing antibodies to the C1, C4 and C5 regions of gp120 (c) Antibodies to CD4-induced epitopes were examined alone and in combination with sCD4. (d) Neutralizing (2F5) and non-neutralizing (7B2, 2.2B and 25C2) anti-gp41 antibodies and MAb 2G12. (e) Radioimmunoprecipitations of gp140WT (odd numbered lanes) and gp140UNC (even numbered lanes).
Figure 9
   Preparation of disulfide bond-stabilized gp140 proteins from various HIV-1 isolates. 293T cells were transfected with plasmids expressing wild type or mutant gp140s in the presence or absence of exogenous furin as indicated. ³⁵S-labeled supernatants were prepared and analyzed by radioimmuno-precipitation with MAb 2G12 as described above. Lane 1: SOS gp140 protein. Lane 2: gp140WT plus furin. Lane 3: gp140WT without furin. (a)HIV-1_{DH123}. (b)HIV-1_{HxB2}.
Figure 10
   Amino acid sequences of the glycoproteins with various deletions in the variable regions. The deleted wild-type sequences are shown in the white shade and include the following: ΔV1: D132-K152; ΔV2: F156-I191; ΔV1V2': D132-K152 and F156-I191; ΔV1V2*: V126-S192; ΔV3: N296-Q324.
Figure 11
   Formation of an intersubunit cysteine bridge in envelope proteins with deletions in variable loop regions. (a) The ΔV1V2*V3 protein and the ΔV1V2*V3 N357Q N398Q protein with two cysteines at positions 492 and 596 (indicated with CC) were precipitated with 2G12 and F91 (Lanes 3 and 7, and 4 and 8, respectively). The appropriate controls without cysteine mutations are shown in Lanes 1, 2, 5, and 6. The wild-type protein without extra cysteines is shown in lanes 9 and 10. All the proteins were cleaved by furin, except for the wild-type protein of lane 10. The approximate sizes in kDa are given on the right. (b) Various loop deleted proteins with two cysteines at positions 492 and 596 (CC) were precipitated with 2G12 (Lanes 3, 5, 7, 9, 11, and 13). Proteins with the same deletions without extra cysteines are given in the adjacent lanes. These control proteins were not cleaved by furin. The full-length SOS gp140 protein is included as a control in Lane 1.
Figure 12
   Antigenic characterization of the Δ492C/T596C mutant in combination with deletions in the variable loops. All mutants were expressed in the presence of exogenous furin. The antibodies used in RIPAs are indicated on top. (a) The A492C/T596C Δ1V2* mutant and (b) the A492C/T596C ΔV3 mutant.
Figure 13
   Nucleotide (a) and amino acid (b) sequences for HIV-1_{JR-FL} SOS gp140. The amino acid numbering system corresponds to that for wild-type JR-FL (Genbank Accession Number U63632). The cysteine mutations are indicated in underlined bold type face.
Figure 14
   Nucleotide (a) and amino acid (b) sequences for HIV-1_{JR-FL} ΔV1V2* SOS gp140. The amino acid numbering system corresponds to that for wild-type JR-FL (Genbank Accession Number U63632). The cysteine mutations are indicated in underlined bold-type face.
Figure 15
   Nucleotide (a) and amino acid (b) sequences for HIV-1_{JR-FL} ΔV3 SOS gp140. The amino acid numbering system corresponds to that for wild-type JR-FL (Genbank Accession Number U63632). The cysteine mutations are indicated in underlined bold-type face.
Figure 16
   SDS-PAGE analysis of purified HIV-1_{JR-FL} SOS gp140, gp140UNC and gp120 proteins. CHO cell-expressed proteins (0.5µg) in Laemmli sample buffer with (reduced) or without (non-reduced) 50mM DTT were resolved on a 3-8% polyacrylamide gradient gel.
Figure 17
   Biophysical analyses of purified, CHO cell-expressed HIV-1_{JR-FL} envelope glycoproteins. (a) Ultracentrifugation analysis of SOS gp140 was performed at protein concentrations ranging from 0.25mM to 1.0mM. The experimental data (open circles) were compared with theoretical curves for ideal monomers, dimers and trimers (labeled 1, 2, and 3). (b) Analytical size exclusion chromatography. Purified SOS gp140, gp140UNC and gp120 proteins were resolved on a TSK G3000SWXL column in PBS buffer, and their retention times were compared with those of known molecular weight standard proteins of 220kDa, 440kDa and 880kDa (arrowed). The main peak retention time of SOS gp140 (5.95 minutes) is consistent with it being a monomer that is slightly larger than monomeric gp120 (retention time 6.24 minutes), whereas gp140UNC (retention time 4.91 minutes) migrates as oligomeric species. (c) The oligomeric status of pure standard proteins, thyroglobulin, ferritin and albumin, were compared with gp120 and gp120 in complex with soluble CD4 using BN-PAGE. The proteins were visualized on the gel using coomassie blue. (d) BN-PAGE analysis of CHO cell-derived, purified HIV-1_{JR-FL} gp120, SOS gp140 and gp140UNC glycoproteins.
Figure 18
   Negative stain electron micrographs of SOS gp140 alone (a) and in complex with MAbs (b-f). Bar = 40nm. In b-f, the panels were masked and rotated so that the presumptive Fc of the MAb is oriented downward. When multiple MAbs were used, the presumptive Fc of MAb 2F5 is oriented downward. In b-f, interpretative diagrams are also provided to illustrate the basic geometry and stoichiometry of the immune complexes. SOS gp140, intact MAb, and F(ab')2 are illustrated by ovals, Y-shaped structures and V-shaped structures, respectively, in the schematic diagrams, which are not drawn to scale. The MAbs used are as follows: (b) 2F5; (c) IgG1b12; (d) 2G12; (e) MAb 2F5 plus F(ab') 2 IgG1b12; (f) MAb 2F5 plus MAb 2G12.
Figure 19
   Individual, averaged and subtracted electron micrographs of SOS gp140 and gp120 in complex with sCD4 and MAb 17b. Bar = 40nm. Panels a and b are individual electron micrographs of ternary complexes of SOS gp140 (a) and YU2 gp120 (b). The Fc region of MAb 17b is aligned downward. Panels c and f are averaged electron micrographs of ternary complexes of SOS gp140 (Panel c) and gp120 (Panel f). Panels d and g are masked and averaged electron micrographs of the SOS gp140 complex (Panel d) and the gp120 complex (Panel g). Panel e represents the density remaining upon subtraction of the gp120 complex (Panel g) from the gp140 complex (Panel d). In Panels d and e, the arrow indicates the area of greatest residual density, which represents the presumptive gp41ECTO moiety that is present in SOS gp140 but not in gp120. Panel h indicates the outline of the gp120 complex (Panel g) overlaid upon a ribbon diagram of the X-ray crystal structure of the gp120 core in complex with sCD4 and the 17b Fab fragment [PDB code 1GC1] (Kwong, 1998). The gp120 complex was enlarged to facilitate viewing.
Figure 20
   Models indicating the approximate location of gp41ECTO in relation to gp120 as derived from electron microscopy data of SOS gp140. (a) Presumptive location of gp41ECTO (represented by the dark blue oval) in relation to the X-ray crystal structure of the gp120 core in complex with sCD4 (yellow) and Fab 17b (light blue) [PDB code 1GC1] (Kwong, 1998). The gp120 core surface was divided into three faces according to their antigenic properties (Moore, 1996; and Wyatt, 1998a); the non-neutralizing face is colored lavender, the neutralizing face is red, and the silent face, green. (b) The IgG1b12 epitope (Saphire, 2001) and the 2G12 epitope (Wyatt, 1998a) are shown in yellow and white, respectively. The residues associated with the gp120 C-terminus are colored blue, to provide a point of reference.
Figure 21
   RIPA analysis of unpurified, CHO cell-expressed HIV-1_{JR-FL} SOS gp140. Stably transfected CHO cells were cultured in the presence of ³⁵S-labeled cysteine and methionine. Culture supernatants were immunoprecipitated with the indicated MAbs and protein G-agarose beads, and bound proteins were resolved by SDS-PAGE and visualized by autoradiography. The MAb and/or CD4-based protein used for capture is indicated above each lane. In Lane 2, the proteins were reduced with DTT prior to SDS-PAGE; the remaining samples were analyzed under non-reducing conditions.
Figure 22
   SPR analysis of CHO cell-expressed HIV-1_{JR-FL} SOS gp140, gp140UNC and gp120 proteins. Anti-gp120 and anti-gp41 MAbs were immobilized onto sensor chips and exposed to buffers containing the indicated gp120 or gp140 glycoproteins in either purified or unpurified form, as indicated. Where noted, Env proteins were mixed with an 8-fold molar excess of sCD4 for 1h prior to analysis. Culture supernatants from stably transfected CHO cells were used as the source of unpurified SOS gp140 and gp140UNC proteins. The concentrations of these proteins were measured by Western blotting and adjusted so that approximately equal amounts of each protein were loaded. Only the binding phases of the sensorgrams are shown; in general, the dissociation rates were too slow to provide meaningful information.
Figure 23
   BN-PAGE analyses of unfractionated cell culture supernatants. (a) Comparison of HIV-1_{JR-FL} gp120, SOS gp140, gp140UNC, and ΔV1V2 SOS gp140 glycoproteins present in culture supernatants from stable CHO cell lines. (b) Proteolytic cleavage destabilizes gp140 oligomers. 293T cells were transfected with furin and plasmids encoding SOS gp140, gp140UNC, SOS gp140UNC. Cell culture supernatants were combined with MOPS buffer containing 0.1% coomassie blue and resolved by BN-PAGE. Proteins were then transferred to PVDF membranes and visualized by Western blotting. Thyroglobulin and the BSA dimer were used as molecular weight markers (see Figure 2c).
Figure 24
   HIV-1_{JR-FL} gp120 immobilization onto PA1-microbeads. HIV-1_{JR-FL} gp120 was immobilized onto PA1 magnetic microbeads as described. 5µl and 12.5µl of the resuspended beads were analyzed under reducing conditions on SDS-PAGE followed by Coomassie staining. 2.5µg of gp120 was loaded for comparison and quantitation.
Figure 25
   HIV-1_{JR-FL} gp120 immobilization onto PA1-Dynabeads, HIV-1_{JR-FL} gp120 was immobilized onto PA1 magnetic Dynabeads as described. Indicated volumes of the resuspended beads were analyzed under reducing conditions on SDS-PAGE followed by Coomassie staining. Increasing amounts of gp120 were loaded for quantitation.
Figure 26
   Temporal analysis of anti-gp120 antibody response elicited by gp120 vaccines. Serum response was analyzed after each immunization, using a native gp120-specific ELISA assay. Dose of gp120 is indicated in parentheses in legend.
Figure 27
   Anti-gp120 titers (50% maximal) in serum from animals immunized with three doses of gp120 vaccine. Data are mean +/- SD of 5 animals per group, and dose of gp120 is in parentheses.
Figure 28
   Mutagenesis strategy for gp41ECTO. (a) The residues at the *a* and *d* positions in the N-terminal heptad repeat of gp41_{ECTO}, depicted in gray in the secondary structure, were substituted. Major MAb epitopes are indicated (Earl, 1997; Parker, 2001; Xu, 1991; and Zwick, 2001). (b) A schematic representation of a cross-section of the 6-helix bundle, post-fusion form of gp41ECTO, and a helical wheel representation of one N-terminal helix, are depicted. The residues at the *a* and *d* positions of the N-terminal heptad repeat (black) form the trimer interface.
Figure 29
   PAGE analysis of SOS gp140 proteins. (a) BN-PAGE of SOS gp140 proteins containing changes at position 559. The gp41UNC protein was included for comparison. (b) SDS-PAGE of SOS gp140 variants under non-reducing conditions. (c) SDS-PAGE of SOS gp140 variants under reducing conditions. (d) The SOSI559G gp140 protein was treated with SDS for 1 hour at 25°C. The SDS concentrations used were 0%, 0.005%, 0.01%, 0.015%, 0.02%, 0.025%, 0.03%, 0.035% and 0.04%, increasing from left to right. BN-PAGE analysis was then performed. Monomeric gp120 (20ng) served as a molecular weight standard.
Figure 30
   Gel-filtration analysis of SOSI559P gp140. (a) The SOS and SOSI559P gp140 proteins were fractionated on a Superdex 200 column. The individual fractions were analyzed by SDS-PAGE and Western blotting. (b) The same SOSI559P gp140 fractions from (a) were analyzed by BN-PAGE and western blotting. The elution positions (peak fractions) of standard proteins are indicated.
Figure 31
   Stability of SOSI559P gp140 trimers. Fractions 6 and 7 from a gel-filtration profile similar to that shown in Figure 3 were pooled as a source of SOSI559P gp140 trimers. The trimers were (a) incubated for 1 hour at the temperatures indicated or (b) exposed to a 0.1% concentration of various detergents for 1 hour at 25°C. The proteins were then analyzed using BN-PAGE and Western blotting.
Figure 32
   Antigenic structure analysis of gp140 proteins. RIPAs were performed using the gp140UNC, SOS gp140 and SOSI559P gp140 proteins and various neutralizing and non-neutralizing MAbs (Binley, 2000a).
Figure 33
   HIV-1 gp41ECTO core structure and mutant peptides. (a)The N36/CD4 crystal structure is shown with one N36 and one C34 helix labeled at the amino terminus. Three C34 helices (red) pack against the N36 trimeric coiled coil (blue).
   The van der Waal surfaces of residues at the *a* (red) and *d* (green) positions are superimposed on the helix backbone of the N36 coiled coil. Amino acids substituted in this study are indicated above the *a* and *d* layers. The figure was prepared using the program GRASP (Nicholls, 1991). (b) Thermal melting transition curves of the N36(L6)C34 (open circles), I559G (closed circles), I559P (open squares), L566V (open rhombs) and T569P peptides (open triangles) were determined by CD spectroscopy at 222nm, and at a peptide concentration of 10µm in PBS (pH 7.0). The increase in the fraction of unfolded molecules is shown as a function of temperature. All melts were reversible. Superimposable folding and unfolding curves were observed. Greater than 90% of the signal was regained upon cooling. (c) Equilibrium sedimentation analysis of the T569P peptide. Representative data for this peptide were collected at 20°C and 20,000 rpm in PBS (pH 7.0), at a peptide concentration of ~30µm. The data fit best to a trimer model (curve 3). Curves for a dimer (curve 2) and a tetramer (curve 4) are depicted for comparison. Analyses of residual differences from curve 3 do not reveal a systematic error.
Figure 34
   Model of gp41ECTO and its transitions during fusion. Left panel: The hypothetical, native pre-fusion configuration of gp41 (Hunter, 1997). Middle panel: The pre-hairpin intermediate form. Right panel: The post-fusion state. In the pre-fusion configuration the N-terminal helix is not present, and the region around position 559-569 is not helical. The I559P and related substitutions are proposed to disrupt either the formation of the N-terminal helix in the pre-hairpin intermediate, or the formation of the 6-helix bundle. By doing so, the modified SOS gp140 proteins are maintained in the pre-fusion configuration. The position of the T605C substitution that creates the SOS gp140 protein is also specified, as is the adjacent intermolecular disulfide bond (yellow bar) and the position of N-linked glycans. Only the two helices from one gp41 molecule are shown, for clarity.
Figure 35
   In vitro enzymatic cleavage of Env with plasmin or soluble furin. Eight micrograms of partially (∼50%) cleaved, purified JR-FL SOS gyp140 or gp120 were incubated with a protease for various times at 37°C. The Env proteins were then analyzed by reducing SDS-PAGE and Western blot. (a) SOS gp140 proteins from a 2 hours or 16 hours plasmin (20µg) digest. The Western blot was probed with the anti-gp41 MAb 2F5. (b) SOS gp140 or gp120 samples from a 16 hours plasmin (20µg) digest. The Western blot was probed with the anti-gp120 MAb B12. (c) SOS gp140 was incubated at pH 7.5 with or without plasmin or soluble furin for 16 hours at 37°C. The Western blot was probed with MAb B12. (d) JR-FL SOS gp140 was incubated with or without furin for 16 hours at the pH indicated. The Western blot was probed with MAb B12. The percent cleavage achieved by soluble furin was calculated as described under Materials and Methods, and expressed with reference to the control in Lane 10 (no furin, 0% cleavage).
Figure 36
   *Cleavage and expression of Env proteins with or without furin.* Culture supernatants containing ³⁵S-labeled Env proteins were immunoprecipitated with either HIVIG or SIVIG as appropriate, then analyzed by reducing SDS-PAGE. The results shown are representative of three repeats. In each panel, the percent cleavage was calculated as per materials and methods. Additionally, the relative expression of the gp120+gp140 or gp120+gp160 bands was calculated and expressed as a ratio relative to a standard (expression defined as 1.00) in each gel. (a) Soluble gp140 proteins were expressed in 293T cells transfected with pPPI4-based plasmids. (b) The JR-FL SOS gp140 or gp140WT proteins were expressed as in Panel a but in the presence of variable amounts of co-expressed, full-length furin. (c) BSC40 cells were infected with vaccinia viruses v-VS4 (expressing SIVₘₙₑ Gag-Pol) and/or v-SE5 (expressing Size Env) at an MOI of 5, as indicated. Some of the cells were co-infected, also at an MOI of 5, with the vaccinia virus vv:hfur expressing full-length furin (Lanes 2 and 4). (d) JR-FL gp140WT and gp140UNC proteins were expressed in 293T cells transfected with pPPI4-based plasmids, with or without co-transfection of full-length furin. (e) JR-FL gp140WT and SOS gp140 proteins were expressed in 293T cells transfected with pPPI4-based plasmids, alone or with co-transfection of either full-length furin (FL) or truncated, soluble furin (∂TC), as indicated.
Figure 37
   Altering the cleavage sequence can increase Env processing by cellular proteases. (a) JR-FL SOS gp140 with the wild type REKR cleavage site (Lanes 1 and 2) or the mutant RRRKKR (Lanes 3 and 4) or RRRRRR (Lanes 5 and 6) cleavage site sequences was expressed in the absence (Lanes 1, 3, and 5) or presence (Lanes 2, 4, and 6) of co-transfected full-length furin. (b) JR-FL SOS gp140_{RRRRRR} was expressed with no (Lane 1), 0.1µg (Lane 2), 1µg (Lane 3) or 10µg (Lane 4) of co-transfected full-length furin. (c) DU151 SOS gp140 (Lanes 1 and 2) and its RRRRRR mutant (Lanes 3 and 4) was expressed in the absence (Lanes 1 and 3) or presence of co-transfected full-length furin (Lanes 2 and 4). All samples were labeled and immunoprecipitated as described in the legend to Figure 36. The percent cleavage and relative expression of Env were calculated as in Figure 36.
Figure 38
   Env cleavage site mutants are functional for infection and fusion. (a) The infectivity for HeLa-CD4-CCR5 cells of pNL-luc viruses pseudotyped with JR-FL gp160WT, the JR-FL gp160_{RRRRRR} mutant or VSV-G was measured using a single round infection assay using a luciferase readout. Normalized luciferase values for negative control viruses lacking envelope (derived from pNL-luc transfection supernatants) were <1 unit. (b) Cell-cell fusion mediated by the gp160WT or gp160_{RRRRRR} proteins was analyzed in a dye transfer assay.

### Detailed Description of the Invention

This invention provides a first stable HIV-1 pre-fusion envelope glycoprotein trimeric complex, according to claim 1 wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into the gp41.

In an embodiment of this invention, gp140 comprises gp120 or a modified form of gp120, according to claim 1 which has modified immunogenicity relative to wild type gp120. In another embodiment, the modified gp120 molecule is characterized by the absence of one or more variable loops present in wild type gp120. In another embodiment, the variable loop comprises V1, V2, or V3. In another embodiment, the modified gp120 molecule is characterized by the absence or presence of one or more canonical glycosylation sites not present in wild type gp120. In another embodiment, one or more canonical glycosylation sites are absent from the V1V2 region of the gp120 molecule.

In an embodiment, the gp41 and the gp120 of each monomeric unit are produced by proteolytic cleavage of a polypeptide, wherein the cleavage occurs at a mutated furin recognition sequence according to claims 4-6.

A disulfide bond is formed between a cysteine residue introduced by an A492C mutation in gp120 and a cysteine residue introduced by a T596C mutation in gp41.

The gp120 is further characterized by (i) the absence of one or more canonical glycosylation sites present in wild-type HIV-1 gp120, and/or (ii) the presence of one or more canonical glycosylation sites absent in wild-type HIV-1 gp120.

This specification also provides a first polypeptide comprising the amino acid sequence of HIV-1 gp120 and HIV-1 gp41, wherein (i) the gp41 sequence has one or more mutations in its N-terminal helix, and (ii) the gp120 and gp41 sequences each have at least one cysteine residue introduced thereinto, permitting the formation of at least one disulfide bond between the gp120 and the gp41 sequences.

The polypeptide comprises a mutated furin recognition sequence permitting the generation of gp120 and gp41 upon proteolytic cleavage thereof.

A cysteine residue is introduced into the gp120 sequence by an A492C mutation and into the gp41 sequence by a T596C mutation.

The gp120 sequence is further characterized by (i) the absence of one or more canonical glycosylation sites present in wild-type HIV-1 gp120, and/or (ii) the presence of one or more canonical glycosylation sites absent in wild-type HIV-1 gp120.

In the first trimeric complex a mutation is located at position A or D in the N-terminal helix of the gp41.

The first trimeric complex may comprise the substitution of a non-helix-breaking amino acid with a helix-breaking amino acid. The helix-breaking amino acid can be, for example, proline or glycine. The amino acid which is mutated may be selected from the group consisting of V583, V580, L576, I573, T569, L566, Q562, Q590 L555, Q552. I548, L545 and I559. In the preferred embodiment, the amino acid mutation is I559P.

This invention further discloses a first composition comprising a pharmaceutically acceptable particle and the first trimeric complex operably affixed thereto.

The first trimeric complex may be operably affixed to the particle via an agent which is operably affixed to the particle.

In a specific embodiment, the disulfide bond in each monomer of the trimer is formed between a cysteines residue introduced by an A492C mutation in gp120 and a cysteine residue introduced by a T596C mutation in gp41.

In a further embodiment, the gp120 in each monomer of the trimer is further characterized by (i) the absence of one or more canonical glycosylation sites present in wild-type HIV-1 gp120, and/or (ii) the presence of one or more canonical glycosylation sites absent in wild-type HIV-1 gp120.

In the first composition, the particle can be, for example, a paramagnetic bead, a non-paramagnetic bead, a liposome, or any combination thereof. The particle can comprise, for example, PLG, latex, polystyrene, polymethyl-methacrylate, or any combination thereof.

The mean diameter of the particle is from about 10nm to 100µ or the mean diameter of the particle is from about 100nm to 10µm, from about 100nm to 1µm or the mean diameter of the particle is from about 1µm to 10µm. The mean diameter of the particle is from about 10µm to 100µm or from about 10µm to 100nm or the mean diameter of the particle is about 50nm.

In the first composition, the agent may be, for example, an antibody, a fusion protein, streptavidin, avidin, a lectin, or a receptor. In one example, the agent is an antibody. In another example, the agent is CD4.

This specification further discloses a nucleic acid which encodes the instant polypeptide, as well as

a vector comprising the instant nucleic acid. In the preferred embodiment, the instant vector further comprises a sequence encoding furin. The instant vector can be, for example, a plasmid, cosmid, λ phage, YAC or α-virus.

This specification further discloses a host cell which comprises the instant vector.

This specification further discloses a method for producing a polypeptide which comprises growing the instant host cell under conditions permitting production of the polypeptide and recovering the polypeptide so produced.

This invention further provides a second composition comprising the trimeric complex of claim 1 or the instant particle composition, and a pharmaceutically acceptable carrier.

In one embodiment, the second composition further comprises a cytokine and/or a chemokine. The cytokine can be, for example, interleukin-2, interleukin-4, interleukin-5, interleukin-12, interleukin-15, interleukin-18, GM-CSF, or any combination thereof. The chemokine can be, for example, SLC, ELC, Mip3α, Mip3β, IP-10, MIG, or any combination thereof.

Cytokines include but are not limited to interleukin-4, interleukin-5, interleukin-2, interleukin-12, interleukin-15, interleukin-18, GM-CSF, and combinations thereof. Chemokines include but are not limited to SLC, ELC, Mip-3α, Mip-3β, interferon inducible protein 10 (IP-10), MIG, and combinations thereof.

This invention further provides a third composition comprising the trimeric complex of claim 1 the first particle composition, and an adjuvant. The adjuvant can be, for example, alum, Freund's incomplete adjuvant, saponin, Quil A, QS-21, Ribi Detox, monophosphoryl lipid A, a CpG oligonucleotide, CRL-1005, L-121, or any combination thereof.

This invention further provides the use of the trimeric complex of claim 1 for the preparation of medicaments for eliciting an immune response in a subject against HIV-1 or an HIT-1 infected cell comprising administering to the subject a prophylactically or therapeutically effective amount of the first trimeric complex of claim 1 or first particle composition.

In this method, said trimeric complex or the composition can be administered in a single dose or in multiple doses.

In one embodiment of this method, the trimeric complex or the composition is administered as part of a heterologous prime-boost regimen.

A vaccination is described with at least three different vaccine compositions, wherein the vaccine compositions differ from each other by the form of the vaccine antigen.

This specification further discloses vaccine which comprises a therapeutically effective amount of the instant trimeric complex or the instant particle composition.

Moreover a vaccine which comprises the above isolated nucleic acid is disclosed. In one embodiment, the vaccine comprises a therapeutically effective amount of the nucleic acid. In another embodiment, the vaccine comprises a therapeutically effective amount of the protein encoded by the above nucleic acid. In another embodiment, the vaccine comprises a combination of the recombinant nucleic acid molecule and the mutant viral envelope protein.

This specification discloses the above vaccine which comprises but is not limited to the following: a recombinant subunit protein, a DNA plasmid, an RNA molecule, a replicating viral vector, a non-replicating viral vector, or a combination thereof.

This specification further discloses a vaccine which comprises a prophylactically effective amount of the first trimeric complex or the first particle composition.

This specification further discloses a method for preventing a subject from becoming infected with HIV-1 comprising administering to the subject a prophylactically effective amount of the first trimeric complex or the first particle composition, thereby preventing the subject from becoming infected with HIV-1.

This specification further discloses a method for reducing the likelihood of a subject's becoming infected with HIV-1 comprising administering to the subject a prophylactically effective amount of the first trimeric complex or the first particle composition, thereby reducing the likelihood of the subject's becoming infected with HIV-1.

The subject may be HIV-1-exposed.

This specification further discloses a method for preventing or delaying the onset of, or slowing the rate of progression of, an HIV-1-related disease in an HIV-1-infected subject which comprises administering to the subject a therapeutically effective amount of the first trimeric complex or the first particle composition, thereby preventing or delaying the onset of, or slowing the rate of progression of, the HIV-1-related disease in the subject.

A first method for producing the first particle composition, comprising contacting a pharmaceutically acceptable particle with a stable HIV-1 pre-fusion envelope glycoprotein trimeric complex under conditions permitting the complex to become operably affixed to the particle, wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gyp120 and a cysteine residue introduced into the gp41 is also disclosed.

Moreover, a second method for producing the first particle composition, comprising contacting (a) a pharmaceutically acceptable particle having operably affixed thereto an agent which binds to a stable HIV-1 pre-fusion envelope glycoprotein trimeric complex and (b) a stable HIV-1 pre-fusion envelope glycoprotein trimeric complex under conditions permitting the complex to bind to the agent, thereby permitting the complex to become operably affixed to the particle, wherein (i) each monomeric unit of the complex comprises HIV-1, gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into the gp41 is disclosed.

This invention provides a stable HIV-1 pre-fusion envelope glycoprotein trimeric complex, wherein (i) each monomeric unit of the complex comprises HIV-1 gp120 and HIV-1 gp41, (ii) the gp120 and the gp41 of each monomeric unit are produced by proteolytic cleavage of a polypeptide having therein a mutated furin recognition sequence, (iii) the gp120 and gp41 are bound to each other by at least one disulfide bond between a cysteine residue introduced into the gp120 and a cysteine residue introduced into the gp41, and (iv) the gp120 has deleted from it at least one V-loop present in wild-type HIV-1 gp120.

The gp120 has deleted from it one or more of variable loops V1, V2, and V3.

In a specific embodiment, the disulfide bond is formed between a cysteine residue introduced by an A492C mutation in gp120 and a cysteine residue introduced by a T596C mutation in gp41.

In a further embodiment, the gp120 is further characterized by (i) the absence of one or more canonical glycosylation sites present in wild-type HIV-1 gp120, and/or (ii) the presence of one or more canonical glycosylation sites absent in wild-type HIV-1 gp120.

A second polypeptide comprising the amino acid sequence of HIV-1 gp120 and HIV-1 gp41, wherein (i) the polypeptide has a mutated furin recognition situated so as to permit the production of gp120 and gp41 upon proteolytic cleavage thereof, (ii) the gp120 and gp41 sequences each have at least one cysteine residue introduced thereto, permitting the formation of at least one disulfide bond between the gp120 and the gp41 sequences, and (iv) the gp120 has deleted from it at least one V-loop present in wild-type HIV-1 gp120 is described. In one embodiment, a cysteine residue is introduced into the gp120 sequence by an A492C mutation and into the gp41 sequence by a T596C mutation. In another embodiment, the gp120 sequence is further characterized by (i) the absence of one or more canonical glycosylation sites present in wild-type HIV-1 gp120, and/or (ii) the presence of one or more canonical glycosylation sites absent in wild-type HIV-1 gp120.

In the second trimeric complex and polypeptide, the gp41 can have one or more mutations in its N-terminal helix, and preferably, in the portion thereof which forms the trimer interface in the post-fusion 6-helix bundle of the trimeric complex. Preferably, the amino acid mutation is I559P.

In the second trimeric complex and polypeptide, the mutated furin recognition sequence can have, for example, the sequence R-X-(R/K)-R, RRRKKR, RRRRKR, or RRRRRR.

This specification also discloses a second particle composition comprising a pharmaceutically acceptable particle and the second trimeric complex operably affixed thereto. In one embodiment, the second trimeric complex is operably affixed to the particle via an agent which is operably affixed to the particle. In another embodiment, the disulfide bond in each monomer of the trimer is formed between a cysteine residue introduced by an A492C mutation in gp120 and a cysteine residue introduced by a T596C mutation in gp41. In a further embodiment, the gp120 in each monomer of the trimer is further characterized by (i) the absence of one or more canonical glycosylation sites present in wild-type HIV-1 gp120, and/or (ii) the presence of one or more canonical glycosylation sites absent in wild-type HIV-1 gp120.

In the second particle composition, the particle can be, for example, a paramagnetic bead, a non-paramagnetic bead, a liposome, or any combination thereof. The particle can comprise, for example, PLG, latex, polystyrene, polymethyl-methacrylate, or any combination thereof.

The mean diameter of the particle is from about 10nm to 100µm, or from about 100nm to 10µm, or or the mean diameter of the particle is from about 100nm to 1µm. Furthermore the mean diameter of the particle is from about 1µm to 10µm or from about 10µm to 100µm, or the mean diameter of the particle is about 50nm.

In the second particle composition, the agent can be, for example, an antibody, a fusion protein, streptavidin, avidin, a lectin, or a receptor. In one embodiment, the agent is an antibody. In another embodiment, the agent is CD1.

This specification further discloses a a second nucleic acid which encodes the second polypeptide.

Furthermore a second vector comprising the second instant nucleic acid is disclosed. The second vector further comprises a sequence encoding furin. The second vector can be, for example, a plasmid, cosmid, λ phage, YAC or α-virus.

This specification further discloses a second host cell which comprises the second vector.

This specification further discloses a second method for producing a polypeptide which comprises growing the second host cell under conditions permitting production of the polypeptide and recovering the polypeptide so produced.

This specification further discloses a fourth composition comprising the second trimeric complex or the second particle composition, and a pharmaceutically acceptable carrier.

The fourth composition further comprises a cytokine and/or a chemokine. The cytokine can be, for example, interleukin-2, interleukin-4, interleukin-5, interleukin-12, interleukin-15, interleukin-18, GM-CSF, or any combination thereof. The chemokine can be, for example, SLC, ELC, Mip3α, Mip3β, IP-10, MIG, or any combination thereof.

This specification further discloses a fifth composition comprising the second trimeric complex or particle composition, and an adjuvant. The adjuvant can be, for example, alum, Freund's incomplete adjuvant, saponin, Quil A, QS-21, Ribi Detox, monophosphoryl lipid A, a CpG oligonucleotide, CRL-1005, L-121, or any combination thereof.

This invention further provides the use of the trimeric complex of claim 1 for the preparation of a medicament for eliciting an immune response in a subject against HIV-1 or an HIV-1 infected cell comprising administering to the subject a prophylactically or therapeutically effective amount of the trimeric complex of claim 1 In this method, the trimeric complex or the composition can be administered in a single dose or in multiple doses. In one embodiment of this method, the trimeric complex or the composition is administered as part of a heterologous prime-boost regimen.

This specification further discloses a vaccine which comprises a therapeutically effective amount of the second trimeric complex or particle composition.

This specification further discloses a vaccine which comprises a prophylactically effective amount of the second trimeric complex or particle composition.

This specification further discloses a method for preventing a subject from becoming infected with HIV-1 comprising administering to the subject a prophylactically effective amount of the second trimeric complex or particle composition, thereby preventing the subject from becoming infected with HIV-1.

This specification further discloses a method for reducing the likelihood of a subject's becoming infected with HIV-1 comprising administering to the subject a prophylactically effective amount of the second trimeric complex or particle composition, thereby reducing the likelihood of the subject's becoming infected with HIV-1.

This specification further discloses a method for preventing or delaying the onset of, or slowing the rate of progression of, an HIV-1-related disease in an HIV-1-infected subject which comprises administering to the subject a therapeutically effective amount of the second trimeric complex or particle composition, thereby preventing or delaying the onset of, or slowing the rate of progression of, the HIV-1-related disease in the subject.

This specification further discloses a first method for producing the second particle composition, comprising contacting a pharmaceutically acceptable particle with a stable HIV-1 pre-fusion envelope glycoprotein trimeric complex under conditions permitting the complex to become operably affixed to the particle, wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into the gp41.

This specification further discloses a second method for producing the second particle composition, comprising contacting (a) a pharmaceutically acceptable particle having operably affixed thereto an agent which binds to a stable HIV-1 pre-fusion envelope glycoprotein trimeric complex and (b) a stable HIV-1 pre-fusion envelope glycoprotein trimeric complex under conditions permitting the complex to bind to the agent, thereby permitting the complex to become operably affixed to the particle, wherein (i) each monomeric unit of the complex comprises HIV-1 gp120 and HIV-1 gp41, (ii) the gp41 has one or more mutations in its N-terminal helix, and (iii) the gp120 and gp41 are bound to each other by at least one disulfide bond between a cysteine residue introduced into the gp120 and a cysteine residue introduced into the gp41.

Finally, this invention provides antibodies directed against the instant trimeric complex.

Set forth below are certain additional definitions and examples which are intended to aid in an understanding of the instant invention.

As used herein, the following standard abbreviations are used throughout the specification to indicate specific amino acids: A=ala=alanine; R=arg=arginine; N=asn=asparagine; D=asp=aspartic acid; C=cys=cysteine; Q=gln=glutamine; E=glu=glutamic acid; G=gly=glycine; H=his=histidine; I=ile=isoleucine; L=leu=leucine; K=lys=lysine; M=met=methionine; F=phe=phenylalanine; P=pro=proline; S=ser=serine; T=thr=threonine; W=trp=tryptophan; Y=tyr=tyrosine; V=val=valine; B=asx=asparagine or aspartic acid; Z=glx=glutamine or glutamic acid.

As used herein, to "enhance the stability" of an entity, such as a protein, means to make the entity more long-lived or resistant to dissociation. Enhancing stability can be achieved, for example, by the introduction of disulfide bonds, salt bridges, hydrogen bonds, hydrophobic interactions, favorable van der Waals contacts, a linker peptide or a combination thereof. Stability-enhancing changes can be introduced by recombinant methods. As used herein, "mutant" means that which is not wild-type.

As used herein, "HIV" shall mean the human immunodeficiency virus. HIV shall include, without limitation, HIV-1. The human immunodeficiency virus (HIV) may be either of the two known types of HIV (HIV-1 or HIV-2). The HIV-1 virus may represent any of the known major subtypes (Classes A, B, C, D E, F, G and H) or outlying subtype (Group O).

The human imunodeficiency virus includes but is not limited to the JR-FL strain. Surface proteins include but are not limited to gp120. An amino acid residue of the C1 or C5 region of gp120 may be mutated. The gp120 amino acid residues which may be mutated include but are not limited to the following amino acid residues: V35; Y39, W44; G462; I482; P484; G486; A488; P489; A492; and E500. The gp120 amino acid residues are also set forth in Figure 3a. The gp41 amino acid residues which may be mutated include but are not limited to the following: D580; W587; T596; V599; and P600. The gp41 amino acid residues are also set forth in Figure 3b.

As used herein, the term "nucleic acid" shall mean any nucleic acid including, without limitation, DNA, RNA and hybrids thereof. The nucleic acid bases that form nucleic acid molecules can be the bases A, C, T, G and U, as well as derivatives thereof. Derivatives of these bases are well known in the art and are exemplified in PCR Systems, Reagents and Consumables (Perkin-Elmer Catalogue 1996-1997, Roche Molecular Systems, Inc, Branchburg, New Jersey, USA).

HIV-1_{JR-FL} is a strain that was originally isolated from the brain tissue of an AIDS patient taken at autopsy and co-cultured with lectin-activated normal human PBMCs (O'Brien, 1990). HIV-_{1JR-FL} is known to utilize CCR5 as a fusion coreceptor and has the ability to replicate in phytohemagglutinin (PHA)-stimulated PBMCs and blood-derived macrophages but does not replicate efficiently in most immortalized T cell lines.

HIV-1_{DH123} is a clone of a virus originally isolated from the peripheral mononuclear cells (PBMCs) of a pateint with AIDS (Shibata, 1995). HIV-1_{DH123} is known to utilize both CCR5 and CXCR4 as fusion coreceptors and has the ability to replicate in PHA-stimulated PBMCs, blood-derived macrophages and immortalized T cell lines.

HIV-1_{Gun-1} is a cloned virus originally isolated from the peripheral blood mononuclear cells of a hemophilia B patient with AIDS (Takeuchi, 1987). HIV-1_{Gun-1} is known to utilize both CCR5 and CXCR4 as fusion coreceptors and has the ability to replicate in PHA-stimulated PBMCs, blood-derived macrophages and immortalized T cell lines.

HIV-1_{89.6} is a cloned virus originally isolated from a patient with AIDS (Collman, 1992). HIV-1_{89.6} is known to utilize both CCR5 and CXCR4 as fusion coreceptors and has the ability to replicate in PHA-stimulated PBMCs, blood-derived macrophages and immortalized T cell lines.

HIV-1_{HXB2} is a TCLA virus that is known to utilize CXCR4 as a fusion coreceptor and has the ability to replicate in PHA-stimulated PBMCs and immortalized T cell lines but not blood derived macrophages.

Although the above strains are used herein to generate the mutant viral envelope proteins of the subject invention, other HIV-1 strains could be substituted in their place as is well known to those skilled in the art.

As used herein, "gp41" shall include, without limitation, (a) whole gp41 including the transmembrane and cytoplasmic domains; (b) gp41 ectodomain (gp41ECTO); (c) gp41 modified by deletion or insertion of one or more glycosylation sites; (d) gp41 modified so as to eliminate or mask the well-known immunodominant epitope; (e) a gp41 fusion protein; and (f) gp41 labeled with an affinity ligand or other detectable marker. As used herein, "ectodomain" means the extracellular region of a transmembrane protein exclusive of the transmembrane spanning and cytoplasmic regions.

As used herein, "HIV gp140 protein" shall mean a protein having two disulfide-linked polypeptide chains, the first chain comprising the amino acid sequence of the HIV gp120 glycoprotein and the second chain comprising the amino acid sequence of the water-soluble portion of HIV gp41 glycoprotein ("gp41 portion"). HIV gp140 protein includes, without limitation, proteins wherein the gp41 portion comprises a point mutation such as I559G, L566V, T569P and I559P. HIV gp140 protein comprising such mutations is also referred to as "HIV SOSgp140", as well as "HIV gp140 monomer."

As used herein, "C1 region" means the first conserved sequence of amino acids in the mature gp120 glycoprotein. The C1 region includes the amino-terminal amino acids. In HIV-1_{JR-FL}, the C1 region consists of the amino acids VEKLWVTVYYGVPVWKEATTTLFCASDAKAYDTEVHNVWATHACVPTDPNPQEVVLEN VTEHFNMWKNNMVEQMQEDIISLWDQSLKPCVKLTPLCVTLN. Amino acid resides 30-130 of the sequence set forth in Figure 3a have this sequence. In other HIV isolates, the C1 region will comprise a homologous amino-terminal sequence of amino acids of similar length. W44C and P600C mutations are as defined above for A492 and T596 mutations. Because of the sequence variability of HIV, W44 and P600 will not be at positions 44 and 600 in all HIV isolates. In other HIV isolates, homologous, non-cysteine amino acids may also be present in the place of the tryptophan and proline. This invention encompasses cysteine mutations in such amino acids, which can be readily identified in other HIV isolates by those skilled in the art.

As used herein, "C5 region" means the fifth conserved sequence of amino acids in the gp120 glycoprotein. The C5 region includes the carboxy-terminal amino acids. In HIV-1_{JR-FL} gp120, the unmodified C5 region consists of the amino acids GGGDMRDNWRSELYKYKVVKIEPLGVAPTKAKRRWQRE. Amino acid residues 462-500 of the sequence set forth in Figure 3a have this sequence. In other HIV isolates, the C5 region will comprise a homologous carboxy-terminal sequence of amino acids of similar length.

As used herein, "A492C mutation" refers to a point mutation of amino acid 492 in HIV-I_{JR-FL} gp120 from alanine to cysteine. Because of the sequence variability of HIV, this amino acid will not be at position 492 in all other HIV isolates. For example, in HIV-_{1NL4-3} the corresponding amino acid is A499 (Genbank Accession Number AAA44992). It may also be a homologous amino acid other than alanine or cysteine. This invention encompasses cysteine mutations in such amino acids, which can be readily identified in other HIV isolates by those skilled in the art.

As used herein, "T596C mutation" refers to a point mutation of amino acid 596 in HIV-1_{JR-FL} gp41 from threonine to cysteine. Because of the sequence variability of HIV, this amino acid will not be at position 596 in all other HIV isolates. For example, in HIV-1_{NL4-3} the corresponding amino acid is T603 (Genbank Accesion Number AAA44992). It may also be a homologous amino acid other than threonine or cysteine. This invention encompasses cysteine mutations in such amino acids, which can be readily identified in other HIV isolates by those skilled in the art.

As used herein, "canonical glycosylation site" includes but is not limited to an Asn-X-Ser or Asn-X-Thr sequence of amino acids that defines a site for N-linkage of a carbohydrate. In addition, Ser or Thr residues not present in such sequences to which a carbohydrate can be linked through an O-linkage are canonical glycosylation sites. In the later case of a canonical glycosylation site, a mutation of the Ser and Thr residue to an amino acid other than a serine or threonine will remove the site of O-linked glycosylation.

As used herein, "I559G" shall mean a point mutation wherein the isoleucine residue at position 559 of a polypeptide chain is replaced by a glycine residue.

As used herein, "L566V" shall mean a point mutation wherein the leucine residue at position 566 of a polypeptide chain is replaced by a valine residue.

As used herein, "T569P" shall mean a point mutation wherein the threonine residue at position 569 of a polypeptide chain is replaced by a proline residue.

As used herein, "I559P" shall mean a point mutation wherein the isoleucine residue at position 559 of a polypeptide chain is replaced by a proline residue.

As used herein, "operably affixed", when in reference to a trimeric complex or other antigen on a particle, means affixed so as to permit recognition of the complex or other antigen by an immune system. A "pharmaceutically acceptable particle" means any particle made of a material suitable for introduction into a subject.

As used herein, non-paramagnetic beads may contain, for example, metal oxides, aluminum phosphate, aluminum hydroxide, calcium phosphate, or calcium hydroxide.

As used herein, "vector" shall mean any nucleic acid vector known in the art. Such vectors include, but are not limited to, plasmid vectors, cosmid vectors and bacteriophage vectors. For example one class of vectors utilizes DNA elements which are derived from animal viruses such as animal papilloma virus, polyoma virus, adenovirus, vaccinia virus, baculovirus, retroviruses (RSV, MMTC or MoMLV), Semliki Forest virus or SV40 virus.

As used herein, "host cells" shall include, but are not limited to, bacterial cells (including gram-positive cells), yeast cells, fungal cells, insect cells and animal cells. Suitable animal cells include, but are not limited to HeLa cells, Cos cells, CV1 cells and various primary mammalian cells. Numerous mammalian cells can be used as hosts, including, but not limited to, the mouse fibroblast cell NIH-3T3 cells, CHO cells, HeLa cells, Ltk- cells and COS cells. Mammalian cells can be transfected by methods well known in the art, such as calcium phosphate precipitation, electroporation and microinjection.

In one embodiment, the plasmid is designated PPI4. The invention is not limited to the PPI4 plasmid and may include other plasmids known to those skilled in the art.

In accordance with the invention, numerous vector systems for expression of recombinant proteins may be employed. For example, one class of vectors utilizes DNA elements which are derived from animal viruses such as bovine papilloma virus, polyoma virus, adenovirus, vaccinia virus, baculovirus, retroviruses (RSV, MMTV or MoMLV), Semliki Forest virus or SV40 virus. Additionally, cells which have stably integrated the DNA into their chromosomes may be selected by introducing one or more markers which allow for the selection of transfected host cells. The marker may provide, for example, prototropy to an auxotrophic host, biocide resistance, (e.g., antibiotics) or resistance to heavy metals such as copper or the like. The selectable marker gene can be either directly linked to the DNA sequences to be expressed, or introduced into the same cell by cotransformation. Additional elements may also be needed for optimal synthesis of mRNA. These elements may include splice signals, as well as transcriptional promoters, enhancers, and termination signals. The cDNA expression vectors incorporating such elements include those described by (Okayama and Berg, Mol Cell Biol 3:280, 1983).

The mutant envelope protein may be produced by a) transfecting a mammalian cell with an expression vector for producing mutant envelope glycoprotein; b) culturing the resulting transfected mammalian cell under conditions such that mutant envelope protein is produced; and c) recovering the mutant envelope protein so produced.

Once the expression vector or DNA sequence containing the constructs has been prepared for expression, the expression vectors may be transfected or introduced into an appropriate mammalian cell host. Various techniques may be employed to achieve this, such as, for example, protoplast fusion, calcium phosphate precipitation, electroporation, retroviral transduction, or other conventional techniques. In the case of protoplast fusion, the cells are grown in media and screened for the appropriate activity. Expression of the gene encoding a mutant envelope protein results in production of the mutant protein.

Methods and conditions for culturing the resulting transfected cells and for recovering the mutant envelope protein so produced are well known to those skilled in the art, and may be varied or optimized depending upon the specific expression vector and mammalian host cell employed.

In accordance with the disclosure, the preferred host cells for expressing the mutant envelope protein of this invention are mammalian cell lines. Mammalian cell lines include, for example, monkey kidney CV1 line transformed by SV40 (COS-7); human embryonic kidney line 293; baby hamster kidney cells (BHK); Chinese hamster ovary-cells-DHFR⁺ (CHO); Chinese hamster ovary-cells DHFR⁻(DXB11); monkey kidney cells (CV1); African green monkey kidney cells (VERO-76); human cervical carcinoma cells (HELA); canine kidney cells (MDCK); human lung cells (W138); human liver cells (Hep G2); mouse mammary tumor (MMT 060562); mouse cell line (C127); and myeloma cell lines.

Other eukaryotic expression systems utilizing non-mammalian vector/cell line combinations can be used to produce the mutant envelope proteins. These include, but are not limited to, baculovirus vector/insect cell expression systems and yeast shuttle vector/yeast cell expression systems.

Pharmaceutically acceptable carriers are well known to those skilled in the art and include, but are not limited to, 0.01-0.1M and preferably 0.05M phosphate buffer, phosphate-buffered saline, or 0.9% saline. Additionally, such pharmaceutically acceptable carriers may include, but are not limited to, aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, chelating agents, inert gases and the like.

As used herein, "CCR5" is a chemokine receptor which binds members of the C-C group of chemokines and whose amino acid sequence comprises that provided in Genbank Accession Number 1705896 and related polymorphic variants. As used herein, CCR5 includes extracellular portions of CCR5 capable of binding the HIV-1 envelope protein.

As used herein, "CXCR4" is a chemokine receptor which binds members of the C-X-C group of chemokines and whose amino acid sequence comprises that provided in Genbank Accession Number 400654 and related polymorphic variants. As used herein, CXCR4 includes extracellular portions of CXCR4 capable of binding the HIV-1 envelope protein.

Cytokines may be provided to a subject by a vector expressing one or more cytokines. Likewise, chemokines may be provided via a vector expressing same.

As used herein, "adjuvants" shall mean any agent suitable for enhancing the immunogenicity of an antigen such as protein and nucleic acid. Adjuvants suitable for use with protein-based vaccines include, but are not limited to, alum, Freund's incomplete adjuvant (FIA), Saponin, Quil A, QS21, Ribi Detox, Monophosphoryl lipid A (MPL), and nonionic block copolymers such as L-121 (Pluronic; Syntex SAF). In a preferred embodiment, the adjuvant is alum, especially in the form of a thixotropic, viscous, and homogenous aluminum hydroxide gel. The vaccines of the subject invention may be administered as an oil-in-water emulsion. Methods of combining adjuvants with antigens are well known to those skilled in the art.

Adjuvants may also be in particulate form. The antigen may be incorporated into biodegradable particles composed of poly-lactide-co-glycolide (PLG) or similar polymeric material. Such biodegradable particles are known to provide sustained release of the immunogen and thereby stimulate long-lasting immune responses to the immunogen. Other particulate adjuvants, include but are not limited to, micellular mixtures of Quil A and cholesterol known as immunostimulating complexes (ISCOMs) and aluminum or iron oxide beads. Methods for combining antigens and particulate adjuvants are well known to those skilled in the art. It is also known to those skilled in the art that cytotoxic T lymphocyte and other cellular immune responses are elicited when protein-based immunogens are formulated and administered with appropriate adjuvants, such as ISCOMs and micron-sized polymeric or metal oxide particles.

Suitable adjuvants for nucleic acid based vaccines include, but are not limited to, Quil A, interleukin-12 delivered in purified protein or nucleic acid form, short bacterial immunostimulatory nucleotide sequence such as CpG-containing motifs, interleukin-2/Ig fusion proteins delivered in purified protein or nucleic acid form, oil in water micro-emulsions such as MF59, polymeric microparticles, cationic liposomes, monophosphoryl lipid A (MPL), immunomodulators such as Ubenimex, and genetically detoxified toxins such as E. coli heat labile toxin and cholera toxin from Vibrio. Such adjuvants and methods of combining adjuvants with antigens are well known to those skilled in the art.

As used herein "prophylactically effective amount" means amount sufficient to reduce the likelihood of a disorder from occurring.

As used herein, "therapeutically effective amount" means an amount effective to slow, stop or reverse the progression of a disorder.

As used herein, "subject" means any animal or artificially modified animal. Artificially modified animals include, but are not limited to, SCID mice with human immune systems. Animals include, but are not limited to, mice, rats, dogs, guinea pigs, ferrets, rabbits, and primates. In the preferred embodiment, the subject is a human.

As used herein, "virally infected" means the introduction of viral genetic information into a target cell, such as by fusion of the target cell membrane with the virus or infected cell. The target may be a cell of a subject. In the preferred embodiment, the target cell is a cell in a human subject.

As used herein, "immunizing" means generating an immune response to an antigen in a subject. This can be accomplished, for example, by administering a primary dose of a vaccine to a subject, followed after a suitable period of time by one or more subsequent administrations of the vaccine, so as to generate in the subject an immune response against the vaccine. A suitable period of time between administrations of the vaccine may readily be determined by one skilled in the art, and is usually on the order of several weeks to months.

Depending on the nature of the vaccine and size of the subject, the dose of the vaccine can range from about 1µg to about 10mg. The preferred dose is about 300µg.

Vaccination may be performed in a manner that biases the immune system in a preferred direction, for example, in the direction of a preferred T helper 1 type of immune response or a more T helper 2 type of immune response. It is now widely accepted that T cell-dependent immune responses can be classified on the basis of preferential activation and proliferation of two distinct subsets of CD4+ T-cells termed T_{H}1 and T_{H}2. These subsets can be distinguished from each other by restricted cytokine secretion profiles. The T_{H}1 subset is a high producer of IFN-γ with limited or no production of IL-4, whereas the T_{H}2 phenotype typically shows high level production of both IL-4 and IL-5 with no substantial production of IFN-γ. Both phenotypes can develop from naive CD4+ T cells and at present there is much evidence indicating that IL-12 and IFN-γ on the one hand and IL-4 on the other are key stimulatory cytokines in the differentiation process of pluripotent T_{H}O precursor cells into T_{H}1 or T_{H}2 effector cells, respectively, in vitro and in vivo. Since IFN-γ inhibits the expansion and function of T_{H}2 effector cells and IL-4 has the opposite effect, the preferential expansion of either IFN-γ producing cells (pc) or IL-4 pc is indicative of whether an immune response mounts into a T_{H}1 or T_{H}2 direction. The cytokine environment, however, is not the only factor driving T_{H} lineage differentiation. Genetic background, antigen dose, route of antigen administration, type of antigen presenting cell (APC) and signaling via TCR and accessory molecules on T cells also play a role in differentiation.

The immune system is directed toward a more T helper 1 or 2 type of immune response through using vaccine compositions with the property of modulating an immune response in one direction or the other. In a preferred aspect at least part of said adjuvant function comprises means for directing the immune system toward a more T helper 1 or 2 type of immune response.

the biasing is accomplished using vectors with the property of modulating an immune response in one direction or the other. Examples of vectors with the capacity to stimulate either a more T helper 1 or a more T helper 2 type of immune response or of delivery routes such as intramuscular or epidermal delivery can be found in Robinson, 1997; Sjolander, 1997; Doe, 1996; Feltquate, 1997; Pertmer, 1996; Prayaga, 1997; and Raz, 1996.

In another aspect the immune system is induced to produce innate immune responses with adjuvant potential in the ability to induce local inflammatory responses. These responses include interferons, B-chemokines, and chemokines in general, capable of attracting antigen processing and presenting cells as well as certain lymphocyte populations for the production of additional specific immune responses. These innate type responses have different characteristics depending on the vector or DNA used and their specific immunomodulating characteristics, including those encoded by CpG motifs, and as such, the site of immunization. By using in a specific sequence vaccine compositions containing at least one common specific vaccine antigen, different kinds of desired protective vaccine responses may be generated and optimized. Different kinds of desired immune responses may also be obtained by combining different vaccine compositions and delivering them at different or the same specific sites depends on the desired vaccine effect at a particular site of entry (i.e. oral, nasal, enteric or urogenital) of the specific infectious agent.

In one aspect, the instant vaccine comprises antigen-presenting cells. Antigen-presenting cells include, but are not limited to, dendritic cells, Langerhan cell, monocytes, macrophages, muscle cells and the like. Preferably said antigen presenting cells are dendritic cells. Preferably, said antigen presenting cells present said antigen, or an immunogenic part thereof, such as a peptide, or derivative and/or analogue thereof, in the context of major histocompatibility complex I or complex II.

The potential exists not only to substantially boost immune responses to the recombinant antigen, but to tailor the nature of the immune responses by priming and then delivering one or more subsequent boosts with different forms of the antigen or by delivering the antigen to different immunological sites and/or antigen-presenting cell populations. Indeed, the ability to induce preferred type-1 or type-2 like T-helper responses or to additionally generate specific responses at mucosal and/or systemic sites are envisioned with such an approach. Such protocols, also known as "Prime-boost" protocols, are described in U.S. Patent No. 6,210,663 B1 and WO 00/44410.

### Examples of Prime Boost Regimens.

| **Priming Composition** | **Boosting Composition** |
|---|---|
| NA | AG |
| NA | AGP |
| NA | AG + AGP |
| AG | NA |
| AGP | NA |
| AG + AGP | NA |
| NA + AG | AGP |
| NA + AG | AG + AGP |
| NA + AG | AGP + NA |
| NA + AG + AGP | NA |
| NA + AG + AGP | NA + AG |
| NA + AG + AGP | NA + AGP |
| NA + AG + AGP | AG |
| NA + AG + AGP | AGP |
| NA + AG + AGP | AG + AGP |
| AG | NA |
| AGP | NA |
| AG + AGP | NA |
| AGP | NA + AG |
| AG + AGP | NA + AG |
| AGP + NA | NA + AG |
| NA | NA + AG + AGP |
| NA + AG | NA + AG + AGP |
| NA + AGP | NA + AG + AGP |
| AG | NA + AG + AGP |
| AGP | NA + AG + AGP |
| AG + AGP | NA + AG + AGP |
| AG | AGP |
| AG + AGP | AGP |
| AGP | AG |
| AGP | AG + AGP |

| | |
|---|---|
| NA = Nucleic acid* AG = Antigen AGP = Particle-bound antigen *The nucleic acid component in the above examples can be in the form of a viral vector component. The viral vector can be replicating or non-replicating. | |

Vaccination is provided with at least three different vaccine compositions, wherein the vaccine compositions differ from each other by the form of the vaccine antigen.

For example, a priming vaccine composition is a replication-competent or replication-defective recombinant virus containing a nucleic acid molecule encoding, the antigen, or a viral-like particle. Particularly, the priming composition is a non-replicating recombinant virus or viral-like particle derived from an α-virus.

One method according to this specification involves "priming" a mammalian subject by administration of a priming vaccine composition. "Priming", as used herein, means any method whereby a first immunization using an antigen permits the generation of an immune response to the antigen upon a second immunization with the same antigen, wherein the second immune response is greater than that achieved where the first immunization is not provided.

The priming vaccine, as with other instant compositions, is administered systemically. This systemic administration includes, for example, any parenteral route of administration characterized by physical breaching of a tissue of a subject and administration of an agent through the breach in the tissue. In particular, parenteral administration is contemplated to include, but is not limited to, intradermal, transdermal, subcutaneous, intraperitoneal, intravenous, intraarterial, intramuscular and intrasternal injection, intravenous, interaarterial and kidney dialytic infusion techniques, and so-called "needleless" injections through tissue. Preferably, the systemic, parenteral administration is intramauscular injection. Alternatively the instant vaccine is administered at a site of administration including the intranasal, oral, vaginal, intratracheal, intestinal and rectal mucosal surfaces.

The priming vaccine, as with other instant compositions, may be administered at various sites in the body in a dose-dependent manner. The invention is not limited to the amount or sites of injection(s) or to the pharmaceutical carrier, nor to this immunization protocol. Rather, the priming step encompasses treatment regimens which include a single dose or dosage which is administered hourly, daily, weekly, or monthly, or yearly.

"Priming amount" as used herein, means the amount of priming vaccine used.

Preferably, a boosting vaccine composition is administered about 2 to 27 weeks after administering the priming vaccine to a mammalian subject. The administration of the boosting vaccine is accomplished using an effective amount of a boosting vaccine containing or capable of delivering the same antigen as administered by the priming vaccine.

As used herein, the term "boosting vaccine" includes, as one embodiment, a composition containing the same antigen as in the priming vaccine or precursor thereof, but in a different form, in which the boosting vaccine induces an immune response in the host. In one particular embodiment, the boosting vaccine comprises a recombinant soluble protein.

In another example, a boosting vaccine composition is a replication-competent or replication-defective recombinant virus containing the DNA sequence encoding the protein antigen or the boosting vaccine is a non-replicating α-virus comprising a nucleic acid molecule encoding the protein antigen or a non-replicating vaccine replicon particle derived from an Alphavirus. Adenoviruses, which naturally invade their host through the airways, infect cells of the airways readily upon intranasal application and induce a strong immune response without the need for adjuvants. In another example the boosting vaccine comprises a replication-defective recombinant adenovirus.

Another example of a boosting vaccine is a bacterial recombinant vector containing the DNA sequence encoding the antigen in operable association with regulatory sequences directing expression of the antigen in tissues of the mammal. One example is a recombinant BCG vector. Other examples include recombinant bacterial vectors based on Salmonella, Shigella, and Listeria, among others.

Still another example of a boosting vaccine is a naked DNA sequence encoding the antigen in operable association with regulatory sequences directing expression of the antigen in tissues of the mammal but containing no additional vector sequences. These vaccines may further contain pharmaceutically suitable or physiologically acceptable carriers.

In still additional examples the boosting vaccines can include proteins or peptides (intact and denatured), heat-killed recombinant vaccines, inactivated whole microorganisms, antigen-presenting cells pulsed with the instant proteins or infected/transfected with a nucleic acid molecule encoding same, and the like, all with or without adjuvants, chemokines and/or cytokines.

Cytokines that may be used in the prime and/or boost vaccine or administered separately from the prime and/or boost vaccine include, but are not limited, to interleukin-4, interleukin-5, interleukin-2, interleukin-12, interleukin-15, interleukin-18, GM-CSF, and combinations thereof. The cytokine may be provided by a vector expressing one or more cytokines.

Representative forms of antigens include a "naked" DNA plasmid, a "naked" RNA molecule, a DNA molecule packaged into a replicating or nonreplicating viral vector, an RNA molecule packaged into a replicating or nonreplicating viral vector, a DNA molecule packaged into a bacterial vector, or proteinaceous forms of the antigen alone or present in virus-like particles, or combinations thereof.

As used herein, "virus-like particles" or VLPs are particles which are non-infectious in any host, nonreplicating in any host, which do not contain all of the protein components of live virus particles. In one embodiment, VLPs contain the instant trimeric and a structural protein, such as HIV-1 gag, needed to form membrane-enveloped virus-like particles.

Advantages of VLPs include (1) their particulate and multivalent nature, which is immunostimulatory, and (2) their ability to present the disulfide-stabilized envelope glycoproteins in a near-native, membrane-associated form.

VLPs are produced by co-expressing the viral proteins (e.g., HIV-1 gp120/gp41 and gag) in the same cell. This can be achieved by any of several means of heterologous gene expression that are well-known to those skilled in the art, such as transfection of appropriate expression vector(s) encoding the viral proteins, infection of cells with one or more recombinant viruses (e.g., vaccinia) that encode the VLP proteins, or retroviral transduction of the cells. A combination of such approaches can also be used. The VLPs can be produced either in vitro or in vivo.

VLPs can be produced in purified form by methods that are well-known to the skilled artisan, including centrifugation, as on sucrose or other layering substance, and by chromatography.

The instant nucleic acid delivery vehicle replicates in a cell of an animal or human being vaccinated. Said replicating nucleic acid has as least a limited capacity to spread to other cells of the host and start a new cycle of replication and antigen presentation and/or perform an adjuvant function. In another example, the nucleic acid is non-replicating in an animal or human being being vaccinated. The nucleic acid can comprise nucleic acid of a poxvirus, a Herpes virus, a lentivirus, an Adenovirus, or adeno-associated virus, in particular the nucleic acid comprises nucleic acid of an α-virus including, but not limited to, Venezuelan equine encephalitis (VEE) virus, Semliki Forest Virus, Sindbis virus, and the like. In another example, said nucleic acid delivery vehicle is a VEE virus particle, Semliki Forest Virus particle, a Sindbis virus particle, a pox virus particle, a herpes virus particle, a lentivirus particle, or an adenovirus particle.

The vaccine can comprise, but is not limited to, the following: a recombinant subunit protein, a DNA plasmid, an RNA molecule, a replicating viral vector, a non-replicating viral vector, or a combination thereof.

As used herein, "reducing the likelihood of a subject's becoming infected with a virus" means reducing the likelihood of the subject's becoming infected with the virus by at least two-fold. For example, if a subject has a 1% chance of becoming infected with the virus, a two-fold reduction in the likelihood of the subject becoming infected with the virus would result in the subject having a 0.5% chance of becoming infected with the virus. In the preferred embodiment of this invention, reducing the likelihood of the subject's becoming infected with the virus means reducing the likelihood of the subject's becoming infected with the virus by at least ten-fold.

As used herein, "exposured" to HIV-1 means contact with HIV-1 such that infection could result.

As used herein, "CDR" or complementarity determining region means a highly variable sequence of amino acids in the variable domain of an antibody. As used herein, a "derivatized" antibody is one that has been modified. Methods of derivatization include, but are not limited to, the addition of a fluorescent moiety, a radionuclide, a toxin, an enzyme or an affinity ligand such as biotin.

As used herein, "humanized" describes antibodies wherein some, most or all of the amino acids outside the CDR regions are replaced with corresponding amino acids derived from human immunoglobulin molecules. In one embodiment of the humanized forms of the antibodies, some, most or all of the amino acids outside the CDR regions have been replaced with amino acids from human immunoglobulin molecules but where some, most or all amino acids within one or more CDR regions are unchanged. Small additions, deletions, insertions, substitutions or modifications of amino acids are permissible as long as they do not abrogate the ability of the antibody to bind a given antigen. Suitable human immunoglobulin molecules include IgG1, IgG2, IgG3, IgG4, IgA, IgE and IgM molecules. A "humanized" antibody would retain an antigenic specificity similar to that of the original antibody.

One skilled in the art would know how to make the humanized antibodies of the subject invention. Various publications, also describe how to make humanized antibodies. For example, the methods described in United States Patent No. 4,816,567 comprise the production of chimeric antibodies having a variable region of one antibody and a constant region of another antibody.

United States Patent No. 5,225,539 describes another approach for the production of a humanized antibody. This patent describes the use of recombinant DNA technology to produce a humanized antibody wherein the CDRs of a variable region of one immunoglobulin are replaced with the CDRs from an immunoglobulin with a different specificity such that the humanized antibody would recognize the desired target but would not be recognized in a significant way by the human subject's immune system. Specifically, site directed mutagenesis is used to graft the CDRs onto the framework.

Other approaches for humanizing an antibody are described in United States Patent Nos. 5,585,089 and 5,693,761 and WO 90/07861 which describe methods for producing humanized immunoglobulins. These have one or more CDRs and possible additional amino acids from a donor immunoglobulin and a framework region from an accepting human immunoglobulin. These patents describe a method to increase the affinity of an antibody for the desired antigen. Some amino acids in the framework are chosen to be the same as the amino acids at those positions in the donor rather than in the acceptor. Specifically, these patents describe the preparation of a humanized antibody that binds to a receptor by combining the CDRs of a mouse monoclonal antibody with human immunoglobulin framework and constant regions. Human framework regions can be chosen to maximize homology with the mouse sequence. A computer model can be used to identify amino acids in the framework region which are likely to interact with the CDRs or the specific antigen and then mouse amino acids can be used at these positions to create the humanized antibody.

The above patents 5,585,089 and 5,693,761, and WO 90/07861 also propose four possible criteria which may be used in designing the humanized antibodies. The first proposal was that for an acceptor, use a framework from a particular human immunoglobulin that is unusually homologous to the donor immunoglobulin to be humanized, or use a consensus framework from many human antibodies. The second proposal was that if an amino acid in the framework of the human immunoglobulin is unusual and the donor amino acid at that position is typical for human sequences, then the donor amino acid rather than the acceptor may be selected. The third proposal was that in the positions immediately adjacent to the 3 CDRs in the humanized immunoglobulin chain, the donor amino acid rather than the acceptor amino acid may be selected. The fourth proposal was to use the donor amino acid reside at the framework positions at which the amino acid is predicted to have a side chain atom within 3Å of the CDRs in a three dimensional model of the antibody and is predicted to be capable of interacting with the CDRs. The above methods are merely illustrative of some of the methods that one skilled in the art could employ to make humanized antibodies.

One method for determining whether a subject has produced antibodies capable of blocking the infectivity of a virus is a diagnostic test examining the ability of the antibodies to bind to the stabilized viral envelope protein. As shown herein, such binding is indicative of the antibodies' ability to neutralize the virus. In contrast, binding of antibodies to non-stabilized, monomeric forms of viral envelope proteins is not predictive of the antibodies' ability to bind and block the infectivity of infectious virus (Fouts et al., J. Virol. 71:2779, 1997). The method offers the practical advantage of circumventing the need to use infectious virus.

Numerous immunoassay formats that are known to the skilled artisan are appropriate for this diagnostic application. For example, an enzyme-linked immunosorbent assay (ELISA) format could be used wherein in the mutant virus envelope glycoprotein is directly or biospecifically captured onto the well of a microtiter plate. After wash and/or blocking steps as needed, test samples are added to the plate in a range of concentrations. The antibodies can be added in a variety of forms, including but not limited to serum, plasma, and a purified immunoglobulin fraction. Following suitable incubation and wash steps, bound antibodies can be detected, such as by the addition of an enzyme-linked reporter antibody that is specific for the subject's antibodies. Suitable enzymes include horse radish peroxidase and alkaline phosphatase, for which numerous immunoconjugates and colorimetric substrates are commercially available. The binding of the test antibodies can be compared with that of a known monoclonal or polyclonal antibody standard assayed in parallel. In this example, high level antibody binding would indicate high neutralizing activity.

As an example, the diagnostic test could be used to determine if a vaccine elicited a protective antibody response in a subject, the presence of a protective response indicating that the subject was successfully immunized and the lack of such response suggesting that further immunizations are necessary.

Methods and conditions for purifying mutant envelope proteins from the culture media are provided in the invention, but it should be recognized that these procedures can be varied or optimized as is well known to those skilled in the art.

This invention will be better understood from the Experimental Details which follow. However, one skilled in the art will readily appreciate that the specific methods and results discussed are merely illustrative of the invention as described more fully in the claims which follow thereafter.

### Experimental Details

### Experimental Set I

### A. Materials and Methods

The plasmid designated PPI4-tPA-gp120JR-FL was deposited pursuant to, and in satisfaction of, the requirements of the Budapest Treaty on the International Recognition of the Deposit of Microorganisms for the Purposes of Patent Procedure with the American Type Culture Collection (ATCC), 12301 Parklawn Drive, Rockville, Maryland 20852 under ATCC Accession Number 75431. The plasmid was deposited with ATCC on March 12, 1993. This eukaryotic shuttle vector contains the cytomegalovirus major immediate-early (CMV/MIE) promoter/enhancer linked to the full-length HIV-1 envelope gene whose signal sequence was replaced with that derived from tissue plasminogen activator. In the vector, a stop codon has been placed at the gp120 C-terminus to prevent translation of gp41 sequences, which are present in the vector. The vector also contains an ampicillin resistance gene, an SV40 origin of replication and a DHFR gene whose transcription is driven by the β-globin promoter.

The epitopes for, and some immunochemical properties of, anti-gp120 Mabs from various donors have been described previously (Moore, 1994a; and Moore, 1996). These include Mab 19b to the V3 locus (Moore, 1995); mABs 50.1 and 83.1 to the V3 loop (White-Scharf, 1993); MAbs IgG1b12 and F91 to the CD4 binding site (CD4bs) (Burton, 1994; and Moore, 1996) Mab 2G12 to a unique C3-V4 glycan-dependent epitope (Trkola, 1996) MAb M90 to the C1 region (diMarzo Veronese, 1992); Mab 23a and Ab D7324 to the C5 region (Moore, 1996); Mab 212A to a conformational C1-C5 epitope (Moore, 1994b); Mab 17b to a CD4-inducible epitope (Moore, 1996); Mab A32 to a CD4-inducible C1-C4 epitope (Moore, 1996; and Sullivan, 1998); Mabs G3-519 and G3-299 to C4 or C4/V3 epitopes (Moore, 1996). Mabs to gp41 epitopes included 7B2 to epitope cluster 1 (kindly provided by Jim Robinson, Tulane University); 25C2 to the fusion peptide region (Buchacher, 1994); 2F5 to a neutralizing epitope encompassing residues 665-690 (Muster, 1994). The tetrameric CD4-IgG2 has been described previously (Allaway, 1995).

Anti-HIV Antibodies were obtained from commercial sources, from the NIH AIDS Reagent Program, or from the inventor. Where indicated, the Antibodies were biotinylated with NHS-biotin (Pierce, Rockford, IL) according to the manufacturer's instructions.

Monomeric gp120_{JR-FL} was produced in CHO cells stably transfected with the PPI4-tPA-gp120JR-FL plasmid as described (U.S. Patents 5,866,163 and 5,869,624). Soluble CD4 was purchased from Bartels Corporation (Issaquah, WA).

### Construction of PPI4-based plasmids expressing wild-type and mutant HIV envelope proteins

*Wild-type gp140s (gp190WT).* The gp140 coding sequences were amplified using the polymerase chain reaction (PCR) from full-length molecular clones of the HIV-1 isolates JR-FL, DH123, Gun-1, 89.6, NL4-3 and HxB2. The 5' primer used was designated Kpn1env (5'-GTCTATTATGGGGTACCTGTGTGGAA AGAAGC-3') while the 3' primer was BstBlenv (5'-CGCAGACGCAGATTCGAATT AATACCACAGCCAGTT-3'). PCR was performed under stringent conditions to limit the extent of Taq polymerase-introduced error. The PCR products were digested with the restriction enzymes Kpn1 and Xho1 and purified by agarose gel electrophoresis. Plasmid PPI4-tPA-gp120JR-FL was also digested with the two restriction enzymes and the large fragment (vector) was similarly gel-purified. The PPI4-tPA-gp120JR-FL expression vector has been described previously (U.S. Patents Numbers 5886163 and 5869624). Ligations of insert and vector were carried out overnight at room temperature. DH5αF'Q10 bacteria were transformed with 1/20 of each ligation. Colonies were screened directly by PCR to determine if they were transformed with vector containing the insert. DNA from three positive clones of each construct was purified using a plasmid preparation kit (Qiagen, Valencia, CA) and both strands of the entire gp160 were sequenced. By way of example, pPPI4-gp140WTJR-FL and pPPI4-gp140WTDH123 refer to vectors expressing wild-type, cleavable gp140s derived from HIV-1_{JR-FL} and HIV-1_{DH123}, respectively.

*gp140UNC.* A gp120-gp41 cleavage site mutant of JR-FL gp140 was generated by substitutions within the REKR motif at the gp120 C-terminus, as described previously (Earl, 1990). The deletions were made by site-directed mutagenesis using the mutagenic primers 5'140M (5'-CTACGACTTCGTCTCCGCCTTCGACTACGG GGAATAGGAGCTGTGTTCCTTGGGT-TCTTG-3') and 3'gp140M (sequence conjunction with Kpnlenv and BstBlenv 5'-TCGAAGGCG GAGACGAAGTCGTAGCCGCAGTGCC-TTGGTGGGTGCTACTCCTAATGGTTC-3'). In conjunction with Kpn1env and BstB1, the PCR product was digested with Kpn1 and BstB1 and subcloned into pPPI4 as described above.

Loop-deleted gp120s and gp140s PPI4-based plasmids expressing variable loop-deleted forms of gp120 and gp140 proteins were prepared using the splicing by overlap extension method as described previously (Binley, 1998). In the singly loop-deleted mutants, a Gly-Ala-Gly spacer is used to replace D132-K152 (ΔV1), F156-I191 (ΔV2), or T300-G320 (ΔV3). The numbering system corresponds to that for the JR-FL clone of HIV-1 (Genbank Accession Number U63632).

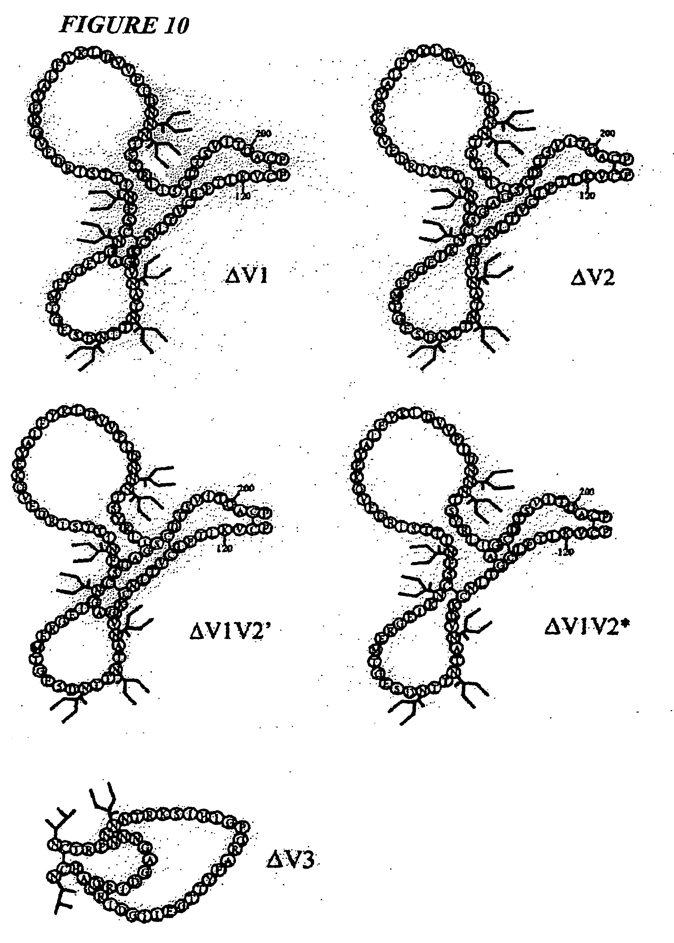

PCR amplification using DGKPN5'PPI4 and 5JV1V2-B (5'-GTCTATTATGGGGTACCTGTGTGGAAAGAAGC-3') on a ΔV1 template and subsequent digestion by Kpn1 and BamH1 generated a 292bp fragment lacking the sequences encoding the V1 loop. This fragment was cloned into a plasmid lacking the sequences for the V2 loop using the Kpn1 and BamH1 restriction sites. The resulting plasmid was designated ΔV1V2' and contained a Gly-Ala-Gly sequences in place of both D132-K152 and F156-I191. Envs lacking the V1, V2 and V3 loops were generated in a similar way using a fragment generated by PCR on a ΔV3 template with primers 3JV2-B (5'-GTCTGAGTCGGATCCTGTGACACCTCAGTCATTACACAG-3') and H6NEW (5'CTCGAGTCTTCGAATTAGTGATGGGTGATGGTGATGATACCACAGCCATTTTGTT A-TGTC-3'). The fragment was cloned into ΔV1V2', using BamH1 and BstB1. The resulting env construct was named ΔV1V2'V3. The glycoproteins encoded by the ΔV1V2' and ΔV1V2'V3 plasmids encode a short sequence of amino acids spanning C125 to C130. These sequences were removed using mutagenic primers that replace T127-I191 with a Gly-Ala-Gly sequence. We performed PCR amplification with primers 3'DV1V2STU1 (5'-GGCTCAAAGGATATCTTTGGACAGGCCTGTGTAATGAC TGAGGTGTCACATCCTGCACCACAGAGTGGGGTTAATTTTACACATGGC-3') and DGKPN5'PPI4, digested the resulting fragment by Stu1 and Kpn1 and cloned it in a PPI4 gp140 vector. The resulting gp140 was named ΔV1V2*. In an analogous manner ΔV1V2*V3 was constructed. The amino acid substitutions are shown schematically in Figure 10.

*Glycosylation site mutants.* Canonical N-linked glycosylation sites were eliminated at positions 357 and 398 on gp120 by point mutations of asparagine to glutamine. These changes were made on templates encoding both wild-type and loop-deleted HIV envelope proteins.

*Disulfide-stabilized gp140s.* The indicated amino acids in gp120 and gp41 were mutated in pairs to cysteines by site-directed mutagenesis using the Quickchange™ kit (Stratagene, La Jolla, CA). As indicated below, additional amino acids in the vicinity of the introduced cysteines were mutated to alanines using similar methods in an attempt to better accommodate the cysteine mutations within the local topology of the envelope glycoproteins. The changes were similarly made on templates encoding both wild-type and loop-deleted HIV envelope proteins.

*Expression of gp140s in transiently transfected 293T cells.* HIV envelope proteins were transiently expressed in adherent 293T cells, a human embryonic kidney cell line (ATCC Cat. Number CRL-1573) transfected with the SV40 large T antigen, which promotes high level replication of plasmids such as PPI4 that contain the SV40 origin. 293T cells were grown in Dulbecco's minimum essential medium (DMEM; Life Technologies, Gaithersburg, MD) containing 10% fetal bovine serum supplemented with L-glutamine, penicillin, and streptomycin. Cells were plated in a 10cm dish and transfected with 10µg of purified PPI4 plasmid using the calcium phosphate precipitation method. On the following day, cells were supplied fresh DMEM containing 0.2% bovine serum albumin along with L-glutamine, penicillin and streptomycin. For radioimmunoprecipitation assays, the medium also contained ³⁵S-labeled cysteine and methionine (200µCi/plate). In certain experiments, the cells were cotransfected with 10µg of a pcDNA3.1 expression vector (Invitrogen, Carlsbad, CA) encoding the gene for human furin.

*ELISA analyses.* The concentration of gp120 and gp140 proteins in 293T cell supernatants was measured by ELISA (Binley, 1997b). Briefly, Immulon II ELISA plates (Dynatech Laboratories, Inc.) were coated for 16-20 hours at 4°C with a polyclonal sheep antibody that recognizes the carboxy-terminal sequence of gp120 (APTKAKRRVVQREKR). The plate was washed with tris buffered saline (TBS) and then blocked with 2% nonfat milk in TBS. Cell supernatants (100µL) were added in a range of dilutions in tris buffered saline containing 10% fetal bovine serum. The plate was incubated for 1 hour at ambient temperature and washed with TBS. Anti-gp120 or anti-gp41 antibody was then added for an additional hour. The plate was washed with TBS, and the amount of bound antibody is detected using alkaline phosphatase conjugated goat anti-human IgG or goat anti-mouse IgG. Alternatively, biotinylated reporter Antibodies are used according to the same procedure and detected using a streptavidin-AP conjugate. In either case, AP activity is measured using the AMPAK kit (DAKO) according to the manufacturer's instructions. To examine the reactivity of denatured HIV envelope proteins, the cell supernatants were boiled for 5 minutes in the presence of 1% of the detergents sodium dodecyl sulfate and NP-40 prior to loading onto ELISA plates in a range of dilutions. Purified recombinant JR-FL gp120 was used as a reference standard.

*Radioimmunoprecipitation assay (RIPA)*. ³⁵S-labeled 293T cell supernatants were collected 2 days post-transfection for RIPA analysis. Culture supernatants were cleared of debris by low speed centrifugation (-300g) before addition of RIPA buffer to a final concentration of 50mM tris-HCl, 150mM NaCl, 5mM EDTA, pH 7.2. Biotinylated antibodies (~10µg) were added to 1mL of supernatant and incubated at ambient temperature for 10 minutes. Samples were then incubated with streptavidin-agarose beads for 12-18 hours at 4°C with gentle agitation. Alternatively, unlabeled antibodies were used in combination with protein G-agarose (Pierce, Rockford, IL). The beads were washed three times with RIPA buffer containing 1% Nonidet-P40 (NP40) detergent. Bound proteins were eluted by heating at 100°C for 5 minutes with SDS-PAGE sample buffer containing 0.05M tris-HCl, 10% glycerol, 2% sodium dodecyl sulfate (SDS), 0.001% bromophenol blue, and where indicated, 100mM dithiothreitol (DTT). Samples were loaded on an 8% polyacrylamide gel and run at 200V for 1 hour. Gels were then dried and exposed to a phosphor screen for subsequent image analysis using a STORM phosphoimager (Molecular Dynamics, Sunnyvale, CA). ¹⁴C-labeled proteins were used as size calibration standards (Life Technologies, Gaithersburg, MD).

The following paragraphs (until p.99, l.16) are published already in D1 and useful for understanding the invention.

### B. Results and Discussion

### Processing of gp140NON is facilitated by co-expression of the furin protease

To minimize the production of gp140NON, pcDNA3.1-furin and pPPI4-gp140WTJR-FL were cotransfected into 293T cells, and RIPA assay was performed using the anti-gp120 MAb 2G12. As indicated in Figure 2, furin eliminated production of gp140NON but had no effect on gp140UNC. Similar results were obtained in RIPAs performed using other anti-gp120 MAbs (data not shown).

Treatment of the samples with DTT prior to SDS-PAGE did not affect the migration or relative amounts of these bands, indicating that the gp140s consist of a single polypeptide chain rather than separate gp120-gp41 molecules linked by an adventitious disulfide bond.

### Stabilization of the gp120-gp41 interaction by introduction of double cysteine mutations

With furin co-transfection, we could now express a soluble gp140 protein in which the gp120 and gp41ECTO components were associated only through a non-covalent linkage, mimicking what occurs in the native trimeric envelope glycoprotein complex on virions. However, on virions or the surface of infected cells, the gp120-gp41 association is weak, so that gp120 is gradually shed (McKeating, 1991). We found this to occur also with the gp140WT protein made in the presence of endogenous furin. Thus, we could detect very little, if any, stable gp120-gp41ECTO complexes in the supernatants from gp140WT-expressing cells after immunoprecipitation. We therefore sought ways to stabilize the non-covalent gp120-gp41 interaction, by the introduction of an intermolecular disulfide bond between the gp120 and gp41 subunits.

We therefore substituted a cysteine residue at one of several different positions in the C1 and C5 regions of gp120, focusing on amino acids previously shown to be important for the gp120-gp41 interaction (Figure 3a). Simultaneously, we introduced a second cysteine mutation at several residues near the intramolecular disulfide loop of gp41 (Figure 3b). The intent was to identify pairs of cysteine residues whose physical juxtaposition in native gp120-gp41 was such that an intermolecular disulfide bond would form spontaneously. In all, >50 different double-cysteine substitution mutants were generated in the context of the JR-FL gp140WT protein, and co-expressed with furin in transient transfections of 293T cells.

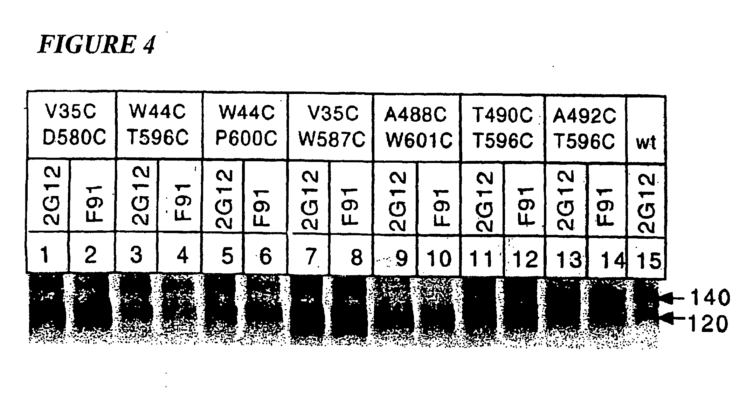

An initial analysis of the transfection supernatants by antigen capture ELISA indicated that all of the mutants were efficiently expressed as secreted proteins, except those which contained a cysteine at residue 486 of gp120 (data not shown). We next characterized the transfection supernatants by immunoprecipitation with the anti-gp120 MAbs 2G12 and F91 (Figure 4). In addition to the expected 120kDa band (gp120), a second band of approximately 140kDa was precipitated by F91 and 2G12 from many of the double-cysteine mutant transfection supernatants. The gp140 bands derived from mutants in which a cysteine was present in the C1 region of gp120 migrated slightly more slowly, and were more diffuse, than the corresponding bands from mutants in which the gp120 cysteine was in the C5 region (Figure 4). The presence of diffuse bands with reduced mobility on SDS-PAGE gels is probably indicative of incomplete or improper envelope glycoprotein processing, based on previous reports (Earl, 1990; and Earl, 1994). The relative intensity of the 140kDa band was highly dependent upon the positions of the introduced cysteines, suggesting that certain steric requirements must be met if a stable intersubunit disulfide bond is to be formed.

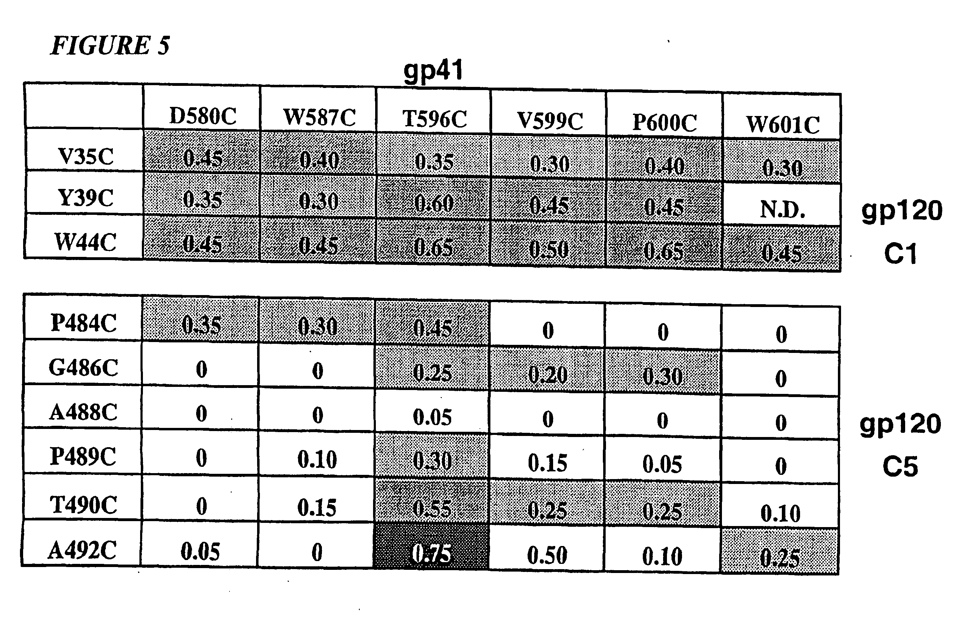

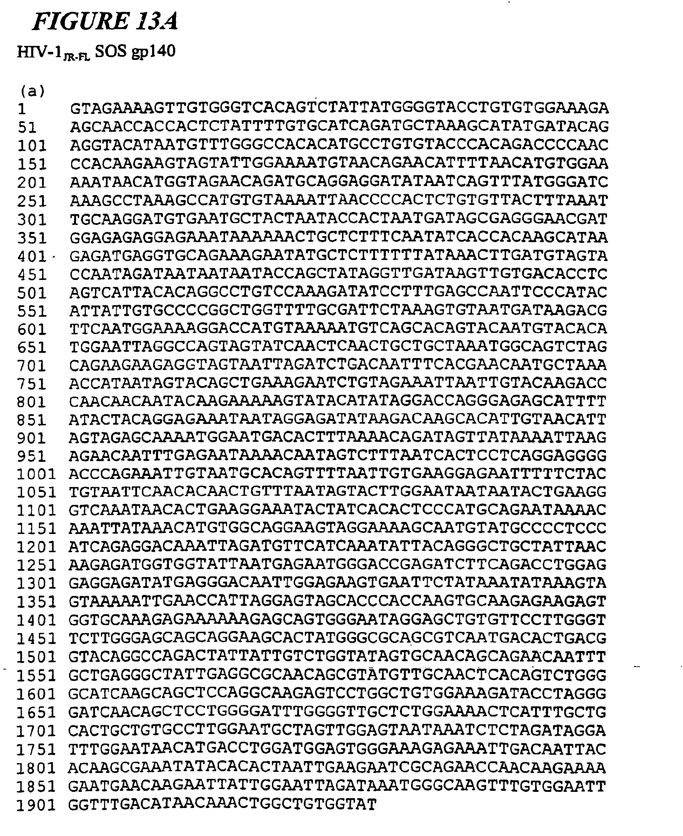

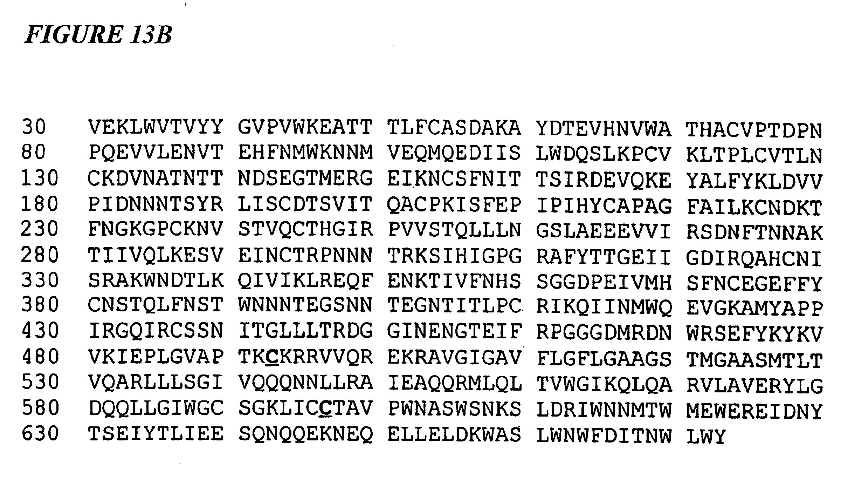

To determine which among the double-cysteine mutants was the most suitable for further analysis, we determined the relative intensities of the gp140 and gp120 bands derived after immunoprecipitation of each mutant by the potently neutralizing anti-gp120 MAb 2G12, followed by SDS-PAGE and densitometry (Figure 5). We sought the mutant for which the gp140/gp120 ratio was the highest, which we interpreted as indicative of the most efficient formation of the intermolecular disulfide bond. From Figure 5, it is clear that mutant A492C/T596C has this property. From hereon, we will refer to this protein as the SOS gp140 mutant. Of note is that the mobility of the SOS gp140 mutant on SDS-PAGE is identical to that of the gp140NON protein, in which the gp120 and gp41ECTO moieties are linked by a peptide bond. The gp140 band derived from the SOS mutant is not quite as sharp as that from the gp140NON protein, but it is less diffuse than the gp140 bands obtained from any of the other double-cysteine mutants (Figure 4). This suggests that the SOS mutant is efficiently processed. The complete nucleic acid and amino acid sequences of the JR-FL SOS gp140 mutant are provided in Figure 13.

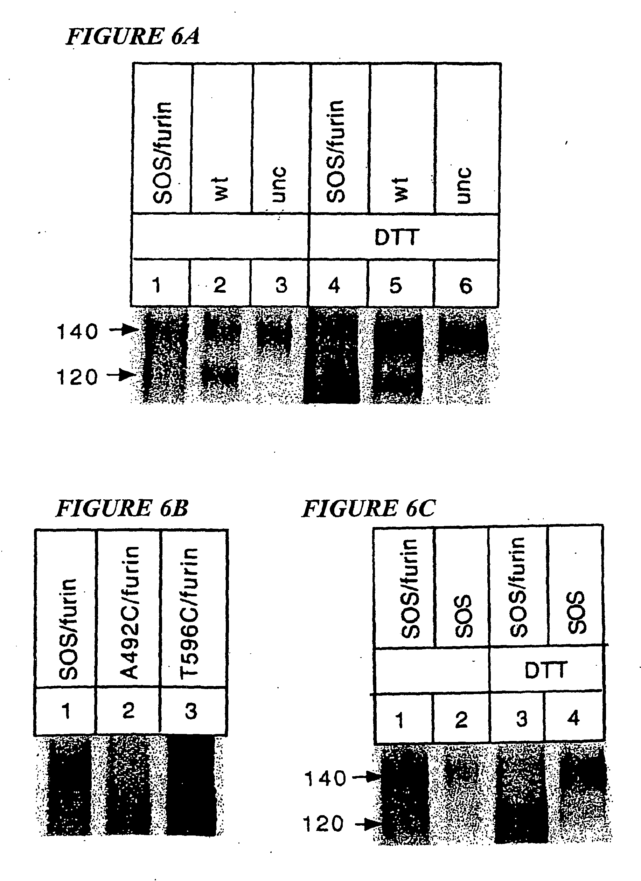

We verified that the 140kDa proteins were stabilized by an intermolecular disulfide bond by treating the immunoprecipitated proteins with DTT prior to gel electrophoresis. In contrast, the 140kDa bands in gp140WT and gp140UNC were unaffected by the DTT treatment as expected for uncleaved single-chain proteins. Of note is that a 140kDa band was never observed for either the A492C or T596C single mutants (Figure 6b). This is further evidence that the 140kDa band in the double-cysteine mutants arises from the formation of an intermolecular disulfide bond between gp120 and gp41ECTO. In the absence of exogenous furin, the 140kDa SOS protein band was not reducible by DTT, suggesting the band is the double cysteine mutant of gp140NON (Figure 6c).

### Approaches to improve the efficiency of disulfide bondformation in the SOS gp140 protein

Disulfide-stabilized gp140 is not the only env species present in the 293T cell supernatants. Discernable amounts of free gp120 are also present. This implies that the disulfide bond between gp120 and the gp41 ectodomain forms with imperfect efficiency. Although the free gp120 can be removed by the purification methods described below, attempts were made to further reduce or eliminate its production. To this end, additional amino acid substitutions were made near the inserted cysteines. In addition, the position of the cysteine in gp120 was varied. We retained the gp41 cysteine at residue 596, as in the SOS gp140 protein, because this position seemed to be the one at which intermolecular disulfide bond formation was most favored.

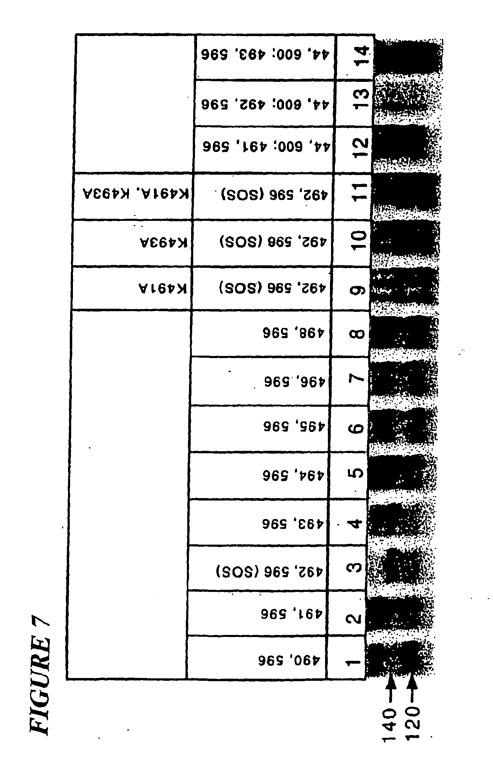

We first varied the position of the cysteine substitution in gp120, by placing it either N-terminal or C-terminal to alanine-492. The gp140/gp140+gp120 ratio was not increased in any of these new mutants; it remained comparable with, or less than, the ratio derived from the SOS gp140 protein (Figure 7). Furthermore, there was usually a decrease in the mobility and sharpness of the gp140 band compared to that derived from the SOS gp140 protein (Figure 7). Next, we considered whether the bulky side chains of the lysine residues adjacent to alanine-492 might interfere with disulfide bond formation. We therefore mutated the lysines at positions 491 and 493 to alanines in the context of the SOS gp140 protein, but these changes neither increased the gp140/gp140+gp120 ratio nor affected the migration of gp140 (Figure 7). Finally, we introduced a second pair of cysteines into the SOS gp140 protein at residues 44 of gp120 and 600 of gp41, since a disulfide bond formed fairly efficiently when this cysteine pair was introduced into the wild-type protein (Figure 5). However, the quadruple-cysteine mutant W44C/A492C/P600C/T596C was poorly expressed, implying that there was a processing or folding problem (Figure 7). Poor expression was also observed with two more quadruple-cysteine mutants W44C/K491C/P600C/T596C and W44C/K493C/P600C/T596C (Figure 7).

Further approaches to optimize the efficiency or overall expression of the disulfide stabilized mutant are possible. For example, cells stably transfected with furin could be created so as to ensure adequate levels of furin in all cells expressing the SOS gp140 proteins. Similarly, furin and the gp140 proteins could be coexpressed from a single plasmid. K491 and K493 could be mutated to non-alanine residues singly or as a pair. To better accommodate the introduced cysteines, other gp120 and/or gp41 amino acids in the vicinity of the introduced cysteines could be mutated as well.

### The antigenicity of the SOS gp140 protein parallels that of virus-associated gp120-gp41

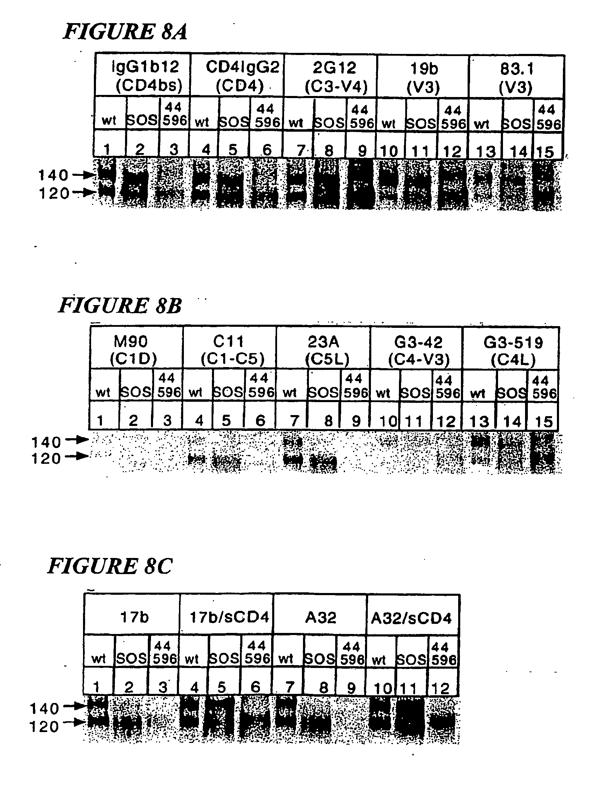

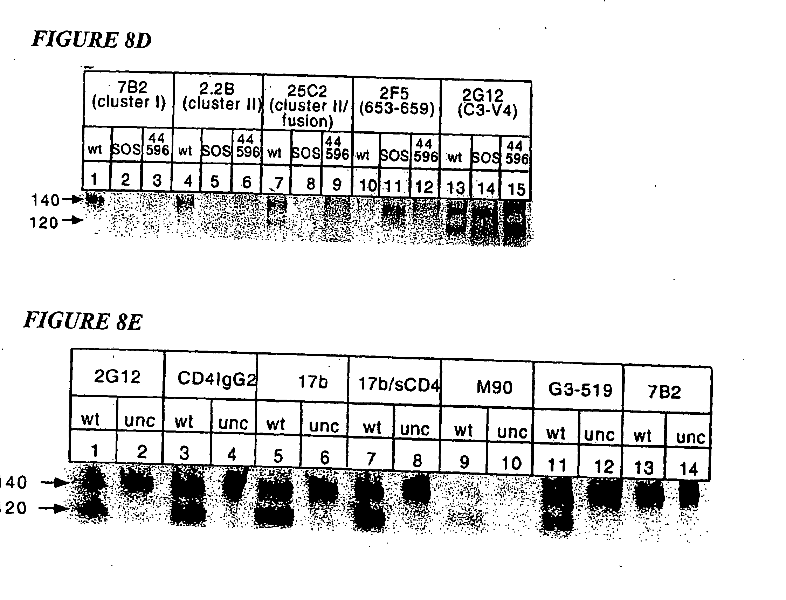

Compared to gp140NON, the SOS gp140 protein has several antigenic differences that we believe are desirable for a protein intended to mimic the structure of the virion-associated gp120-gp41 complex. These are summarized below.
1) The SOS gp140 protein binds strongly to the potently neutralizing MAbs IgG1b12 and 2G12, and also to the CD4-IgG2 molecule (Figure 8a). Although the RIPA methodology is not sufficiently quantitative to allow a precise determination of relative affinities, the reactivities of these MAbs and of the CD4-IgG2 molecule with the SOS gp140 protein appear to be substantially greater than with the gp140NON and gp120 proteins (Figure 8a). Clearly, the SOS gp140 protein has an intact CD4-binding site. V3 loop epitopes are also accessible on the SOS gp140 protein, shown by its reactivity with MAbs 19b and 83.1 (Figure 8a).
2) Conversely, several non-neutralizing anti-gp120 MAbs bind poorly, or not at all, to the SOS gp140 protein whereas they react strongly with gp140NON and gp120 (Figure 8b). These MAbs include ones directed to the C1 and C5 domains, regions of gp120 that are involved in gp41 association and which are considered to be occluded in the context of a properly formed gp120-gp41 complex (Moore, 1994a; and Wyatt, 1997). Conversely, the C1- and C5-directed MAbs all reacted strongly with the gp140NON protein (Figure 8b).
3) The exposure of the epitope for MAb 17b by the prior binding of soluble CD4 occurs far more efficiently on the SOS gp140 protein than on the gp140NON or gp120 proteins (Figure 8c). Indeed, in the absence of soluble CD4, there was very little reactivity of 17b with the SOS gp140 protein. The CD4-induced epitope for MAb 17b overlaps the coreceptor binding site on gp120; it is considered that this site becomes exposed on the virion-associated gp120-gp41 complex during the conformational changes which initiate virus-cell fusion after CD4 binding. Induction of the 17b epitope suggests that the gp120 moieties on the SOS gp140 protein possess the same static conformation and conformational freedom as virus-associated gp120-gp41. The gp140NON protein bound 17b constitutively, and although there was some induction of the 17b epitope upon soluble CD4 binding, this was less than occurred with the SOS gp140 protein.
4) Another CD4-inducible epitope on gp120 is that recognized by MAb A32 (Moore, 1996; and Sullivan, 1998). There was negligible binding of A32 to the SOS gp140 mutant in the absence of soluble CD4, but the epitope was strongly induced by soluble CD4 binding (Figure 8c). As observed with 17b, the A32 epitope was less efficiently induced on the gp140NON protein than on the SOS gp140 protein.
5) There was no reactivity of any of a set of non-neutralizing gp41 MAbs with the SOS gp140 protein, whereas all of these MAbs bound strongly to the gp140NON protein. These anti-gp41 MAbs recognize several regions of the gp41 ectodomain, all of which are thought to be occluded by gp120 in the virion-associated g-p120-gp41 complex (Moore, 1994a; and Sattentau, 1995). Their failure to bind to the SOS gp140 protein is another strong indication that this protein adopts a configuration similar to that of the native trimer; their strong recognition of the gp140NON protein is consistent with the view that these proteins have an aberrant conformation because of the peptide bond linking gp120 with gp41 (Edinger, 1999) (Figure 8d).
6) In marked contrast to what was observed with the non-neutralizing MAbs, the neutralizing anti-gp41 MAb 2F5 bound efficiently to the SOS gp140 protein, but not to the gp140NON protein. Of note is that the 2F5 epitope is the only region of gp41 thought to be well exposed in the context of native gp120-gp41 complexes (Sattentau, 1995). Its ability to bind 2F5 is again consistent with the adoption by the SOS gp140 protein of a configuration similar to that of the native trimer.

The antigenic properties of the SOS gp140 protein were compared with those of the W44C/T596C gp140 mutant. Among the set of mutants that contained a cysteine substitution within the C1 domain, this was the most efficient at gp140 formation. Although the W44C/T596C gp140 reacted well with the 2G12. MAb, it bound CD4-IgG2 and IgG1b12 relatively poorly. Furthermore, there was little induction of the 17b epitope on the W44C/T596C gp140 by soluble CD4, yet strong reactivity with non-neutralizing anti-gp41 MAbs (Figure 8). We therefore judge that this mutant has suboptimal antigenic properties. Indeed, the contrast between the properties of the W44C/T596C gp140 protein and the SOS gp140 protein demonstrates that the positioning of the intermolecular disulfide bonds has a significant influence on the antigenic structure of the resulting gp140 molecule.

In contrast to the antigenic character of the gp140SOS protein, the 140kDa proteins of gp140WT and gp140UNC reacted strongly with non-neutralizing anti-gp120 and anti-gp41 MAbs such as G3-519 and 7B2. In addition, the epitope recognized by MAb 17B was constitutively exposed rather than CD4-inducible (Figure 8e).

Overall, there was a strong correlation between the binding of MAbs to the SOS gp140 protein and their ability to neutralize HIV-1_{JR-FL}. This correlation was not observed with the gp140NON, gp140UNC or gp120 proteins.

### The formation of intersubunit disulfide bonds is not isolate-dependent

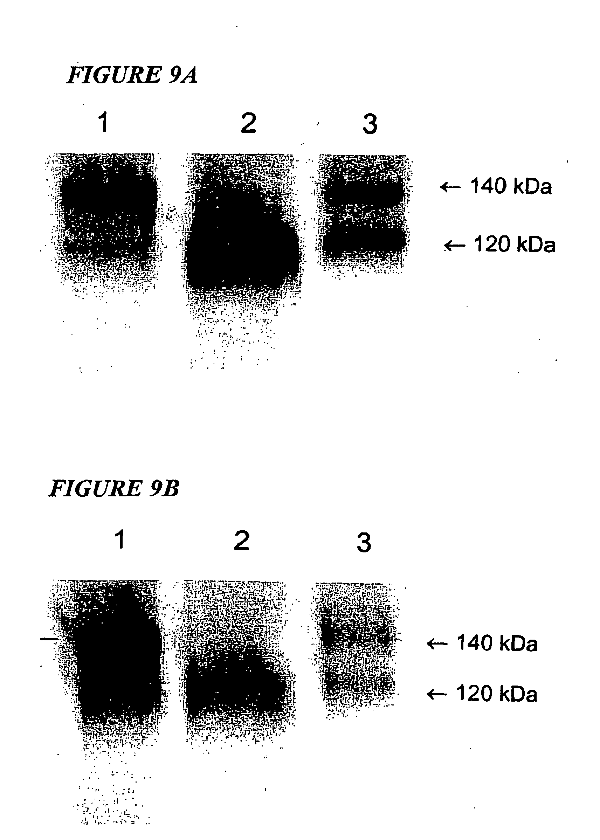

To assess the generality of our observations with gp140 proteins derived from the HIV-1 isolate JR-FL, we generated double-cysteine mutants of gp140's from other HIV-1 strains. These include the R5X4 virus DH123 and the X4 virus HxB2. In each case, the cysteines were introduced at the residues equivalent to alanine-492 and threonine-596 of JR-FL. The resulting SOS proteins were transiently expressed in 293T cells and analyzed by RIPA to ascertain their assembly, processing and antigenicity. As indicated in Figure 9, 140kDa material is formed efficiently in the DH123 and HxB2 SOS proteins, demonstrating that our methods can successfully stabilize the envelope proteins of diverse viral isolates.

### Disulfide stabilization of HIV envelope proteins modified in variable loop and glycosylation site regions

Since there is evidence to suggest that certain variable loop and glycosylation site mutations provide a means to better expose underlying conserved neutralization epitopes, we examined the assembly and antigenicity of disulfide-stabilized forms. In initial studies, A492C/T596C JR-FL gp140 mutants were created for each of the ΔV1, ΔV2, ΔV3, ΔV1V1*, and ΔV1V2*V3 molecules described above. For the ΔV1V2*V3 protein, glycosylation site mutants were also synthesized by N-Q point mutations of amino acids 357 and 398.

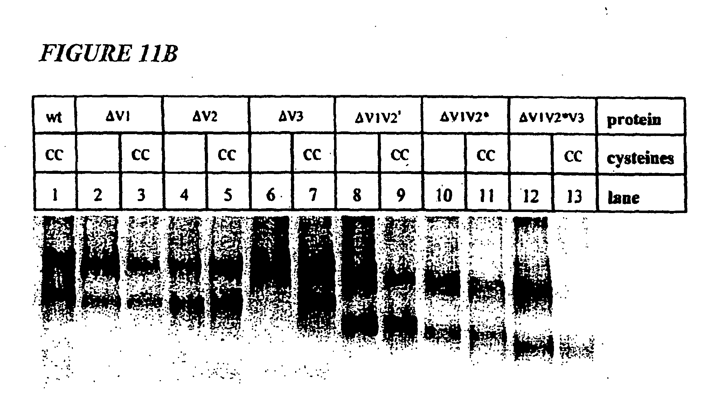

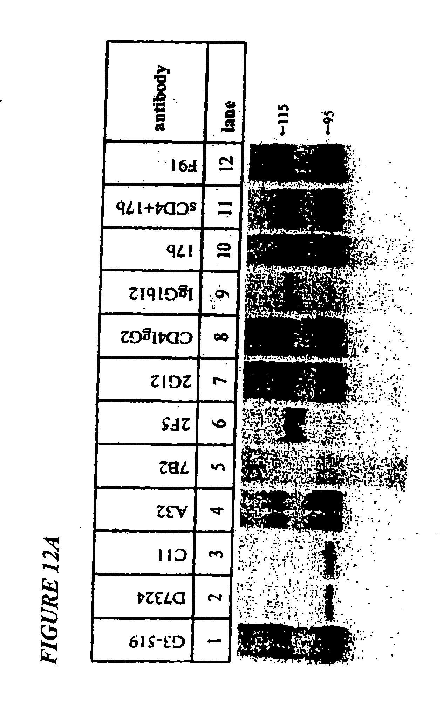

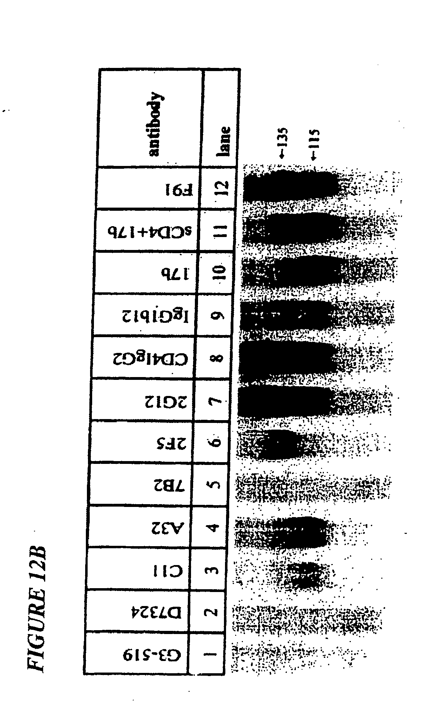

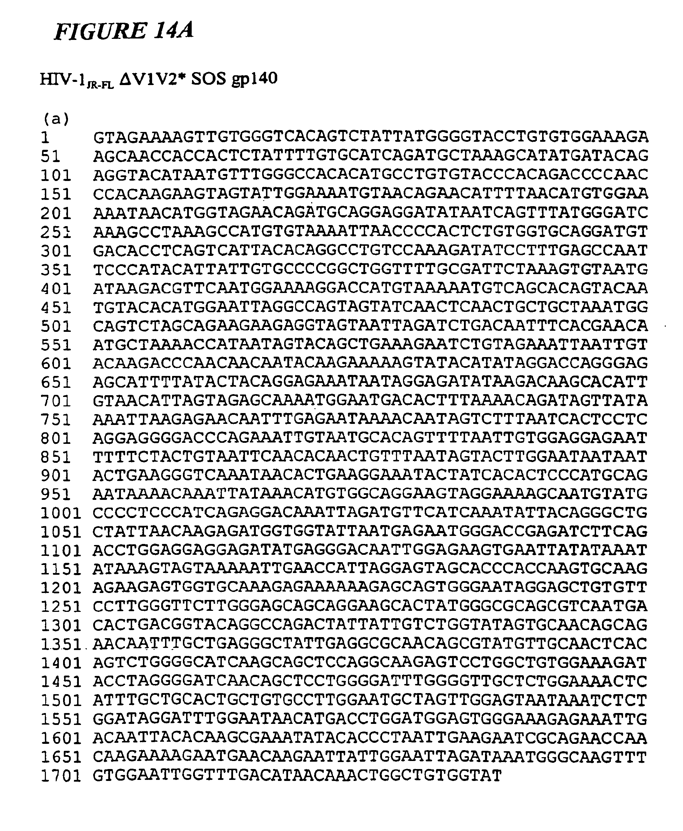

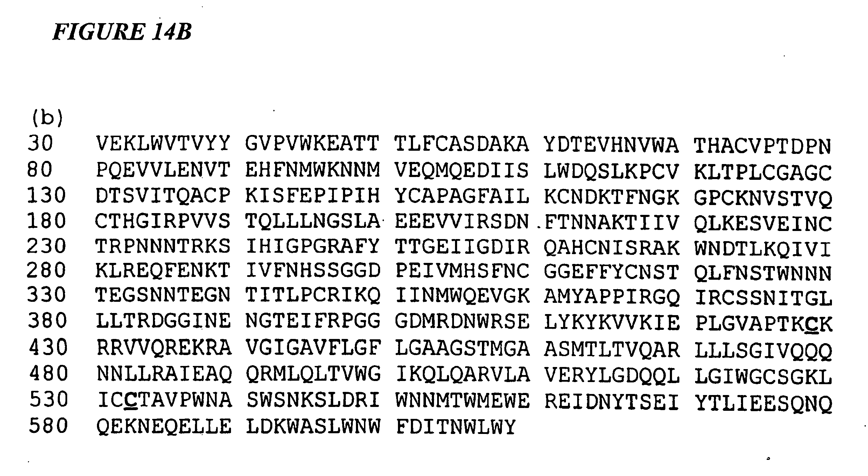

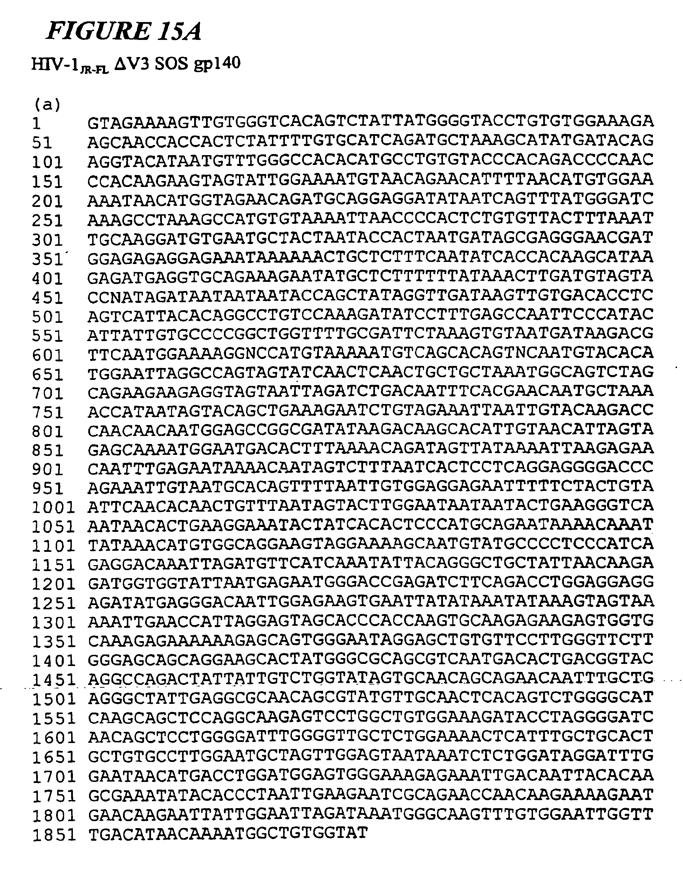

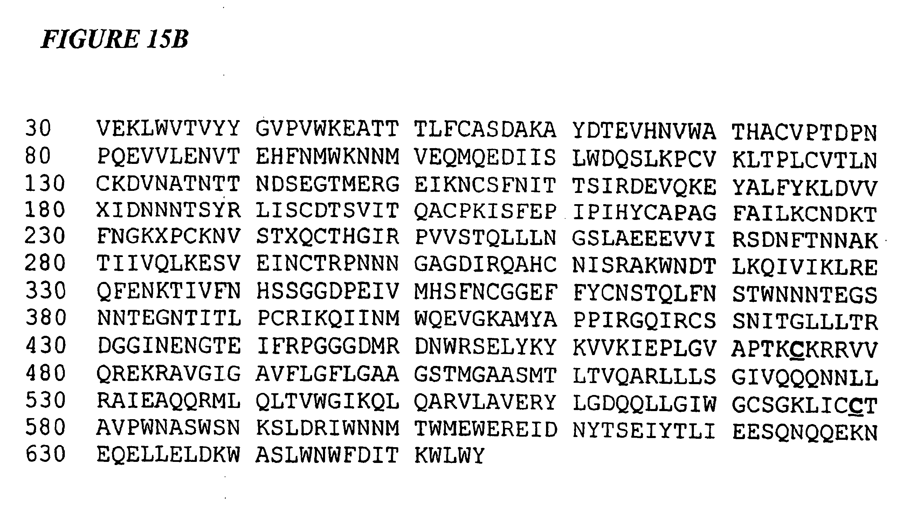

For each of the singly and doubly loop-deleted mutants, we could detect gp140 bands in comparable quantities as for the full-length SOS gp140 protein (Figure 11b). To see whether deletion of the variable loops altered antigenicity in an oligomeric context, we precipitated the ΔV3 and ΔV1V2* SOS proteins with a panel of MAbs (Figure 12). MAbs to gp41 except 2F5 did not bind to loop deleted versions of the cysteine stabilized protein, indicating that those epitopes are still occluded. MAbs to C1 and C5 epitopes were similarly non-reactive. The neutralizing antibody 2F5 did bind to the mutants and was particularly reactive with the ΔV3 SOS protein. MAbs to the CD4BS (IgG1b12, F91) as well as 2G12 bound avidly to these mutants as well. Of note is that CD4-IgG2 and 2G12 bound with very high affinity to the oligomeric ΔV3 SOS protein. Furthermore, consistent with data indicating that the CD4i epitopes are constitutively exposed on the ΔV1V2* protein, binding of MAbs 17b and A32 to the ΔV1V2* SOS mutant was not inducible by sCD4. The ΔV3 SOS mutant, however, bound 17b and A32 weakly in the absence of sCD4 and strongly in its presence. These results are consistent with observations that the V1/V2 and V3 loop structures are involved in occlusion of the CD4i epitopes (Wyatt, 1995). Taken together, the results demonstrate that variable loop-deleted gp140s can be disulfide-stabilized without loss of conformational integrity. Figures 14 and 15, respectively, contain the complete nucleic acid and amino acid sequences of the ΔV1V2* and ΔV3 JR-FL SOS proteins.

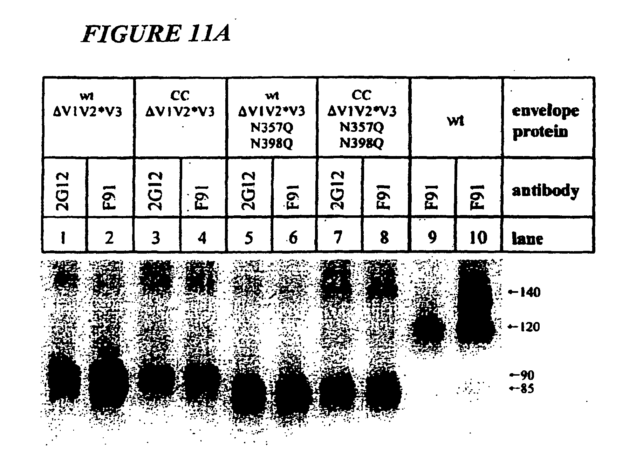

For the ΔV1V2*V3 and ΔV1V2*V3 N357Q N398Q SOS mutants, we could not precipitate a gp140 (110 kDa and 105 kDa) with any of a variety of neutralizing and non-neutralizing MAbs (Figure 11a, Lanes 3, 4, 7 & 8). We did, however, observe strong 90kDa and 85kDa bands, which correspond to the mutant gp120 domains. These preliminary experiments suggest a variety of approaches for disulfide-stabilizing triply-loop deleted gp140s, including adjusting the location(s) of one or more introduced cysteines, adding additional pairs of cysteines, modifying amino acids adjacent to the introduced cysteines, and modifying the manner in which the loops are deleted. Alternatively, triply loop deleted gp140s derived from other HIV isolates may be more readily stabilized by cysteines introduced at residues homologous to 496/592.

### Production and purification of recombinant HIV-1 envelope glycoproteins

Milligram quantities of high quality HIV-1 envelope glycoproteins are produced in CHO cells stably transfected with PPI4 envelope-expressing plasmids (U.S. Patent 5,886,163 and 5,869,624). The PPI4 expression vector contains the dhfr gene under the control of the ß-globin promoter. Selection in nucleoside-free media of dhfr+ clones is followed by gene amplification using stepwise increases in methotrexate concentrations. The cytomegalovirus (CMV) promoter drives high level expression of the heterologous gene, and the tissue plasminogen activator signal sequence ensures efficient protein secretion. A high level of gp120 expression and secretion is obtained only upon inclusion of the complete 5' non-coding sequences of the CMV MIE gene up to and including the initiating ATG codon. To produce milligram quantities of protein, recombinant CHO cells are seeded into roller bottles in selective media and grown to confluency. Reduced serum-containing media is then used for the production phase, when supernatants are harvested twice weekly. A purification process comprising lectin affinity, ion exchange, and/or gel filtration chromatography is carried out under non-denaturing conditions.

### A protocol for determining the immunogenicity of stabilized HIV-1 envelope subunit proteins

Purified recombinant HIV-1 envelope proteins are formulated in suitable adjuvants (e.g., Alum or Ribi Detox). For alum, formulation is achieved by combining the mutant HIV-1 envelope glycoprotein (in phosphate buffered saline, normal saline or similar vehicle) with preformed aluminum hydroxide gel (Pierce, Rockford, IL) at a final concentration of approximately 500µg/mL aluminum. The antigen is allowed to adsorb onto the alum gel for two hours at room temperature. Guinea pigs or other animals are immunized 5 times, at monthly intervals, with approximately 100µg of formulated antigen, by subcutaneous intramuscular or intraperitoneal routes. Sera from immunized animals are collected at biweekly intervals and tested for reactivity with HIV-1 envelope proteins in ELISA as described above and for neutralizing activity in well established HIV-1 infectivity assays (Trkola, 1998). Vaccine candidates that elicit the highest levels of HIV-1 neutralizing Antibodies can be tested for immunogenicity and efficacy in preventing or treating infection in SHIV-macaque or other non-human primate models of HIV infection, as described below. The subunit vaccines could be used alone or in combination with other vaccine components, such as those designed to elicit a protective cellular immune response.

For these studies, the HIV-1 envelope proteins also may be administered in complex with one or more cellular HIV receptors, such as CD4, CCR5, and CXCR4. As described above, the binding of soluble CD4 exposes formerly cryptic conserved neutralization epitopes on the stabilized HIV-1 envelope protein. Antibodies raised to these or other neoepitopes could possess significant antiviral activity. As described above, interaction of CD4-env complexes with fusion coreceptors such as CCR5 and CXCR4 is thought to trigger additional conformational changes in env required for HIV fusion. Trivalent complexes comprising the stabilized env, CD4, and coreceptor could thus adopt additional fusion intermediary conformations, some of which are thought to be sufficiently long-lived for therapeutic and possibly immunologic interventions (Kilby, 1998). Methods for preparing and administering env-CD4 and env-CD4-coreceptor complexes are well-known to the skilled artisan (LaCasse, 1999; Kang, 1994; and Gershoni, 1993).

### A protocol for determining the immunogenicity of nucleic acid-based vaccines encoding stabilized HIV-1 envelope proteins

PCR techniques are used to subclone the nucleic acid into a DNA vaccine plasmid vector such as pVAX1 available from Invitrogen (catalog number V260-20). PVAX1 was developed according to specifications in the FDA document "Points to Consider on Plasmid DNA Vaccines for Preventive Infectious Disease Indications" published on December 22, 1996. PVAX1 has the following features: Eukaryotic DNA sequences are limited to those required for expression in order to minimize the possibility of chromosomal integration, Kanamycin is used to select the vector in E.coli because ampicillin has been reported to cause an allergic response in some individuals, Expression levels of recombinant proteins from pVAX1 is comparable to those achieved with its parent vector, pc DNA3.1, and the small size of pVAX1 and the variety of unique cloning sites amplify subcloning of even very large DNA fragments.

Several methods can be used to optimize expression of the disulfide stabilized protein in vivo. For example, standard PCR cloning techniques could be used to insert into pVAX1 certain elements of the optimized PPI4 expression vector, including Intron A and adjoining regions of the CMV promoter. In addition, the genomic DNA sequences of the HIV-1 envelope are biased towards codons that are suboptimal for expression in mammalian cells (Haas, 1996). These can be changed to more favorable codons using standard mutagenesis techniques in order to improve the immunogenicity of nucleic acid based HIV vaccines (Andre, 1998). The codon optimization strategy could strive to increase the number of CpG motifs, which are known to increase the immunogenicity of DNA vaccines (Klinman, 1997). Lastly, as for the transient transfection systems described above, env processing into gp120-gp41 may be facilitated by the heterologous expression of furin introduced on the same or separate expression vectors.

The insert containing plasmid can be administered to the animals by such means as direct injection or using gene gun techniques. Such methods are known to those skilled in the art.

In one protocol, Rhesus macaques are individually inoculated with five approximately 1mg doses of the nucleic acid. The doses are delivered at four week intervals. Each dose is administered intramuscularly. The doses are delivered at four week intervals. After four months, the animals receive a single immunization at two separate sites with 2mg of nucleic acid with or without 300µg of mutant HIV-1 envelope glycoprotein. This series may be followed by one or more subsequent recombinant protein subunit booster immunizations. The animals are bled at intervals of two to four weeks. Serum samples are prepared from each bleed to assay for the development of specific antibodies as described in the subsequent sections.

### SHIV Challenge Experiments

Several chimeric HIV-SIV viruses have been created and characterized for infectivity in Rhesus monkeys. For Virus challenge experiments, the Rhesus monkeys are injected intravenously with a pre-titered dose of virus sufficient to infect greater than 9/10 animals. SHIV infection is determined by two assays. ELISA detection of SIV p27 antigen in monkey sera is determined using a commercially available kit (Coulter). Similarly, Western blot detection of anti-gag antibodies is performed using a commercially available kit (Cambridge Biotech).

A reduction in either the rate of infection or the amount of p27 antigen produced in immunized versus control monkeys would indicate that the vaccine or vaccine combination has prophylactic value.

### Experimental Set II

### A. Synopsis of Results

The gp120 and gp41 subunits of the human immunodeficiency virus type 1 (HIV-1) envelope glycoprotein associate via weak, non-covalent interactions, which can be stabilized by an intersubunit disulfide bond between cysteine residues introduced at appropriate sites in gp120 and gp41. The properties of such a protein, designated SOS gp140, are described herein. HIV-1_{JR-FL} SOS gp140, proteolytically uncleaved gp140 (gp140UNC) and gp120 were expressed in stably transfected Chinese hamster ovary (CHO) cells and analyzed for antigenic and structural properties before and after purification. In surface plasmon resonance (SPR) and radioimmunoprecipitation assays, SOS gp140 avidly bound the broadly neutralizing monoclonal antibodies (MAbs) 2G12 (anti-gp120) and 2F5 (anti-gp41), whereas gp140UNC bound these MAbs less avidly. In addition, MAb 17b against a CD4-induced epitope that overlaps the CCR5-binding site bound more strongly and rapidly to SOS gp140 than to gp140UNC. In contrast, gp140UNC displayed the greater reactivity with non-neutralizing anti-gp120 and anti-gp41 MAbs. A series of immunoelectron microscopy studies suggested a model for SOS gp140 wherein the gp41 ectodomain (gp41ECTO) occludes the "non-neutralizing" face of gp120, consistent with the antigenic properties of this protein. Also discussed is the application of Blue Native polyacrylamide gel electrophoresis (BN-PAGE), a high-resolution molecular sizing method, to the study of viral envelope proteins in purified and unpurified form. BN-PAGE and other biophysical studies demonstrated that SOS gp140 was monomeric, whereas gp140UNC comprised a mixture of non-covalently associated and disulfide-linked oligomers that could be resolved into dimers, trimers and tetramers by BN-PAGE. The oligomeric and antigenic properties of these proteins were largely unaffected by purification. An uncleaved gp140 protein containing the SOS cysteine mutations (SOS gp140UNC) was also oligomeric, indicating that cleavage of an oligomeric gp140 protein into gp120 and gp41 subunits destabilizes the gp41-gp41 interactions. This may be necessary for fusion to occur, but hinders the production of recombinant envelope glycoprotein complexes that mimic the native, virion-associated structure. Surprisingly, variable-loop-deleted SOS gp140 proteins were expressed as cleaved, non-covalently associated oligomers that were significantly more stable than the full-length protein. This suggests one path for producing proteolytically mature forms of the HIV-1 envelope glycoproteins in purified, oligomeric form. Overall, our findings have relevance for rational vaccine design.

### B. Introduction

HIV vaccine development targeting HIV envelope glycoproteins has been hindered by the inherent instability of the native envelope glycoprotein complex. Therefore, more stable forms of the envelope glycoprotein complex that better mimic the native structure need to be developed.

An approach to resolving the instability of the native complex is to remove the cleavage site that naturally exists between the gp120 and gp41 subunits. Doing so means that proteolysis of this site does not occur, leading to the expression of gp140 glycoproteins in which the gp120 subunit is covalently linked to the gp41 ectodomain (gp41ECTO) by means of a peptide bond (Berman, 1990; Berman, 1988; Earl, 1997; Earl, 1994; and Earl, 1990). Such proteins can be oligomeric, sometimes trimeric (Chen, 2000; Earl, 1997; Earl, 1994; Earl, 1990; Earl, 2001; Edinger, 2000; Farzan, 1998; Richardson, 1996; Stamatatos, 2000; Yang, 2000a; Yang, 2000b; Yang, 2001; and Zhang, 2001).

However, it is not clear that they truly represent the structure of the native, fusion-competent complex in which the gp120-gp41 cleavage site is fully utilized. Hence the receptor-binding properties of uncleaved gp140 (gp140UNC) proteins tend to be impaired, and non-neutralizing antibody epitopes are exposed on them that probably are not accessible on the native structure (Binley, 2000a; Burton, 1997; Hoffman, 2000; Sattentau, 1995; and Zhang, 2001).

An alternative approach to the problem of gp120-gp41 instability, is to retain the cleavage site but to introduce a disulfide bond between the gp120 and gp41ECTO subunits (Binley, 2000a; and Sanders, 2000). Properly positioned, this intermolecular disulfide bond forms efficiently during envelope glycoprotein (Env) synthesis, allowing the secretion of gp140 proteins that are proteolytically processed but in which the association between the gp120 and gp41ECTO subunits is maintained by the disulfide bond.

Here we show that the gp41-gp41 interactions are unstable in the SOS gp140 protein, which is expressed and purified primarily as a monomer. In contrast, gp140UNC proteins, with or without the SOS cysteine substitutions, are multimeric, implying that cleavage of the peptide bond between gp120 and gp41 destabilizes the native complex. Despite being monomeric, the purified and unpurified forms of SOS gp140 are better antigenic structural mimics of the native, fusion-competent Env structure than are the corresponding gp120 or gp140UNC proteins. This may be because the presence and orientation of gp41ECTO occludes certain non-neutralization epitopes on SOS gp140 while preserving the presentation of important neutralization sites. This explanation is consistent with immunoelectron microscopy studies of the protein. Unexpectedly, proteolytically mature, but variable-loop-deleted, SOS gp140 glycoproteins have enhanced oligomeric stability, so these molecules warrant further study for their structural and immunogenic properties.

### C. Materials and Methods

*Plasmids.* The pPPI4 eukaryotic expression vectors encoding SOS and uncleaved forms of HIV-1_{JR-FL} gp140 have been described previously (Binley, 2000a; and Trkola, 1996). The SOS gp140 protein contains cysteine substitutions at residues A501 in the C5 region of gp120 and T605 in gp41 (Binley, 2000a; and Sanders, 2000). In gp140UNC, the sequence KRRVVQREKRAV at the junction between gp120 and gp41ECTO has been replaced with a hexameric LR motif to prevent scission of gp140 into gp120 and gp41ECTO (Binley, 2000a). Plasmids encoding variable-loop-deleted forms of HIV-l_{JR-FL} SOS gp140 have been described (Sanders, 2000). In these constructs, the tripeptide GAG is used to replace V1 loop sequences (D133-K155) and V2 loop sequences (F159-I194), alone or in combination. The SOS gp140UNC protein contains the same cysteine substitutions that are present in SOS gp140, but the residues REKR at the gp120-gp41ECTO cleavage site have been replaced by the sequence IEGR, to prevent gp140 cleavage. The furin gene (Thomas, 1988) was expressed from plasmid pcDNA3.1furin (Binley, 2000a).

*MAbs and CD4-based proteins.* The following anti-gp120 MAbs were used: IgG1b12 [against the CD4 binding site (Burton, 1994)], 2G12 [against a unique C3-V4 glycan-dependent epitope (Trkola, 1996)], 17b [against a CD4-inducible epitope (Thali, 1993), 19b [against the V3 loop (Moore, 1995)], and 23A [against the C5 region (Moore, 1996)]. The anti-gp41 MAbs were 2F5 [against a cluster 1 epitope centered on the sequence ELDKWA (Muster, 1993; and Parker, 2001)] and 2.2B [against epitope cluster II]. MAbs IgG1b12, 2G12 and 2F5 are broadly neutralizing (Trkola, 1995). MAb 17b weakly neutralizes diverse strains of HIV-1, more so in the presence of soluble CD4 (Thali, 1993), whereas the neutralizing activity of MAb 19b against primary isolates is limited (Trkola, 1998). MAbs 23A and 2.2B are non-neutralizing. Soluble CD4 (sCD4) and the CD4-based molecule CD4-IgG2 have been described elsewhere (Allaway, 1995).

*HIV-1 gp140 and gp120 glycoproteins.* To create stable cell lines that secrete full-length HIV-1_{JR-FL} SOS gp140 or ΔV1V2 SOS gp140, we co-transfected DXB-11 dihydrofolate reductase (dhfr)-negative CHO cells with pcDNA3.1furin and either pPPI4-SOS gp140 (Binley, 2000a) or pPPI4-ΔV1V2* SOS gp140 (Sanders, 2000), respectively, using the calcium phosphate precipitation method. Doubly transformed cells were selected by passaging the cells in nucleoside-free α-MEM media containing 10% fetal bovine serum (FBS), geneticin (Life Technologies, Rockville, MD) and methotrexate (Sigma, St. Louis, MO). The cells were amplified for gp140 expression by stepwise increases in methotrexate concentration, as described elsewhere (Allaway, 1995). Clones were selected for SOS gp140 expression, assembly, and endoproteolytic processing based on SDS-PAGE and Western blot analyses of culture supernatants. CHO cells expressing SOS gp140UNC were created using similar methods, except that pcDNA3.1furin and geneticin were not used. Full-length SOS gp140 was purified from CHO cell culture supernatants by Galanthus nivalis lectin affinity chromatography (Sigma) and Superdex 200 gel filtration chromatography (Amersham-Pharmacia, Piscataway, NJ), as described elsewhere (Trkola, 1996). The gp140UNC glycoprotein was purified by lectin chromatography only. The concentration of purified Envs was measured by UV spectroscopy as described (Scandella, 1993), and was corroborated by ELISA and densitometric analysis of SDS-PAGE gels. Recombinant HIV-1_{JR-FL}, HIV-1_{LAI} and HIV-1_{YD2} gp120 glycoproteins were produced using methods that have been previously described (Trkola, 1996; and Wu, 1996).

Where indicated, HIV-1 envelope glycoproteins were transiently expressed in adherent 293T cells by transfection with Env- and furin-expressing plasmids, as described previously (Binley, 2000a). For radioimmunoprecipitation assays, the proteins were metabolically labeled with [³⁵S]cysteine and [³⁵S]methionine for 24 hour prior to analysis.

*SDS-PAGE, radioimmunoprecipitation, Blue Native PAGE, and Western blot analyses.* Sodium dodecyl sulfate polyacrylamide gel electrophoresis (SDS-PAGE) analyses were performed as described elsewhere (Binley, 2000a). Reduced and non-reduced samples were prepared by boiling for 2 minutes in Laemmli sample buffer (62.5mM Tris-HCl, pH 6.8, 2% SDS, 25% glycerol, 0.01% bromophenol blue) in the presence or absence, respectively, of 50mM dithiothreitol (DTT). Protein purity was determined by densitometric analysis of the stained gels followed by the use of ImageQuant software (Molecular Devices, Sunnyvale, CA). Radioimmunoprecipitation assays (RIPA) were performed on Env-containing cell culture supernatants, as previously described (Binley, 2000a; and Sanders, 2000).

Blue Native (BN)-PAGE was carried out with minor modifications to the published method (Schägger, 1994; and Schägger, 1991). Thus, purified protein samples or cell culture supernatants were diluted with an equal volume of a buffer containing 100mM 4-(N-morpholino)propane sulfonic acid (MOPS), 100mM Tris-HCl, pH 7.7, 40% glycerol, 0.1% coomassie blue, just prior to loading onto a 4-12% Bis-Tris NuPAGE gel (Invitrogen). Typically, gel electrophoresis was performed for 2h at 150V (∼0.07A) using 50mM MOPS, 50mM Tris, pH 7.7, 0.002% coomassie blue as cathode buffer, and 50mM MOPS, 50mM Tris, pH 7.7 as anode buffer. When purified proteins were analyzed, the gel was destained with several changes of 50mM MOPS, 50mM Tris, pH 7.7 subsequent to the electrophoresis step. Typically, 5µg of purified protein were loaded per lane.

For Western blot analyses, gels and polyvinylidine difluoride (PVDF) membranes were soaked for 10 minutes in transfer buffer (192mM glycine, 25mM Tris, 0.05% SDS, pH 8.8 containing 20% methanol). Following transfer, PVDF membranes were destained of coomassie blue dye using 25% methanol and 10% acetic acid and air-dried. Destained membranes were probed using the anti-V3 loop MAb PA1 (Progenics) followed by horseradish peroxidase (HRP)-labeled anti-mouse IgG (Kirkegaard & Perry Laboratories, Gaithersburg, MD), each used at 0.2µg/mL final concentration. Luminometric detection of the envelope glycoproteins was obtained with the Renaissance7 Western Blot Chemiluminescence Reagent Plus system (Perkin Elmer Life Sciences, Boston, MA). Bovine serum albumin (BSA), apo-ferritin, and thyroglobulin were obtained from Amersham Biosciences (Piscataway, NJ) and used as molecular weight standards.

*Matrix-assisted laser desorption*/*ionization time-of-flight (MALDI-TOF) mass spectrometry.* Proteins were dialyzed overnight against water prior to analysis. Where indicated, SOS gp140 (1mg/ml) was reduced with 10mM DTT (Sigma), after which iodoacetamide (Sigma) was added to a final concentration of 100mM, before dialysis. The samples were mixed with an equal volume of sinapinic acid matrix solution, dried at room temperature, and analyzed by MALDI-TOF mass spectrometry (Lewis, 1998). MALDI-TOF mass spectra were acquired on a PerSeptive Biosystems Voyager-STR mass spectrometer with delayed extraction. Samples were irradiated with a nitrogen laser (Laser Science Inc.) operated at 337nm. Ions produced in the sample target were accelerated with a deflection voltage of 30,000V.

*Sedimentation equilibrium analysis.* Sedimentation equilibrium measurements were performed on a Beckman XL-A Optima analytical ultracentrifuge with an An-60 Ti rotor at 20°C. Protein samples were dialyzed overnight against 50mM sodium phosphate (pH 7.0) and 150mM NaCl, loaded at initial concentrations of 0.25mM, 0.5mM and 1mM, then centrifuged in a six-sector cell at rotor speeds of 6,000 and 9,000 rpm. Data were acquired at two wavelengths per rotor speed and processed simultaneously with a nonlinear least squares fitting routine (Johnson, 1981). Solvent density and protein partial specific volume were calculated according to solvent and protein composition, respectively (Laue, 1992).

*Size exclusion chromatography.* Purified, CHO cell-expressed SOS gp140, gp140UNC and gp120 proteins were analyzed by size exclusion chromatography on a TSK G3000SWXL HPLC column (TosoHaas, Montgomeryville, PA) using phosphate buffered saline (PBS) as the running buffer. The protein retention time was determined by monitoring the UV absorbance of the column effluent at a wavelength of 280 nm. The column was calibrated using ferritin as a model protein that exists in oligomeric states of 220 kDa, 440 kDa and 880 kDa (Gerl, 1988).

### Surface plasmon resonance measurements

*Immunoelectron microscopy.* Immunoelectron-microscopic analyses of SOS gp140 and gp120 alone and in complex with MAb, MAb fragments and sCD4 were performed by negative staining with uranyl formate as previously described (Roux, 1989; and Roux, 1996). The samples were examined on a JEOL JEM CX-100 electron microscope and photographed at 100,000 diameters magnification.

*Immune complex image digitalizing and averaging.* The electron micrographs of immune complex images were digitalized on an AGFA DUOSCAN T2500 Negative Scanner (Ridgefield Park, NJ). Potentially informative complexes were selected and windowed as 256 H 256 pixel images. These randomly oriented complexes were then brought into approximate alignment utilizing the multi-reference alignment function of the SPIDER program (Frank, 1996). The aligned images were subsequently averaged to improve the signal-to-noise ratio.

*Molecular modeling.* The SwissPDBviewer program (Guex, 1997) was used to enhance the EM-based interpretations and to investigate the likely location of the gp41 domain in SOS gp140.

### D. Results

### Assembly and cleavage of purified SOS gp140

We have previously described the antigenic properties of unpurified HIV-1_{JR-FL} SOS gp140 proteins produced via transient transfection of 293T cells (Binley, 2000a). To facilitate preparation of larger amounts of this protein for evaluation in purified form, we constructed a stable CHO cell line that expresses both SOS gp140 and human furin. Heterologous furin was expressed to facilitate efficient proteolytic processing of SOS gp140 (Binley, 1997b).

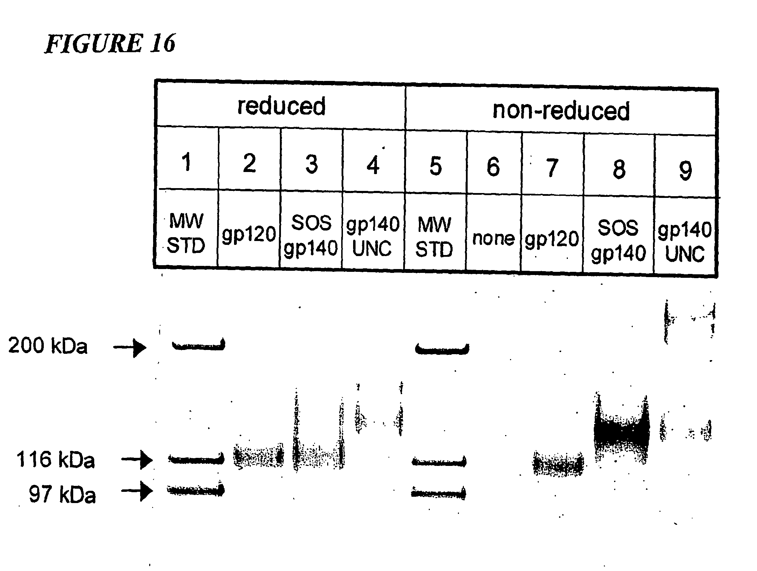

The SOS gp140 protein was purified from CHO cell supernatants to -90% homogeneity (Figure 16, Lane 8). Only minor amounts of free gp120 were present in the SOS gp140 preparation, indicating that the inter-subunit disulfide bond remained substantially intact during purification. No high molecular weight SOS gp140 oligomers or aggregates were observed (Figure 16, Lane 8). Under non-reducing conditions, SOS gp140 migrated as a predominant 140 kDa band. The major contaminant was bovine alpha 2-macroglobulin, which migrates as an ∼170kDa band on a reducing SDS-PAGE gel (Figure 16, Lane 3) and can be eliminated by adaptation of the CHO cell line to serum-free culture (data not shown). Upon reduction with DTT, the purified SOS gp140 protein migrated as a predominant 120kDa band, with a minor (-10%) fraction of the 140kDa band present (Figure 16, Lane 3). These data indicated that approximately 90% of the SOS gp140 protein was proteolytically processed.

The HIV-1_{JR-FL} gp140UNC protein was expressed in CHO cells using similar methods, although without co-transfected furin, and was also obtained at -90% purity. It too contained alpha 2-macroglobulin as the major contaminant, but no free gp120 was detectable (Figure 16, Lanes 4 and 9). In the absence of DTT, alpha 2-macroglobulin migrates as a -350kDa dimer and is not clearly resolved from gp140UNC oligomers (Figure 16, Lane 9). Under non-reducing conditions, bands consistent with gp140UNC monomers (140kDa), dimers (280kDa), and trimers (420kDa) were observed in roughly equal amounts (Figure 16, Lane 9). These proteins were reactive with anti-gp120 MAbs in Western blot analysis (data not shown). When treated with DTT, gp140UNC gave rise to an intensified monomer band at 140kDa and an alpha 2-macroglobulin monomer band at ∼170kDa; but gp140 oligomers were absent (Figure 16, compare Lanes 4 and 9). Thus, disulfide-linked, reducible oligomers comprise half or more of the gp140UNC preparation. Comparable amounts of reducible oligomers have been observed in gp140UNC protein preparations derived from subtype A, B and E viruses, with minor strain-to-strain differences (Owens, 1999; and Staropoli, 2000). Reducible gp160 oligomers of this type have been proposed to contain aberrant intermolecular disulfide bonds (Owens, 1999). If so, at least some of the oligomers present in gp140UNC preparations represent misfolded protein aggregates.

### Biophysical properties of purified SOS gp140

Matrix-assisted laser desorption ionization mass spectrometry. This technique was used to determine the absolute molecular masses of HIV-1_{JR-FL} gp120 and SOS gp140. As indicated in Table 1 (shown below), the measured molecular masses were 121.9kDa for SOS gp140 and 91.3kDa for gp120.

**Table 1.**

| Molecular masses of recombinant HIV-1_{JR-FL} envelope glycoproteins as determined by MALDI-TOF mass spectrometry | | |
|---|---|---|
| **HIV-1_{JR-FL} envelope glycoprotein** | | **mass, kDa** |
| gp120 | | 91.3 |
| SOS gp140 | | 121.9 |
| SOS gp140, reduced: | | |
| | *uncleaved gp140* | 118.5 |
| | *gp120* | 91.8 |
| | *gp41ECTO* | 27.0 |

Reduced SOS gp140 gave rise to a small peak of uncleaved gp140 at 118.5kDa, a gp120 peak at 91.8kDa and a gp41ECTO peak at 27kDa. Differences in glycosylation between cleaved and uncleaved SOS gp140 proteins could account for the 3.4kDa difference in their measured masses. A smaller difference (∼500Da) was observed in the mass of gp120 when it was expressed alone and in the context of SOS gp140. The alanine 6 cysteine SOS mutation would be expected to increase the mass of gp120 by only 32Da (one sulfur atom), so again a minor difference in glycosylation patterns may be responsible. The measured mass of HIV-1_{JR-FL} gp120 is comparable to previously reported molecular masses of CHO cell-expressed HIV-1_{GB8} gp120 (91.8kDa) and Drosophila cell-expressed HIV-1_{WD61} gp120 (99.6kDa) (Jones, 1995; and Myszka, 2000). The anomalously high molecular weights (∼120kDa and ∼140kDa, respectively, Figure 16) observed for gp120 and SOS gp140 by SDS-PAGE reflect the high carbohydrate content of these proteins. The extended structure of the glycans and their poor reactivity with the dodecyl sulfate anion retard the electrophoretic migration of the glycoproteins through SDS-PAGE gel matrices (Jones, 1995).

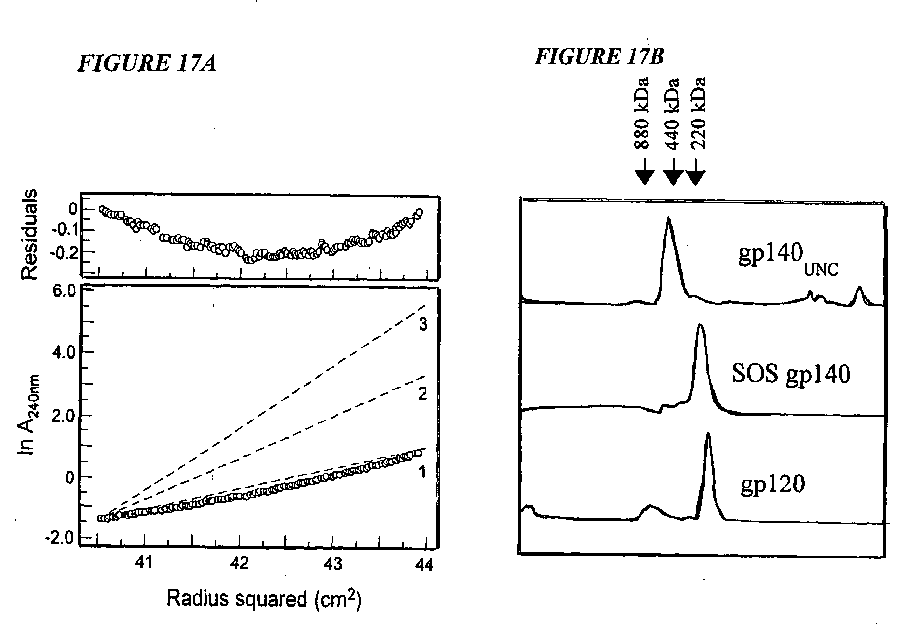

Ultracentrifugation sedimentation equilibrium measurements were used to examine the oligomeric state of purified SOS gp140. Over protein concentrations ranging from 0.25-1.0mM, the apparent molecular weight of SOS gp140 was consistently found to be 155kDa (Figure 17a). Hence, the purified SOS gp140 protein is monomeric in solution. There was no systematic dependence of molecular weight on protein concentration over the range studied. However, the residuals (the difference between the data and the theoretical curve for a monomer) deviated from zero in a systematic fashion (Figure 17a), suggesting the presence of small amounts of oligomeric material.

Analytical gel filtration chromatography Purified HIV-1_{JR-FL} SOS gp140, gp140UNC and gp120 proteins were also examined using size exclusion chromatography. Monomeric gp120 eluted with a retention time of 6.24 minutes and an apparent molecular weight of -200kDa (Figure 17b). The apparently large size of this protein reflects the extended structures of its carbohydrate moieties. The retention time (5.95 minutes) and apparent molecular weight (-220kDa) of the SOS gp140 protein are consistent with it being a monomer that is slightly larger than gp120. In contrast, the gp140UNC protein eluted at 4.91 minutes as a broad peak with an average molecular weight of >500kDa, which is consistent with it comprising a mixture of oligomeric species. Although the chromatogram suggests the existence of multiple species in the gp140UNC preparation, this gel-filtration technique cannot resolve mixtures of gp140 dimers, trimers and tetramers.

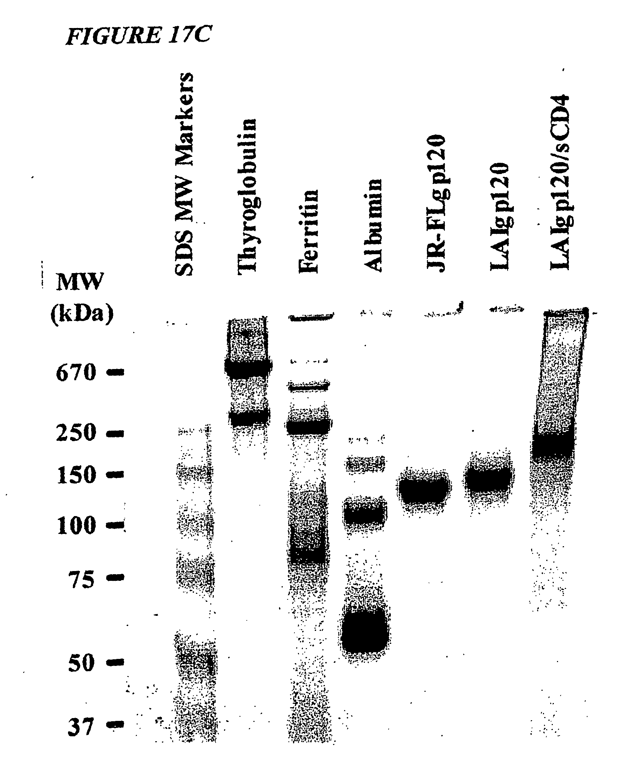

Blue Native polyacrylamide gel electrophoresis BN-PAGE was used to examine the oligomeric state of the purified SOS gp140 and gp140UNC proteins. In BN-PAGE, most proteins are fractionated according to their Stokes' radius. We first applied this technique to a model set of soluble proteins, including gp120 alone and in complex with sCD4 (Figure 17c). The model proteins included thyroglobulin and ferritin, which naturally comprise a distribution of non-covalent oligomers of varying size. The oligomeric states of these multi-subunit proteins, as determined by BN-PAGE, are similar to those observed using other non-denaturing techniques (Gerl, 1988; and Venkatesh, 1999). BSA exists as monomers, dimers, and higher order species in solution (Lambin, 1982); the same ladder of oligomers was observed in BN-PAGE. Not surprisingly, the gp120/sCD4 complex, which has an association constant in the nanomolar range (Allaway, 1995), remained intact during BN-PAGE analysis.

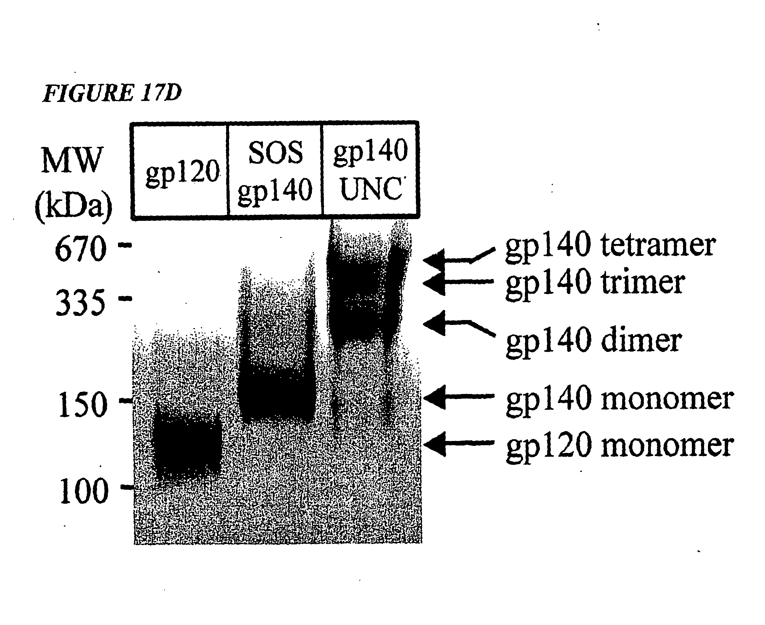

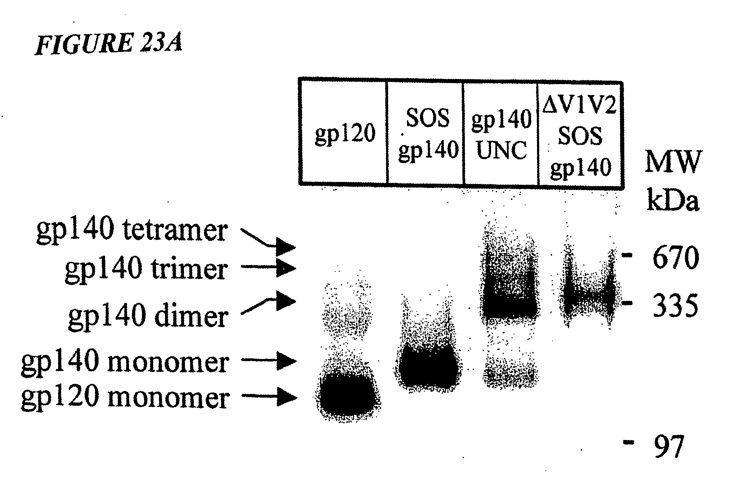

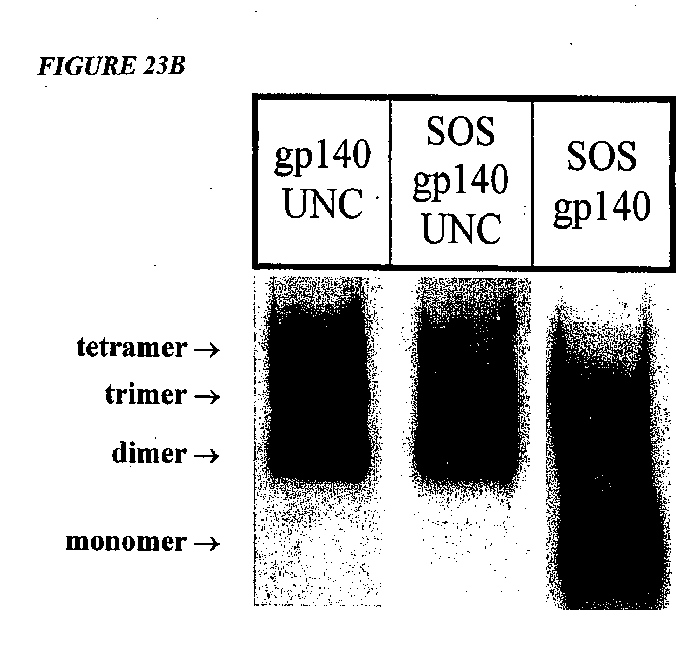

The purified SOS gp140 protein was largely monomeric by BN-PAGE (Figure 17d), although a minor amount (<10%) of dimeric species was also observed. The purified gp140UNC protein migrated as well-resolved dimers, trimers and tetramers, with trace amounts of monomer present (Figure 17d). The gp140UNC dimer represented the major oligomeric form of the protein present under non-denaturing conditions. Although tetrameric gp140UNC is a distinct minor species on BN-PAGE gels (Figure 17d), it is absent from non-reduced SDS-PAGE gels (Figure 16). Upon treatment with SDS and heat, the gp140UNC tetramers probably revert to lower molecular weight species, such as monomers and/or disulfide-linked dimers. As expected, HIV-1_{FR-FL} gp120 migrated as a predominant 120 kDa monomeric protein. BN-PAGE analyses of unpurified gp140 proteins are described below (see Figure 23).

Overall, ultracentrifugation, gel filtration and BN-PAGE analyses were in excellent agreement as to the oligomeric states of these purified Env proteins. BN-PAGE, however, was the only method capable of clearly resolving the mixture of oligomeric species contained in the gp140UNC preparation.

### Immunoelectron microscopy of SOS gp140 and SOS gp140-MAb complexes

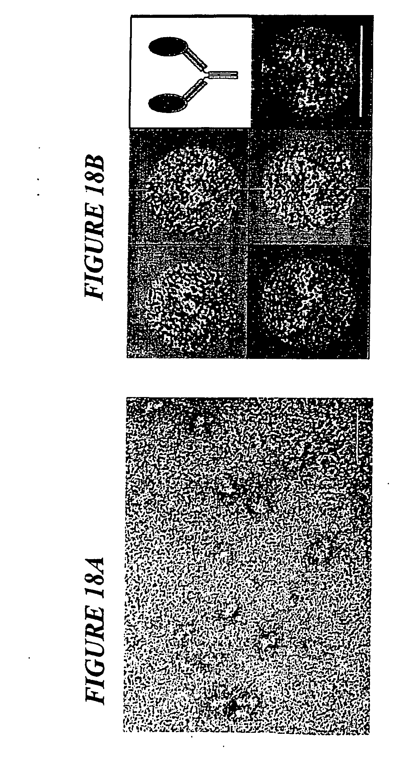

In the absence of antibodies, the electron micrographs revealed SOS gp140 to be mostly monomeric, randomly oriented and multi-lobed (Figure 18a). Qualitatively similar images were obtained with HIV-1_{JR-FL} gp120 (data not shown), and the two proteins could not be clearly distinguished in the absence of MAbs or other means of orienting the images.

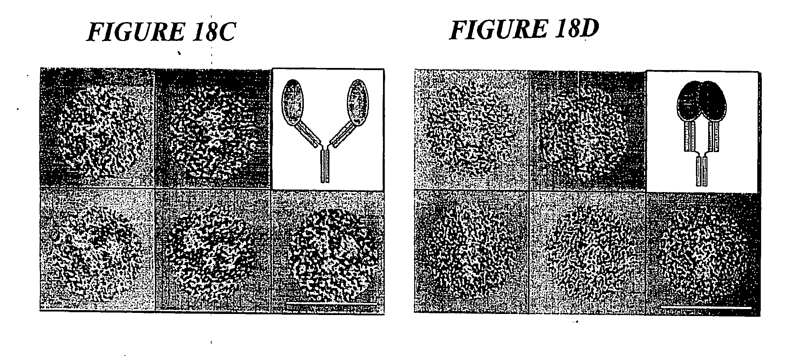

Electron micrographs were also obtained of SOS gp140 in complex with MAbs 2F5 (Figure 18b), IgG1b12 (Figure 18c) and 2G12 (Figure 18d). To aid in interpretation, the complexes were masked and rotated such that the presumptive Fc of the MAb points downward. Schematic diagrams are also provided for each complex in order to illustrate the basic geometry and stoichiometry observed. In each case, the complexes shown represent the majority or plurality species present. However, other species, such as free MAb and monovalent MAb-SOS gp140 complexes, were also present in each sample (data not shown).

When combined with IgG1b12 or 2F5, SOS gp140 formed rather typical immune complexes composed of a single MAb and up to two SOS gp140s (Figure 18b and 18c). The complexes adopted the characteristic Y-shaped antibody structure, with a variable angle between the Fab arms of the MAb. In contrast, the 2G12/SOS gp140 complexes produced strikingly different images (Figure 18d). Y-shaped complexes comprising two distinct Fab arms with bound SOS gp140s were rare. Instead the 2G12-SOS gp140 images were strongly linear and appeared to represent one MAb bound to two SOS gp140 proteins aligned in parallel. The parallel alignment of the SOS gp140s forces the two Fab arms into similar alignment, resulting in an overall linear structure. These complexes are unprecedented in our immunoelectron microscopy studies of Env-MAb complexes (Roux, 1989; Roux, 1996; and Zhu, 2001) and KHR, unpublished observations). Of note is that the HIV-1_{JR-FL} gp120-2G12 complexes do not adopt this parallel configuration but instead resemble the SOS gp140-2F5 and SOS gp140-IgG1b12 complexes (data not shown). One hypothesis is that 2G12 binds to SOS gp140 in an orientation that promotes residual weak interactions between the gp41ECTO moieties, which then stabilize the complex in the parallel configuration observed. Additional studies are ongoing to further explore this finding.

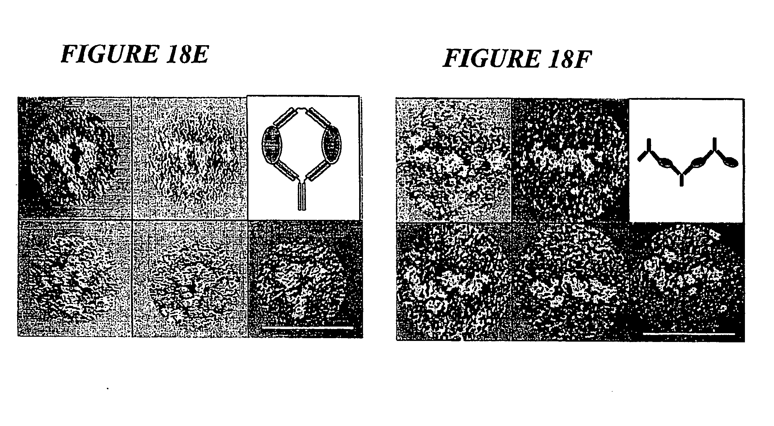

Combinations of the above, well-characterized MAbs were used to examine the relative placement of their epitopes on SOS gp140. In the first combination, SOS gp140-2F5-IgG1b12, multiple ring structures were observed which appeared to be composed of two SOS gp140 proteins bridged by two antibody molecules (data not shown). To distinguish between the 2F5 and IgG1b12 MAbs, we examined complexes formed between IgG1b12 F(ab')2, SOS gp140 and the intact 2F5 MAb. Characteristic ring structures were again observed (Figure 18e). The ring complexes were then subjected to computational analysis using the SPIDER program package to yield several categories of averaged images (data not shown). The MAb 2F5 and IgG1b12 F(ab')2 components can clearly be delineated in the images, as can the SOS gp140 molecule. When bound to a given SOS gp140 molecule, the Fab arms of 2F5 and IgG1b12 lie at approximately right angles, as indicated in the schematic diagram (Figure 18e).

In marked contrast to the IgG1b12-containing ternary complexes, those composed of SOS, 2F5 and 2G12 formed extended chains rather than closed rings (Figure 18f). These observations place the 2F5 and 2G12 epitopes at opposite ends of the SOS gp140 molecule. There was significant heterogeneity in the stoichiometry of the 2F5/2G12/SOS gp140 complexes, just one example of which is indicated in the schematic diagram.

### Immunoelectron microscopy of SOS gp140 and gp120 in complex with sCD4 and MAb 17b.

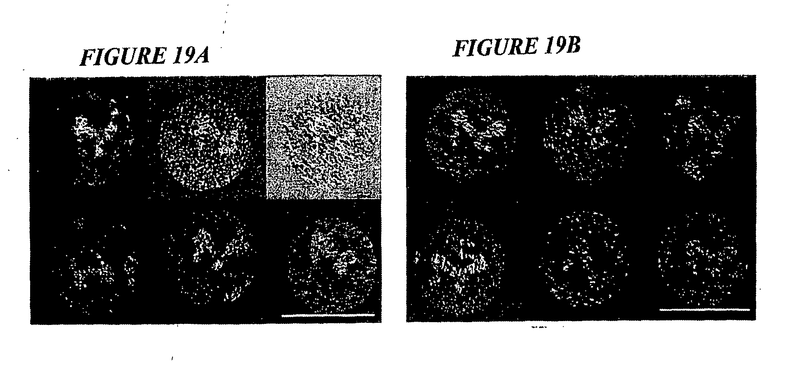

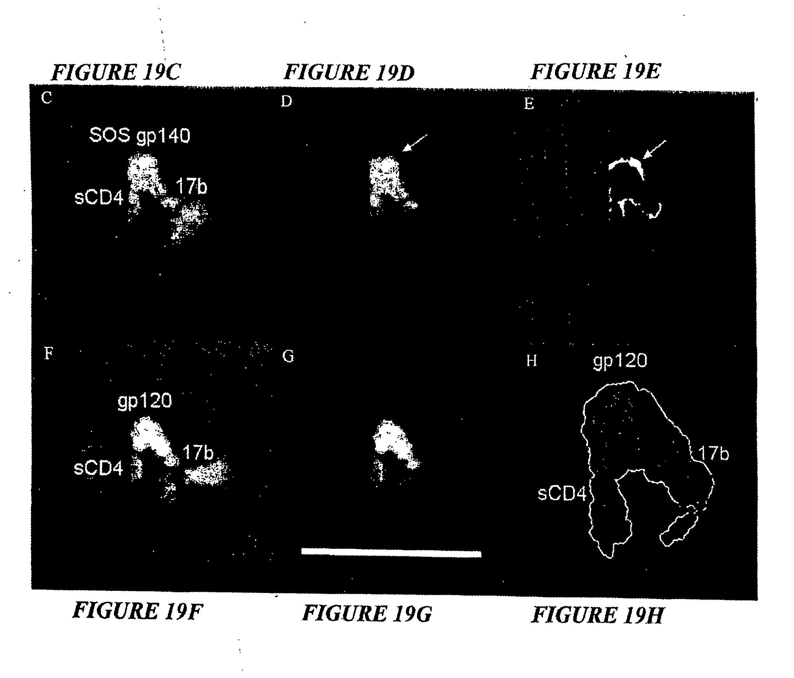

In an effort to further characterize the topology of SOS gp140, we reacted it with MAb 17b and/or sCD4 (Figure 19). We generated the corresponding YU2 gp120 complexes for comparison. As expected, the combination of MAb 17b plus SOS gp140 or gp120 alone did not form complexes, consistent with the need for sCD4 to induce the 17b epitope. Similarly, unremarkable complexes were obtained when sCD4 was mixed with SOS gp140 or gp120 in the absence of MAb 17b (data not shown). However, complexes with clearly defined geometry were obtained for sCD4/Env/17b (Figure 19a and 19b).

These complexes were composed of 17b with one or two attached SOS gp140s or gp120s, together with tangentially protruding sCD4 molecules. These complexes were then subjected to computer-assisted averaging (Figure 19c and 19f). The free arm and the Fc region of MAb 17b were disordered in these images due to the flexibility of the MAb, so the averaged images were masked to highlight the better-resolved sCD4, Env and 17b Fab structures (Figure 19d and 19g). The gp120 and SOS gp140 images were qualitatively similar, but an image subtraction of one from the other revealed the presence of additional mass on the SOS gp140 protein (arrowed in Figure 19d and 19e). This additional mass may represent gp41ECTO, although we cannot strictly exclude other explanations, such as differences in the primary sequence and/or glycosylation of the gp120 and SOS gp140 proteins used.

In order to orient the putative gp41ECTO moiety in relation to the remaining structures seen in the electron micrographs, the X-ray structure of the gp120 core in complex with the D1D2 domain of sCD4 and Fab 17b (Kwong, 1998) was docked, using Program O, into the profile map obtained for the sCD4/gp120/MAb 17b complex (Figure 19h). Given that there are differences in the gp120 (whole vs. core) and CD4 (four domain vs. two domain) molecules used for the electron microscopy and crystallization studies, there is reasonable agreement in the overall topology of the structures generated.

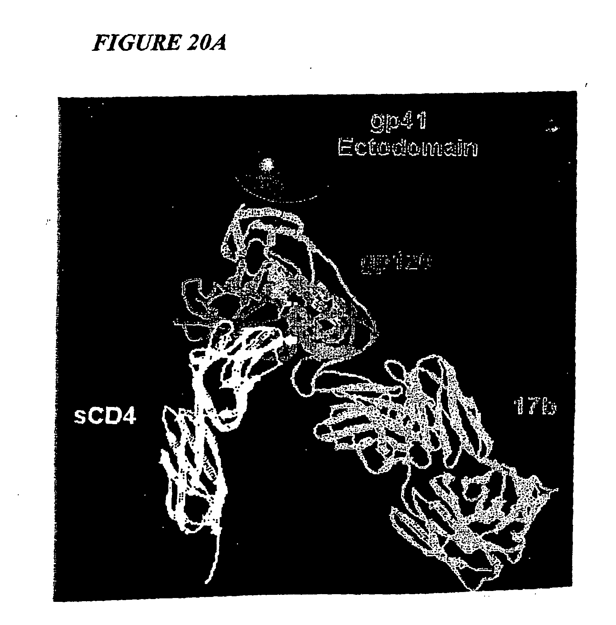

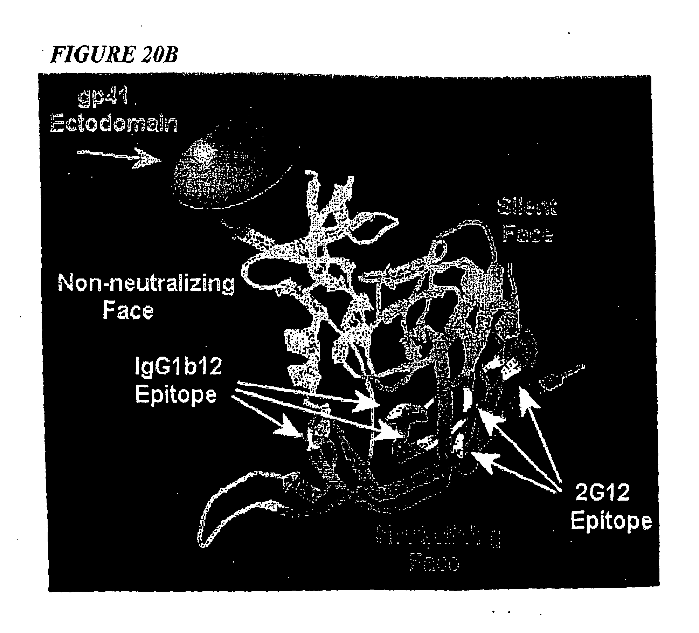

This agreement in structures (Figure 19h) enabled us to position the putative gp41ECTO moiety in relation to the core gp120 structure (Figure 20). The previously defined neutralizing, non-neutralizing, and silent faces of gp120 (Moore, 1996; and Wyatt, 1998a) are illustrated, as are the IgG1b12 (Saphire, 2001) and 2G12 (Wyatt, 1998a) epitopes. According to this model, the gp41ECTO moiety recognized by MAb 2F5 is located at ∼90B relative to the IgG1b12 epitope and -180B from the 2G12 epitope (Figure 20b). This model is in broad agreement with the independently derived electron microscopy images of the complexes formed between SOS gp140 and combinations of these MAbs (Figure 18e and 18f). This putative placement of gp41ECTO would cause it to largely occlude the non-neutralizing face of gp120, a result that is consistent with the MAb reactivity patterns observed for SOS gp140 both here and elsewhere (Binley, 2000a).

### Antigenic properties of unpurified SOS gp140 and gp140UNC proteins

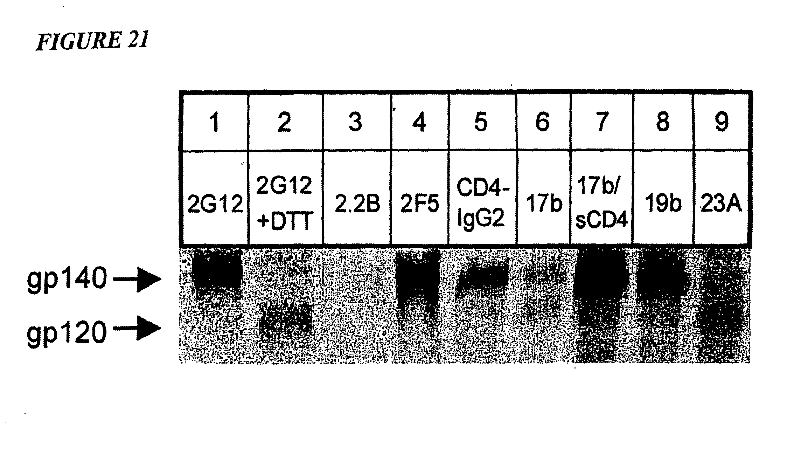

Radioimmunoprecipitation assays (RIPA) was used to determine whether the antigenicity of HIV-I_{JR-FL} SOS gp140 differed when the protein was expressed in stably transfected CHO cells, compared to what was observed previously when the same protein was expressed in transiently transfected 293T cells (Binley, 2000a). The SOS gp140 proteins in unpurified supernatants expressed from CHO cells were efficiently recognized by neutralizing agents to gp120 epitopes located in the C3/V4 region (MAb 2G12), the CD4 binding site (the CD4-IgG2 molecule), and the V3 loop (MAb 19b) (Figure 21). In addition, the conserved CD4-induced neutralization epitope defined by MAb 17b was strongly induced on SOS gp140 by sCD4. SOS gp140 was also efficiently immunoprecipitated by the broadly neutralizing gp41 MAb 2F5. In contrast, SOS gp140 was largely unreactive with the non-neutralizing MAbs 23A and 2.2B to gp120 and gp41, respectively (Figure 21, Lanes 3 and 9). A comparison of these analyses with our previous observations (Binley, 2000a) indicates that CHO and 293T cell-derived HIV-1_{JR-FL} SOS gp140 proteins possess similar antigenic properties.

Relatively minor amounts of free gp120 were observed in the unpurified SOS gp140 CHO cell supernatants (Figure 21, Lanes 1, 5, 7, and 8). This free gp120 was preferentially recognized by MAb 23A, suggesting that its C5 epitope is largely obscured in SOS gp140 (Figure 21, Lane 9). This is consistent with the electron microscopy-derived topology model described above (Figure 20b), and with what is known about the gp120-gp41 interface (Helseth, 1991; Moore, 1996; and Wyatt, 1997). Processing of SOS gp140 at the gp120-gp41 cleavage site was efficient, as determined by RIPAs performed under reducing and non-reducing conditions (Figure 21, compare Lanes 1 and 2). Similar levels of assembly and proteolytic processing were observed when unpurified SOS gp140 was analyzed by Western blotting rather than RIPA (data not shown). These findings also are comparable to those seen with 293T cell-derived HIV-1_{JR-FL} SOS gp140 (Binley, 2000a). Thus the folding, assembly, and processing of this protein appear to be largely independent of the cell line used for its production.

Surface plasmon resonance assays SPR was used to further characterize the antibody and receptor-binding properties of unpurified, CHO cell-expressed SOS gp140 and gp140UNC proteins. A comparison of results obtained using SPR and RIPA with the same MAbs allows us to determine if the antigenicity of these proteins is method-dependent. Whereas SPR is a kinetically-limited procedure that is completed in one or more minutes, RIPA is an equilibrium method in which Env-MAb binding occurs over several hours. SPR analysis was also performed on purified and unpurified forms of the SOS gp140 and gp140UNC proteins, to assess whether protein antigenicity was significantly altered during purification. Purified HIV-1_{JR-FL} gp120 was also studied. Although the purified SOS gp140 protein is a monomer, it does contain the gp120 subunit linked to the ectodomain of gp41. Since there is evidence that the presence of gp41 can affect the antigenic structure of gp120 (Klasse, 1993; and Reitz, 1988), we thought it worth determining whether monomeric SOS gp140 behaved differently than monomeric gp120 in its interactions with neutralizing and non-neutralizing MAbs.

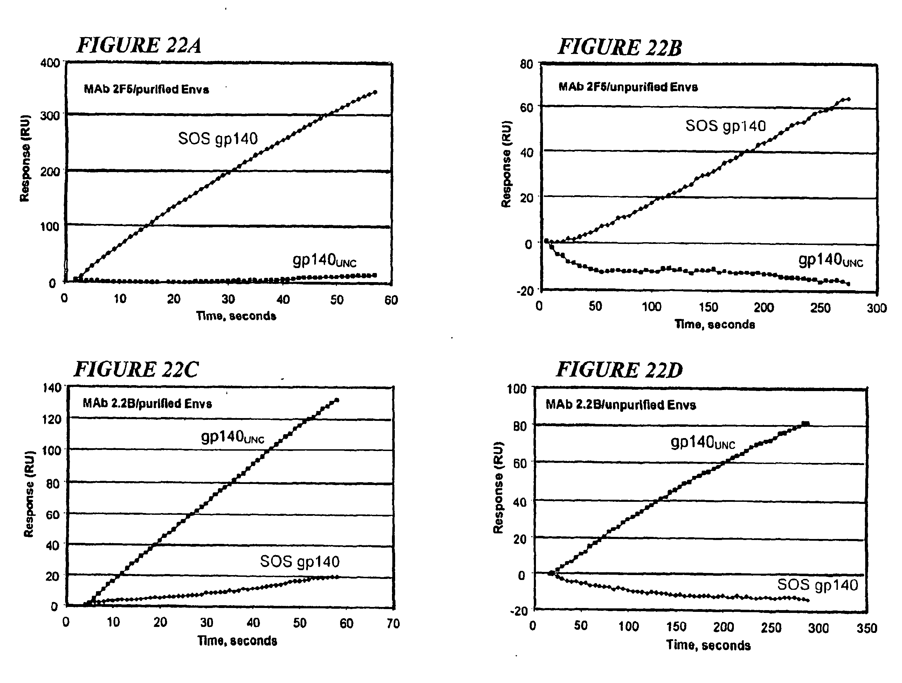

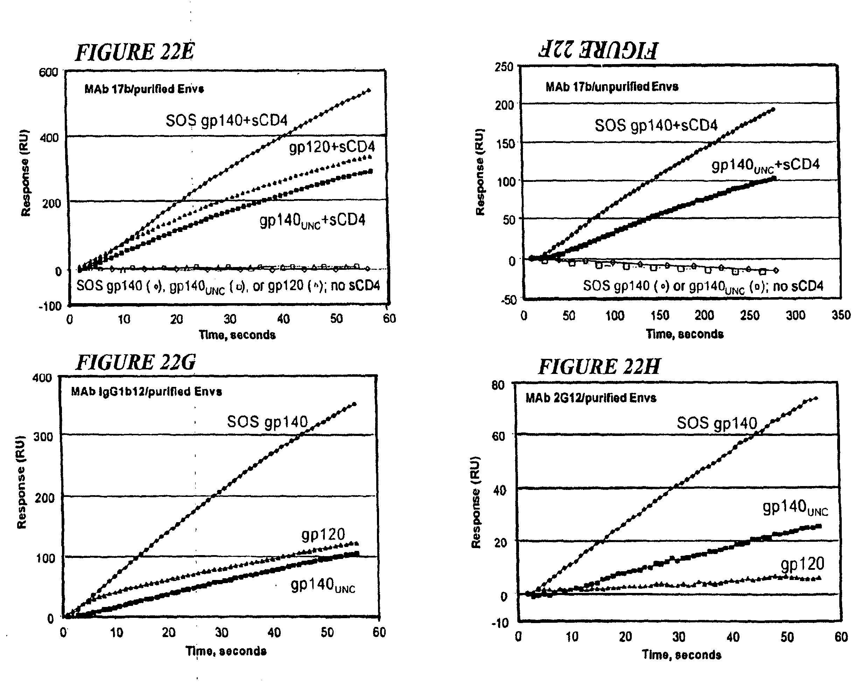

There was good concordance of results between RIPA-(Figure 21) and SPR-based (Figure 22) antigenicity analyses of unpurified SOS gp140 in CHO cell supernatants. For example, SOS gp140 bound the broadly neutralizing anti-gp41 MAb 2F5 (Figure 21, Lane 4 and Figure 22b) but not the non-neutralizing anti-gp41 MAb 2.2B (Figure 21, Lane 3 and Figure 22d). Similarly, binding of MAb 17b was strongly potentiated by sCD4 (Figure 21, Lanes 6-7 and Figure 22f). Unpurified SOS gp140 bound the neutralizing anti-gp120 MAbs 2G12 and 19b, but not the non-neutralizing anti-gp120 MAb 23A in both SPR (data not shown) and RIPA (Figure 21, Lanes 1, 8, and 9) experiments. Taken together, the RIPA and SPR data indicate that unpurified, CHO cell-derived SOS gp140 rapidly and avidly binds neutralizing anti-gp120 and anti-gp41 MAbs, whereas binding to the present set of non-neutralizing MAbs is not measurable by either technique.

SPR revealed some significant differences in the reactivities of SOS gp140 and gp140UNC proteins with anti-gp41 MAbs. Thus, SOS gp140 but not gp140UNC bound MAb 2F5 but not MAb 2.2B, whereas the converse was true for gp140UNC. Notable, albeit less dramatic, differences were observed in the reactivity of SOS gp140 and gp140UNC with some anti-gp120 MAbs. Of the two proteins, SOS gp140 had the greater kinetics and magnitude of binding to the neutralizing MAbs IgG1b12 (Figure 22g), 2G12 (Figure 22h) and 22b in the presence of sCD4 (Figure 22e, and 22f). The binding of gp140UNC to 17b was clearly potentiated by sCD4, as has been reported elsewhere (Zhang, 2001). Neither SOS gp140 nor gp140UNC bound the anti-gp120 MAb 23A (data not shown). This was expected for gp140UNC since the C5 amino acid substitutions that eliminate the cleavage site directly affect the epitope for MAb 23A (Moore, 1994b).

Qualitatively, the antigenicities of SOS gp140 and gp140UNC were little changed upon purification (Figure 22, compare Panels a, c and e with Panels b, d and f). Hence the lectin affinity and gel filtration columns used for purification do not appear to significantly affect, or select for, a particular conformational state of these proteins. However, these studies do not allow for direct, quantitative comparisons of SPR data derived using purified and unpurified materials.

Compared with monomeric gp120, the purified gp140UNC protein reacted more strongly with MAb 2G12 but less strongly with MAb IgG1b12. Prior SPR studies have demonstrated that 2G12 avidly binds to oligomeric forms of Env, and it is possible that MAb 2G12 is capable of undergoing bivalent binding to oligomeric Envs. It will be informative to perform electron microscopy analyses of 2G12 in complex with gp140UNC or other oligomeric Env in future studies, given the unusual nature of the 2G12-SOS gp140 complex (Figure 18d).

### Oligomeric properties of unpurified SOS gp140 and gp140UNC proteins

BN-PAGE was used to examine the oligomeric state of the SOS gp140 and gp140UNC proteins present in freshly prepared, CHO cell culture supernatants. The SOS gp140 protein was largely monomeric by BN-PAGE, with only a minor proportion of higher order proteins present (Figure 23a). In some, but not all, 293T cell preparations, greater but highly variable amounts of dimers and higher-order oligomers were observed using BN-PAGE (data not shown, but see Figure 23b below). This probably accounts for our previous report that oligomers can be observed in unpurified SOS gp140 preparations using other techniques (Binley, 2000a).

The unpurified gp140UNC protein typically migrated as well-resolved dimers, trimers and tetramers, with trace amounts of monomer sometimes present (Figure 23a). Qualitatively similar banding patterns were observed for purified (Figure 17d) and unpurified gp140UNC proteins (Figure 23a). In each case, dimers of gp140UNC were the most abundant oligomeric species. HIV-1_{JR-FL} gp120 ran as a predominant 120 kDa monomeric band, although small amounts of gp120 dimers were observed in some unpurified supernatants. In general, the BN-PAGE analyses indicate that the oligomeric properties of the various Env proteins did not change appreciably upon purification (compare Figure 23a and Figure 17d).

The same CHO cell supernatants were also analyzed by analytical gel filtration, the column fractions being collected in 0.2mL increments and analyzed for Env content by Western blotting. The retention times of unpurified gp120, SOS gp140 and gp140UNC proteins were determined to be -6.1, -5.9 and -5.2 minutes, respectively (data not shown). These values agree with those observed for the purified proteins (Figure 17b) to within the precision of the method. The gel filtration studies thus corroborate the BN-PAGE data in that unpurified gp120 and SOS gp140 were mostly monomeric, while gp140UNC was mostly oligomeric (data not shown). However, unlike BN-PAGE, this analytical gel filtration procedure does not have sufficient resolving power to characterize the distribution of the oligomeric species present in the gp140UNC preparation.

SDS-PAGE followed by Western blot analyses of supernatants containing unpurified SOS gp140 and gp140UNC proteins yielded banding patterns similar to those shown in Figure 16 for the purified proteins (data not shown). The gp120 preparation contained -10% dimer, which was observed only when SDS-PAGE analyses were carried out under non-reducing conditions. Thus the gp120 dimer represents disulfide-linked and presumably misfolded material (Owens, 1999).

### Variable loop-deleted SOS gp140 glycoproteins form more stable oligomers

We previously described HIV-1_{JR-FL} SOS gp140 glycoproteins from which one or more of the gp120 variable loops were deleted to better expose underlying, conserved regions around the CD4- and coreceptor-binding sites. It was possible to remove the V1, V2 and V3 loop structures individually or in pairs without adversely affecting the formation of the intersubunit disulfide bond, proper proteolytic cleavage, or protein folding. However, the triple loop-deletant was not efficiently cleaved (Sanders, 2000). In order to explore the oligomeric properties of these modified SOS gp140 glycoproteins, the supernatants of 293T cells transiently co-transfected with these gp140 constructs and furin were analyzed by BN-PAGE. Unexpectedly, deletion of the variable loops, both alone and in combination, significantly enhanced the stability of the SOS gp140 oligomers. The ΔV1V2 SOS gp140 preparation contained almost exclusively trimeric and tetrameric species, whereas ΔV1 SOS gp140 formed a mixture of dimers, trimers and tetramers similar to that seen with gp140UNC (data not shown). The ΔV2 SOS gp140 protein was predominantly oligomeric, but it also contained significant quantities of monomer. Thus, in terms of oligomeric stability, the SOS proteins can be ranked as follows: ΔV1V2 SOS gp140 > ΔV1 SOS gp140 > ΔV2 SOS gp140 > full-length SOS gp140. The reasons for this rank order are not yet clear, but are under investigation.

Based on the above observations, we chose to generate a CHO cell line that stably expresses the ΔV1V2 SOS gp140 protein. Supernatants from the optimized CHO cell line were first analyzed by SDS-PAGE under reducing and non-reducing conditions, followed by Western blot detection. The major Env band was seen at 120kDa (ΔV1V2 gp140 protein) in the non-reduced gel and at 100kDa (ΔV1V2 gp120 protein) in the reduced gel (data not shown). These results are consistent with our prior findings that deletion of the V1V2 loops decreases the apparent molecular weight of the protein by -20kDa. Notably, the ΔV1V2 SOS gp140 protein was largely free both of disulfide-linked aggregates and of the ∼100kDa loop-deleted, free gp120 protein. Thus proteolytic cleavage and SOS disulfide bond formation occur efficiently in the ΔV1V2 SOS gp140 protein (data not shown).

CHO cell supernatants containing ΔV1V2 SOS gp140, full-length SOS gp140 and gp140UNC were also analyzed by BN-PAGE and Western blotting (Figure 23a). As was observed with the transiently transfected 293T cells, unpurified CHO cell-derived material was oligomeric. The CHO cell-derived ΔV1V2 SOS gp140 migrated as a distinct single band with a molecular weight consistent with that of a trimer (360kDa); the ΔV1V2 SOS gp140 band lies between those of gp140UNC dimer (280kDa) and gp140UNC trimer (420kDa) (Figure 23a). Hence the ΔV1V2 SOS gp140 protein represents a proteolytically mature form of HIV-1 Env that oligomerizes into presumptive trimers via non-covalent interactions. Purification and additional biophysical studies of this protein are now in progress, and immunogenicity studies are planned.

### The uncleaved SOS gp140 and gp140UNC proteins possess similar oligomeric properties

Overall, the above analyses reveal a clear difference in the oligomeric properties of the SOS gp140 and gp140UNC proteins. One structural difference between these proteins is their proteolytic cleavage status, another is the presence or absence of the intersubunit disulfide bond that defines SOS gp140 proteins. To address the question of whether it is gp120-gp41 cleavage or the introduced cysteine residues that destabilize the SOS gp140 oligomers, we made the SOS gp140UNC protein. Here, the cysteines capable of intersubunit disulfide bond formation are present, but the cleavage site between gp120 and gp41ECTO has also been modified to prevent cleavage. The SOS gp140UNC, SOS gp140 and gp140UNC proteins were all expressed transiently in 293T cells and analyzed by BN-PAGE (Figure 23b). In this and multiple repeat experiments, SOS gp140UNC and gp140UNC had similar migration patterns on the native gel, with the dimer band predominating and some monomers, trimers and tetramers also present. In contrast, SOS gp140 was primarily monomeric, although small amounts of dimeric and trimeric species were also observed in this particular analysis (Figure 23b).

The above results suggest that the SOS gp140UNC protein behaves more like the gp140UNC protein than the SOS gp140 protein. This, in turn, implies that the cleavage of gp140 into gp120 and gp41ECTO has a substantial effect on how gp140 is oligomerized via interactions between the gp41ECTO moieties, whereas the presence of the cysteine substitutions in gp120 and gp41 has little effect on these interactions. We believe that this observation is central to understanding the relative instability of SOS gp140 oligomers, compared to those of the gp140UNC protein. We note, however, that we have not determined whether or not the intermolecular disulfide bond actually forms in SOS gp140UNC; the simple method of DTT treatment to reduce this bond is inadequate, because the uncleaved peptide bond between the gp120 and gp41ECTO moieties still holds the two subunits together. To address this issue will require characterizing purified SOS gp140UNC by methods such as peptide mapping. Such studies are now in progress, to further explore the effect of gp140 cleavage on the structure of the gp120-gp41ECTO complex.

### E. Discussion

We have previously described the antigenic properties of SOS gp140, an HIV-1 envelope glycoprotein variant in which an intermolecular disulfide bond has been introduced to covalently link the gp120 and gp41ECTO subunits (Binley, 2000a; and Sanders, 2000). In the original report, we demonstrated that the SOS gp140 protein, as contained in supernatants of transiently transfected 293T cells, was an antigenic mimic of virion-associated Env (Binley, 2000a). In that report, the methods employed were not sufficiently robust to conclusively determine the oligomeric state of unpurified 293T-derived SOS gp140 (Binley, 2000a). Here we show that purified and unpurified CHO cell-derived SOS gp140 proteins also mimic native Env in terms of their patterns of antibody reactivity. However, unlike virus-associated Env, SOS gp140 is a monomeric protein.

Antigenicity and immunoelectron microscopy studies support a model for SOS gp140 in which the neutralizing face of gp120 is presented in a native conformation, but the non-neutralizing face is occluded by gp41ECTO. The immunoelectron microscopy data suggest a model in which the gp41ECTO moiety of SOS gp140 occludes the non-neutralizing face of the gp120 subunit (Figure 20). The evidence for this model is derived from several independent studies. In the first of these, SOS gp140 was examined in complex with combinations of anti-gp120 and anti-gp41 MAbs to defined epitopes (Figure 18). The gp41ECTO subunit, as defined by the position of the anti-gp41 MAb 2F5, was located -180B from the MAb 2G12 epitope and ∼90B from the MAb IgG1b12 epitope, as is the non-neutralizing face. A second set of studies compared SOS gp140 and gp120 in complex with sCD4 and MAb 17b (Figure 19). Here, a region of additional mass in the gp140 complex defined the presumptive gp41ECTO; its location was similarly adjacent to the non-neutralizing face of gp120. This model of the geometry of the gp120-gp41 interaction is consistent with previous models based on mutagenesis techniques and the mapping of MAb epitopes (Helseth, 1991); Moore, 1996; and Wyatt, 1997). It also provides a basis for interpreting the patterns of MAb reactivity described above and discussed below.

The antigenicity of CHO-derived SOS gp140 was explored from a number of perspectives: (1) in comparison with gp140UNC and gp120; (2) before and after purification; (3) in an equilibrium-based assay (RIPA) vs. a kinetics-based assay (SPR). SOS gp140 proteins expressed in stably transfected CHO cells or transiently transfected 293T cells possessed qualitatively similar antigenic properties that were largely unaffected by purification. We observed that most neutralizing anti-gp120 MAbs bound more strongly and more rapidly to SOS gp140 than to the gp120 or gp140UNC proteins, whereas the converse was true of non-neutralizing MAbs (Figures 21 and 22). These results were largely independent of the analytical methodology used (RIPA or SPR), or the purification state of the glycoproteins, and thus extend our earlier studies on the antigenicity of unpurified Env glycoproteins determined by RIPA (Binley, 2000a). We have addressed these issues on a largely qualitative basis in the present study; quantitative comparisons of MAb reactivities are now being explored.

It is not obvious why neutralizing MAbs recognize monomeric SOS gp140 better than monomeric gp120. One possibility relates to differences in the conformational freedom of the two glycoproteins. Monomeric gp120 has considerable conformational flexibility, such that Afreezing@ of the conformation by CD4 binding results in an unexpectedly large loss in entropy (Myszka, 2000). Indeed, it has been suggested that reducing the conformational freedom of a gp120 immunogen may provide a means of generating broadly neutralizing antibodies, which generally recognize conformational epitopes (Myszka, 2000). The presence of gp41ECTO may serve to minimize the conformational flexibility of the gp120 subunit of SOS gp140, stabilizing the protein in conformations recognized by neutralizing antibodies. However, the induction of 17b binding by sCD4 demonstrates that SOS gp140 is still capable of sampling multiple, relevant conformations. Studies are in progress to address these issues.

Variations in conformational flexibility may also underlie the antigenic differences observed between the SOS gp140 and gp140UNC proteins. Other possible explanations include the effect that cleavage may have on the overall structure of Env, and differences in the oligomerization state of the two proteins. Further studies using additional Env protein variants (e.g., SOS gp140UNC), a broader range of anti-Env MAbs, and purified or size-fractionated proteins of a homogenous subunit composition, will be required to explore these issues more thoroughly.

Standard biophysical techniques were used to demonstrate that the purified HIV-1_{JR-FL} SOS gp140 glycoprotein is a monomer comprising one gp120 subunit disulfide-linked to gp41ECTO. Since it is generally accepted that the gp41 subunits are responsible for Env trimerization (Caffrey, 1998; Chan, 1997; Lu, 1995; Tan, 1997; and Weissenhorn, 1997), we assume that the gp41-gp41 interactions within the cleaved SOS gp140 glycoprotein are weak, and that this instability precludes the purification of cleaved trimers.

We also report the application of a rapid, simple and high-resolution electrophoretic technique, BN-PAGE, for exploring the oligomeric state of HIV-1 envelope glycoproteins in unpurified as well as purified form. In this technique, the proteins of interest are combined with the dye coomassie blue, which binds to the exposed hydrophobic surfaces of proteins and usually enhances their solubility. In the presence of the dye, most proteins adopt a negative charge, migrate towards the anode in an electric field, and so can be sieved according to their Stokes= radius in a polyacrylamide gradient gel. Whereas traditional native PAGE methods are typically performed under alkaline conditions (pH 9.5), BN-PAGE uses a physiological pH (pH 7.5), which is more compatible with protein stability. We demonstrate that a gp120/sCD4 complex and a variety of purified, oligomeric model proteins all remain associated during BN-PAGE analysis. When combined with Western blot detection, BN-PAGE can be used to determine the oligomeric state of HIV-1 envelope glycoproteins at all stages of purification. This high resolution technique can resolve monomeric, dimeric, trimeric and tetrameric forms of gp140.

As determined by BN-PAGE and other methods, the SOS gp140 protein was secreted in mostly monomeric form. In contrast, gp140UNC proteins, in which the peptide bond between gp120 and gp41 still attaches the two subunits, form oligomers that are significantly more stable. Thus, we show that HIV-1_{JR-FL} gp140UNC comprises a mixture of dimers, trimers and tetramers, with dimers representing the major oligomeric form present under non-denaturing conditions. Although non-covalently associated oligomers constitute a significant percentage of the gp140UNC preparation, half or more of the material consists of disulfide-linked and presumably misfolded material (Owens, 1999). Others have made similar observations with uncleaved gp140 proteins from other HIV-1 strains, and from SIV (Chen, 2000; Earl, 1997; Earl, 1994; Earl, 1990; Earl, 2001; Edinger, 2000; Farzan, 1998; Hoffman, 2000; Owens, 1999; Richardson, 1996; Stamatatos, 2000; Staropoli, 2000; Yang, 2000a; Yang, 2000b); and Yang, 2001). The question then arises as to why the SOS gp140 protein is a monomer, but the uncleaved proteins are oligomeric. We believe that the cleavage of the gp120-gp41 peptide bond alters the overall conformation of the envelope glycoprotein complex, rendering it fusion-competent but also destabilizing the association between the gp41 subunits. Support for this argument is provided by the evidence that the SOS gp140UNC protein behaves identically to the gp140UNC protein, but very differently from the SOS gp140 protein; cleavage is clearly more important than the engineered, intermolecular disulfide bind in determining the oligomeric stability of gp140 proteins. Destabilization of gp41-gp41 interactions might be necessary for gp41-mediated fusion to occur efficiently upon activation of the Env complex by gp120-receptor interactions. Moreover, having cleavage/activation take place late in the synthetic process minimizes the risk of fusion events occurring prematurely, i.e. during intracellular transport of the envelope glycoprotein complex. Additional studies are in progress to explore the effect of cleavage on Env structure.

Taken together, the antigenic and biophysical data of SOS gp140, gp120 and gp140UNC suggest that SOS gp140 represents an improved yet clearly imperfect mimic of native Env. It is perhaps surprising that an SOS gp140 monomer mimics virus-associated Env in its reactivity with a diverse panel of MAbs. Immunochemical studies and the X-ray crystal structure of the gp120 core in complex with CD4 and MAb 17b have together defined the surface of gp120 in terms of neutralizing, non-neutralizing and silent faces (Kwong, 1998; and Wyatt, 1998a). The data presented here and elsewhere (Binley, 2000a) demonstrate the neutralizing face is readily accessible on SOS gp140, whereas the non-neutralizing face is not. There are still no immunologic ways to probe the exposure of the silent face of gp120 (Moore, 1996). A source of purified SOS gp140 glycoprotein, as described herein, will facilitate further studies of the antigenic structure of SOS gp140 in comparison with that of native Env.

Do gp140UNC proteins mimic the structure of the native, fusion-competent envelope glycoprotein complex on virions? We believe not, based on their exposure of non-neutralizing epitopes in both gp120 and gp41 that are not accessible on the surface of native envelope glycoprotein complexes (Binley, 2000a; and Sattentau, 1995). Neutralization epitopes overlapping the CD4 binding site are poorly presented on HIV-1_{BH8} gp140UNC relative to virus-associated Env (Parren, 1996), and only one CD4 molecule can bind to the SIVmac32H gp140UNC protein. The lack of correlation between the binding of MAbs to uncleaved envelope glycoprotein complexes on the surface of Env-transfected cells and neutralization of the corresponding viruses again argues that uncleaved complexes have an abnormal configuration (York, 2001). However, in the absence of definitive and comparative structural information on native and uncleaved Env complexes, this is an unresolved point. At present it is not possible to predict what antigenic structures will elicit a desired immune response; that can only be defined empirically, and it may be that one or more uncleaved forms of Env will be effective immunogens even if they do not properly mimic the structure of the native Env complex. Given this situation, we believe it is relevant to design and rigorously test different Envs, such as SOS gp140, that possess distinct antigenic properties.

Given that SOS gp140 is monomeric, what can be done to further stabilize the structure of fully cleaved, envelope glycoprotein complexes? The immunoelectron microscopy data of the 2G12/SOS gp140 complex suggest that appropriately directed antibodies could strengthen weak oligomeric interactions. The immunogenicity of such complexes may be worth testing, although a bivalent MAb might be expected to promote formation of Env dimers rather than trimers. We have already attempted to combine the SOS gp140 disulfide bond stabilization strategy with one in which the gp41 subunits were also stabilized by an intermolecular disulfide bond B this was unsuccessful, in that the mutated protein was poorly expressed and could not be cleaved into gp120 and gp41 subunits, even in the presence of co-transfected furin. Similarly, adding GCN-4 domains onto the C-terminus of gp41 hindered the proper cleavage of gp140 into gp120 and gp41 furin. Other approaches, based on site-directed mutagenesis of selected gp41 residues, are presently being evaluated.

Fortuitously, we have found that variable-loop-deleted forms of HIV-1_{JR-FL} SOS gp140 form more stable oligomers than their full-length counterparts. Thus, the SOS gp140 proteins lacking either the V1 or V2 variable loops contain a greater proportion of oligomers than the full-length protein, and the V1V2 double loop-deletant is expressed primarily as noncovalently-associated trimers. One hypothesis is that the extended and extensively glycosylated variable loops sterically impede the formation of stable gp41-gp41 interactions in the context of the full-length SOS gp140 protein. Indeed, using the crystal structure of the gp120/CD4/17b complex, Kwong et al. have developed a model of oligomeric gp120 that places V1V2 sequences at the trimer interface (Kwong, 2000). The variable-loop-deleted SOS gp140 proteins may therefore represent proteolytically mature HIV-1 envelope glycoproteins that can perhaps eventually be produced and purified as oligomers. We previously demonstrated that unpurified forms of variable-loop-deleted SOS gp140 proteins possess favorable antigenic properties (Sanders, 2000). These proteins are therefore worth further evaluation in structural and immunogenicity studies.

### Experimental Set III - Particle vaccines

### A. Materials and Methods

*Antibodies and Recombinant HIV-1 envelope antigen.* The expression vectors designated CD4-IgG2HC-pRcCMV and CD4-kLC-pRcCMV were deposited pursuant to, and in satisfaction of, the requirements of the Budapest Treaty with ATCC under ATCC Accession Nos. 75193 and 75104. CD4-IgG2 protein was produced in purified form as described from Chinese hamster ovary cells stably co-transfected with CD4-IgG2HC-pRcCMV and CD4-kLC-pRcCMV (Allaway, 1995).

The expression vector designated PPI4-tPA-gp120_{JR-FL} was deposited pursuant to, and in satisfaction of, the requirements of the Budapest Treaty with ATCC under ATCC Accession Number. 75432. Recombinant HIV-1_{JR-FL} gp120 protein was produced in purified form as described from Chinese hamster ovary cells stably co-transfected with PPI4-tPA-gp120JR-FL as described previously (U.S. Patent Number 5,869,624).

The mouse monoclonal antibody to the V3 loop of HIV-1_{JR-FL} gp120 was prepared from the hybridoma cell line by passaging the cell line in mouse ascites fluid and isolating the monoclonal antibody by protein A affinity chromatography as described (Olson, 1999).

The human monoclonal antibody IgG1b12 (National Institutes of Health AIDS Research and Reference Reagent Program [ARRRP] Cat. Number 2640) binds an epitope on gp120 that overlaps the CD4 binding site (Burton, 1991). The human monoclonal antibody 2G12 (ARRRP Cat. Number 1476) binds a glycan-dependent epitope on gp120 (Trkola, 1996b). The human monoclonal antibody 2F5 (ARRRP Cat. Number 1475) binds the HIV-1 envelope transmembrane glycoprotein gp41 (Muster, 1993).

*Preparation of* Miltenyi µ*-MACS Protein G Microbeads.* 2mg of purified PA1 (1mg/ml) were incubated overnight with 4 ml of a suspension of Miltenyi µ-MACS Protein G microbeads (Miltenyi Biotec; Cat. Number 130-071-101) at 4°C. The next day the microbeads were pelleted in a Sorvall RC5C ultracentrifuge (SS-34 rotor) at 12,000 rpm (∼20,000 x g) for 15 minutes. The isolated microbeads were washed once with 400µl PBS and pelleted in a microcentrifuge at 15,000 rpm (∼16,000 x g) for 15 minutes. If protein was to be immunoprecipitated with the mAb bound to the beads, the beads were resuspended with the protein solution (see below). Otherwise, the PA1-beads were gently resuspended in PBS at a concentration of 1mg/ml PA1. Using this method, ∼150µg of PA1 could be immobilized per ml of microbead suspension.

The immobilization of CD4-IgG2, 2G12, IgG1b12, and 2F5 was performed essentially as described for PA1. The capacity was ∼40µg of CD4-IgG2, ∼55µg of IgG1b12, ∼60µg of 2G12, or ∼95µg of 2F5 per ml of microbead suspension.

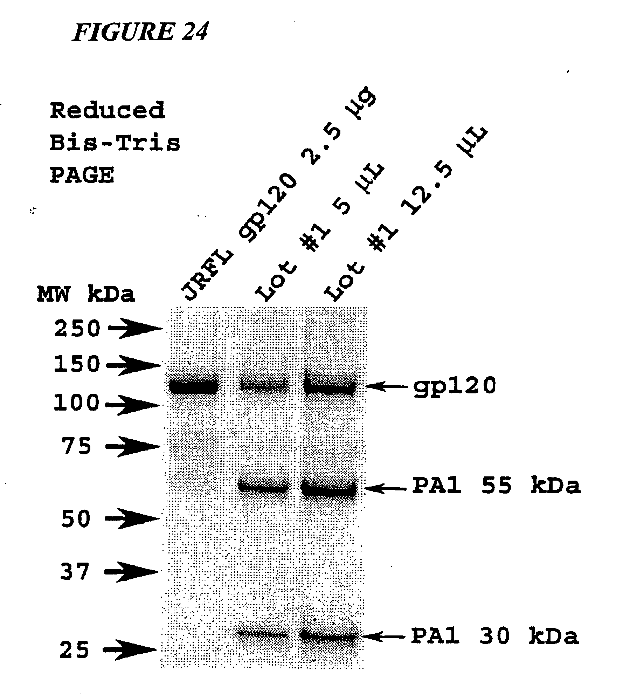

*Capture of HIV-1_{JR-FL} gp120 onto PA1 microbeads.* Beads containing 600µg of PA1 were carefully resuspended with 3mg of HIV-1_{JR-FL} gp120 and incubated overnight at 4°C. The efficiency of gp120 binding to the PA1-beads was increased when the incubation was performed over 3 days. Following the capture of gp120, the microbeads were pelleted in a Sorvall RC5C ultracentrifuge (SS-34 rotor) at 12,000 rpm (∼20,000 x g) for 15 minutes. The isolated microbeads were washed once with 400µl PBS and pelleted in a microcentrifuge at 15,000 rpm (∼16,000 x g) for 15 minutes. Subsequent to the wash the gp120-loaded beads were thoroughly resuspended with PBS at a concentration of 1mg/ml gp120 (as determined by SDS-PAGE and Coomassie staining of the protein bands). Using this method, ∼800µg of gp120 were routinely immobilized with 600µg of PA1 (Figure 24). Efficient capture of antigen was obtained using both purified gp120 in PBS buffer and gp120 in cell culture media (Sigma Chemical Company, St. Louis, MO, Cat. Number C1707) containing 1% L-glutamine (Life Technologies, Gaithersburg, MD, Cat. Number 25030-081) and 0.02% bovine serum albumin (Sigma Cat. Number A7409).

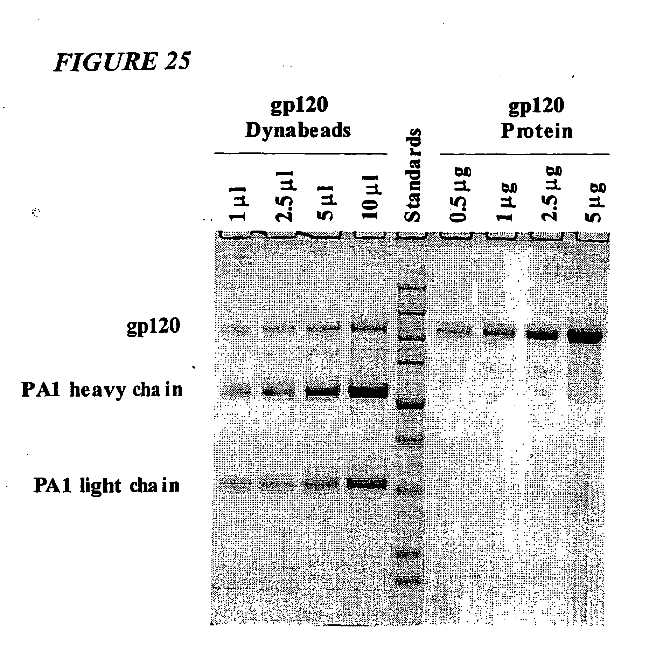

*Immobilization of antibody onto Dynabeads^{®} Protein G.* The PA1 antibody was produced as described above. 0.5mg of purified PA1 (1mg/ml) were incubated overnight with 0.1ml of a suspension of Dynabeads^{®} Protein G (Dynal Biotech Inc., Cat. Number 100.04) at 4°C. The next day the Dynabeads were collected with a magnet (Dynal Magnetic Particle Concentrator, Dynal MPC^{®}) and washed once with PBS. If protein was to be immunoprecipitated with the mAb bound to the beads, the Dynabeads were resuspended directly with the protein solution (see below). Otherwise, the PA1-beads were carefully resuspended in PBS at a concentration of 1mg/ml PA1. Using this method, ∼150µg of PA1 could be immobilized per ml of Dynabeads suspension (Figure 25).

*Capture of HIV-1_{JR-FL} gp120 onto Dynabeads.* Beads containing 15µg of PA1 were gently resuspended with 200µg of HIV-1_{JR-FL} gp120 and incubated overnight at 4°C. Following capture, the gp120-loaded Dynabeads were collected with a magnet and washed twice with PBS. Subsequent to the wash steps, the gp120-beads were carefully resuspended in PBS at a concentration of 1mg/ml gp120. Using this method, ∼3.3µg of gp120 were routinely immobilized with 15µg of PA1.

*SDS-PAGE analysis of biospecific bead vaccines.* HIV-1_{JR-FL} gp120 immobilized to the beads was analyzed by SDS-PAGE as follows: 20µl of resuspended beads were mixed with the same volume of 2x LDS/DTT sample buffer (140mM Tris Base, 106mM Tris/HCl, 2% SDS, 10% glycerol, 25mM DTT, 0.5mM EDTA, pH 8.5) and incubated at 70°C for 5 minutes. 10µl and 25µl of the Miltenyi microbeads samples or 2µl, 5µl, 10µl, and 20µl of the Dynabeads samples were loaded onto a 4-12% NuPAGE Bis-Tris gel (Invitrogen) and electrophoresed at 175V for 50 minutes using the MES/SDS running buffer system (50mM MES, 50mM Tris Base, 0.1% SDS, 1mM EDTA, pH 7.7). Included in the gels were known concentrations of gp120 treated as described for the beads for quantitation purposes. Following electrophoresis, the gels were fixed in 10% acetic acid/40% methanol and subsequently stained according to the manufacturers' protocol using the Gelcode Blue staining solution (Pierce). The stained protein bands were analyzed and quantitated by densitometry (Molecular Dynamics).

*Immunization of mice with biospecific bead vaccines.*

Successful vaccination relies on the induction of a protective immune response to an antigen of interest. Effective presentation of antigen to the immune system can be achieved by delivery of highly purified protein with an immunostimulatory adjuvant. We describe a novel dual-purpose approach using magnetic beads that (1) enables efficient purification of antigen for immunization and (2) enhanced immune responses to the antigen in animals.

*Immunogens.* Purified gp120 (Subtype B, JR-FL; 1mg/ml) was used at the indicated doses. Gp120 was admixed with the adjuvant, QS-21 (10µg per dose; Antigenics), or captured on Miltenyi MACS magnetic beads by the anti-gp120 mAb, PA1 as described above. Groups of animals received beads either with or without QS-21.

*Immunizations.* Groups of 5 female Balb/C mice (6-10 weeks of age at the onset of studies; Charles River Laboratories, Boston, MA) were used for each vaccine. Three immunizations were administered in 200µl volume at 2-week intervals by subcutaneous injection in the flank-region using ½ cc insulin syringes and 28G gauge needles (Becton Dickinson, Franklin Lakes, NJ).

*Sera and tissue collection*. Mice were bled through the retro orbital plexus one day prior to each immunization, and the sera separated by centrifugation in blood-collection Capiject tubes (Terumo; Somerset, NJ). Aliquots of the separated sera were cryopreserved at -80°C before analysis.

Spleens were harvested and pooled from the 5 mice per group and single cell suspensions prepared by gently teasing the tissue through a 70µm nylon mesh filter. Cells were cryopreserved at -196°C before analysis.

*ELISA assay*. HIV-1 gp120 specific antibodies in sera were quantified by a standard ELISA assay (Binley, 1997). Briefly, 96-well ELISA plates were coated with HIV-1_{JR-FL} gp120 via adsorbed sheep anti-gp120 mAb D7324 (Aalto BioReagents, Dublin, Ireland) and blocked before addition of serial dilutions of serum samples from individual mice in triplicate wells. After incubation, the wells were washed and incubated with a dilution of anti-mouse IgG-detection antibody conjugate before addition of chromogenic substrate. Binding was measured using an ELISA plate reader at OD490. Titers (50% maximal) were calculated for each group as defined by the antibody dilution giving half-maximal binding after background subtraction (wells with no antigen). The mean values +/-SD of replicate wells are represented.

*ELISPOT assay.* HIV-gp120 specific T cells are quantified using an IFNγ-ELISPOT assay, essentially as described (Miyahira, 1995). Briefly, mixed cellulose ester membrane 96-well plates (Millipore) are coated with an anti-mouse anti-IFNγ antibody (5µg/ml; MABTech) for 2 hours at 37°C and washed thrice in PBS. The wells are blocked in complete RPMI medium (RPMI 1640, α-MEM, FBS (10%, Gibco) HEPES (10mM Gibco), L-Gln (2mM), 2-mercaptoethanol (50µM) for a further 2 hours at 37°C. After washing the wells thrice with PBS, single cell suspensions of splenocytes are added at 1-5 x 10⁵ cells per well in the presence of gp120 protein (5µM) or H-2^{d} restricted gp120 peptide (RGPGRAFVTI (2µM)) for 16-20 hours. Plates are washed extensively in PBS/Tween-20 (PBS-T; 0.05%) and incubated for 1 hour with biotinylated anti-IFNγ antibody (2µg/ml; MABTech) at room temperature. The plates are washed thrice in PBS/T and incubated for 2 hours with streptavidin-HRP (Vectastain Elite ABC Kit). After washing with PBS/T, the HRP-substrate, AEC (3-amino-9-ethylcarbazole; Sigma), is added for 15 minutes at room temperature. The reaction is stopped by added de-ionized water, and the wells are washed before drying in air for 24 hours. The spots are enumerated using an automatic ELISPOT plate reader (Carl Zeiss, Germany) and software. Each condition is performed in triplicate with serial dilutions of splenocytes and the frequency of spot-forming cells (SFCs) per 10⁶ splenocytes is calculated. Negative controls samples with splenocytes and complete medium alone are used to determine background levels, and a positive signal is defined as >2-fold SFCs in control wells.

### B. Results and Discussion

The ability of magnetic beads to potentiate immune responses to an antigen of interest was examined using HIV-1 envelope protein, gp120, attached to magnetic beads with an anti-gp120-specific antibody (PA1). Preparations of beads were administered thrice subcutaneously to groups of mice to achieve a gp120 dose of 25µg or 5µg, with or without the immunostimulatory adjuvant, QS-21. Control groups of animals received gp120 (25µg or 5µg) with QS-21, gp120 admixed with QS21 and PA1 (no beads), or magnetic beads with PA1 (no gp120). Sequential bleeds after each dose were performed and serum separated for analysis of the anti-gp120 humoral response with a standard ELISA assay. Temporal analysis of sera demonstrated that immune responses increased after each immunization, and that anti-gp120 antibody titers were maximal after three doses

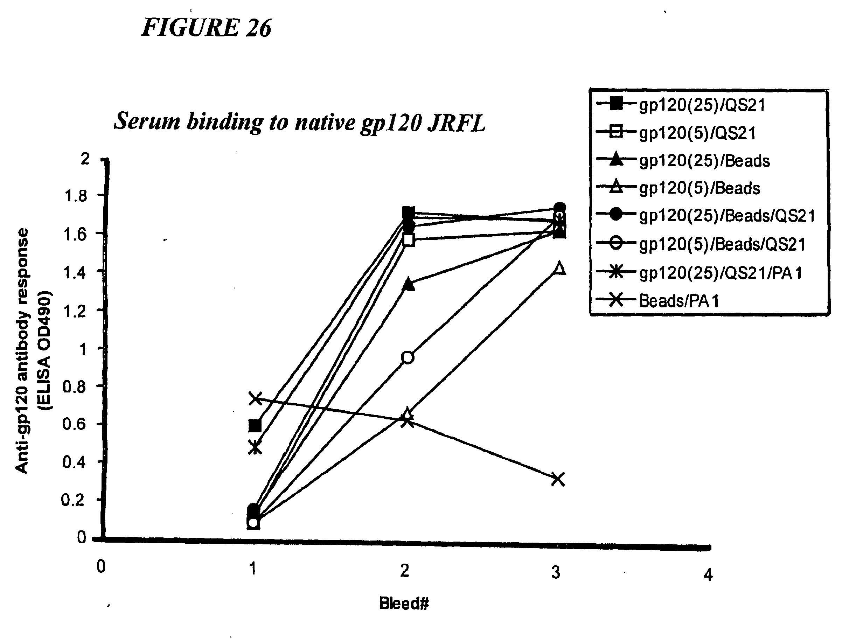

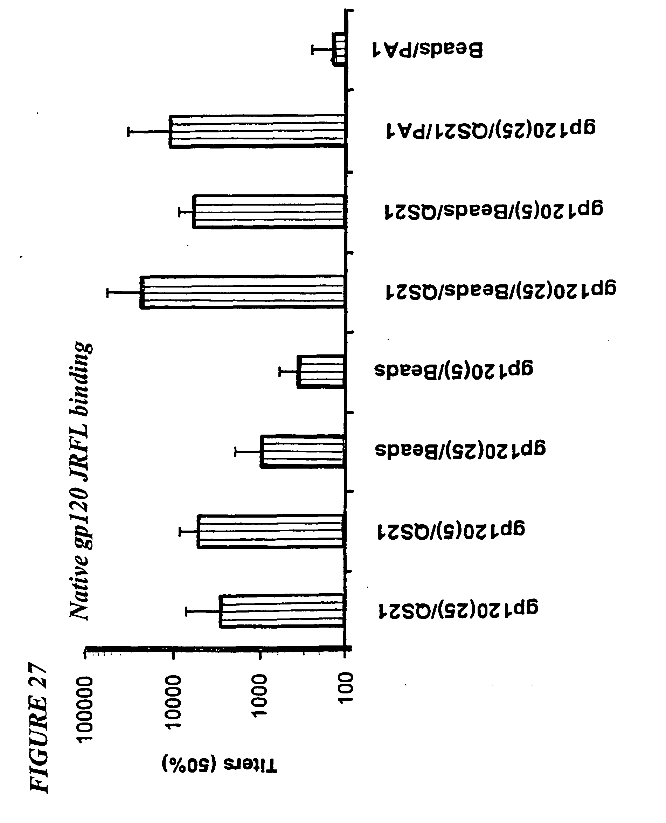

(Figure 26). The titers of antibodies were measured with serial dilutions of the sera, and indicated that bead-captured gp120 with QS-21 was the most potent immunogen (Figure 27). This response was correlated with the dose of gp120, and animals immunized with 25µg gp120 had higher levels of serum antibodies. Importantly, these responses were approximately one order of magnitude greater than those in animals receiving gp120 and QS21 without beads. These data indicate that magnetic beads augment the immune response to captured antigen, and this technology may have utility for vaccine development.

### Experimental Set IV

### A. Introduction

The envelope glycoprotein (Env) complex of human immunodeficiency virus type 1 (HIV-1) mediates viral entry into CD4⁺ cells. The sequential binding of the surface subunit gp120 to the CD4 receptor and a co-receptor, usually CCR5 or CXCR4, induces conformational changes in the Env complex. These alterations in protein structure eventually enable the insertion of the hydrophobic fusion peptide of the transmembrane subunit, gp41, into the cell membrane. Subsequently, the viral and cell membranes fuse, allowing the release of the viral core into the cytoplasm and the initiation of a new cycle of infection (for reviews see Chan, 1997; Doms, 2000a; Doms, 2000b; Eckert, 2001; Moore, 2000; and Wyatt, 1998b).

The gp120 and gp41 proteins are synthesized as a gp160 precursor that is cleaved within the cell to yield the native, pre-fusion form of the envelope glycoprotein complex (Hunter, 1997; McCune, 1988; and Moulard, 2000). This is generally considered to be a trimeric structure, containing three gp120 and three gp41 moieties, held together by non-covalent interactions (Eckert, 2001; Poignard, 2001; and Wyatt, 1998b). The native Env complex is unstable because the non-covalent intersubunit interactions that hold gp120 onto gp41 are weak, as are the intermolecular interactions between the gp41 moieties (Eckert, 2001; Poignard, 2001; and Wyatt, 1998b). This instability is probably essential for the receptor-triggered conformational changes to occur, but it does cause a problem for attempts to express the native complex as a recombinant protein (Binley, 2000a).

Recombinant forms of the native Env complex have been prepared to permit structural studies thereupon. At present, structural information on HIV-1 Env is limited to core fragments of gp120 in the CD4-associated configuration, and the 6-helix bundle form of the gp41 core which represent its terminal, most stable configuration (Caffrey, 1998; Chan, 1997; Kwong, 2000b, Kwong, 1998; Lu, 1999; Malashkevic, 1998; Tan, 1997; and Weissenhorn, 1997). Although the 6-helix bundle is often referred to as the "fusogenic" form of gp41, this term can be misleading because it is the formation, and not the mere presence, of the 6-helix bundle that drives membrane fusion (Doms, 2000b; Gallo, 2001; and Melikyan, 2000). Hence, antibodies to the 6-helix bundle cannot interfere with fusion and are non-neutralizing (Jiang, 1998; Moore, 2001; Nyambi, 1998; Parren, 1999; and Taniguchi, 2000). The term "post-fusion" form of gp41 is therefore used when referring to the 6-helix bundle, to reflect its persistence on infected cells as a major immunogen after the fusion process is complete and on virions when conformational changes in Env leading to gp120 shedding have occurred prematurely or abortively (Doms, 2000b). Most antibodies to gp41 in HIV-1-infected individuals recognize this post-fusion conformation (Gorny, 2000; Moore, 2001; Parren, 1999; Robinson, 1990; Taniguchi, 2000; and Xu, 1991).

A second purpose for recombinant forms of the native Env complex is to study their immunogenicity and to determine their suitability as vaccine antigens. The few monoclonal antibodies (MAbs) that potently neutralize HIV-1 all recognize epitopes exposed on the native Env complex, and may well have been induced by such a complex (Burton, 2000; Fouts, 1998; Moore, 1995b; Parren, 1997; Parren, 1998; Parren, 1999, Poignard, 2001; and Sattentau, 1995a). In contrast, non-neutralizing MAbs do not bind to the native complex, and probably represent immune responses to non-native forms of Env, such as uncleaved gp160 precursors, dissociated gp120 subunits or the 6-helix bundle, post-fusion form of gp41 (Burton, 2000; Moore, 2001; Parren, 1997; Parren, 1998; and Poignard, 2001).

Eliciting neutralizing antibodies by vaccination with any form of Env is problematic because of the mechanisms that the native Env complex has evolved to shield its most critical sites and to limit its overall immunogenicity. Thus, conserved regions of gp120 involved in receptor binding are shielded by variable loops and by extensive glycosylation. The CD4 binding site is recessed, and the co-receptor binding site is only formed or exposed for a short period after CD4 has already bound, thereby limiting the time and space available for antibody interference (Moore, 1998; Moore, 1995b; Olofsson, 1998; and Wyatt, 1998a). Whether such defense mechanisms can be overcome by vaccine-induced antibodies remains uncertain (Moore, 2001; and Parren, 1999). One approach to this problem has involved attempts to form a stabilized native Env complex, which may then have to be further modified to improve its immunogenicity.

The lability of the non-covalent interaction between gp120 and the gp41 ectodomain (gp41ECTO) is an obstacle to the production of stable, fully processed HIV-1 Env trimers. The association between gp120 and gp41ECTO can be stabilized by the introduction of a correctly positioned intermolecular disulfide bond, in order to form the SOS gp120 protein (Binley, 2000a; and Sanders, 2000). In the presence of co-transfected furin, the peptide bond linking gp120 to gp41_{ECTO} is cleaved, thus permitting production of properly processed gp140 proteins (Binley, 2000a; and Sanders, 2000). It was initially reported that oligomeric proteins were present in supernatant from 293T cells transiently expressing SOS gp140 (Binley, 2000a). However, these oligomers were not abundant, and they did not survive purification. Purified SOS gp140 is a monomeric protein (Schulke, 2002).

### B. Overview

The invention relates to the generation and characterization of soluble, cleaved RIV-1 envelope glycoprotein trimers. In these gp140 proteins, the gp120-gp41 interactions are stabilized by an intermolecular disulfide bond, and the gp41-gp41 interactions are stabilized by specific amino-acid substitutions in the N-terminal heptad repeat of gp41_{ECTO}, most notably at position 559. This work is based upon the observation that the SOS gp140 protein was unstable, i.e., it dissociated into gp140 monomers and could not be purified in trimeric form

(Schulke, 2002). The fragility of the SOS gp140 trimer is created by the proteolytic cleavage event that eliminates the peptide bond between gp120 and gp41ECTO as the gp140 precursor is processed to maturity. Thus, gp140UNC proteins form stable oligomers, whether or not the disulfide bond that characterizes the SOS gp140 proteins is also present (Barnett, 2001; Schulke, 2002; Stamatatos, 2000; Yang, 2000b, Yang, 2000a; and Zhang, 2001). The focus is upon formation of cleaved, stable Env trimers for structural and immunogenicity studies. To achieve this result, a way to overcome the instability of the.gp41-gp41 ectodomain interactions in the pre-fusion form of the gp140 protein has been developed.

Destabilization of the post-fusion state of gp41 might lead to stabilization of the native, trimeric SOS gp140 complex. The native HIV-1 Env complex is metastable and undergoes a transition to the post-fusion, 6-helix bundle structure after activation by receptor-binding, probably losing gp120 in the process (Chan, 1998; Doms, 2000a; Doms, 2000b; Eckert, 2001; Moore, 2000; and Wyatt, 1998b). The gp120 moiety of the SOS gp140 protein can bind CD4 and undergo conformational changes within gp120 that expose the co-receptor binding site. It is not known whether any additional conformational changes are initiated in the gp41ECTO moiety of the SOS gp140 protein upon CD4 and/or co-receptor binding. If so, these changes cannot be completed. However, whether or not the SOS gp140 protein has bound to CD4, the gp41-gp41 interactions that are responsible for its trimerization are unstable, i.e., the protein readily becomes a monomer (Schulke, 2002).

A delicate balance exists between the metastable, pre-fusion state of gp140 and its stable, post-fusion, 6-helix bundle configuration. The balance involves not only the trimeric interactions between gp41ECTO moieties, but also the association between gp120 and gp41ECTO.

The SIV_{mac} gp140 protein seems to be a more stable trimeric protein than HIV-1 gp140 (Center, 2001 and Chen, 2000). In contrast, the post-fusion state of SIV_{mac} Env is less stable than that of HIV-1 Env (Liu, 2001). It may or may not be relevant that the SIV_{mac} Env glycoprotein contains a valine and not an isoleucine residue at position 559. Furthermore, a SIV_{mac} envelope glycoprotein with an unusually strong gp120-gp41 association has a destabilized, post-fusion, 6-helix bundle conformation compared to the parental virus from which it evolved (LaBranche, 1995; and Liu, 2002). Variant HIV-1_{LAI} viruses have been engineered to contain the SOS substitutions so to have gp120 covalently linked to gp41. These viruses are minimally infectious, but evolve in culture to more infectious forms via reversions at a region of gp41ECTO that corresponds to the trimer interface of the post-fusion form. These changes in gp41ECTO may influence the interactions both between gp120 and gp41 and between the gp41 moieties.

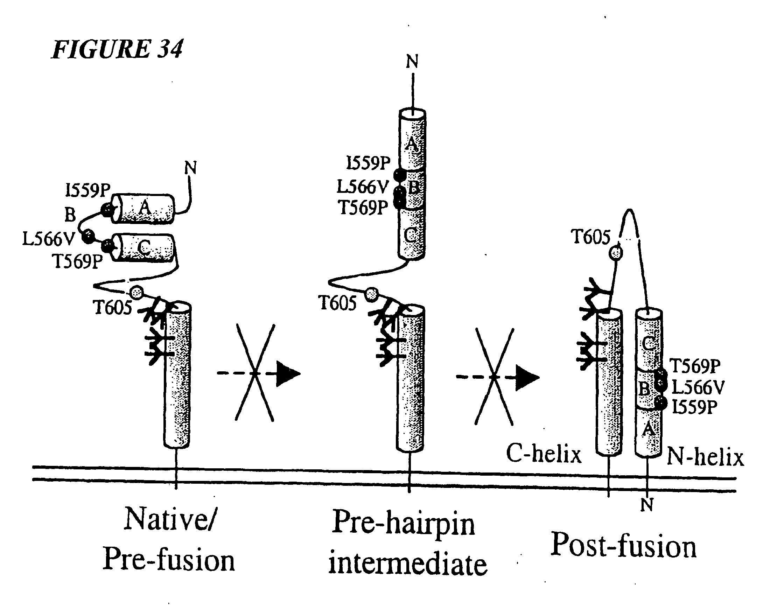

Substitutions introduced at position 559 to make the SOSI559P or SOSI559G gp140 proteins block one or more of the conformational transitions in gp41ECTO (Hunter, 1997). A model of how the substitutions might act is presented in Figure 34. Preventing these transitions stabilizes the SOS gp140 protein as a trimer. It is not yet known which among the several conformational transitions that gp41ECTO undergoes during fusion is impaired by the changes at residue 559. Possibilities include, first, that the transition from the elusive native state to the pre-hairpin intermediate could be effected, or second, that the subsequent transition from the pre-hairpin intermediate to the 6-helix bundle structure might be prevented (Figure 34). The observation that the N36(L6)C34 I559P peptide is only -75% helical argues in favor of the first possibility, i.e., interference with the formation of the N-terminal helix. Peptide-based studies on the gp41 N-terminal helix have shown that the first part of this helix, including the region around residue 559, is more flexible than the last part (Chang, 2001).

Stabilizing the pre-fusion, trimeric structure of a fusogenic viral glycoprotein, by destabilizing or disrupting its N-terminal helix via a proline substitution, is not without precedent. In the influenza HA₂ glycoprotein, a stretch of 22 amino acids is not helical in the pre-fusion form. However, upon exposure to low pH to trigger fusion, this region of HA₂ undergoes a loop-to-helix transition to form the fusion-active configuration of the protein (Bullough, 1994). Proline substitutions at the indicated location in HA₂ allow the expression of properly processed, but fusion-incompetent proteins (Qiao, 1998). Similarly, a proline substitution at position 559 in HIV-1 gp41 is known to abolish the fusogenicity of an otherwise infectious virus (Chen, 1994; and Chen, 1998).

The SOSI559P, SOSI559G, SOS L566V and SOS T569P gp140 proteins do not suffer from any proteolytic cleavage defects. This is in contrast with the cleavage defects that are caused by amino-acid substitutions at the same positions in the context of wild-type gp160 proteins (Cao, 1993; Chen, 1994; Chen, 1998; and Poumbourios, 1997). An explanation for the apparent discrepancy may be provided by the earlier observations that the presence of the SOS inter-subunit disulfide bond can rescue some cleavage defects in gyp14 proteins (Sanders, 2000). Moreover, the various SOS gp140 proteins are expressed in the presence of co-transfected furin, so the increased concentration of this enzyme may compensate for any partial reduction in cleavage efficiency.

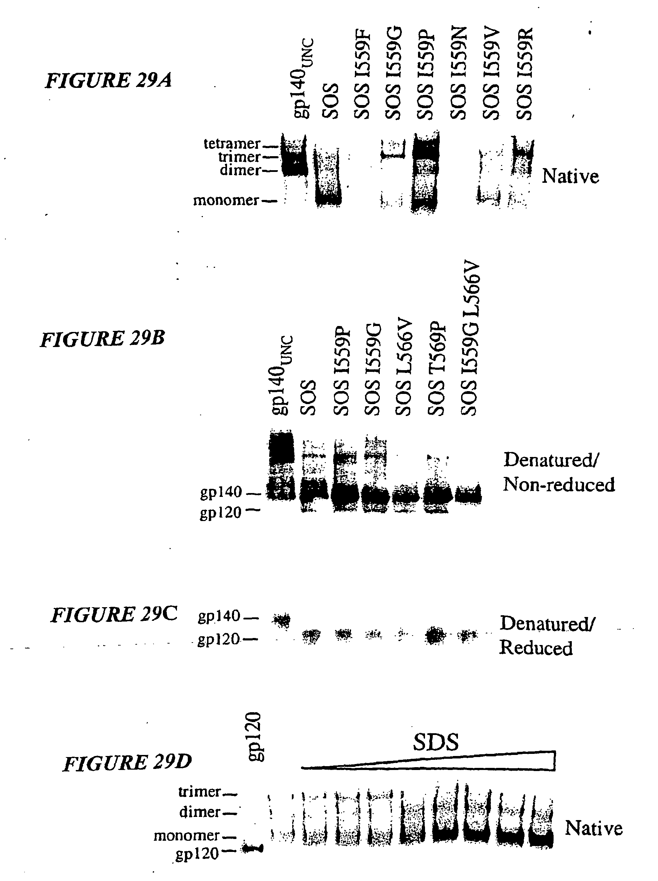

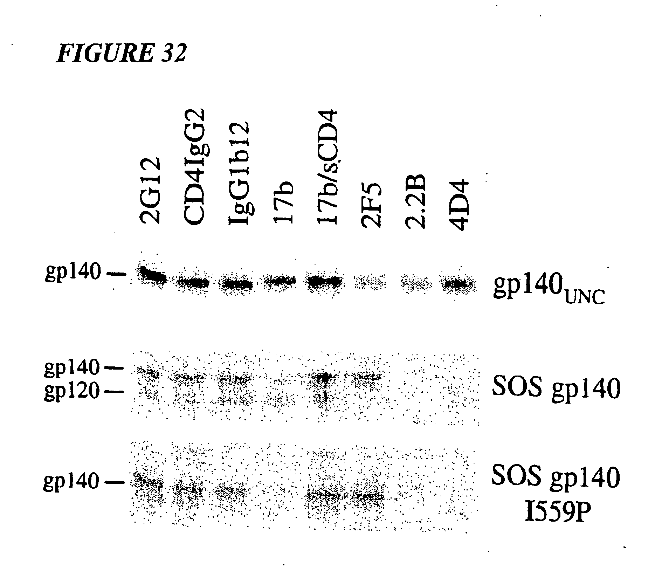

Several unpurified and purified gp140UNC proteins from both HIV-1 and SIV_{mac} have been described previously (Barnett, 2001; Center, 2001; Chen, 2000; Earl, 1994b; Earl, 1990; Srivastava, 2002; Stamatatos, 2000; and Zhang, 2001). There is general agreement that SIV_{mac} gp140UNC proteins are predominantly trimeric (Center, 2001; and Chen, 2000). In contrast, the oligomeric state of HIV-1 gp140UNC proteins varies from study to study. After a purification procedure that included an earlier size exclusion chromatography step, HIV-1 ADA gp140UNC proteins were eluted homogeneously from size exclusion columns with a molecular weight that indicates they are trimers (Zhang, 2001). The production of an oligomeric US4 gp140UNC protein that might be trimeric has also been reported (Srivastava, 2002). The presence of both dimers and tetramers in uncleaved versions of both membrane-bound gp160 and soluble gp140 molecules has been observed in several previous studies (Earl, 1994b; Earl, 1990; Earl, 1993; Earl, 1991; Farzan, 1998; Yang, 2000a; and Yang, 2000b). Experience, using the BN-PAGE assay, has been that a mixture of dimers, trimers and tetramers is present in unpurified and purified HIV-1_{JR-FL} gp140UNC preparations, with dimers being the most abundant species (Schulke, 2002). Moreover, the dimeric and tetrameric forms of HIV-1 gp140UNC and gp160 proteins are probably oligomerized by aberrant intermolecular disulfide bonds (Figure 29) (Owens, 1990; and Schulke, 2002). It is unlikely that oligomeric g140 proteins of this type will fully mimic the native conformation of Env. Indeed, it has been found that unpurified gp140UNC proteins have a different antigenic structure than unpurified SOS gp140 monomers and SOSI559P gp140 trimers. Thus, non-neutralizing antibody epitopes in both the gp120 and gp41ECTO moieties are exposed to a much greater extent in gp140UNC proteins than in the SOS gp140 proteins (Figure 32) (Binley, 2000a; Sanders, 2000; and Schulke, 2002). Future efforts involve comparing purified, cleaved and uncleaved trimers, as well as purified, uncleaved dimers and tetramers, to assess their antigenic structures, their migration on BN-PAGE gels after various treatments, and their receptor-binding properties.

### C. Materials and Methods

*Env Expression.* Various forms of the JR-FL gp140 envelope glycoproteins were expressed in 293T cells from the pPPI4 vector, and furin was expressed-from pcDNA3.1-Furin, as described previously (Binley, 2000a; and Sanders, 2000). The uncleaved JR-FL gp140 protein (gp140UNC) with amino acid substitutions to prevent its proteolytic processing has also been described elsewhere (Binley, 2000a). Specific mutations were made using the Quickchange^{™} mutagenesis kit (Stratagene, La Jolla, CA). Random mutations were generated with primers that could contain any nucleotide at the relevant positions. Numbering is based on the HXB2 Env sequence. Recombinant JR-FL gp120 has been described elsewhere (Trkola, 1996a).

*Antibody binding assays*. The procedures and MAbs used to perform radioimmunoprecipitation assays (RIPA) have all been described (Allaway, 1995; Binley, 2000a; Moore, 1996; Sanders, 2002; and Trkola, 1996b). MAbs were provided by James Robinson (17b, 2.2B), George Lewis (B12), Hermann Katinger (2F5, 2G12, 4D4, 4E10) and Dennis Burton (IgG1b12).

*SDS-PAGE, BN-PAGE and Western blot analyses.* Sodium dodecyl sulfate polyacrylamide gel electrophoresis (SDS-PAGE), Blue Native (BN)-PAGE and Western blot analyses were performed as described previously (Binley, 2000a; Sanders, 2000; and Schulke, 2002). Culture supernatants from transiently transfected 293T cells were concentrated 10-fold before gel electrophoresis, using Ultrafree-15 concentrators (Millipore, Bedford, MA).

*Gel-filtration analysis of envelope glycoproteins.* Supernatants from 293T cells, transfected with pPPI4-gp140 plus pcDNA3.1-Furin, were concentrated 100-fold, then size-fractionated using an analytical Superdex 200 HR 10/30 column equilibrated with phosphate-buffered saline (PBS; Amersham-Pharmacia, Piscataway, NJ). Fractions (300µl) were analyzed using SDS-PAGE and BN-PAGE; in both cases, Western blotting was used to detect Env glycoproteins. The column was calibrated using protein standards of known size (HMW-standard; Amersham-Pharmacia).

*Trimer stability experiments.* Eluates from gel filtration columns, containing SOSI559P gp140 trimers, were incubated with various reagents or under different conditions, then analyzed using BN-PAGE and western blot procedures. The following detergents were obtained from the following suppliers SDS, Sigma, St Louis, MO; t-octylphenoxypolyethoxyethanol (Triton X-100), Sigma; polyoxyethylene sorbitan monolaurate (Tween-20), Sigma; ethylphenyl-polyethylene glycol (NP-40), United States Biochemicals, Cleveland, OH; n-octyl ß-D-glucopyranoside (oct-glucoside), Sigma; Empigen BB 30% solution, (Empigen) Calbiochem, La Jolla, CA.

*Peptide production.* Plasmid pN36/C34JR-FL, encoding the HIV-1_{JR-FL} N36(L6)C39 model peptide, was derived from pN36/C34HXB2 (Lu, 1999). Amino acid substitutions were introduced into the N36 segment of pN36/34JR-FL using the method of Kunkel et al. (Kunkel, 1987), then verified by DNA sequencing. All recombinant peptides were expressed in *Escherichia coli* strain BL21(DE3)/pLysS (Novagen, Madison, WI). The bacteria were grown at 37°C in LB medium to an optical density of 0.8 at 600nm, and induced with isopropylthio-ß-D-galactoside for 3-4 hours. Cells were lysed at 0°C with glacial acetic acid. The bacterial lysate was centrifuged (35,000 g for 30 minutes) to separate the soluble fraction from inclusion bodies. The soluble fraction, containing denatured peptide, was dialyzed into 5% acetic acid overnight at room temperature. Peptides were purified from the soluble fraction to homogeneity by reverse-phase high-performance liquid chromatography (Waters, Milford, MA) using a Vydac C-18 preparative column (Vydac, Hesperia, CA) and a water-acetonitrile gradient in the presence of 0.1% trifluoroacetic acid, then lyophilized. The molecular weight of each peptide was confirmed by using matrix-assisted laser desorption ionization-time-of-flight mass spectrometry (PerSeptive Biosystems, Framingham, MA). The concentration of each peptide was determined at 280nm after solubilization in 6M guanidinium chloride (Edelhoch, 1967).

*Circular dichroism (CD) spectroscopy.* HPLC-purified peptides were solubilized in 6M guanidinium chloride and 10mM Tris-HCl (pH 7.0), and refolded by dilution into PBS at neutral pH. The single-point substituted variant peptides were named according to the position of the substitution. CD experiments were performed using an Aviv 62A DS circular dichroism spectrometer. The wavelength dependence of molar ellipticity, [θ], was monitored at 4°C, using a 10µM peptide solution in 100mM NaCl, 50mM sodium phosphate, pH 7.0 (PBS). Helix content was calculated by the method of Chen et al. (Chen, 1974). Thermal stability was determined by monitoring the change in the CD signal at 222nm ([θ]₂₂₂) as a function of temperature. Thermal melts were performed in 2°C increments with an equilibration time of 2 minutes at the desired temperature and an integration time of 30 seconds. All melts were reversible. Superimposable folding and unfolding curves were observed, and >90% of the signal was regained upon cooling. The melting temperatures, or midpoints of the cooperative thermal unfolding transitions (*T*ₘ₎, were determined from the maximum of the first derivative, with respect to the reciprocal of the temperature, of the [θ]₂₂₂ values (Cantor, 1980). The error in estimation of *T*ₘ is ± 0.5°C.

*Sedimentation equilibrium analysis.* A Beckman XL-A (Beckman Coulter, Fullerton, CA) analytical ultracentrifuge equipped with an An-60 Ti rotor (Beckman Coulter) was used. Peptide solutions were dialyzed overnight against PBS (pH 7.0), loaded at initial concentrations of 10, 30, and 100µM. and analyzed at rotor speeds at 20,000 and 23,000 rpm at 20°C. Data sets were fitted simultaneously to a single-species model of In(absorbance) versus (radial distances)² using the program NONLIN (Johnson, 1981). Protein partial specific volume and solvent density were calculated as described by Laue et al. (Laue, 1992).

### D. Results and Discussion

### Stabilization of the SOS gp140 trimer by mutagenesis

Although the purified, cleaved SOS gp140 protein is a monomer, oligomeric forms of this protein have been observed in freshly prepared supernatants from transiently transfected 293T cells (Binley, 2000a; and Schulke, 2002). This suggests that the oligomeric form of SOS gp140, although clearly unstable, might not be far from stability. A mutagenesis strategy to try to increase the stability of the cleaved, oligomeric SOS gp140 protein was therefore adopted. The focus was on altering the gp41ECTO sequence, because of the clear preponderance of evidence that interactions between gp41ECTO subunits are responsible for the oligomerization of gp140 (Earl, 1993; Shugars, 1996; and Wyatt, 1998b). However, no structural or other data are available on the likely points of contact between the gp41 moieties in the native, pre-fusion form of the envelope glycoprotein trimer. Destabilization of the post-fusion state of gp41 may stabilize its pre-fusion configuration, by shifting the conformational equilibrium in favor of the pre-fusion state (Jelesarov, 2001; Ji, 2000; Liu, 2001; Liu, 2002; and Lu, 1999). Structural and genetic data on the post-fusion, 6-helix bundle structure of gp41 are available (Bernstein, 1995; Caffrey, 1998; Cao, 1993; Chan, 1997; Chen, 1994; Chen, 1998; Hunter, 1997; Liu, 2001; Lu, 1999; Malashkevich, 1998; Poumbourios, 1997; Tan, 1997; Weissenhorn, 1997; Weng, 1998; and Weng, 2000).

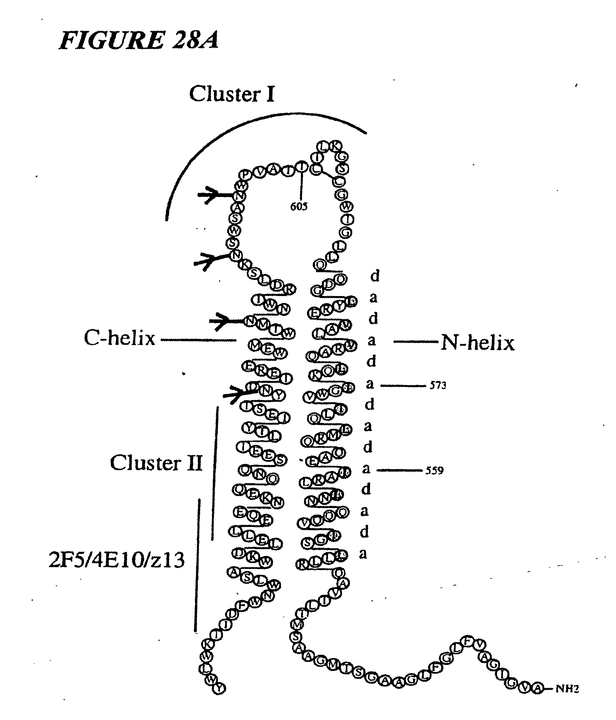

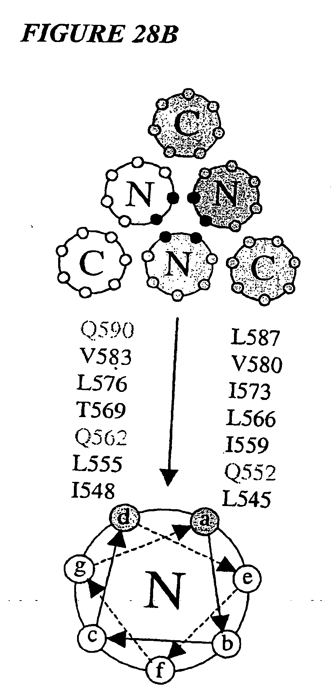

The trimeric stability of gp41 in the 6-helix bundle form of the protein is determined by the residues at the a and d positions of the N-terminal heptad-repeat region (Jelesarov, 2001; Ji, 2000; and Lu, 2001) (Figure 28). Most of these amino acids are absolutely conserved. Hydrophobic Val, Leu and Ile residues form the apolar interface between the three N-terminal helices. These residues are critical determinants of the folding and thermal stability of the 6-helix bundle (Shu, 1999). It was assumed that making non-conservative substitutions at these conserved, hydrophobic residues would destabilize the 6-helix bundle and so, by reducing the probability of its formation, cause the gp41ECTO subunits to remain in their desired, pre-fusion configuration. The hydrophilic glutamine residues at positions 552, 562 and 590 were initially left unaltered, since buried polar interactions involving these residues confer structural specificity for formation of the N-terminal coiled-coil trimer at the expense of its thermal stability (Ji, 2000) (Figure 28). Substitutions at residue Thr-569 were also evaluated.

Because it could not be predicted what amino acids would be tolerated in the pre-fusion configuration of gp41_{ECTO}, random mutagenesis was performed. Hence various amino acids with different biochemical properties were introduced in place of the targeted Val, Ile and Leu residues. An emphasis of the mutagenesis approach was to alter the Ile-559 and Ile-573 residues, because core isoleucines are known to confer the greatest stability to trimeric coiled coils (also known as isoleucine zippers) (Harbury, 1994). In most simian immunodeficiency virus (SIV) strains, Val and Thr residues are found, respectively, at these positions, where they may serve to destabilize the post-fusion, coiled-coil structure (Jelesarov, 2001; Liu, 2001: and Liu, 2002). Of note is that the SIV gp140UNC protein has a greater tendency to be trimeric than the corresponding HIV-1 protein (Center, 2001; and Chen, 2000).

The use of blue native (BN)-PAGE to monitor the oligomeric state of HIV-1 gp140 proteins is described elsewhere (Schulke, 2002). This technique served as a screening assay to identify more stable SOS gp140 variants, i.e., ones that remained trimeric under conditions in which the unmodified SOS gp140 protein ran mainly as a monomer. The variant SOS gp140 proteins were expressed in transiently transfected 293T cells, in the presence of co-transfected furin, to facilitate gp120-gp41 cleavage. The effects of various, single-residue substitutions on the expression and trimer stability of these SOS gp140 proteins are summarized in Table 2 (see below). An example of how BN-PAGE was used to derive this information is shown in Figure 29a.

Many of the randomly generated SOS gp140 mutants were not expressed, or were expressed poorly, particularly those with substitutions at residues 555, 576 and 587. This is probably because amino-acid changes at these positions have adverse effects on protein folding. However, other conserved residues (e.g., 548, 573, 580 and 583) were more tolerant of substitution, in that the altered SOS gp140 proteins were still expressed efficiently. The effect on SOS gp140 expression of substitutions at other positions, notably at residues 545, 559, 566 and 569, was dependent on the identity of the amino acid introduced. Thus, at position 545, a Leu-to-Phe change (L545F) reduced SOS gp140 expression, whereas the introduction of Asn (L545N), Pro (L545P) or Gly (L545G) at this position had little effect. Similarly, Ile-to-Phe or Ile-to-Asn substitutions at position 559 (I559F, I559N) severely diminished gp140 expression (Figure 2a, Lanes 3, and 6). In contrast, the introduction of Val (ISS9V), Gly (I559G) or Arg (I559R) residues at position 559 had a lesser effect on SOS gp140 expression, and a Pro (I559P) substitution had no adverse effect at all (Figure 29a, Lanes 4, 7, and 8). Other examples of how the introduced residue can have a variable effect on SOS gp140 expression include changes at residues 566 and 569 (Table 2).

BN-PAGE was used to determine whether there were differences in oligomer stability among the subset of altered SOS gp140 proteins that were efficiently expressed. Under native conditions, the wild-type SOS gp140 protein migrates predominantly as a monomer, with some dimeric and trimeric species also present (Figure 29a, Lane 2). The proportion of the SOS gp140 protein that is oligomeric varies from experiment to experiment, but the gel shown in Figure 29a is typical of what is most commonly observed. In contrast, the gp140UNC protein migrates as oligomeric forms, with the dimer predominating (Figure 29a, Lane 1).

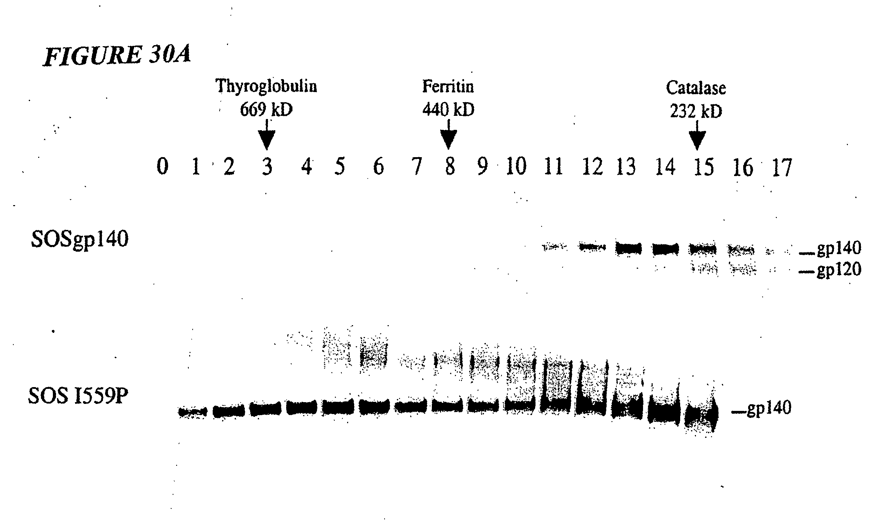

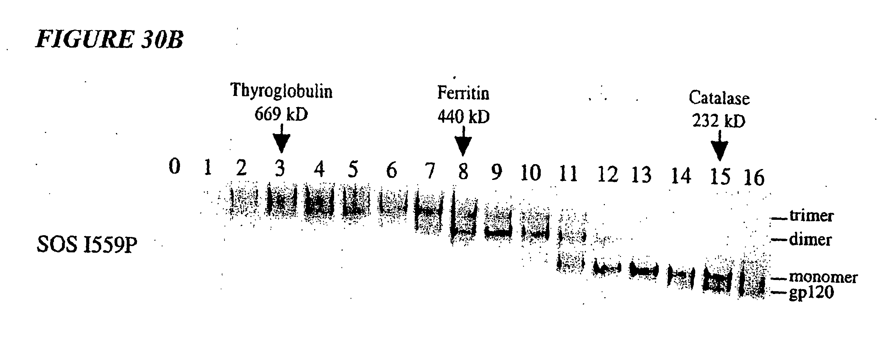

Most of the amino-acid substitutions consistent with the efficient expression of SOS gp140 had little effect on the extent of its oligomerization (Table 2). However, several substitutions at position 559 clearly altered the oligomerization state of SOS gp140 (Figure 29a). Thus, when Ile-559 was replaced by a non-conservative Gly (I559G), Arg (I559R) or Pro (I559P) residue, the most abundant protein form was consistently the trimer (Figure 29a, Lanes 4, 5, and 8). In contrast, the conservative Ile-to-Val substitution at position 559 had no effect, in that the 1559V and wild-type SOS gp140 proteins were indistinguishable (Figure 29a, Lanes 2, and 7). Some substitutions at positions 566 and 569 (e.g., L566V, T569P) marginally increased the proportion of SOS gp140 proteins that were trimeric (Table 2). The percentage of trimers varied per transfection, but the amount of trimer in the SOS gp140 I559P preparations shown in Figure 29a (and Figure 30, see below) was typical. Sometimes, higher amounts of trimer, up to 90% were observed.

There appeared to be a correlation between the expression of some SOS gp140 mutants and the extent to which they were oligomeric. Thus, substitutions at positions 559, 566 and 569 affected both the expression and the oligomerization of SOS gp140 (Table 2, Figure 29a). These residues may be particularly important for the correct folding of the trimeric, pre-fusion form of gp140 glycoproteins.

The above experiments demonstrate that the introduction of the helix-destabilizing residues Gly or Pro at position 559 increased the tendency of the SOS gp140 protein to form trimers. Hence this region of gp140 may not be helical in the pre-fusion configuration of the protein. In the influenza HA₂ protein, a loop-to-helix transition induced by exposure to low pH is essential for the formation of the fusion-intermediate, extended coiled-coil conformation (Bullough, 1994). Various proline substitutions in this particular region of HA₂ permit the production of proteolytically cleaved trimers. These mutants are fusion-impaired, however, because they cannot undergo the critical loop-to-helix transition (Qiao, 1998). It was therefore investigated whether proline substitutions at other residues near position 559 of HIV-1 gp41 could have the same, trimer-stabilizing effect as the I559P substitution. However, although most of these mutants could be efficiently expressed, none of them stabilized the trimeric form of SOS gp140 in the same manner as I559P substitution (Table 3, see below).

Various combinations of amino-acid substitutions were tested. Generally, in all the double or triple mutants that were evaluated, the presence of one or other of the I559P or I559G changes was essential to obtain the trimer-stabilized phenotype. A few other substitutions (e.g., L566V and T569P) had a marginally stabilizing effect on the SOS gp140 trimers.

**Table 2.**

| Summary of characteristics of variant SOS gp140 proteins. | | | | |
|---|---|---|---|---|
| **Residue** | **Mutation^{a}** | **Expxession^{b}** | **Trimer Stability^{c}** | **Cleavage^{d}** |
| | F | +/-(2/2) | +/-(2/2) | |
| L545 | N | ++(2/2) | +/-(2/2) | |
| | P | ++(2/2) | +/-(2/2) | |
| | G | ++(2/2) | +/-(2/2) | |
| | V | ++(2/2) | +/-(2/2) | |
| I548 | L | ++(2/2) | +/-(2/2) | |
| | H | ++(2/2) | +/-(2/2) | |
| | S | ++(2/2) | +/-(2/2) | |
| | G | ++(1/2) | +/-(2/2) | |
| | R | ++(1/2) | +/-(2/2) | |
| | V | --(2/3) | ND^{e} | |
| L555 | W | --(3/3) | ND | |
| | Y | --(3/3) | ND | |
| | S | --(3/3) | ND | |
| | P | --(4/4) | ND | |
| | V | +(1/3) | +/-(2/2) | |
| I559 | F | +/-(2/3) | ++(3/3) | |
| | N | ? | ++(1/1) | |
| | P | ++(6/6) | ++(6/6) | ± ± |
| | G | +(3/3) | ++(3/3) | ±± |
| | R | +(2/2) | ++(2/2) | |
| | V | +(3/3) | +(3/3) | ± ± |
| L566 | I | --? | ? | |
| | N | ++(3/3) | +(3/3) | |
| | T | +(2/2) | +(3/3) | |
| | P | +/- | - | |
| | K | +(2/2) | +(2/2) | |
| | S | +(2/3) | -?? | |
| T569 | P | +(2/3) | +(2/3) | |
| | K | +(2/3) | +(3/3) | |
| | E | --(3/3) | ND | |
| | L | ++(3/3) | +/-(3/3) | |
| I573 | F | ++(2/3) | +/-(3/3) | |
| | Y | ++(3/3) | +/-(3/3) | |
| | Q | ++(3/3) | +/-(3/3) | |
| | N | ++(3/3) | +/-(3/3) | |
| | T | | | |
| | P | | | |
| | G | | | |
| | K | ++(3/3) | +/-(3/3) | |
| | V | +? | +/-(2/2) | |
| L576 V580 | F | +? | +/-(2/2) | |
| | Y | +/-? | +/-(2/2) | |
| | Q | +/-? | +/-(2/2) | |
| | N | +/-? | +/-(2/2) | |
| | G | +/-? | +/-(2/2) | |
| | K | +/-? | +/-(2/2) | |
| | L | ++? | ? | |
| | H | +/-? | ? | |
| | T | +(2/2) | +/-(1/1) | |
| | P | +(2/2) | +/-(1/1) | |
| | G | --(2/2) | ? | |
| V583 | L | --(2/2) | ND | |
| | Q | --(2/2) | ND | |
| | N | --(2/2) | ND | |
| | S | --(2/2) | ND | |
| | P | --(2/2) | ND | |
| | R | --(2/2) | ND | |
| | K | --(2/2) | ND | |
| L587 | A | --(2/2) | ND | |
| | P | --(2/2) | ND | |
| | R | --(2/2) | ND | |
| | D | +/-(1/2) | -(1/1) | |
| | E | ++(1/2) | -(1/1) | |

| | | | | |
|---|---|---|---|---|
| ^{a}In SOS gp140. ^{b}Relative Scale: No expression (-); minimal expression (+/-); expression level less than wild-type SOS gp140 (++); expression level greater than wild-type SOS gp140 (+++). All proteins were expressed (Binley, 2000a; Binley, 2002; and Sanders, 2000). The data were derived from at least 3 independent transfections. ^{c}As assessed in BN-PAGE. The trimer stability of the SOS gp140 protein was set at (+/-) (some trimers were present in some transfections). The maximum amount of trimers (++) were only found in gp140 proteins containing substitutions at residue Iie-559, and ranged from 40%->90% of total Env expression in independent transfections. The data were derived from at least 3 independent transfections. ^{d}Data derived from Figure 29c. ^{e}Not determined (no expression). | | | | |

**Table 3**

| Summary of characteristics of variant SOS gp140 proteins | | | | |
|---|---|---|---|---|
| Residue | Mutation^{a} | Expression^{b} | Trimer Stability^{c} | Cleavage^{d} |
| L555 | P^{f} | --(4/4) | ND^{e} | |
| 556 | P | ++ | | |
| 557 | P | ++ | | |
| 558 | P | ++ | | |
| I559 | P^{f} | ++(6/6) | ++(6/6) | ++ |
| 560 | P | +++ | | |
| 561 | P | +++ | | |
| 562 | P | +++ | | |
| 563 | P | +++ | | |
| 564 | P | +++ | | |
| 565 | P | ++ | | |
| 566 | P^{f} | +/- | - | |

| | | | | |
|---|---|---|---|---|
| ^{a}In SOS gp140. ^{b}Relative Scale: No expression (-); minimal expression (+/-); expression level less than wild-type SOS gp140 (++); expression level greater than wild-type SOS gp140 (+++). All proteins were expressed (Binley, 2000a; Binley, 2002; and Sanders, 2000). The data were derived from at least 3 independent transfections. ^{c}As assessed in BN-PAGE. The trimer stability of the SOS gp140 protein was set at (+/-) (some trimers were present in some transfections). The maximum amount of trimers (++) were only found in gp140 proteins containing substitutions at residue Iie-559, and ranged from 40%->90% of total Env expression in independent transfections. The data were derived from at least 3 independent transfections. ^{d}Data derived from Figure 29c. ^{e}Not determined (no expression). ^{f}Also present in Table 2. | | | | |

### Stabilized SOS trimers form non-covalently and are cleaved

Based on the above results, the SOS I559P and I559G gp140 proteins were focused upon for further analysis. These proteins are designated herein as the SOSI559P and SOSI559G gp140 proteins, respectively. Although the SOSI559P gp140 protein was consistently expressed at higher levels than SOSI559G gp140, the presence of a glycine residue at position 559 might confer flexibility to the latter protein. It was considered that any such flexibility might prove useful if and when the I559G substitution were combined with other modifications. The SOS L566V and T569P gp140 proteins were also further studied to see whether similar results were obtained with trimers that had been stabilized, even to only a limited extent, by substitutions at positions other than residue 559.

To investigate whether the oligomeric species present in the preparations of the SOSI559P, SOSI559G, SOSL566V and SOST569P gp140 proteins were covalently or non-covalently associated, they were analyzed using denaturing, but non-reducing, SDS-PAGE (Figure 29b). The gp140UNC protein was included for comparison (Figure 29b, Lane 1). As expected, SOS gp140 migrated predominantly as a 140kDa species (Figure 29b, Lane 2). However, some higher molecular weight, SDS-resistant species were also present in both the SOS gp140 and the gp140UNC preparations. The higher molecular weight species were only a minor component of the SOS gp140 preparation, but they were the predominant form of the gp140UNC protein (Figure 29b, Lane 1). It is believed that the higher molecular weight forms of these proteins are predominantly covalently associated dimers and, in some cases, tetramers that could be dimers of dimers (see below).

The SOS gp140 variants were all indistinguishable from the wild-type SOS gp140 protein, in that the predominant species after SDS treatment were always 140kDa monomers (Figure 29b, Lanes 3-7). Thus, the trimeric forms of the SOSI559P and SOSI559G gp140 proteins are not created by the aberrant formation of intermolecular disulfide bonds. Instead, the protein is associated by non-covalent interactions.

The goal of the invention is to make stable, oligomeric gp140 proteins that are properly processed at the cleavage site between the gp120 and gp41 subunits. A determination was therefore made as to whether the SOSI559P, SOSI559G, SOSL566V and SOST569P gp140 variants were processed appropriately. The proteins were boiled in the presence of SDS and dithiothreitol (DTT), to achieve both denaturation and reduction, prior to SDS-PAGE analysis (Figure 29c). Under these conditions, each of the various SOS gp140 proteins was converted to gp120 and gp41ECTO forms (Figure 29c, and data not shown). Thus, each of the gp140 proteins was substantially (>90%) cleaved, in that the 140kDa bands did not survive DTT treatment (Figure 29c, Lanes 2-7). In contrast, the gp140UNC protein was unaffected by DTT and still migrated as a 140kDa band, because it possesses a peptide bond between the gp120 and gp41 subunits (Figure 29c, Lane 1) (Binley, 2000a). Thus, the increased trimer stability of the SOSI559P, SOSI559G, SOSL566V and SOST569P gp140 proteins is not caused by, or associated with, any cleavage defect.

Most of the SOS gp140 preparations analyzed by SDS-PAGE contained a small percentage of SDS-resistant oligomers, and these forms of gp140 were relatively abundant in the gp140UNC preparation (Figure 29b). Since these higher molecular weight forms of gp140 were not observed when DTT was also present to reduce disulfide bonds (Figure 29c), they presumably represent protein forms that are linked via aberrant, intermolecular disulfide bonds (Schulke, 2002). To determine whether the higher molecular weight proteins were dimers or trimers, the SOSI559G gp140 protein was treated with increasing amounts of SDS, and then a BN-PAGE analysis was performed (Figure 29d). In the absence of SDS, trimers, dimers and monomers were present at roughly equal proportions in this preparation of the SOSI559G gp140 protein (Figure 29d, Lane 2). As the SDS concentration increased, however, the trimer band completely disappeared, whereas the dimer band survived exposure to SDS concentrations even as high as 1% (Figure 29d; Figure 29b, Lane 4). The stronger intensity of the dimer band on the western blot is probably attributable to an increased reactivity of the detecting MAb once the gp140 protein has been denatured with SDS. The more pronounced increase in the intensity of the monomer band suggests that trimers dissociate to three monomers, rather than to a dimer and a monomer (see below). It was concluded that the SOSI559G gp140 trimers are formed by non-covalent, SDS-sensitive bonds, but that the dimers are associated via aberrant, intersubunit disulfide bonds. Similar results were obtained with the SOSI559P gp140 protein (Figure 32). The wild-type SOS gp140 protein could not, of course, be tested in this manner as its trimeric form was too unstable.

### Fractionation of oligomeric gp140 species by gel filtration

The BN-PAGE analyses showed that amino-acid substitutions, in particular at the 559 position, can stabilize SOS gp140 trimers. To corroborate this by an independent technique, gp140 proteins secreted from Env-transfected 293T cells were studied by analytical gel-filtration chromatography using a Superdex 200 column. Proteins with known molecular weight provided reference standards. These were catalase (232kDa), ferritin (440kDa) and thyroglobulin (669kDa). However, it should be noted that fully glycosylated gp120 and gp140 molecules are non-globular in shape, so gel filtration cannot precisely determine their absolute molecular weights (Center, 2000; Center, 2001; and Schulke, 2002).

The eluate fractions were collected and then analyzed by SDS-PAGE and Western blotting to identify the migration positions of various Env protein forms (Figure 30). The SOS gp140 protein was predominantly found in fractions Center, 2001, 14 and 15, corresponding to an apparent molecular weight similar to that of catalase (232kDa) (Figure 3a, top panel). Thus, the average apparent molecular weight of the eluted SOS gp140 monomer was -240kDa, which is consistent with the value of -220kDa reported previously, also using gel filtration (Schulke, 2002). The small amount of gp120 present in this preparation of SOS gp140 had a slightly lower apparent molecular weight of -220kDa (Figure 30a). In most preparations of SOS gp140, a small quantity of covalently linked oligomers, probably dimers, was also seen, centered around fractions 9 and 10 (data not shown). These results confirm the earlier finding that SOS gp140 is predominantly a monomeric protein (Schulke, 2002).

In contrast, the SOSI559P gp140 proteins eluted over a broad range in fractions 1-16, indicating that both oligomeric and monomeric species are present (Figure 30a, bottom panel). A small amount of covalently linked oligomers was also observed (see also Figure 29b, Lane 3), just as similar oligomers were usually present in SOS gp140 preparations (Figure 29b, Lane 2). To resolve the different oligomeric species, the same SOSI559P gp140 gel-filtration fractions were then analyzed by BN-PAGE. This showed that trimeric, dimeric and monomeric proteins had been clearly resolved on the Superdex 200 column. The trimers were predominantly in fractions 4-9, the dimers in fractions 7-11 and the monomers in fractions 11-15 (Figure 30b). Similar results were obtained with an SOS gp140 triple mutant containing the I559P, L566V and T569P mutations (data not shown).

The apparent molecular weight of the SOSI559P gp140 monomer corresponds to what was observed with wild-type SOS gp140 (-240kDa, see above). The retention of dimers, centered around fraction 9, corresponds to an average apparent molecular weight of ~410kDa, whereas the trimer (peak fraction 6) has an average apparent molecular weight of -520kDa. It is notable that the trimers do not elute in a position consistent with their expected size of three times the size of a gp140 monomer. Thus they elute at a position corresponding to a molecular weight of ~520kDa, as opposed to the "expected" ~660-720kDa (i.e., 3 x -220-240kDa). The same is true, to a lesser extent, of the dimers, which elute at ~410kDa, compared to the "expected" ~440-480kDa (i.e., 2 x ~220-240kDa). The explanation for this is probably that the trimers, and perhaps also the dimers, are folded into a conformation which is more compact than that of gp140 monomers. Electron microscopy studies may be able to confirm this view. Alternatively, the nature and extent of glycosylation of the different oligomeric forms of gp140 may vary, because glycosylation sites on the trimer could be less accessible to modifying enzymes than the same sites on the monomer. Overall, given the limitations of gel filtration for estimating the molecular weights of non-globular proteins (Center, 2000; Center, 2001; and Schulke, 2002) other techniques (e.g., mass spectrometry) will need to be used to establish the absolute molecular weights of the various, purified oligomeric species of SOS gp140 proteins.

### Stability of SOSI559P gp140 trimers

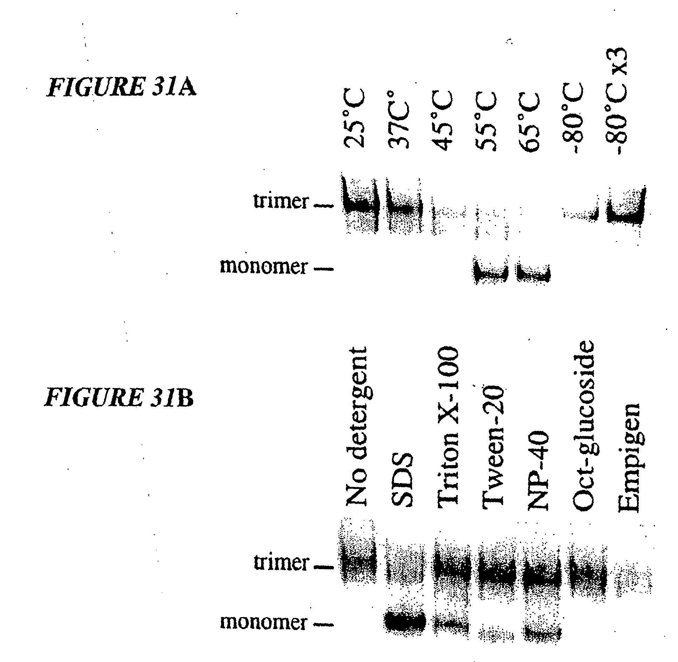

The Superdex 200 column fractions corresponding to the trimer peak of the SOS I559P gp140 protein (fractions 6 and 7) were pooled for analysis of their stability (Figure 31). The trimers were stable to incubation for 1 hour at 25°C and 37°C (Figure 31a, Lanes 1 and 2), but some monomers became visible after 1 hour at 45°C (Figure 31a, Lane 3) and almost all of the protein was in monomeric form after heating for 1 hour at 55°C or 65°C (Figure 31a, Lanes 4 and 5). Three freeze-thaw cycles at -80°C did not convert the trimer into a monomer (Figure 31a, Lane 7). Next, the fractionated trimers were incubated with various detergents for 1 hour at 25°C (Figure 31b). The trimers dissociated into monomers upon incubation with 0.1% SDS, an anionic detergent (Figure 31b, Lane 2), but they were at least partially resistant to the same concentration of the nonionic or zwitterionic detergents Triton X-100, Tween-20, NP-40, Octyl-glucoside and Empigen (Figure 31b, Lanes 3-7). It was also observed that SOSI559P gp140 trimers did not dissociate into monomers in the presence of NaCl concentrations of up to 1.0M, or after exposure to mild acid (pH 4.0) (data not shown).

Dimers were present at only very low levels in heat- or detergent-treated SOSI559P gp140 trimers. This suggests that the assembly units of the trimers are three equivalent monomers, rather than a monomer and a dimer.

### Antigenic structure analysis of the stabilized SOS gp140 proteins

Since the goal of the invention is to form cleaved, stable gp140 trimers that mimic as closely as possible the antigenic structure of virion-associated Env, the reactivity of the unpurified, SOSI559P gp140 protein was studied with a panel of MAbs and CD4-based reagents. The SOS gp140 and gp140UNC proteins were also studied, for comparison (Figure 32). Both the wild-type SOS gp140 and the SOSI559P gp140 proteins were immunoprecipitated by the CD4-IgG2 molecule, indicating that the CD4-binding site (CD4BS) was intact on the gp120 subunits of both proteins (Figure 32, Lane 2). The neutralizing MAb IgG1b12 to a CD4BS-associated epitope also bound to both proteins efficiently, as did the neutralizing MAb 2G12 to a mannose-dependent gp120 epitope (Sanders, 2002; and Trkola, 1996b) (Figure 32, Lanes 1 and 3). Furthermore, sCD4 induction of the 17b epitope was highly efficient in both the SOS and SOSI559P gp140 proteins (Figure 32, Lanes 4 and 5). This epitope overlaps the CD4-inducible, co-receptor binding site on gp120 (Rizzuto, 1998; Thali, 1993; Wyatt, 1998a; and Wyatt, 1995). Thus, the I559P substitution in gp41ECTO does not affect the ability of the gp120 subunits of an SOS gp140 protein to bind the CD4 receptor, and to then undergo receptor-mediated conformational changes within the gp120 subunits. The SOSI559P gp140 protein appears indistinguishable from the wild-type SOS gp140 protein in this regard.

There are three predominant epitope clusters in gp41ECTO. One cluster is recognized by the neutralizing MAbs 2F5, 4E10 and z13, and is located close to the C-terminus of gp41ECTO (Parker, 2001; Stiegler, 2001; and Zwick, 2001) (Figure 28a). This region of gp41ECTO is well exposed on the SOS and SOSI559P gp140 proteins, as indicated by their efficient binding of 2F5 (Figure 32, Lane 6) and 4E10 (data not shown). The cluster I and cluster II gp41ECTO epitopes are highly immunogenic. However, antibodies to these regions of gp41 are non-neutralizing because their epitopes are occluded in the native, pre-fusion form of the envelope glycoprotein complex, either by interactions between gp41ECTO moieties or because of the presence of the gp120 subunits (Binley, 2000a; Jiang, 1998; Moore, 2001; Nyambi, 1998; Parren, 1999; Robinson, 1990; Sattentau, 1995b; and Taniguchi, 2000). MAbs to cluster I (2.2B) and cluster II (4D4) epitopes interact with the gp140UNC protein efficiently but do not bind to the SOS or SOSI559P gp140 proteins (Figure 32, Lanes 7 and 8). Similar results were obtained with other MAbs to these epitope clusters (data not shown). This pattern of results is consistent with previous reports on the antigenic structure of SOS gp140 (Binley, 2000a; Sanders, 2000; and Schulke, 2002). It is possible that the introduction of the cysteine substitution at residue 605 in the SOS gp140 proteins directly perturbs the nearby epitopes for some or all of the cluster I MAbs. However, this cannot be the case for the cluster II MAb epitopes, since these are located in the C-terminal helical region, approximately 40 residues from the Cys-605 substitution (Figure 28a).

The SOS gp140 variants I559G, L566V and T569P were also tested for reactivity with the above MAbs and CD4-based reagents. Each of them behaved similarly to the SOS and SOSI559P gp140 proteins (data not shown).

### Destabilization of the 6-helix bundle form of gp41

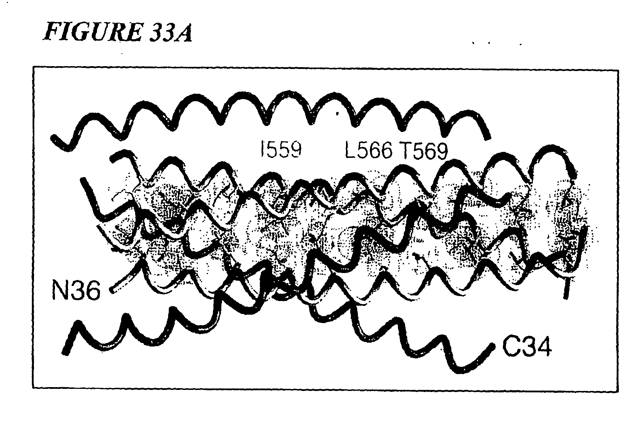

The mutagenesis results obtained indicate that the SOS gp140 trimers can be stabilized by the I559G, I559P and, to some extent, L566V and T569P substitutions in the N-terminal heptad-repeat region of the gp41ECTO subunit. Given that hydrophobic interactions are a dominant factor in the stabilization of the gp41 core (Jelesarov, 2001 and Lu, 1999), it would appear that these amino-acid substitutions destabilize the 6-helix bundle structure. To directly test this hypothesis, the effects of each of the four amino-acid substitutions were determined on the overall structure and stability of the JR-FL gp41ECTO core. Therefore, a recombinant peptide model of this soluble gp41 core was constructed. This model peptide, designated N36(L6)C34, consists of the N36 and C34 peptides connected via a short peptide linker that replaces the disulfide-bonded loop region of gp41ECTO (Figure 33a) (Lu, 1999). The N36 peptide consists of residues 546-581, whereas the C34 peptide consists of residues 628-661 of the JR-FL gp41 protein sequence. Each of the above four amino-acid changes was introduced into the N36(L6)C34 peptide. Sedimentation equilibrium analysis showed that the molecular weights of N36(L6)C34 variants, except for the I559P mutant, were all within 10% of those calculated for an ideal trimer, with no systematic deviation of the residuals (data not shown).

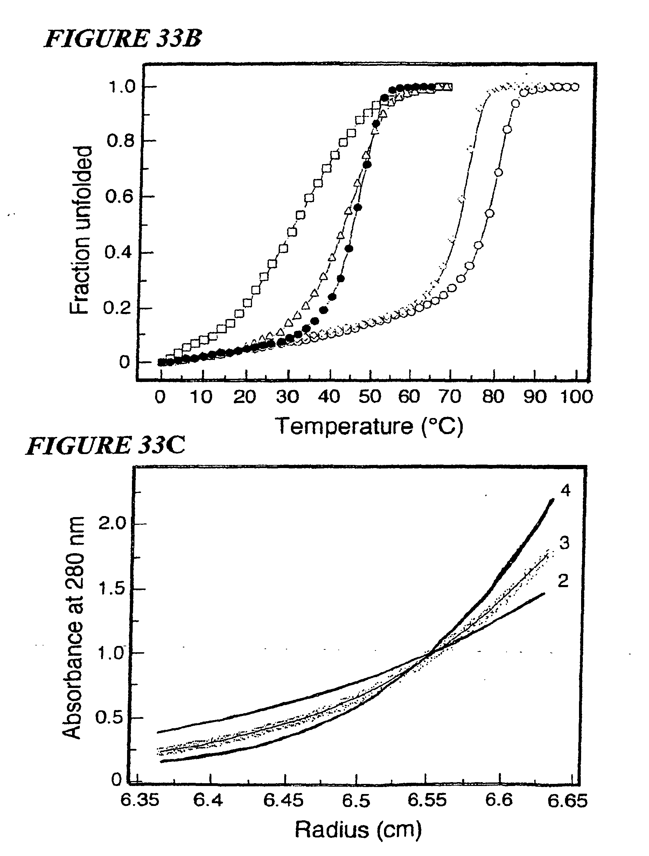

Circular dichroism (CD) measurements indicated that the N36(L6)C34 wild-type, and the I559G, L566V and T569P variant peptides were each >95% -helical at 4°C, whereas the I559P variant peptide was apparently only -75% - helical (Table 4, see below). Under these conditions, the midpoints of thermal denaturation (*T*ₘ's) of the I559G, I559P, L566V, and T569P peptides are 46, 34, 72 and 44°C, respectively, as compared to a *T*ₘ of 78°C for the wild-type peptide (Table 4). The pre- and post-transitional slopes and the steepness of the main transition are very similar for the N36(L6)C34, I559G, and L566V peptides (Figure 33b). In contrast, the I559P and T569P peptides display broad thermal unfolding transitions (Figure 33b). Sedimentation equilibrium experiments indicate that the N36(L6)C34 peptide and its I559G, L566V and T569P variants each sediment as discrete trimers over a ten-fold range of peptide concentration (10 to 100µM) (Table 4; Figure 33c). The I559P peptide is also trimeric in solution at concentrations below 15µM, but it exhibits a systematic deviation from the trimer molecular weight between 10 to 100µM, indicating that I559P is prone to aggregation. Taken together, these results indicate that the Ile-559 to Gly and Thr-569 to Pro substitutions each lead to an appreciable destabilization of the 6-helix bundle structure, but do not affect its overall fold. In contrast, the Ile-559 to Pro substitution essentially disrupts the 6-helix bundle formation. Moreover, the Leu-566 to Val change is associated with a small, unfavorable, residual destabilization of the 6-helix bundle.

**Table 4**

| Biophysical characterization of JR-FL gp41 core mutants | | | |
|---|---|---|---|
| | *-[*θ*] _{222 nm}^{a}* (deg cm² dmol⁻¹⁾ | *Tₘ^{a}* (°C) | *M_{obs}*/*M_{calc}^{b}* |
| N36(L6)C34 | 32,500 | 78 | 3.1 |
| I559G | 32,300 | 46 | 2.9 |
| I559P | 25,600 | 34 | ND^{c} |
| L566V | 32,100 | 72 | 3.1 |
| T569P | 29,400 | 44 | 3.0 |

| | | | |
|---|---|---|---|
| ^{a}All CD scans and melts were performed on 10µM peptide solutions in PBS (pH 7.0). The CD signal at 222nm ([θ]₂₂₂ₙₘ) is reported. The midpoint of thermal denaturation (*T_{M}*) was estimated from the thermal dependence of the CD signal at 222nm. ^{b}Sedimentation equilibrium results are reported as a ration of the experimental molecular weight to the calculated molecular weight for a monomer (M_{obs}/M_{calc}). ^{c}aggregated, as determined by sedimentation equilibrium. | | | |

Overall, these experiments confirm that the I559P, I559G, L566V and T569P substitutions do, in fact, destabilize the 6-helix bundle, post-fusion conformation of gp41ECTO. Indeed, the I559P change appears sufficient to completely prevent the formation of the post-fusion state, by destabilizing the N-terminal helix. The results obtained using model peptides are therefore consistent with what was observed when the corresponding amino acid substitutions were introduced into the SOS gp140 protein. Moreover, both sets of results support the underlying hypothesis that destabilizing, or otherwise preventing the formation of the 6-helix bundle form of gp41ECTO helps maintain the gp140 protein in its native, trimeric, pre-fusion configuration.

The trimer-stabilizing substitutions described herein simplify the production of significant amounts of cleaved Env trimers. These materials are useful in vaccine design and for structural studies.

### Experimental Set V

### A. Synopsis

In virus-infected cells, the envelope glycoprotein (Env) precursor, gp160, of human immunodeficiency virus type 1 (HIV-1) is cleaved by cellular proteases into a fusion-competent gp120/gp41 heterodimer in which the two subunits are non-covalently associated. However, cleavage can be inefficient when recombinant Env is expressed at high levels, either as a full-length gp160 or as a soluble gp140 truncated immediately N-terminal to the transmembrane domain. We have explored several methods for obtaining fully cleaved Env for use as a vaccine antigen. We tested whether purified Env could be enzymatically digested with purified protease in vitro. Plasmin efficiently cleaved the Env precursor, but also cut at a second site in gp120, most probably the V3 loop. In contrast, a soluble form of furin was specific for the gp120/gp41 cleavage site, but cleaved inefficiently. Co-expression of Env with the full-length or soluble forms of furin enhanced Env cleavage but also reduced Env expression. When the Env cleavage site (REKR) was mutated to see if its use by cellular proteases could be enhanced, several mutants were more efficiently processed than the wild-type protein. The optimal cleavage-site sequences were RRRRRR, RRRRKR and RRRKKR. These mutations did not significantly alter the capacity of the Env protein to mediate fusion, so did not radically perturb the Env structure. Furthermore, unlike wild-type Env, expression of the cleavage-site mutants was not significantly reduced by furin co-expression. The co-expression of Env cleavage site mutants and furin is therefore a useful method for obtaining high-level expression of processed Env.

### B. Introduction

The Env glycoprotein complex mediates receptor binding and membrane fusion during human immunodeficiency virus type 1 (HIV-1) infection of susceptible cells (Poignard, 2001). It is synthesized as a polypeptide precursor (gp160) that oligomerizes to form a heavily glycosylated trimer (Doms, 1993; and Earl, 1994). At a late stage of synthesis, most probably in the trans-Golgi network (TGN), gp160 is cleaved by furin (Decroly, 1994; Decroyly, 1996; Morikawa, 1993, Moulard, 1998; Moulard, 1999; and Moulard, 2000) or other, related subtilisin-like proteases (Decroly, 1994; Decroyly, 1996; Franzusoff, 1995; Inocencio, 1997; Moulard, 2000; and Vollenweider, 1996) into the surface (SU; gp120) and transmembrane (TM; gp41) subunits (Hallenberger, 1997; Kozarsky, 1989; Morikawa, 1993; Moulard, 1998; Moulard, 1999; Moulard, 2000; and Stein, 1990). Cleavage occurs at a motif at the gp120-gp41 juncture that contains a basic amino acid residue tetramer, R-X-R/K-R (where X is any amino acid). The gp120 and gp41 proteins then remain non-covalently associated, forming the functional, native (gp120-gp41)₃ complex (Doms, 1993; Earl, 1994; and Poignard, 2001).

During fusion, the gp120 protein interacts with the virus receptor and co-receptor on target cells. This triggers conformational changes that lead to the insertion of a hydrophobic fusion peptide, located at the N-terminus of gp41, into the target cell membrane (Poignard, 2001). Cleavage of gp160 is essential for fusion, since uncleaved gp160 is fusion-incompetent (Bosch, 1990; Guo, 1990; Iwantani, 2001; and McCune, 1988). Generally, only cleaved Env is incorporated into virions (Dubay, 1995), although uncleaved Env can be virion-associated (Iwantani, 2001; and McCune, 1988). By analogy with other enveloped viruses such as influenza A (Bender, 1999; Goto, 1998; Horimoto, 1995; Kawaoka, 1991; Kawakowa, 1988; Klenk, 1994; and Ohuchi, 1989), Semliki forest virus (Ferlenghi, 1998; and Salminen, 1992) and Newcastle disease virus (Sergel, 2001), gp160 cleavage may induce a shift from a low-energy state to a metastable Env configuration that is capable of fusion. The common requirement for cleavage of an Env precursor in many families of enveloped viruses is an indication of the general importance of this event in virus assembly (Bolt, 2000; Ferlenghi, 1998; Klenk, 1994; Sakurai, 1999; Stadler, 1997; Sugrue, 2001; Volchkov, 1998; and Weidmann, 2000).

HIV-1 Env is the focus of vaccine design strategies intended to elicit virus-neutralizing antibodies. To neutralize HIV-1, an antibody must be able to bind to the native, trimeric virus-associated Env complex (Burton, 1997; Burton, 2000; Parren, 1997; and Parren, 2001). Most Env-based vaccine candidates tested to date have been either monomeric gp120 subunits or various forms of the uncleaved gp160 or gp140 (gp120 plus gp41 ectodomain) precursor proteins (Barnett, 2001; Earl, 2001; Farzan, 1998; Hu, 1991; Ly, 2000; Richardson, 1996; Stamatatos, 2000; Yang, 2000a; Yang, 2000b; and Yang, 2001). The use of uncleaved gp140 or gp160 proteins has been considered necessary because the labile, non-covalent gp120-gp41 association in cleaved Env leads to the dissociation of gp120 from gp41 (Gelderblom, 1985; McKeating, 1991; Moore, 1990; Schneider, 1986). However, gp120, uncleaved gp140 and gp160 proteins do not fully mimic the structure of the native trimeric Env complex. As a result, antibodies elicited to gp120 and uncleaved Env proteins can sometimes neutralize the homologous HIV-1 isolate, but generally do not cross-neutralize heterologous primary isolates (Barnett, 2001; Earl, 2001; Richardson, 1996; and Yang, 2001).

Effects of cleavage on the overall structure of viral proteins have been studied in various viruses. Cleavage of the influenza A hemagglutinin precursor (HA₀), causes only localized refolding with little impact on its overall structure (Chen, 1998). However, it is not clear how precise a model influenza A HA₀ is for HIV-1 gp160: The two viruses are distant relatives, and their fusion potential is triggered by quite different mechanisms, so it may not be appropriate to extrapolate what that has been learned from HA₀ to predict all aspects of gp160 structure and function. Indeed, uncleaved HIV-1 gp140 proteins are antigenically and, by implication structurally, different from cleaved proteins (Binley, 2000a). Moreover, the Env proteins of several other enveloped viruses exhibit dramatic refolding of their envelope proteins upon cleavage (Dutch, 2001; Ferlenghi, 1998; Heinz, 1995; Paredes, 1998; Salminen, 1992; Schalich, 1996; Sergel, 2001; and Stadler, 1997). Thus, the projecting domains of the trimeric spike precursor of Semliki Forest virus (SFV) coalesce to form a compact, mature spike (Ferlenghi, 1998; and Salminen, 1992). The structures of the mature forms of the tick-borne encephalitis virus (TBEV) E protein and the SV5 paramyxovirus F protein, as probed by antibodies, appears to be significantly different from the immature form (Dutch, 2001; Heinz, 1995; Schalich, 1996; and Stadler, 1997). Of note is that antibodies against the heptad repeat regions of the transmembrane domain of the SV5 F protein only recognized the uncleaved form (Dutch, 2001). Overall, whether the above examples represent better paradigms than HA₀ for the structural impact of cleavage on HIV-1 Env is unknown, but clearly they support further analysis of cleaved forms of HIV-1 Env.

Mimicking the native structure of Env may be a useful HIV-1 vaccine design strategy. The production of a native Env complex as a recombinant protein has, however, been hampered by the limited efficiency of Env cleavage (Binley, 2000a; Morikawa, 1993; Moulard, 1999; Moulard, 2000; Vollenweider, 1996; and Yamshchikov, 1995), and by the instability of the complex after cleavage has occurred (Gelderblom, 1985; McKeating, 1991; Moore, 1990; and Schneider, 1986). The [gp120-gp41] association in cleaved forms of Env can be stabilized by the introduction of appropriately positioned cysteine residues that form an intermolecular disulfide bond between gp120 and gp41 (Binley, 2000a). However, to achieve full cleavage of the gp140 precursor in Env-transfected cells, it was necessary to co-express furin (Binley, 2000a). A disadvantage of this approach is that furin co-expression significantly reduced Env expression (Binley, 2000a; Morikawa, 1993; Moulard, 1999; Moulard, 2000; and Yamshchikov, 1995). Moreover, cleavage of some Env proteins was still not complete even with furin co-expression (Decroly, 1994; Decroly, 1996; Morikawa, 199; Moulard, 2000; and Yamshchikov, 1995). Changes in the gp120 variable loops (Sanders, 2000; and Travis, 1992), elsewhere in Env (Dedera, 1992; Merat, 1999; Syu, 1991; Tschachler, 1990; and Willey, 1991), and at residues proximal to the cleavage site (Adams, 2000; Freed, 1989; Guo, 1990; and Syu, 1991), can all affect Env cleavage efficiency, usually unpredictably. Overall, cleavage efficiency is a function of the folding, oligomerization and glycosylation of gp160, factors that influence the access of furin to its binding site at the gp120-gp41 juncture.

### C. Materials and Methods

*Plasmids and mutagenesis.* The pPPI4 plasmid that expresses soluble gp140 lacking the transmembrane and intracytoplasmic domains of gp41 has been described elsewhere (Binley, 2000a; Sanders, 2000; and Trkola, 1996a). Unless specified otherwise, the Env glycoproteins expressed in this study were derived from the HIV-1_{JR-FL} molecular clone, a subtype B, R5 primary isolate. However, we also expressed gp140 proteins from the molecular clones HXB2, 89.6, 89.6_{KB9}, DH123 and Gun-1_{WT}, as previously described (Binley, 2000a), and from a South African isolate, DU151, using a pT7blue-based source plasmid provided by Drs. Lynn Morris, Carolyn Williamson and Maria Papathanopoulous (National Institute of Virology, Johannesburg, South Africa). The gp140 proteins from SIV_{mac} and SIVₘₙₑ were expressed in a similar manner to HIV-1_{JR-FL}. Some of the above gp140 proteins were also made as mutants that contained cysteine substitutions designed to introduce an intermolecular disulfide bond between gp120 and gp41; the positioning of this disulfide bond corresponds to the one introduced into JR-FL gp140, to make the protein designated as SOS gp140 (Binley, 2000a). Wild-type gp140 proteins that lack the SOS mutations but retain the native gp120-gp41 cleavage site are denoted gp40WT. Other gp140 proteins were mutated to replace the wild-type gp120-gp41 cleavage site REKR and are designated as follows: gp140_{RRRKKR}, gp140_{RRRRKR}, gp140_{RRRRRR}, gp140_{KKRKKR} and gp140_{RERRRKKR}. All amino-acid substitutions were made using the Quickchange^{™} site-directed mutagenesis kit (Stratagene Inc) using appropriate primers. The plasmid pSV was used to express full-length JR-FL gp160 for infectivity and fusion assays (Dragic, 1996). Mutants of this protein were constructed and named analogously to the pPPI4 gp140 mutants. Vesicular stomatitis virus (VSV) G protein was also expressed by the pSV plasmid (Dragic, 1996). Furin was expressed from the plasmid pcDNA3.1-Furin as previously described (Binley, 2000a). A stop codon was introduced within the furin gene in place of that for residue E-684, to make the plasmid pCDNA-furin∂TC. This mutation truncates furin close to the C-terminal end of its ectodomain, leading to the expression of a secreted, active form of furin (Plaimauer, 2001). A pGEM furin source plasmid was obtained from Dr. Gary Thomas and Sean Molloy (Vollum Institute, Portland, OR) (Molloy, 1992; and Molloy, 1994).

*Anti-HIV-1 and SIV antibodies and sera*. Monoclonal antibody (MAb) B12 recognizes an epitope in the C2 domain of gp120 that is preferentially exposed on denatured forms of the molecule (Abacioglu, 1994). This was provided by Dr. George Lewis (Institute of Human Virology, Baltimore, MD). MAb 2F5 recognizes a neutralizing epitope in the C-terminal region of the gp41 ectodomain (Muster, 1993), and was provided by Dr. Hermann Katinger (Polymun Scientific Inc., Vienna, Austria). Purified SIV immune globulin (SIVIG) was purified from the serum of SIVmac251-infected rhesus macaques as previously described (Binley, 2000b). Purified human immune globulin from HIV-1-infected people (HIVIG) was obtained from Dr. John Mascola (Vaccine Research Center, NIH, Washington, DC).

### Transfection, immunoprecipitation and Western blotting.

Transfection and metabolic labeling of 293T cells and immunoprecipitations were performed as described previously (Binley, 2000a; and Sanders, 2000) using HIVIG or SIVIG to precipitate the labeled HIV-1 or SIV proteins, respectively. Ten micrograms of each plasmid were used for transfections in 10cm cell culture plates, unless otherwise stated. In other experiments, purified gp140 proteins were analyzed by denaturing SDS-PAGE and Western blotting, using either MAb 2F5 or MAb B12 as probes (Binley, 2000a; and Sanders, 2000).

### Measurement of Env expression and cleavage efficiency.

Densitometry measurements were performed using ImageQuant™ and NIH Image software. Env cleavage efficiency was calculated by the following formula (density of gp120 band)/(combined density of gp120+gp140 bands or gp120+gp160 bands), after subtracting the background density in each case. The values obtained were reproducible for each protein within a 6% deviation from the value presented in each case. Env protein expression levels were calculated by combining the densities of the gp120+gp140 or gp120+gp160 bands and subtracting the background density. In each gel, expression is recorded as a ratio relative to the standard used for normalization in that particular experiment.

*Vaccinia viruses.* Viruses v-VSE5 (expressing full length SIVₘₙₑ Env under control of the 7.5K promoter) and v-VS4 (expressing SIVₘₙₑ gag-pol under the control of the 11K promoter) have been described previously (Polacino, 1999). The vaccinia recombinant VV:hfur, expressing full length human furin, was obtained from Drs. Gary Thomas and Sean Molloy (Molloy, 1994). For protein production, BSC40 cells were infected at a multiplicity of infection (MOI) of 5. Supernatant proteins metabolically labeled with [³⁵S]-cysteine and [³⁵S]-methionine were collected 2 days later. Samples were processed in a similar manner to the transfected cell supernatants above.

*Purified Env proteins, enzymes and in vitro enzymatic digestion.* Purified human furin was purchased from Affinity Bioreagents Inc. (Golden, CO). This is a soluble form of furin with the transmembrane and cytoplasmic tail removed. The specific activity of 1 unit of furin is the amount required to release 1pmol of fluorogenic substrate peptide in 1 minute. Purified human plasmin was purchased from Sigma Chemical Co. For determining optimal digestion conditions, a highly purified SOS gp140 protein was used (prepared by Progenics Pharmaceuticals Inc.). This particular, early production batch of protein was approximately 50% cleaved. As a control, JR-FL gp120 produced and purified in the same manner was used (Trkola, 1996a). For plasmin digestions, 8µg (approximately 60pmol) of SOS gp140 or gp120 was incubated at 37°C with 200pmol (approximately 0.2U) of plasmin in 0.1M Tris-HCl pH 7.0 in a total volume of 80µl. For furin digestions, 8µg (approximately 60pmol) of SOS gp140 was incubated at 37°C with 20U of furin in 100mM HEPES, 1mM CaCl₂, pH 7.5 in a total volume of 80µl. The digests were then analyzed by SDS-PAGE and Western blot.

*Viral infectivity and cell-cell fusion assays.* Pseudotyped luciferase reporter viruses were produced by calcium phosphate transfection. Thus, 293T cells were co-transfected with 5µg of the Env-deficient NL4-3 HIV-1 virus construct pNL-luc and with 15µg of a pSV vector expressing either the full-length JR-FL Env glycoproteins or the positive control VSV-G protein (Dragic, 1996). The pNL-luc virus carries the luciferase reporter gene. The pSV plasmids expressed either wild-type gp160 (gp160WT) or a mutant with a cleavage site modified from REKR to RRRRRR, designated as JR-FL gp160_{RRRRRR}. The supernatants containing pseudotyped viruses from transfected cells were harvested after 48 hours and filtered through a 0.45µm filter. The viral stocks were then standardized for p24 protein content by ELISA (Dragic, 1996), and infections were performed using HeLa-CD4-CCR5 cells. Infectivity was expressed as light units per nanogram of p24 protein in the viral inoculum (Dragic, 1996).

Cell-cell fusion activity was measured using a fluorescent cytoplasmic dye transfer assay, as described elsewhere (Melikyan, 2000). Briefly, 293T cells on a 6cm dish were transfected with 10µg of the pSV7D vector expressing full-length JR-FL Env, then labeled with 1.5µM calcein AM (Molecular Probes, Inc., Eugene, OR) in 2mL of phosphate buffered saline (PBS), according to the manufacturer's instructions. Cells were detached from the dish by incubating in PBS supplemented with 0.5mM EDTA and 0.5mM EGTA, then transferred into a centrifuge tube. Approximately 5x10⁶ CEM.NKR.CCR5 cells (Trkola, 1999) were suspended in 2 ml of Opti-MEM (Gibco) containing 100µM 7-amino-4-chloro methylcoumarin (CMAC, Molecular Probes) and incubated for 30 min at 37°C. After extensive washing to remove the remaining free dye, the effector and target cells were mixed, transferred into poly-lysine coated, 8-well chambered slides and incubated for 2 hours at 37°C. The extent of fusion was determined by fluorescence video microscopy by normalizing the number of fusion products (stained with both cytoplasmic markers) against the number of target cells that were in contact with the effector cells.

### D. Results

### Enzymatic processing of purified, uncleaved SOS gp140

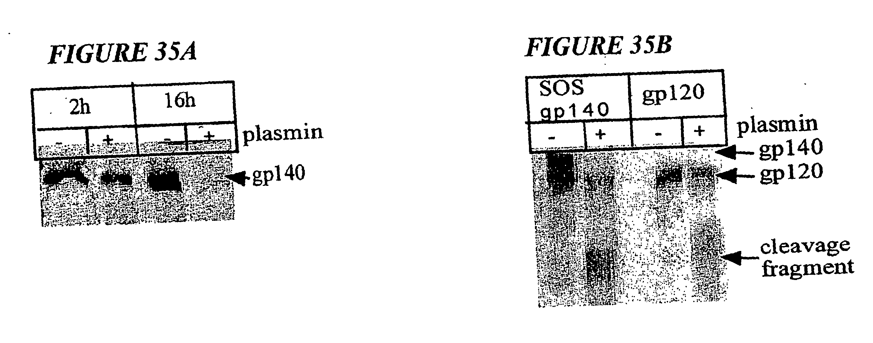

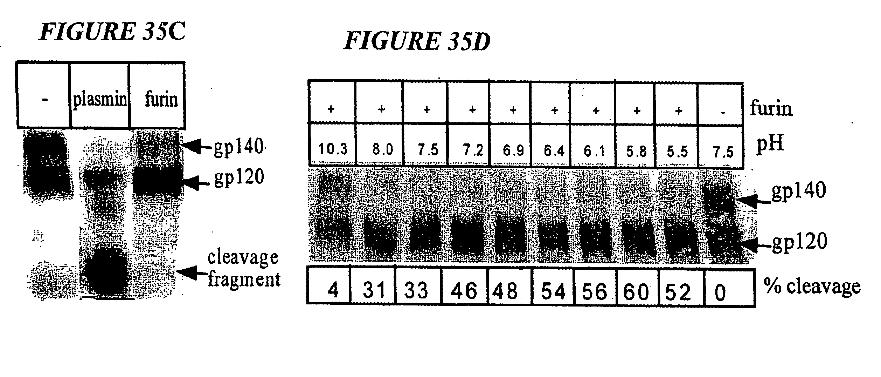

In principle, one way to achieve Env cleavage is to treat purified Env proteins in vitro with proteases capable of recognizing the gp120-gp41 cleavage site. The highly active subtilisin-family protease plasmin cleaves recombinant gp160 into gp120-gp41, whereas other trypsin-like proteases lacked this ability (Okumura, 1999). Plasmin is also capable of processing influenza HA₀ at the cell surface (Goto, 1998). We therefore evaluated the effect of plasmin on a preparation of purified, soluble SOS gp140 that was 50% cleaved. The partially cleaved SOS gp140 preparation was incubated with an excess of plasmin for 2 hours or 16 hours at 37°C, and the proteins were analyzed by SDS-PAGE and Western blotting using the 2F5 anti-gp41 MAb (Figure 35). After 2 hours of plasmin treatment, there was a reduction in the intensity of the uncleaved gp140 band, but the longer reaction time (16 hours) was required for processing to be complete (Figure 35a). This is consistent with the previous report on gp160 cleavage by plasmin (Okumura, 1999). However, when a Western blot of the 16 hours plasmin digest was probed with the gp120-specific MAb, B12, it was clear that plasmin also digests gp120 into fragments, one of which is of about 70kDa (Figure 35b). MAb B12 recognizes an epitope in the second conserved domain of gp120, N-terminal to the V3 loop (Abacioglu, 1994). Thus, plasmin cleaves gp120 internally, most likely at the site in the V3 loop that is a substrate for other tryptic proteases and which typically yields 50kDa and 70kDa fragments (Clements, 1991; McKeating, 1991; and Schulz, 1993). Although plasmin does process the gp120-gp41 cleavage site, the use of this enzyme to enhance Env cleavage is not, therefore, a practical technique.

We next investigated whether soluble furin would cleave gp140 efficiently but with greater specificity. During a 16 hour incubation, soluble furin significantly, albeit incompletely, cleaved SOS gp140 into gp120 without causing additional gp120 degradation (Figure 35c, compare Lanes 1 and 3). The efficiency of cleavage of gp140 by soluble furin was low, as shown by the following calculation: One unit (U) of soluble furin can process 1pmol of fluorogenic peptide substrate in 1 minute (Angliker, 1995). If gp140 were an equally efficient substrate, the 8µg of gp140, containing approximately 4µg (30pmol) of uncleaved gp140, would be digested by 20U of furin within 2 minutes. However, only 50% of the gp140 was actually processed after 16 hours. If we assume the rate of processing was uniform over this period, gp140 was cleaved at 0.7fmol/min; i.e. gp140 is -1000-fold less efficiently cleaved by furin than are model peptides.

The pH of the furin digest may affect its efficiency. For example, the mildly acidic pH of the exocytic pathway alters the structure of the TEBV Env precursor to permit an increase in cleavage efficiency (Stadler, 1997). Hence furin is able to cleave the TEBV Env precursor in vitro at pH 6.2, but not at pH 7.5 (Stadler, 1997). Furthermore, NH₄Cl treatment of cells, which raises the pH of the secretory pathway, can interfere with HIV-1 Env processing (Willey, 1988). We therefore investigated whether a mildly acidic pH might allow more efficient cleavage of gp140 by soluble furin during a 16 hour incubation. The optimal pH for Env cleavage was found to be 5.8 (Figure 35d), in contrast to a report that furin was most active (>80%) at a pH in the range 6.5 to 8 (Decroly, 1996). However, even at pH 5.8, gp140 was only cleaved by about 60% (Figure 35d), so optimizing the reaction pH was insufficient to achieve complete cleavage. Overall, we conclude that because a large excess of furin is required to achieve only a modest increase in gp140 cleavage efficiency, this also is not a practical technique for routine use.

### Recombinant Env proteins are incompletely processed by cellular proteases; effect of co-expressing furin and Env

We examined the extent of endogenous gp120-gp41 cleavage of seven HIV-1 and four SIV gp140 proteins by immunoprecipitation using HIVIG or SIVIG, as appropriate. Although the cleavage site (REKR) was conserved among all seven HIV-1 isolates, the SOS gp140 cleavage efficiency (defined under Materials and Methods) varied from 38-58%, and in no case was cleavage complete (Figure 36a). Similar results were obtained with the corresponding seven HIV-1 gp140WT proteins that lack the SOS cysteine substitutions (data not shown). These proteins are secreted as a mixture of gp120 and uncleaved gp140 despite retaining the REKR cleavage site, because proteolysis is inefficient in the absence of co-transfected furin (Binley, 2000a). The cleavage efficiency was generally slightly higher for each gp140WT protein than for the corresponding SOS gp140 mutant (data not shown).

Incomplete cleavage was also observed with the SIV_{mac251} and SIVₘₙₑ SOS gp140 proteins and the SIVₘₙₑ gp140WT protein, each of which has an RNKR cleavage site motif. In contrast, cleavage of the SIV_{mac251} gp140WT protein was almost complete (Figure 36a). Since the cleavage site motif in the latter protein is identical, indirect factors such as differences in folding must influence cleavage efficiency.

### Co-expression of furin reduces the expression of Env proteins

We next examined the effects of co-expressing furin with JR-FL SOS gp140 and gp140WT proteins, since this has previously been shown to increase cleavage efficiency (Binley, 2000a; Morikawa, 1993; and Yamshchikov, 1995). Varying amounts of the full-length, furin-expressing plasmid, pCDNA-furin, were co-transfected with a constant amount of Env-expressing plasmid (Figure 36b). The expression of sufficient furin resulted in almost complete (>90%) cleavage of both forms of gp140 protein, but it also caused a significant reduction in overall Env expression as measured by a decrease in the combined intensity of the gp140 and gp120 bands.

To verify the effect of Env and furin co-expression using a different form of Env protein and a different expression system, we expressed SIVₘₙₑ E11S gp160 in BSC40 cells from a recombinant vaccinia virus, both alone and together with Gag and Pol. Co-expression of Env with Gag-Pol enabled us to examine the efficiency with which full-length, membrane-bound gp160, secreted as pseudovirions, was cleaved into gp120/gp41 complexes (Figure 36c). The expression of SIVₘₙₑ gp160 was approximately 10-fold higher after vaccinia virus v-VSE5 infection than after transfection of the pPPI4-based plasmid encoding the identical gp160 (data not shown). In the absence of furin, gp160 cleavage was very low (Figure 36c, Lanes 1 and 3). The extent of cleavage was increased only modestly by furin co-expression, but there was a substantial reduction in the overall expression of Env (Figure 36c, Lanes 2 and 4). Indeed, when Gag-Pol and Env were co-expressed along with furin, the Env proteins were barely detectable (Figure 36c, Lane 2). Gag-Pol was also immunoprecipitated at diminished levels when furin was co-expressed (Figure 36c, Lane 2). This may be because the precipitation of Gag-Pol from pseudovirions occurs indirectly via antibody reactivity with surface Env, and Env expression is reduced by furin. Alternatively, this could be explained by nonspecific competition for expression of proteins from the various plasmids. Some full-length gp160 was present in the supernatant even in the absence of co-expressed Gag-Pol (Figure 36c, Lanes 3 and 4). This may be associated with cellular vesicles (Gluschankof, 1997) or could have been released from dead cells.

Overall, it is clear that furin expression has qualitatively similar effects on both gp140 and gp160 proteins irrespective of the expression system. Moreover, the increase in Env expression in the vaccinia virus system is associated with a further reduction in the extent of Env cleavage.

### Influence of the furin substrate sequence on Env expression in the presence of co-expressed furin

The co-expression of furin reduces the expression of several furin substrates, perhaps due to the complexing and retention of the nascent proteins with furin in the TGN rather than to any overtly toxic effect of furin on the cells (Molloy, 1994; Moulard, 2000; and Staropoli, 2000). To investigate this, we determined whether the reduction in Env expression caused by furin co-expression required that the Env protein exhibit a furin-recognition motif (Figure 36d). We observed that furin co-expression had little effect on expression of the JR-FL gp140UNC protein (Figure 36d, Lanes 3 and 4) in which the KRRWQREKRAV furin-recognition sequence had been replaced by LRLRLRLRLRLR (Binley, 2000a). Although, in this experiment, gp140UNC expression was slightly increased in the presence of furin, the increase was not usually observed in repeat assays (data not shown). The lack of effect of furin on gp140UNC expression contrasts markedly with its substantial inhibition of the expression of the gp140WT and SOS gp140 proteins that have unmodified cleavage site sequences (Figure 36d, Lanes 1 and 2, Figure 36b). These results are consistent with the hypothesis that furin-induced reduction in Env expression is attributable to the formation of Env-furin complexes that are retained within the cell.

### A soluble form of furin allows efficient Env cleavage without dramatically reducing Env expression

In an attempt to overcome the apparent formation of furin-Env complexes in the TGN, we co-expressed Env with a soluble form of furin. The proteolytic activity of furin is contained entirely in its lumenal domain, and soluble forms of the enzyme retain enzymatic activity (Molloy, 1994; Molloy, 1992; and Plaimauer, 2001). When we expressed JR-FL gp140WT and SOS gp140 in the presence of full-length furin, we saw the expected reduction in Env expression (Figure 36e, Lanes 2 and 5). However, essentially the same result was observed when a soluble form of furin (furin∂TC) was used instead of the full-length, membrane-bound enzyme (Figure 36e, Lanes 3 and 6). Thus, although the presence of the furin recognition sequence is important (Figure 36d), direct retention of Env in complex with furin in the TGN may not be the entire explanation for the reduction in Env expression upon furin co-expression (Inocencio, 1997; Molloy, 1994; Moulard, 1999; and Willey, 1988).

### Altering the cleavage sequence can increase Env processing by cellular proteases

Our next approach towards increasing the efficiency of gp140 cleavage was to vary the furin recognition sequence. The rationale for this is partly derived from studies of other RNA viruses. For example, some influenza A virus variants have evolved proteolytic cleavage sites in the HA₀ precursor protein that contain basic residue insertions. This is associated with increased cleavage efficiency and a gain in viral virulence. Thus, whereas avirulent clones contain only a single arginine residue within the HA₀ cleavage site, the corresponding sites of virulent clones contain multiple basic residues, leading to motifs such as RRRKKR (Bender, 1999; Goto, 1998; Horimoto, 1995; Kawaoka, 1991; Kawaoka, 1988; and Ohuchi, 1989). Biochemical evidence using peptide-cleavage assays has confirmed that multi-arginine stretches are highly efficient targets for furin (Cameron, 2000). The most efficiently recognized target sequences consist of hexa- or hepta-arginine repeats; for example, a peptide with the recognition sequence RRRRRR was cleaved approximately 50 times more efficiently than one with the RRRR motif (Cameron, 2000).

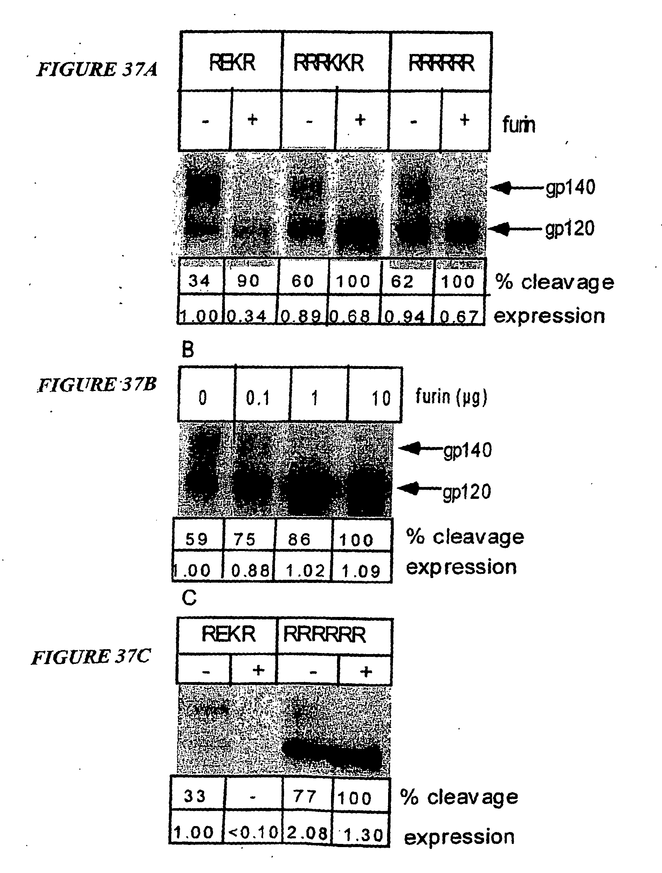

In contrast to influenza A, HIV-1 and SIV strains contain only simple R-X-R/K-R furin-recognition sequences. We therefore introduced basic amino acids into the cleavage site of the JR-FL SOS gp140 and gp140WT proteins. The mutated gp140 proteins were processed more efficiently than those containing the normal REKR motif, although none of the mutants was completely cleaved by endogenous, cellular proteases (Figure 37a, Table 5).

**Table 5.**

| Summary of expression and cleavage efficiencies of gp140 proteins with mutant cleavage sites. | | | | | | | | |
|---|---|---|---|---|---|---|---|---|
| | gp140WT | | gp140WT + furin | | SOS gp140 | | SOS gp140 + furin | |
| Cleavage site | Cleavage (%) | Expression | Cleavage (%) | Expression | Cleavage (%) | Expression | Cleavage (%) | Expression |
| REKR | 35 | 1.0 | 91 | 0.4 | 34 | 1.0 | 92 | 0.3 |
| RRRKKR | 66 | 0.9 | 100 | 0.7 | 60 | 0.9 | 100 | 0.8 |
| RRRRKR | 65 | 0.9 | 100 | 0.7 | 60 | 0.9 | 100 | 0.7 |
| RRRRRR | 71 | 1.0 | 100 | 0.9 | 62 | 0.9 | 100 | 0.9 |
| KKRKKR | 59 | 0.2 | 96 | 0.1 | 57 | 0.3 | 98 | 0.3 |
| RERRRKKR | 58 | 0.4 | 97 | 0.3 | 55 | 0.4 | 100 | 0.4 |

| | | | | | | | | |
|---|---|---|---|---|---|---|---|---|
| The cleavage efficiency of various gp140 cleavage site mutants is given as a percentage derived from densitometric analysis. The percent cleavage value recorded represents the mean from at least 3 individual experiments in which the individual values did not deviate by more than 6% from the mean. Combined expression of gp140 and gp120 is also given as a ratio relative to the level of expression of the parental gp140+gp120 observed in transfections with gp140WT or SOS gp140. Mean ratios from 3 repeats are given to the nearest decimal place and did not deviate more than 25% from this value. Data are shown for both gp140WT and SOS gp140 proteins expressed both in the presence and absence of co-transfected furin. | | | | | | | | |

Two of the most efficiently cleaved mutants contained the RRRRRR or RRRKKR motifs (Figure 37a; compare Lanes 1, 3 and 5). When furin was co-expressed, these mutants were 100% cleaved, compared to only about 90% cleavage for wild-type Env (or less than 90% in other experiments; data not shown). An unexpected finding was that furin did not reduce the overall expression of the cleavage-site mutant gp140 proteins, whereas, as noted above, it significantly diminished the expression of the wild-type gp140 (Figure 37a; compare Lanes 2, 4 and 6). This was confirmed when the RRRRRR gp140 mutant was co-expressed with variable amounts of pCDNA-furin (0.1µg, 1µg or 10µg) (Figure 37b). In this experiment, furin co-expression actually increased the overall amount of Env protein secreted, although an increase was not always seen with this or related mutants in other experiments.

The expression levels and cleavage efficiencies of a selection of gp140 mutants with basic insertions into the REKR cleavage site are summarized in Table 5. The closely related mutants RRRKKR, RRRRKR and RRRRRR all had similar properties, in that cleavage was enhanced in the absence of co-transfected furin, and was complete in the presence of furin, but without a significant decrease in the extent of Env expression. The mutants KKRKKR and RERRRKKR were also better cleaved than the wild-type protein, and their expression was unaffected by furin co-transfection. However, they were expressed at lower levels than the other mutants and less well than wild-type gp140 proteins containing the standard REKR motif. The effects of the basic residue insertions were similar whether the test protein was gp140WT or SOS gp140, although some of the gp140WT proteins were expressed at slightly higher levels than the corresponding SOS gp140 proteins (Table 5).

To address whether the insertion of basic amino acids into the proteolytic cleavage site had a general effect on cleavage efficiency (i.e. was not restricted to the JR-FL clone), we mutated the cleavage site of SOS gp140 of the subtype C primary isolate, DU151, from REKR to RRRRRR. In the absence of co-transfected furin, the unmodified DU151 SOS gp140 protein was partially cleaved (Figure 37c, Lane 1). When furin was co-expressed, Env expression was significantly reduced, in some experiments to the extent that the Env proteins were no longer visible on the gel (Figure 37c, Lane 2; and data not shown). In contrast, the RRRRRR mutant was more efficiently cleaved in the absence of furin, and was fully cleaved in the presence of furin. Furthermore, the overall expression of Env being greater than that of the wild-type gp140 (Figure 37c, compare Lanes 2 and 4).

### Effect of cleavage sites mutations on HIV-1 infectivity

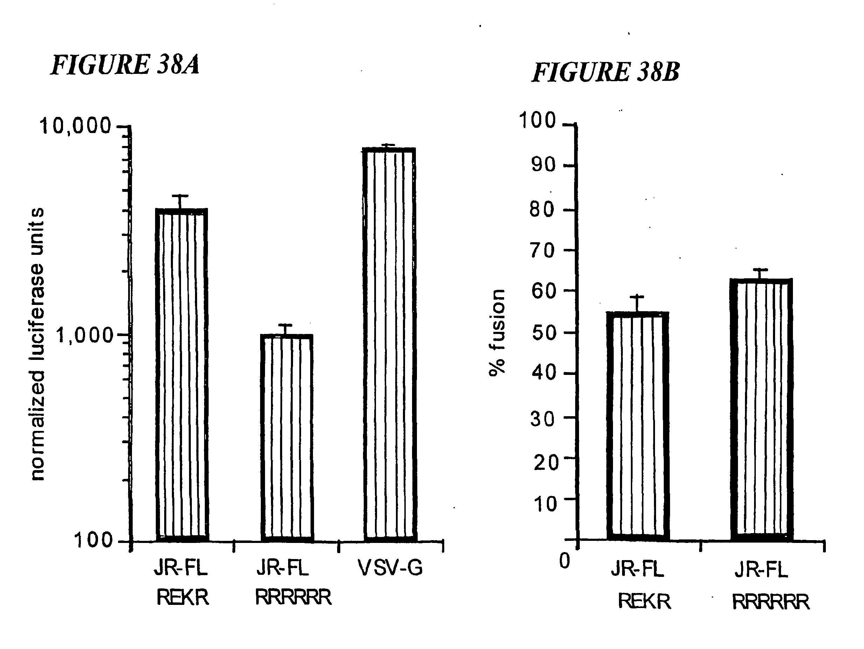

We examined whether Env mutants containing basic cleavage site insertions were still functional for virus infection, using an Env-pseudotype assay (Dragic, 1996). The JR-FL gp160_{RRRRRR} mutant expressed by the pSV plasmid in 293T cells could successfully pseudotype pNL-luc, producing a virus capable of infecting HeLa-CD4-CCR5 cells. The infectivity of the JR-FL gp160_{RRRRRR} Env pseudotype was about 3-4 fold lower than the JR-FL gp160WT pseudotype, but still in the range we find to be typical of pseudotyped virus stocks (Figure 38a). In an independent test of the functional activity of the mutant Env, we examined the ability of the JR-FL gp160_{RRRRRR} mutant to mediate cell-cell fusion, using a fluorescent cytoplasmic dye transfer assay (Melikyan, 2000). The modest, and statistically insignificant, increase in fusion with the RRRRRR mutant (Figure 38b) may be because it is expressed at 5 fold higher levels than the wild type gp160 by Western blot analysis of cell lysates. Overall, the REKR to RRRRRR substitution does not globally disrupt the Env conformation required for fusion and infection.

### E. Discussion

The Env proteins of most enveloped viruses, including HIV-1, are synthesized as inactive precursors that are proteolytically processed to attain full functional activity. In the case of HIV-1, the gp160 precursor is cleaved into a fusion-active gp120/gp41 complex. The structures of a monomeric gp120 core fragment (Kwong, 1998) and a post-fusion form of gp41 (Chan, 1997; Lu, 1995; and Weissenhorn, 1997) have been determined. However, little is known about the structure of either uncleaved gp160 or the gp120/gp41 complex, although the latter is considered to be trimeric (Chan, 1997; Doms, 1993; Lu, 1995; Poignard, 2001; and Weissenhorn, 1997). The fusion-active complex is unstable, principally because the gp120-gp41 interaction is weak and gp120 is shed. Introducing a disulfide bond between gp120 and gp41 can prevent gp120-gp41 dissociation (Binley, 2000a). However, the purified form of this protein (SOS gp140) is monomeric, probably because of a further instability between the associated gp41 subunits.

Here we investigated methods to produce proteolytically processed proteins. The expression of gp140 or gp160 proteins at high levels usually leads to the production of a mixture of cleaved and uncleaved proteins, implying that processing of the cleavage site by host cell proteases is incomplete. Partial cleavage is a common phenomenon when Env is expressed in a variety of recombinant systems and cell lines (Inocencio, 1997; Morikawa, 1993; Moulard, 1998; Moulard, 1999; Moulard, 2000; Rodriguez, 1995; Spies, 1994; and Yamshchikov, 1995). Differences in folding among natural and mutant Env proteins (Dedera, 1992; Merat, 1999; Syu, 1991; Travis, 1992; Tschachler, 1990; Willey, 1991) may affect the exposure of what is likely to be a loop structure containing the cleavage site (Chen, 1998). Another influence on Env cleavage is the direct or indirect masking of the furin recognition site by glycans (Paredes, 1998). Overall, the accessibility of this site to the protease is a complex function of both Env folding and glycosylation (Chen, 1998; Moulard, 2000; and Singh, 2000).

We first evaluated the possibility of cleaving unprocessed, purified Env proteins by adding a purified protease in vitro. Although plasmin could efficiently process the gp120/gp41 cleavage site, as has been previously reported (Okumura, 1999), it also cleaved gp120 at a second site, most probably within the V3 loop. This renders its use impractical. Purified furin can also cleave secreted Env (Decroly, 1994; Decroly, 1996; Inocencio, 1997; and Moulard, 1998), albeit at low efficiency (Decroly, 1994; and Vollenweider, 1996). Our own findings were similar: Even when furin digestion of SOS gp140 was performed at optimal pH with the enzyme in large excess, approximately 40% of the Env substrate remained uncleaved, suggesting that there may be a subpopulation of gp140 that is more resistant to cleavage. This may perhaps represent hyperglycosylated or misfolded proteins. Furin is not an inherently inefficient enzyme - it is highly effective at cleaving synthetic peptides (Cameron, 20001; and Plaimauer, 2001) - but the conformation of its recognition site on gp160 limits its ability to cleave this particular substrate. That gp160 is an inherently poor substrate for furin is exemplified by a comparison of gp160 and anthrax toxin, the latter being cleaved by furin several orders of magnitude more efficiently than gp160 at pH 7.2 (Molloy, 1992).

One way to augment gp160 cleavage is to co-express exogenous furin, but this can lead to a reduction in overall Env expression. Primary protein expression (including, but not limited to, HIV-1 Env) is reduced upon furin co-expression (Binley, 2000a; Morikawa, 1993; Moulard, 1999; Staropoli, 2000; and Vollenweider, 1996). One possible explanation is that furin may form stable complexes with Env proteins that it cleaves poorly, with these complexes being retained in the TGN or recycled to lysosomes, rather than secreted (Inocencio, 1997; Molloy, 1994; Moulard, 1999; Staropoli, 2000; and Willey, 1988). This idea is supported by our observation that furin co-expression with Env mutants containing optimized cleavage sites caused very little reduction in Env expression. The co-expression of Env with either the full-length (membrane-bound) or the soluble form of furin reduced gp140 expression, so this reduction can occur without a direct association of the furin-Env complex with a membrane. Perhaps another, membrane-associated cellular protein is involved in the removal and degradation of complexes between uncleaved Env and furin. It has been shown that although truncated furin is shed into the culture medium, it can also still be isolated from membrane fractions like the full-length counterpart (Molloy, 1994).

Although furin co-expression increases the cleavage of secreted gp140 proteins, it has been reported that this does not occur with full-length gp160 molecules expressed on the cell surface (Inocencio, 1997; and York, 2001). In contrast, we and others (Vollenweider, 1996; and Yamshchikov, 1995) have found that gp160 cleavage can be at least partially augmented by furin, at least under some experimental conditions. Clearly, then, there are poorly understood variables that affect different experimental systems differently, perhaps including the expression vectors, the particular Env gene and the cell line used.

A successful strategy for improving Env cleavage efficiency involved mutating the furin-recognition site. Studies of naturally occurring influenza A virus variants have revealed that insertion of basic amino acids in and near the cleavage site of the HA₀ protein is associated with enhanced proteolysis (Bender, 1999), and frequently also with increased host cell range and virulence (Bender, 1999; Goto, 1998; Horimoto, 1995; Kawaoka, 1991; Kawaoka, 1988; and Ohuchi, 1989). Moreover, improved cleavage of the influenza B glycoprotein was previously achieved by Brassard and Lamb, who substituted the conserved monobasic cleavage site with the multibasic cleavage sites found in virulent influenza A clones (Brassard, 1997). We therefore considered it possible that altering the conserved, tetrameric cleavage recognition sequence of HIV-1 Env might increase cleavage efficiency. We found that several variant furin recognition sequences, based on those found in HA₀ proteins from pathogenic influenza A strains, allowed enhanced cleavage of HIV-1 Env in the absence of co-expressed furin. The best of these variant sequences were RRRKKR, RRRRKR and RRRRRR, which approximately doubled the extent of Env cleavage compared to that achieved when the standard REKR sequence was present. Furthermore, co-expression of furin did not reduce the expression of Env proteins containing these mutated sequences, but did allow the cleavage efficiency to now approach 100%. A consequence of the more efficient cleavage of these improved furin substrates may be the more rapid egress of Env from the secretory pathway, allowing a higher overall expression of fully processed Env. Furthermore, the REKR to RRRRRR mutation had little impact on the infectivity of Env-complemented reporter viruses, or on Env-mediated membrane fusion. The cleavage site mutations do not, therefore, affect the overall folding of Env in any adverse manner, which is relevant to any consideration of the use of such Env mutants as vaccine antigens.

Although furin recognition of gp160 is rather inefficient, the strict conservation of the REKR sequence in HIV-1 (or of RNKR in SIV) suggests that this sequence confers a selective advantage to the virus. There are no examples of Env sequences with basic residue insertions adjacent to the consensus cleavage-site motif (Kuiken, 2000), so a higher rate of Env cleavage may be disadvantageous. For example, a too-rapid destruction of the infected cell by fusion caused by high levels of processed, cell-surface Env could reduce the yield of progeny virions from that cell. An immunological mechanism might be that uncleaved Env is actually beneficial to the virus by acting as a decoy that causes the induction of predominantly non-neutralizing antibodies (Burton, 2000; Parren, 1997; and Sakurai, 1999).

Here we have demonstrated that we can produce HIV-1 Env mutants containing polybasic cleavage sites that are more efficient substrates for furin than the consensus, REKR, sequence. Co-expression of Env cleavage site mutants with furin is a useful method for obtaining significant amounts of processed Env. The use of these Env mutants should simplify the production of significant amounts of cleaved Env, which may be useful in HIV-1 vaccine design.

### References

1. Abacioglu, Y.H., Fouts T.R., Laman J.D., Claassen E., Pincus S.H., Moore J.P., Roby C.A., Kamin-Lewis R. and Lewis G.K. (1994) Epitope mapping and topology of baculovirus-expressed HIV-1 gp160 determined with a panel of murine monoclonal antibodies. AIDS Res. Hum. Retroviruses 10:371-381.
2. Adams, O., Schall H. and Scheid A. (2000) Natural variation in the amino acid sequence around the HIV-1 glycoprotein gp160 cleavage site and its effect on cleavability, subunit association and membrane fusion. AIDS Res. Hum. Retroviruses 16:1235-1245.
3. Allaway, G.P., Davis-Bruno K.L., Beaudry G.A., Garcia E.B., Wong E.L., Ryder A.M., Hasel K.W., Gauduin M.C., Koup R.A., McDougal J.S., and Maddon P.J. (1995) Expression and characterization of CD4-IgG2, a novel heterotetramer which neutralizes primary HIV-1 isolates. AIDS Res. Hum. Retroviruses. 11:533-539.
4. Allaway, G.P., Ryder A.M., Beaudry G.A., and Maddon P.J. (1993) Synergistic inhibition of HIV-1 envelope-mediated cell fusion by CD4-based molecules in combination with antibodies to gp120 or gp41. AIDS Res. Hum. Retroviruses 9:581-587.
5. Alving, C.R., Koulchin V., Glenn G.M., and Rao M. (1995) Liposomes as carriers of peptide antigens: induction of antibodies and cytotoxic T lymphocytes to conjugated and unconjugated peptides. Immunol. Rev. 145:5-31.
6. Andre, S., Seed B., Eberle J., Schraut W., Bultmann A. and Haas J. (1998) Increased immune response elicited by DNA vaccination with a synthetic gp120 sequence with optimized codon usage. J. Virol. 72:1497-503.
7. Angliker, H., Neumann U., Molloy S.S. and Thomas G. (1995) Internally quenched fluorogenic substrate for furin. Anal. Biochem. 224:409-412.
8. Atwell, S., Ridgway J.B., Wells J.A. and Carter P. (1997) Stable heterodimers from remodeling the domain interface of a homodimer using a phage display library. J. Mol. Biol. 270:26-35.
9. Barnett, S. W., Lu S., Srivastava I., Cherpelis S., Gettie A., Blanchard J., Wang S., Mboudjeka I., Leung L., Lian Y., Fong A., Buckner C., Ly A., Hilt S., Ulmer J., Wild C.T., Mascola J.R. and Stamatatos L.. (2001) The ability of an oligomeric human immunodeficiency virus type 1 (HIV- 1) envelope antigen to elicit neutralizing antibodies against primary HIV-1 isolates is improved following partial deletion of the second hypervariable region. J. Virol. 75:5526-5540.
10. Bender, C., Hall H., Huang J., Klimov A., Cox N., Hay A., Gregory V., Cameron K., Lim W. and Subbarao K. (1999) Characterization of the surface proteins of influenza A (H5N1) viruses isolated from humans in 1997-1998. Virology 254:115-123.
11. Berman, P.W., P.W., Gregory T.J., Riddle L, Nakamura G.R., Champe, M.A., Porter J.P., Wurm F.M., Hershberg R.D., Cobb E.K. and Eichberg J.W. (1990) Protection of chimpanzees from infection by HIV-1 after vaccination with recombinant glycoprotein gp120 but not gp160. Nature 345:622-625.
12. Berman, P.W., P.W., Nunes W.M. and Haffar O.K. (1988) Expression of membrane-associated and secreted variants of gp160 of human immunodeficiency virus type 1 in vitro and in continuous cell lines. J. Virol. 62:3135-3142.
13. Berman, P.W., Riddle L., Nakamura G., Haffar O.K., Nunes W.M., Skehel P., Byrn R., Groopman J., Matthews T. and Gregory T. (1989) Expression and immunogenicity of the extracellular domain of the human immunodeficiency virus type 1 envelope glycoprotein, gp160. J. Virol. 63: 3489-98.
14. Bernstein, H.B., Tucker S.P., Kar S.R., McPherson S.A., McPherson D.T., Dubay J.W., Lebowitz J., Compans R.W. and Hunter E. (1995) Oligomerization of the hydrophobic heptad repeat of gp41. J. Virol. 69:2745-2750.
15. Binley, J., and Moore J.P. (1997b) HIV-cell fusion. The viral mousetrap. 387:346-348.
16. Binley, J.M., Clas B., Gettie A., Vesanen M., Montefiori D.C., Sawyer L., Booth J., Lewis M., Marx P.A., Bonhoeffer S. and Moore J.P. (2000b) Passive infusion of immune serum into simian immunodeficiency virus-infected rhesus macaques undergoing a rapid disease course has minimal effect on plasma viremia. Virology 270:237-249.
17. Binley, J.M., Klasse P.J., Cao Y., Jones I., Markowitz M., Ho D.D., and Moore J.P. (1997a). Differential regulation of the antibody responses to Gag and Env proteins of human immunodeficiency virus type 1. J. Virol. 71:2799-2809.
18. Binley, J.M., Sanders R.W., Clas B., Schulke N., Master A., Guo Y., Kajumo F., Anselma D.J., Maddon P.J., Olson W.C. and Moore J.P. (2000a) A recombinant HIV-1 envelope glycoprotein complex stabilized by an intermolecular disulfide bond between the gp120 and gp41 subunits is an antigenic mimic of the trimeric virion-associated structure. J. Virol. 74:627-643.
19. Binley, J.M., Sanders R.W., Master A., Cayanan C.S., Wiley C.L., Schiffner L., Travis B., Kuhmann S., Burton D.R., Hu S.L., Olson W.C. and. Moore J.P. (2002) Enhancing the proteolytic maturation of human immunodeficiency virus type 1 envelope glycoproteins. J. Virol. 76:2606-2616.
20. Binley, J.M., Wyatt R., Desjardins E, Kwong P.D., Hendrickson W., Moore J.P. and Sodroski J. (1998) Analysis of the interaction of antibodies with a conserved enzymatically deglycosylated core of the HIV type 1 envelope glycoprotein 120.AIDS Res. Human Retrovir. 14:191-98.
21. Bolmstedt, A., Hinkula J., Rowcliffe E., Biller M., Wahren B. and Olofsson S. (2001) Enhanced immunogenicity of a human immunodeficiency virus type 1 env DNA vaccine by manipulating N-glycosylation signals. Effects of elimination of the V3 N306 glycan. Vaccine 20:397-405.
22. Bolt, G., Pederson L.O. and Birkeslund H.H. (2000) Cleavage of the respiratory syncytial virus fusion protein is required for its surface expression: role of furin. Virus Res. 68:25-33.
23. Bosch, V. and Pawlita M. (1990) Mutational analysis of the human immunodeficiency virus type 1 env gene product proteolytic cleavage site. J. Virol. 64:2337-2344.
24. Brassard, D.L. and Lamb R.A. (1997) Expression of influenza B virus hemagglutinin containing multibasic residue cleavage sites. Virology 236:234-248.
25. Buchacher, A., Predl R., Strutzenberger K., Steinfellner W., Trkola A., Purtscher M., Gruber G., Tauer C., Steindl F., Jungbauer A., et al. (1994) AIDS Res. Human Retrov. 10:359-369.
26. Bullough, P.A., Hughson F.M., Skehel J.J., and Wiley D.C. (1994) Structure of influenza haemagglutinin at the pH of membrane fusion. Nature 371:37-43.
27. Burton, D.R. (1997b) A vaccine for HIV type 1: the antibody perspective. PNAS 94:10018-10023.
28. Burton, D.R. and Montefiori D.C., (1997) The antibody response in HIV-1 infection. AIDS. 11 Suppl A:S87-S98.
29. Burton, D.R. and Parren P.H.W.I. (2000) Vaccines and the induction of functional antibodies: time to look beyond the molecules of natural infection? Nat. Med. 6:123-125.
30. Burton, D.R., Barbas C.F., Persson M.A., Koenig S., Chanock R.M., and Lerner R.A.(1991) A large array of human monoclonal antibodies to type 1 human immunodeficiency virus from combinatorial libraries of asymptomatic seropositive individuals. Proc. Nat. Acad. Sci. U.S.A. 88:10134-10137.
31. Burton, D.R., Pyati J., Koduri R., Sharp S.J., Thornton G.B, Parren P.W., Sawyer L.S., Hendry R.M., Dunlop N., Nara P.L., et al. (1994) Efficient neutralization of primary isolates of HIV-1 by a recombinant human monoclonal antibody. Science. 266:1024-1027.
32. Caffrey, M., Cai M., Kaufman J., S.J. Stahl, Wingfield P.T., Covell D.G., Gronenborn A.M., and Clore G.M. (1998) Three-dimensional solution structure of the 44 kDa ectodomain of SIV gp41. EMBO J. 17:4572-4584.
33. Cameron, A, Appel J., Houghten R.A. and Lindberg I. (2000) Polyarginines are potent furin inhibitors. J. Biol. Chem. 275:36741-36749.
34. Cantor, C. and Schimmel P. (1980) Biophysical chemistry, part III. W.H. Freeman and company, New York, N.Y.
35. Cao, J., L. Bergeron, E. Helseth, M. Thali, H. Repke and J. Sodroski. (1993) Effects of amino acid changes in the extracellular domain of the human immunodeficiency virus type 1 gp41 envelope glycoprotein. J. Virol. 67:2747-2755.
36. Cao, J., Sullivan N., Desjardin E., Parolin C., Robinson J., Wyatt R. and Sodroski J. (1997) Replication and neutralization of human immunodeficiency virus type 1 lacking the V1 and V2 variable loops of the gp120 envelope glycoprotein. J. Virol. 71: 9808-9812.
37. Center, R.J., Earl P.L., Lebowitz J., Schuck P., and Moss B. (2000) The human immunodeficiency virus type 1 gp120 V2 domain mediates gp41- independent intersubunit contacts. J. Virol. 74:4448-4455.
38. Center, R.J., Schuck P., Leapman R.D., Arthur L.O., Earl P.L., Moss B., and Lebowitz J. (2001) Oligomeric structure of virion-associated and soluble forms of the simian immunodeficiency virus envelope protein in the prefusion activated conformation. Proc. Natl. Acad. Sci. U.S.A 98:14877-14882.
39. Chan, D.C. and Kim P.S. (1998) HIV entry and its inhibition. Cell 93:681-684.
40. Chan, D.C., Fass D., Berger J.M. and Kim P.S. (1997) Core structure of gp41 from the HIV envelope glycoprotein. Cell 89:263-273.
41. Chang, D.K., Trivedi V.D., Cheng S.F. and Francis S. (2001) The leucine zipper motif of the envelope glycoprotein ectodomain of human immunodeficiency virus type 1 contains conformationally flexible regions as revealed by NMR and circular dichroism studies in different media. J. Pept. Res. 57:234-239.
42. Chen, B., Zhou G., Kim M., Chishti Y., Hussey R.E., Ely B., Skehel J.J., Reinherz E.L., Harrison S.C. and Wiley D.C. (2000) Expression, purification, and characterization of gp160e, the soluble, trimeric ectodomain of the simian immunodeficiency virus envelope glycoprotein, gp160. J. Biol. Chem. 275:34946-34953.
43. Chen, J., Lee K.H., Steinhauer D.A., Stevens D.J., Skehel J.J. and Wiley D.C. (1998) Structure of the hemagglutinin precursor cleavage site, a determinant of influenza pathogenicity and the origin of labile conformation. Cell 95:409-417.
44. Chen, S.S. (1994) Functional role of the zipper motif region of human immunodeficiency virus type 1 transmembrane protein gp41. J. Virol. 68:2002-2010.
45. Chen, S.S., Lee S.F., Hao H.J., and Chuang C.K. (1998) Mutations in the leucine zipper-like heptad repeat sequence of human immunodeficiency virus type 1 gp41 dominantly interfere with wild-type virus infectivity. J. Virol. 72:4765-4774.
46. Chen, Y.H., Yang J.T. and Chau K.H. (1974) Determination of the helix and beta form of proteins in aqueous solution by circular dichroism. Biochemistry 13:3350-3359.
47. Chen, Y.T., Scanlan M.J., Sahin U., Tureci O., Gure A.O., Tsang S., Williamson B., Stockert E., Pfreundschuh M., and Old J.J. (1997) A testicular antigen aberrantly expressed in human cancers detected by autologous antibody screening. Proc. Natl. Acad. Sci. U.S.A. 94:1914-1918.
48. Cherpelis, S., Shrivastava I., Gettie A., Jin X., Ho D.D., Barnett S.W., and Stamatatos L. (2001) DNA vaccination with the human immunodeficiency virus type 1 SF162DeltaV2 envelope elicits immune responses that offer partial protection from simian/human immunodeficiency virus infection to CD8(+) T-cell-depleted rhesus macaques. J. Virol. 75:1547-1550.
49. Cleland, J.L., Powell M.F., Lim A., Barron L., Berman P.W., Eastman D.J., Nunberg J.H., Wrin T., and Vennari J.C. (1994) Development of a single-shot subunit vaccine for HIV-1. AIDS Res. Hum. Retroviruses 10 Suppl 2:S21-26.
50. Clements, G.J., Price-Jones M.J., Stephens P.E., Sutton C., Schulz T.F., Clapham P.R., McKeating J.A., McClure M.O., Thomson S., Marsh M., Kay J., Weiss R.A. and Moore J.P. (1991) The V3 loops of the HIV-1 and HIV-2 surface glycoproteins contain proteolytic cleavage sites: a possible function in viral fusion? AIDS Res. Hum. Retroviruses 7:3-16.
51. Collman, R., Balliet J.W., Gregory S.A., Friedman H., Kolson D.L., Nathanson N. and Srinivasan A. (1992) An infectious molecular clone of an unusual macrophage-tropic and highly cytopathic strain of human immunodeficiency virus type 1. J. Virol. 66: 7517-21.
52. Coombes, A.G., Lavelle E.C., and David S.S. (1999) Biodegradable lamellar particles of poly(lactide) induce sustained immune responses to a single dose of adsorbed protein. Vaccine. 2410-2422.
53. Coombes, A.G., Lavelle E.C., Jenkins P.G., and Davis S.S. (1996) Single dose, polymeric, microparticle-based vaccines: the influence of formulation conditions on the magnitude and duration of the immune response to a protein antigen. Vaccine 14:1429-1438.
54. Decroly, E., Vandenbranden M., Ruysschaert J.M., Cogniaux J., Jacob G.S., Howard S.C., Marshall G., Kompelli A., Basak A., Jean F., Lazure C., Benjannet S., Chretien M., Day R., Seidah N.G. (1994) The convertases furin and PC1 can both cleave the human immunodeficiency virus (HIV-1) envelope glycoprotein gp160 into gp120 (HIV-1 SU) and gp41 (HIV-I TM). J. Biol. Chem. 269:12240-12247.
55. Decroly, E., Wouters S., Di Bello C., Lazure C., Ruysschaert J.M. and Seidah N.G. (1996) Identification of the paired basic convertases implicated in HIV gp160 processing based on in vitro assays and expression in CD4+ cell lines. J. Biol. Chem. 271:30442-30450.
56. Dedera, D., Gu R.L. and Ratner L. (1992) Conserved cysteine residues in the human immunodeficiency virus type 1 transmembrane envelope protein are essential for precursor envelope cleavage. J. Virol. 66:1207-1209.
57. diMarzo Veronese et al. (1992) AIDS Res. Human Retrov. 8:1125.
58. Doe, B., Selby M., Barnett S., Baenziger J. and Walker C.M. (1996) Induction of cytotoxic T lymphocytes by intramuscular immunization with plasmid DNA is facilitated by bone marrow-derived cells. Proc. Natl. Acad. Sci. U.S.A. 93(16):8578-8583.
59. Doms, R.W. (2000a) Beyond receptor expression: the influence of receptor conformation, density, and affinity in HIV-1 infection. Virol. 276:229-237.
60. Doms, R.W. and Moore J.P. (2000b) HIV-1 membrane fusion: targets of opportunity. J. Cell Biol. 151:F9-14.
61. Doms, R.W., Lamb R.A., Rose J.K. and Helenius A. (1993) Folding and assembly of viral membrane proteins. Virology 193:545-562.
62. Dragic, T., Litwin V., Allaway G.P., Martin S.R., Huang Y., Nagashima K.A., Cayanan C., Maddon P.J., Koup R.A., Moore J.P. and Paxton W.A. (1996) HIV-1 entry into CD4+ cells is mediated by the chemokine receptor CC-CKR-5. Nature 381:667-673.
63. Dubay, J.W., Dubay S.R., Shin H.J. and Hunter E. (1995) Analysis of the cleavage site of the human immunodeficiency virus type 1 glycoprotein: requirement of precursor cleavage for glycoprotein incorporation. J. Virol. 69:4675-4682.
64. Dutch, R.E., Hagglund R.N., Nagel M.A., Paterson R.G. and Lamb R.A. (2001. Paramyxovirus fusion (F) protein: a conformational change on cleavage activation. Virology 281:138-50.
65. Earl P.L., Doms R.W. and Moss B. (1990) Oligomeric structure of the human immunodeficiency virus type 1 envelope glycoprotein. Proc. Nat. Acad. Sci. U.S.A. 87:648-652.
66. Earl, P.L. and Moss B. (1993) Mutational analysis of the assembly domain of the HIV-1 envelope glycoprotein. AIDS Res. Hum. Retrovir. 9:589-594.
67. Earl, P.L., Moss B. and Doms R.W. (1994a) Folding, interaction with GRP78-BiP, assembly and transport of the human immunodeficiency virus type 1 envelope protein. J. Virol. 65:2047-2055.
68. Earl, P.L., Broder C.C., Doms R.W. and Moss B. (1997) Epitope map of human immunodeficiency virus type 1 gp41 derived from 47 monoclonal antibodies produced by immunization with oligomeric envelope protein. J. Virol. 71:2674-2684.
69. Earl, P.L., Broder C.C., Long D., Lee S.A., Peterson J., Chakrabarti S., Doms R.W. and Moss B. (1994b) Native oligomeric human immunodeficiency virus type 1 envelope glycoprotein elicits diverse monoclonal antibody reactivities. J. Virol. 68:3015-3026.
70. Earl, P.L., Doms R.W. and Moss B. (1990) Oligomeric structure of the human immunodeficiency virus type 1 envelope glycoprotein. Proc. Natl. Acad. Sci. U.S.A 87:648-652.
71. Earl, P.L., Moss B. and Doms R.W. (1991) Folding, interaction with GRP78-BiP, assembly, and transport of the human immunodeficiency virus type 1 envelope protein. J. Virol. 65:2047-2055.
72. Earl, P.L., Sugiura W., Montefiori D.C., Broder C.C., Lee S.A., Wild C., Lifson J. and Moss B. (2001) Immunogenicity and protective efficacy of oligomeric human immunodeficiency virus type 1 gp140. J. Virol. 75:645-653.
73. Eckert, D.M. and Kim P.S. (2001) Mechanisms of viral membrane fusion and its inhibition. Annu. Rev. Biochem. 70:777-810.
74. Edelhoch, H. (1967) Spectroscopic determination of tryptophan and tyrosine in proteins. Biochemistry 6:1948-1954.
75. Edinger, A.L., Ahuja M., Sung T., Baxter K.C., Haggarty B., Doms R.W. and Hoxie J.A. (2000) Characterization and epitope mapping of neutralizing monoclonal antibodies produced by immunization with oligomeric simian immunodeficiency virus envelope protein. J. Virol. 74:7922-7935.
76. Edinger, A.L., Blanpain C., Kunstman K.J., Wolinsky S.M., Parmentier M. and Doms R.W. (1999) Functional dissection of CCR5 coreceptor function through the use of CD4-independent simian immunodeficiency virus strains. J. Virol. 73:4062-73.
77. Farzan, M., Choe H., Desjardins E., Sun Y., Kuhn J., Cao J., Archambault D., Kolchinsky P., Koch M., Wyatt R., and Sodroski J. (1998) Stabilization of human immunodeficiency virus type 1 envelope glycoprotein trimers by disulfide bonds introduced into the gp41 glycoprotein ectodomain. J. Virol. 72:7620-7625.
78. Feltquate, D.M., Heaney S., Webster R.G. and Robinson H.L. (1997) Different T helper cell types and antibody isotypes generated by saline and gene gun DNA immunization. J. Immunol. 158(5):2278-2284.
79. Ferlenghi, I., Gowen B., de Haas F., Mancini E.J., Garoff H., Sjoberg M. and Fuller S.J. (1998) The first step: activation of the Semliki Forest virus spike protein precursor causes a localized conformational change in the trimeric spike. J. Mol. Biol. 283:71-81.
80. Fouts, T.R., Binley J.M., Trkola A., Robinson J.E. and Moore J.P. (1997) Neutralization of the human immunodeficiency virus type 1 primary isolate JR-FL by human monoclonal antibodies correlates with antibody binding to the oligomeric form of the envelope glycoprotein complex. J Virol 71: 2779-85.
81. Fouts, T.R., Trkola A., Fung M.S. and Moore J.P. (1998) Interactions of polyclonal and monoclonal anti-glycoprotein 120 antibodies with oligomeric glycoprotein 120-glycoprotein 41 complexes of a primary HIV type 1 isolate: relationship to neutralization. AIDS Res. Hum. Retrovir. 14:591-597.
82. Frank, J., Radermacher M., Penczek P., Zhu J., Li Y., Ladjadj M. and Leith A. (1996) SPIDER and WEB: processing and visualization of images in 3D electron microscopy and related fields. J. Struct. Biol. 116:190-199.
83. Franzusoff, A., Volpe A.M., Josse D., Pichuantes S. and Wolf J.R. (1995) Biochemical and genetic definition of the cellular protease required for HIV-1 gp160 processing. J. Biol. Chem. 270:3154-3159.
84. Freed, E.O., Myers D.J. and Risser R. (1989) Mutational analysis of the cleavage sequence of the human immunodeficiency virus type 1 envelope glycoprotein precursor gp160. J. Virol. 63:4670-4675.
85. Gallaher, W.R., Ball J.M., Garry R.F., Martin-Amedee A.M. and Montelaro R.C. (1995) A general model for the surface glycoproteins of HIV and other retroviruses. AIDS Res. Human Retrovir. 11: 191-202.
86. Gallo, S.A., Puri A. and Blumenthal R. (2001) HIV-1 gp41 6-helix bundle formation occurs rapidly after the engagement of gp120 by CXCR4 in the HIV-1 Env-mediated fusion process. Biochemistry 40:12231-12236.
87. Gelderblom, H.R., Reupke H. and Pauli G. (1985) Loss of envelope antigens of HTLV-III/LAV, a factor in AIDS pathogenesis? Lancet 11:1016-1017.
88. Gerl, M., Jaenicke R., Smith J.M. and Harrison P.M. (1988) Self-assembly of apoferritin from horse spleen after reversible chemical modification with 2,3-dimethylmaleic anhydride. Biochemistry 27:4089-4096.
89. Gershoni, J.M., Denisova G., Raviv D., Smorodinsky N.I. and Buyaner D. (1993) HIV binding to its receptor creates specific epitopes for the CD4/gp120 complex. FASEB J. 7:1185-1187.
90. Gluschankof, P., Mondor I., Gelderblom H.R. and Sattentau Q.J. (1997) Cell membrane vesicles are a major contaminant of gradient-enriched human immunodeficiency virus type-1 preparations. Virology 230:125-133.
91. Gorny, M.K. and Zolla-Pazner S. (2000) Recognition by human monoclonal antibodies of free and complexed peptides representing the prefusogenic and fusogenic forms of human immunodeficiency virus type 1 gp41. J. Virol. 74:6186-6192.
92. Gorse, G.J., McElrath M.J., Matthews T.J., Hsieh R.H., Belshe R.B., Corey L., Frey S.E., Kennedy D.J., Walker M.C. and Eibl M.M. (1998) Modulation of immunologic responses to HIV-1MN recombinant gp160 vaccine by dose and schedule of administration. National Institute of Allergy and Infectious Diseases AIDS Vaccine Evaluation Group. Vaccine 16:493-506.
93. Goto, H. and Kawaoka Y. (1998) A novel mechanism for the acquisition of virulence by a human influenza A virus. Proc. Natl. Acad. Sci. USA 95:10224-10228.
94. Guo, H.G., Veronese F.M., Tschachler E., Pal R., Kalyanaraman V.S., Gallo R.C. and Reitz Jr M.S. (1990) Characterization of an HIV-1 point mutant blocked in envelope glycoprotein cleavage. Virology 174:217-24.
95. Haas, J., Park E.C. and Seed B. (1996) Codon usage limitation in the expression of HIV-1 envelope glycoprotein. Current Biol. 6:315-24.
96. Hallenberger, S., Moulard M., Sordel M., Klenk H.D. and Garten W. (1997) The role of eukaryotic subtilisin-like endoproteases for the activation of human immunodeficiency virus glycoproteins in natural host cells. J. Virol. 71:1036-1045.
97. Hanes, J., Cleland J.L. and Langer R. (1997) New advances in microsphere-based single-dose vaccines. Advanced Drug Delivery Reviews 28:97-119.
98. Harbury, P.B., Kim P.S. and Alber T. (1994) Crystal structure of an isoleucine-zipper trimer. Nature 371:80-83.
99. He, Q., Mitchell A.R., Johnson S.L., Wagner-Bartak C., Morcol T. and Bell S.J. (2000) Calcium phosphate nanoparticle adjuvant. Clinical and Diagnostic Laboratory Immunology 7:899-903.
100. Heinz, F.X., Allison S.L., Stiasny K., Schalich J., Holzmann H., Mandl C.W. and Kunz C. (1995) Recombinant and virion-derived soluble and particulate immunogens for vaccination against tick-borne encephalitis. Vaccine 13:1636-1642.
101. Helseth, E., Olshevsky U., Furman C and Sodroski J. (1991) Human immunodeficiency virus type 1 gp120 envelope glycoprotein regions important for association with the gp41 transmembrane glycoprotein. J. Virol. 65:2119-2123.
102. Hoffman, T.L., Canziani G., Jia L., Rucker J. and Doms R.W. (2000) A biosensor assay for studying ligand-membrane receptor interactions: binding of antibodies and HIV-1 Env to chemokine receptors. Proc. Natl. Acad. Sci. U.S.A. 97:11215-11220.
103. Hollstein, M., Rice K., Greenblatt M.S., Soussi T., Fuchs R., Sorlie T., Hovig E., Smith-Sorenson B., Montesano R. and Harris C.C. (1994) Database of p53 gene somatic mutations in human tumors and cell lines. Nucleic Acids Res. 22:3551-3555.
104. Horimoto, T., Rivera E., Pearson J., Senne D., Krauss S., Kawaoka Y. and Webster R.G. (1995) Origin and molecular changes associated with emergence of a highly pathogenic H5N2 influenza virus in Mexico. Virology 213:223-230.
105. Hu, S.L., Klaniecki J., Dykers T., Sridhar P. and Travis B.M. (1991) Neutralizing antibodies against HIV-1 BRU and SF2 isolates generated in mice immunized with recombinant vaccinia virus expressing HIV-1 (BRU) envelope glycoproteins and boosted with homologous gp160. AIDS Res. Hum. Retroviruses 7:615-620.
106. Hunter, E. (1997) gp41, a multifunctional protein involved in HIV entry and pathogenesis, p. III-55-III-73. In B. Korber, B. Hahn, B. Foley, J. W. Mellors, T. Leitner, G. Myers, F. McCutchan, and C. L. Kuiken (eds.), Human Retroviruses and AIDS 1997. Theoretical Biology and Biophysics Group, Los Alamos National Laboratory, Los Alamos, NM.
107. Inocencio, N.M., Sucic J.F., Moehring J.M., Spence M.J. and Moehring T.J. (1997) Endoprotease activities other than furin and PACE4 with a role in processing of HIV-I gp160 glycoproteins in CHO-K1 cells. J. Biol. Chem. 272:1344-1348.
108. Israeli, R.S., Powell C.T., Fair W.R. and Heston W.D. (1993) Molecular cloning of a complementary DNA encoding a prostate-specific membrane antigen. Cancer Res. 53:227-230.
109. Iwatani, Y., Kawano K., Ueno T., Tanaka M., Ishimoto A., Ito M. and Sakai H. (2001) Analysis of dominant-negative effects of mutant env proteins of human immunodeficiency virus type 1. Virology 286:45-53.
110. Jackson, I.J., Chambers D.M., Tsukamoto K., Copeland N.G., Gilbert D.J., Jenkins N.A., and Hearing V. (1992) A second tyrosinase-related protein, TRP-2, maps to and is mutated at the mouse slaty locus. EMBO J. 11:527-535.
111. Jelesarov, I. and Lu M. (2001) Thermodynamics of trimer-of-hairpins formation by the SIV gp41 envelope protein. J. Mol. Biol. 307:637-656.
112. Ji, H., Bracken C. and Lu M. (2000) Buried polar interactions and conformational stability in the simian immunodeficiency virus (SIV) gp41 core. Biochemistry 39:676-685.
113. Jiang, S., Lin K. and Lu M. (1998) A conformation-specific monoclonal antibody reacting with fusion-active gp41 from the human immunodeficiency virus type 1 envelope glycoprotein. J. Virol. 72:10213-10217.
114. Johnson, M.L., J.J. Correia, Yphantis D.A. and Halvorson H.R. (1981) Analysis of data from the analytical ultracentrifuge by nonlinear least- squares techniques. Biophys. J. 36:575-588.
115. Jones, D.H., McBride B.W., Roff M.A. and Farrar G.H. (1995) Efficient purification and rigorous characterization of a recombinant gp120 for HIV vaccine studies. Vaccine 13:991-999.
116. Jones, P.L., Korte T. and Blumenthal R. (1998) Conformational changes in cell surface HIV-1 envelope glycoproteins are triggered by cooperation between cell surface CD4 and co-receptors. J. Biol. Chem. 273:404-409.
117. Kang, C.Y., Hariharan K., Nara P.L., Sodroski J. and Moore J.P. (1994) Immunization with a soluble CD4-gp120 complex preferentially induces neutralizing anti-human immunodeficiency virus type 1 antibodies directed to conformation-dependent epitopes of gp120. J. Virol. 68:5854-62.
118. Kawakami, Y., Eliyahu S., Delagado C.H., Robbins P.F., Sakaguchi K., E. Appella, J.R. Yannelli, G.J. Adema, and S.A. Rosenberg. (1994b) Identification of a human melanoma antigen recognized by tumor-infiltrating lymphocytes associated with in vivo tumor rejection.Proc. Natl. Acad. Sci. U.S.A. 91:6458-6462.
119. Kawakami, Y., Eliyahu S., Sakaguchi K., Robbins P.F., Rivoltini L., Yannelli J.R. , Appella E., and Rosenberg S.A. (1994a) Identification of the immunodominant peptides of the MART-1 human melanoma antigen recognized by the majority of HLA-A2-restricted tumor infiltrating lymphocytes. J. Exp. Med. 180:347-352.
120. Kawaoka, Y. (1991) Structural features influencing hemagglutinin cleavability in a human influenza A virus. J. Virol. 65:1195-1201.
121. Kawaoka, Y. and Webster R.G. (1988) Sequence requirements for cleavage activation of influenza virus hemagglutinin expressed in mammalian cells. Proc. Natl. Acad. Sci. U.S.A. 85:324-328.
122. Kilby J.M., Hopkins S., Venetta T.M., DiMassimo B., Cloud G.A., Lee J.Y., Alldredge L., Hunter E., Lambert D., Bolognesi D., Matthews T., Johnson M.R., Nowak M.A., Shaw G.M. and Saag M.S. (1998) Potent suppression of HIV-1 replication in humans by T-20, a peptide inhibitor of gp41-mediated virus entry. Nat. Med. 4:1302-7.
123. Klasse, P.J., McKeating J.A., Schutten M., Reitz M.S. Jr. and Robert-Guroff M. (1993) An immune-selected point mutation in the transmembrane protein of human immunodeficiency virus type 1 (HXB2-Env:Ala 582(->Thr)) decreases viral neutralization by monoclonal antibodies to the CD4-binding site. Virology 196:332-337.
124. Klenk, H.D. and Garten W. (1994) Activation of cleavage of viral spike proteins by host proteases, p241-280. In E.Wimmer (ed.), Cellular receptors for animal viruses. Cold Spring Harbor Laboratory Press, Cold Spring Harbor, N.Y.
125. Klinman, D.M., Yamshchikov G and Ishigatsubo Y. (1997) Contribution of CpG motifs to the immunogenicity of DNA vaccines. J. Immunol. 158:3635-3639.
126. Kovacsovics-Bankowski, M. and Rock K.L. (1995) A phagosome-to-cytosol pathway for exogenous antigens presented on MHC class I molecules. Science 267:243-246.
127. Kozarsky, K., Penman M., Barsiripour L., Haseltine W., Sodroski J. and Krieger M. (1989) Glycosylation and processing of the human immunodeficiency virus type 1 envelope protein. J. Acquir. Immune Defic. Syndr. 2:163-169.
128. Kuiken C., Foley B., Hahn B., Marx P., McCutchan F., Mellors J., Mullins J., Wolinsky S. and Korber B. (2000) HIV Sequence Compendium. Theoretical Biology and Biophysics Group, Los Alamos National Laboratory
129. Kunkel, T.A., Roberts J.D. and Zakour R.A. (1987) Rapid and efficient site-specific mutagenesis without phenotypic selection, p. 367-382. In R. Wu and L. Grossman (eds.), Methods in Enzymology. Academic Press, San Diego.
130. Kwon, B.S., Haq A.K., Pomerantz S.H. and Halaban R. (1997) Isolation and sequence of a cDNA clone for human tyrosinase that maps at the mouse c-albino locus. Proc. Natl. Acad. Sci. U.S.A. 84:7473-7477.
131. Kwong, P.D., Wyatt R., Majeed S., Robinson J., Sweet R.W., Sodroski J., and Hendrickson W.A.. (2000b) Structures of HIV-1 gp120 envelope glycoproteins from laboratory- adapted and primary isolates. Structure. Fold. Des 8:1329-1339.
132. Kwong, P.D., Wyatt R., Robinson J., Sweet R.W., Sodroski J., and Hendrickson W.A. (1998) Structure of an HIV gp120 envelope glycoprotein in a complex with the CD4 receptor and a neutralizing human antibody. Nature 393:648-659.
133. Kwong, P.D., Wyatt R., Sattentau Q.J., Sodroski J. and Hendrickson W.A. (2000) Oligomeric modeling and electrostatic analysis of the gp120 envelope glycoprotein of human immunodeficiency virus. J. Virol. 74:1961-1972.
134. LaBranche, C.C., Sauter M.M., Haggarty B.S., Vance P.J., J. Romano, T. K. Hart, P. J. Bugelski, M. Marsh, and J. A. Hoxie. 1995. A single amino acid change in the cytoplasmic domain of the simian immunodeficiency virus transmembrane molecule increases envelope glycoprotein expression on infected cells. J. Virol. 69:5217-5227.
135. LaCasse, R.A., Follis K.E., Trahey M., Scarborough J.D., Littman D.R. and Nunberg J.H. (1999) Fusion-competent vaccines: broad neutralization of primary isolates of HIV. Science 283:357-362.
136. Lambin, P., Rochu D., Herance N. and Fine J.M. (1982) High molecular-weight polymers in human albumin solutions. Quantitative determination by polyacrylamide gradient electrophoresis Rev. Fr. Transfus. Immunohematol. 25:487-498. French.
137. Langhein, C. and Newman J.F. (1987) Antibody response to bacterial antigens covalently bound to biodegradable polymerized serum albumin beads. Journal of Applied Bacteriology 63:443-448.
138. Laue, T.M., Shah B.D., Ridgeway T.M. and Pelletier S.L. (1992) Computer-aided interpretation of analytical sedimentation data for proteins, p. 90-125. In S. E. Harding, A. J. Rowe, and J. C. Horton (eds.), Analytical ultracentrifugation in biochemistry and polymer science. Royal Society of chemistry, Cambridge, England.
139. Lewis, J.K., Bendahmane M., Smith T.J., Beachy R.N. and Siuzdak G. (1998) Identification of viral mutants by mass spectrometry. Proc. Nat. Acad. Sci. U.S.A. 95:8596-8601.
140. Liu, J., Shu W., Fagan M.B., Nunberg J.H. and Lu M. (2001) Structural and functional analysis of the HIV gp41 core containing an Ile573 to Thr substitution: implications for membrane fusion. Biochemistry 40:2797-2807.
141. Liu, J., Wang S., LaBranche C.C., Hoxie J.A. and Lu M. (2002) Mutations that destabilize the gp41core: determinants for stabilizing the SIV/CPmac envelope glycoproteins complex. J. Biol. Chem. in press.
142. Lu et al, (1998) AIDS Res. Human Retrovir. 14:151-155.
143. Lu, M., Ji H. and Shen S. (1999) Subdomain folding and biological activity of the core structure from human immunodeficiency virus type 1 gp41: implications for viral membrane fusion. J. Virol. 73:4433-4438.
144. Lu, M., S. Blackow, and P. Kim. 1995. A trimeric structural domain of the HIV-1 transmembrane glycoprotein. Nat. Struct. Biol. 2:1075-1085.
145. Lu, M., Stoller M.O., Wang S., Liu J., Fagan M.B. and Nunberg J.H. (2001) Structural and Functional Analysis of Interhelical Interactions in the Human Immunodeficiency Virus Type 1 gp41 Envelope Glycoprotein by Alanine-Scanning Mutagenesis. J. Virol. 75:11146-11156.
146. Lu, S., Wyatt R., Richmond J.F., Mustafa F., Wang S., Weng J., Montefiori D.C., Sodroski J., and Robinson H.L. (1998) Immunogenicity of DNA vaccines expressing human immunodeficiency virus type 1 envelope glycoprotein with and without deletions in the V1/2 and V3 regions. AIDS Res. Hum. Retrovir. 14:151-155.
147. Ly, A. and Stamatatos L. (2000) V2 loop glycosylation of the human immunodeficiency virus type 1 SF162 envelope facilitates interaction of this protein with CD4 and CCR5 receptors and protects the virus from neutralization by anti-V3 loop and anti-CD4 binding site antibodies. J. Virol. 74:6769-76.
148. Malashkevich, V.N., Chan D.C., Chutkowski C.T. and Kim P.S. (1998) Crystal structure of the simian immunodeficiency virus (SIV) gp41 core: conserved helical interactions underlie the broad inhibitory activity of gp41 peptides. Proc. Natl. Acad. Sci. U.S.A 95:9134-9139.
149. Mascola, J.R., Snyder S.W., Weislow O.S., Belay S.M., Belshe R.B., Schwartz D.H., Clements M.L., Dolin R., Graham B.S, Gorse G.J., Keefer M.C., McElrath M.J., Walker M.C., Wagner K.F., McNeil J.G., McCutchan F.E. and Burke D.S. (1996) Immunization with envelope subunit vaccine products elicits neutralizing antibodies against laboratory-adapted but not primary isolates of human immunodeficiency virus type 1. The National Institute of Allergy and Infectious Diseases AIDS Vaccine Evaluation Group. Infec. Dis. 173:340-348.
150. McCune, J.M., Rabin L.B., Feinberg M.B., Lieberman M., Kosek J.C., Reyes G.R., and Weissman I.L. (1988) Endoproteolytic cleavage of gp160 is required for the activation of human immunodeficiency virus. Cell 53:55-67.
151. McKeating, J.A., A. McKnight, and J.P. Moore. 1991. Differential loss of envelope glycoprotein gp120 from virion of human immunodeficiency virus type 1 isolates: effects on infectivity and neutralization. J. Virol. 65:852-860.
152. Melikyan, G.B., Markosyan R.M., Hemmati H., Delmedico M.K., Lambert D.M., and Cohen F.S. (2000) Evidence that the transition of HIV-1 gp41 into a 6-helix bundle, not the bundle configuration, induces membrane fusion. J. Cell Biol. 151:413-423.
153. Men, Y., Gander B., Merkle H.P., and Corradin G. (1996) Induction of sustained and elevated immune responses to weakly immunogenic synthetic malarial peptides by encapsulation in biodegradable polymer microspheres. Vaccine 14:1442-1450.
154. Merat, R., Raoul H., Leste-Lasserre T., Sonigo P. and Pancino G. (1999) Variable constraints on the principal immunodominant domain of the transmembrane glycoprotein of human immunodeficiency virus type 1. J. Virol. 73:5698-5706.
155. Miyahira, Y., Murata K., Rodriguez D., Rodriguez J.R., Esteban M., Rodrigues M.M. and Zavala F. (1995) Quantification of antigen specific CD8+ T cells using an ELISPOT assay. J. Immunol. Methods 181:45-54.
156. Molloy, S., Thomas L., VanSlyke J.K., Stenberg P.E. and Thomas G. (1994) Intracellular trafficking and activation of the furin proprotein convertase: localization to the TGN and recycling from the cell surface. EMBO J. 13:18-33.
157. Molloy, S.S., Bresnahan P.A., Leppla S.H., Klimpel K.R. and Thomas G. (1992) Human furin is a calcium-dependent serine endoprotease that recognizes the sequence Arg-X-X-Arg and efficiently cleaves anthrax toxin protective antigen. J. Biol. Chem. 267:16396-16402.
158. Moore, J.P. and Binley J. (1998) HIV. Envelope's letters boxed into shape. Nature 393:630-631.
159. Moore, J.P. and Sodroski J. (1996) Antibody cross-competition analysis of the human immunodeficiency virus type 1 gp120 exterior envelope glycoprotein. J. Virol. 70:1863-1872.
160. Moore, J.P. and Stevenson M. (2000) New targets for inhibitors of HIV-1 replication. Nat. Rev. Mol. Cell Biol. 1:40-49.
161. Moore, J.P., and Ho D.D. (1995b) HIV-1 neutralization: the consequences of viral adaptation to growth on transformed T cells. AIDS 9 (Suppl. A):S117-S136.
162. Moore, J.P., McKeating J.A., Weiss R.A. and Sattentau Q.J. (1990) Dissociation of gp120 from HIV-1 virions induced by soluble CD4. Science 250:1139-1142.
163. Moore, J.P., Parren P.W.H.I. and Burton D.R. (2001) Genetic Subtypes, Humoral Immunity, and Human Immunodeficiency Virus Type 1 Vaccine Development. J. Virol. 75:5721-5729.
164. Moore, J.P., Sattentau Q.J., Wyatt R. and Sodroski J. (1994a) Probing the structure of the human immunodeficiency virus surface glycoprotein gp120 with a panel of monoclonal antibodies. J. Virol 68:469-484.
165. Moore, J.P., Trkola A., Korber B., Boots L.J., Kessler J.A. 2nd, McCutchan F.E., Mascola J., Ho D.D., Robinson J. and Conley A.J. (1995) HIV-1 neutralization: the consequences of viral adaptation to growth on transformed T cells. AIDS. 9 [Suppl A]: S117-S136.
166. Moore, J.P., Trkola A., Korber B., Boots L.J., Kessler J.A. 2nd, McCutchan F.E., Mascola J., Ho D.D., Robinson J. and Conley A.J. (1995a) A human monoclonal antibody to a complex epitope in the V3 region of gp120 of human immunodeficiency virus type 1 has broad reactivity within and outside clade B. J. Virol. 69:122-130.
167. Moore, J.P., Willey R.L., Lewis G.K., Robinson J., and Sodroski J. (1994b) Immunological evidence for interactions between the first, second, and fifth conserved domains of the gp120 surface glycoprotein of human immunodeficiency virus type 1. J. Virol. 68:6836-6847.
168. Mori, K., Yasutomi Y., Ohgimoto S., Nakasone T., Takamura S., Shioda T. and Nagai Y. (2001) Quintuple deglycosylation mutant of simian immunodeficiency virus SIVmac239 in rhesus macaques: robust primary replication, tightly contained chronic infection, and elicitation of potent immunity against the parental wild-type strain. J. Virol. 75:4023-4028.
169. Morikawa, Y., Barsov E. and Jones I. (1993) Legitimate and illegitimate cleavage of human immunodeficiency virus glycoproteins by furin. J. Virol. 67:3601-3604.
170. Moulard, M. and Decroly E. (2000) Maturation of HIV envelope glycoprotein precursors by cellular endoproteases. Biochim. Biophys. Acta 1469:121-132.
171. Moulard, M. and Decroly E. (2000) Maturation of HIV envelope glycoprotein precursors by cellular proteases. Biochem. Biophys. Acta 1469:121-132.
172. Moulard, M., Chaloin L, Canarelli L., Mabrouk K., Darbon H. and Challoin L. (1998) Retroviral envelope processing: structural investigation of the cleavage site. Biochemistry 37:4510-7.
173. Moulard, M., Hallenberger S., Garten W. and Klenk H.D. (1999) Processing and routage of HIV glycoproteins by furin to the cell surface. Virus Res. 60:55-65.
174. Muster, et al. (1994) Cross-neutralizing activity against divergent human immunodeficiency virus type 1 isolates induced by the gp41 sequence ELDKWAS. J. Virol. 68:4031-4034.
175. Muster, T., Steindl F., Purtscher M., Trkola A., Klima A., Himmler G., Ruker F. and Katinger H. (1993) A conserved neutralizing epitope on gp41 of human immunodeficiency virus type 1. J. Virol. 67:6642-6647.
176. Myszka, D.G., Sweet R.W., Hensley P., Brigham-Burke M., Kwong P.D., Hendrickson W.A., Wyatt R., Sodroski J. and Doyle M.L. (2000) Energetics of the HIV gp120-CD4 binding reaction. Proc. Natl. Acad. Sci. U.S.A. 97:9026-9031.
177. Nicholls, A., K.A. Sharp and Honig B. (1991) Protein folding and association: insights from the interfacial and thermodynamic properties of hydrocarbons. Proteins 11:281-296.
178. Nyambi, P.N., Gorny M.K., Bastiani L., van der G.G., Williams C., and Zolla-Pazner S. (1998) Mapping of epitopes exposed on intact human immunodeficiency virus type 1 (HIV-1) virions: a new strategy for studying the immunologic relatedness of HIV-1. J. Virol. 72:9384-9391.
179. O'Brien, W.A., Koyanagi, Namazie A., Zhao J.Q., Diagne A., Idler K., Zack J.A. and Chen I.S. (1990) HIV-1 tropism for mononuclear phagocytes can be determined by regions of gp120 outside the CD4-binding domain. Nature 348:69-73.
180. O'Hagan, D.T., Jeffery H. and Davis S.S. (1993) Longterm antibody responses in mice following subcutaneous immunization with ovalbumin entrapped in biodegradable microparticles. Vaccine 11:965-969.
181. Ohuchi, M., Orlich M., Simpson B. and Rott R. (1989) Mutations at the cleavage site of the hemagglutinin alter the pathogenicity of influenza virus A/Chick/Penn/83 (H5/N2). J. Virol. 68:274-280.
182. Okayama, H. and Berg P. (1983) A cDNA cloning vector that permits expression of cDNA inserts in mammalian cells. Mol. Cell. Biol. 3(2):280-289.
183. Okumura, Y., Yano M., Murakami M., Mori S., Towatari T. and Kido H. (1999) The extracellular processing of HIV-1 envelope glycoprotein gp160 by human plasmin. FEBS Lett. 442: 39-42.
184. Olofsson, S. and Hansen J.E. (1998) Host cell glycosylation of viral glycoproteins--a battlefield for host defence and viral resistance. Scand. J. Infect. Dis. 30:435-440.
185. Olson, W.C., Rabut G.E., Nagashima K.A., Tran D.N., Anselma D.J., Monard S.P., Segal J.P., Thompson D.A., Kajumo F., Guo Y., Moore J.P., Maddon P.J. and Dragic T. (1999) Differential inhibition of human immunodeficiency virus type 1 fusion, gp120 binding, and CC-chemokine activity by monoclonal antibodies to CCR5. J. Virol. 73:4145-4155.
186. Overwijk, W.W., Tsung A., Irvine K.R., Parkhurst M.R., Goletz T.J., Tsung K., Carroll M.W., Liu C., Moss B., Rosenberg S.A., and Restifo N.P. (1998) Gp100/pmel 17 is a murine tumor rejection antigen: induction of "self"-reactive, tumoricidal T cells using high-affinity, altered peptide ligand. J. Exp. Med. Vol 188(2):277-286.
187. Owens, J.M., Matsuo K., Nicholson G.C., Wagner E.F. and Chambers T.J. (1999) Fra-1 potentiates osteoclastic differentiation in osteoclast-macrophage precursor cell lines. Virology 179:827-833.
188. Owens, R.J., and R.W. Compans. (1990) The human immunodeficiency virus type 1 envelope glycoprotein precursor acquires aberrant intermolecular disulfide bonds that may prevent normal proteolytic processing. Virology 179:827-833.
189. Paredes, A.M., Heidner H., Thuman-Commike P., Venkataram Prasad B.V., Johnston R.E. and Chiu W. (1998) Structural localization of the E3 glycoprotein in attenuated sindbis virus mutants. J. Virol. 72:1534-1541.
190. Parker, C.E., Deterding L.J., Hager-Braun C., Binley J.M., Schulke N., Katinger H., Moore J.P., and Tomer K.B. (2001) Fine definition of the epitope on the gp41 glycoprotein of human immunodeficiency virus type 1 for the neutralizing monoclonal antibody 2F5. J. Virol. 75:10906-10911.
191. Parren, P.W., Burton D.R. and Sattentau Q.J. (1997) HIV-1 antibody--debris or virion? Nat. Med. 3:366-367.
192. Parren, P.W., Fisicaro P., Labrijn A.F., Binley J.M., Yang W.P., Ditzel H.J., Barbas C.F. 3rd and Burton D.R. (1996) In vitro antigen challenge of human antibody libraries for vaccine evaluation: the human immunodeficiency virus type 1 envelope. J. Virol. 70:9046-9050.
193. Parren, P.W., Mondor I., Naniche D., Ditzel H.J., Klasse P.J., Burton D.R. and Sattentau Q.J. (1998) Neutralization of human immunodeficiency virus type 1 by antibody to gp120 is determined primarily by occupancy of sites on the virion irrespective of epitope specificity. J. Virol. 72:3512-3519.
194. Parren, P.W.H.I. and Burton D.R. (2001) The antiviral activity of antibodies in vitro and in vivo. Adv. Immunol. 77:195-262.
195. Parren, P.W.H.I., Moore J.P., Burton D.R. and Sattentau Q.J. (1999) The neutralizing antibody response to HIV-1: viral evasion and escape from humoral immunity. AIDS 13 (Suppl. A):S137-S162.
196. Partidos, C.D., Vohra P., Anagnostopoulou C., Jones D.H., Farrar G.H. and Steward M.W. (1996) Biodegradable microparticles as a delivery system for measles virus cytotoxic T cell epitopes. Mol. Immunol. 33:485-491.
197. Pertmer, T.M., Roberts T.R. and Haynes J.R. (1996) Influenza virus nucleoprotein-specific immunoglobulin G subclass and cytokine responses elicited by DNA vaccination are dependent on the route of vector DNA delivery. J. Virol. 70(9):6119-6125.
198. Plaimauer, B., Mohr G. Wernhart W., Himmelspach M., Dorner F. and Schlokat U. (2001) 'Shed' furin: mapping of the cleavage determinants and identification of its C-terminus. Biochem. J. 354:689-695.
199. Poignard, P., Saphire E.O., Parren P.W. and Burton D.R. (2001) gp120: Biologic aspects of structural features. Annu. Rev. Immunol. 19:253-274.
200. Polacino, P.S., Stallard V., Klaniecki J.E., Pennathur S., Montefiori D.C., Langlois A.J., Richardson B.A., Morton W.R., Benveniste R.E. and Hu S.L. (1999) Role of immune responses against the envelope and the core antigens of simian immunodeficiency virus SIVmne in protection against homologous cloned and uncloned virus challenge in macaques. J. Virol. 73:8201-8215.
201. Poumbourios, P., Wilson K.A., Center R.J., El Ahmar W. and Kemp B.E. (1997) Human immunodeficiency virus type 1 envelope glycoprotein oligomerization requires the gp41 amphipathic alpha-helical/leucine zipper-like sequence. J. Virol. 71:2041-2049.
202. Powell, M.F., Cleland J.L., Eastman D.J., Lim A., Murthy K., Newman M.J., Nunberg J.H., Weissburg R.P., Vennari J.C. and Wrin T. (1994) Immunogenicity and HIV-1 virus neutralization of MN recombinant glycoprotein 120/HIV-1 QS21 vaccine in baboons [published erratum appears in AIDS Res Hum Retroviruses 1995 11(5):661]. AIDS Res. Hum. Retroviruses 10 Suppl 2:S105-8:S105-S108.
203. Prayaga, S.K., Ford M.J. and Haynes J.R. (1997) Manipulation of HIV-1 gp120-specific immune responses elicited via gene gun-based DNA immunization. Vaccine. 15(12-13):1349-52.
204. Qiao, H., Pelletier S.L., Hoffman L., Hacker J., Armstrong R.T., and White J.M. (1998) Specific single or double proline substitutions in the "spring-loaded" coiled-coil region of the influenza hemagglutinin impair or abolish membrane fusion activity. J. Cell Biol. 141:1335-1347.
205. Raychaudhuri, S. and Rock K.L. (1998) Fully mobilizing host defense: building better vaccines. Nat. Biotechnol. 16:1025-1031.
206. Raz, E., Tighe H., Sato Y., Corr M., Dudler J.A., Roman M., Swain S.L., Spiegelberg H.L. and Carson D.A. (1996) Preferential induction of a Th1 immune response and inhibition of specific IgE antibody formation by plasmid DNA immunization. Proc. Natl. Acad. Sci. U.S.A. 93(10):5141-5145.
207. Reitter, J.N., Means R.E. and Desrosiers R.C. (1998) A role for carbohydrates in immune evasion in AIDS. Nat. Med. 4:679-684.
208. Reitz, M.S. Jr, Wilson C., Naugle C., Gallo R.C. and Robert-Guroff M. (1988) Generation of a neutralization-resistant variant of HIV-1 is due to selection for a point mutation in the envelope gene. Cell 54:57-63.
209. Richardson, T.M., Jr., Stryjewski B.L., Broder C.C., Hoxie J.A., Mascola J.R., Earl P.L. and Doms R.W. (1996) Humoral response to oligomeric human immunodeficiency virus type 1 envelope protein. J. Virol. 70:753-62.
210. Rizzuto, C.D., Wyatt R., Hernandez-Ramos N., Sun Y., Kwong P.D., Hendrickson W.A. and Sodroski J. (1998) A conserved HIV gp120 glycoprotein structure involved in chemokine receptor binding. Science 280:1949-1953.
211. Robinson, H.L. (1997) Nucleic acid vaccines: an overview. Vaccine. 15(8):785-787.
212. Robinson, W.E.Jr., Kawamura T., Gorny M.K., Lake D., Xu J.Y., Matsumoto Y., Sugano T., Masuho Y., Mitchell W.M., Hersh E. and Zolla-Pazner S. (1990) Human monoclonal antibodies to the human immunodeficiency virus type 1 (HIV-1) transmembrane glycoprotein gp41 enhance HIV-1 infection in vitro. Proc. Natl. Acad. Sci. U.S.A. 87:3185-3189.
213. Rock, K.L. and Clark K. (1996) Analysis of the role of MHC class II presentation in the stimulation of cytotoxic T lymphocytes by antigens targeted into the exogenous antigen- MHC class I presentation pathway. J. Immunol. 156:3721-3726.
214. Rodriguez, D., Rodriguez J.R. and Esteban M. (1995) Enhanced proteolytic processing of the human immunodeficiency virus type 1 envelope protein in murine Ltk(-) cells. AIDS Res. Hum. Retroviruses 11:81-85.
215. Roux, K.H. (1989) Immunoelectron microscopy of idiotype-anti-idiotype complexes. Methods Enzymol. 178:130-144.
216. Roux, K.H. (1996) Negative-Stain Immunoelectron-Microscopic Analysis of Small Macromolecules of Immunologic Significance Methods 10:247-256.
217. Sakurai, H., R.A. Williamson, Crowe J.E., Beeler J.A., Poignard P., Bastidas R.B., Chanock R.M. and Burton D.R. (1999) Human antibody responses to mature and immature forms of viral envelope in respiratory syncytial virus infection: significance for subunit vaccines. J. Virol. 73:2956-2962.
218. Salminen, A., Wahlberg J.M., Lobigs M., Liljestrom P. and Garoff H. (1992) Membrane fusion process of Semliki Forest virus. II: cleavage-dependent reorganization of the spike protein complex controls virus entry. J. Cell Biol. 116:349-357.
219. Sanders, R.W., Schiffner L., Master A., Kajumo F., Guo Y., Dragic T., Moore J.P. and Binley J.M. (2000) Variable-loop-deleted variants of the human immunodeficiency virus type 1 envelope glycoprotein can be stabilized by an intermolecular disulfide bond between the gp120 and gp41 subunits. J. Virol. 74:5091-5100.
220. Sanders, R.W., Venturi M., Schiffner L., Kalyanaraman R., Katinger H., Kwong P.D., and Moore J.P. (2002) The mannose-dependent epitope for the neutralizing antibody 2G12 on the gp120 glycoprotein of human immunodeficiency virus type 1. J. Virol. 76:7293-7305.
221. Saphire, E.O., Parren P.W., Pantophlet R., Zwick M.B., Morris G.M., Rudd P.M., Dwek R.A., Stanfield R.L., Burton D.R. and Wilson I.A. (2001) Crystal structure of a neutralizing human IGG against HIV-1: a template for vaccine design. Science 293:1155-1159.
222. Sattentau, Q.J. and Moore J.P. (1991) Conformational changes induced in the human immunodeficiency virus envelope glycoprotein by soluble CD4 binding. J. Exp. Med. 174:407-415.
223. Sattentau, Q.J., and Moore J.P. (1995a) Human immunodeficiency virus type 1 neutralization is determined by epitope exposure on the gp120 oligomer. J. Exp. Med. 182:185-196.
224. Sattentau, Q.J., Zolla-Pazner S., and Poignard P. (1995b) Epitope exposure on functional, oligomeric HIV-1 gp41 molecules. Virology 206:713-717.
225. Scandella, C.J., Kilpatrick J., Lidster W., Parker C., Moore J.P., Moore G.K., Mann K.A., Brown P., Coates S., Chapman B., et al. (1993) Nonaffinity purification of recombinant gp120 for use in AIDS vaccine development. AIDS Res. Hum. Retroviruses 9:1233-1244.
226. Schägger, H., Cramer W.A. and von Jagow G. (1994) Analysis of molecular masses and oligomeric states of protein complexes by blue native electrophoresis and isolation of membrane protein complexes by two-dimensional native electrophoresis. Analytical Biochemistry 217:220-230.
227. Schalich, J., Allison S.L., Stiasny K., Mandl C.W., Kunz C. and Heinz F.X. (1996) Recombinant subviral particles from tick-borne encephalitis virus are fusogenic and provide a model system for studying flavivirus envelope glycoprotein functions. J. Virol. 70:4549-4557.
228. Schneider, J., Kaaden O., Copeland T.D., Oroszlan S. and Hunsmann G. (1986) Shedding and interspecies type sero-reactivity of the envelope glycopolypeptide gp120 of the human immunodeficiency virus. J. Gen. Virol. 67:2533-2538.
229. Schulke, N., Vesanen M.S., Sanders R.W., Zhu P., Anselma D.J., Villa A.R., Parren P. W. H. I., Binley J.M., Roux K.H., Maddon P.J., Moore J.P. and Olson W.C. (2002) Oligomeric and conformational properties of a proteolytically mature, disulfide-stabilized human immunodeficiency virus type 1 gp140 envelope glycoprotein.submitted. J. Virol. 76:8875-8889.
230. Schulz, T.F., Reeves J.D., Hoad J.G., Tailor C., Stephens P., Clements G., Ortlepp S., Page K.A., Moore J.P. and Weiss A. (1993) Effect of mutations in the V3 loop of HIV-1 gp120 on infectivity and susceptibility to proteolytic cleavage. AIDS Res. Hum. Retroviruses 9:159-166.
231. Schulz, T.F., Jameson B.A., Lopalco L., Siccardi A.G., Weiss R.A. and Moore J.P. (1992) Conserved structural features in the interaction between retroviral surface and transmembrane glycoproteins? AIDS Res. Human Retrovir. 8: 1571-1580.
232. Sergel, T.A., McGinnes L.W. and Morrison T.G. (2001) Mutations in the fusion peptide and adjacent heptad repeat inhibit folding or activity of the Newcastle Disease Virus fusion protein. J. Virol. 75:7934-7943.
233. Shibata, R., Hoggan M.D., Broscius C., Englund G., Theodore T.S., Buckler-White A., Arthur L.O., Israel Z., Schultz A., Lane H.C., et al. (1995) Isolation and characterization of a syncytium-inducing, macrophage/T-cell line-tropic human immunodeficiency virus type 1 isolate that readily infects chimpanzee cells in vitro and in vivo. J. Virol 69:4453-4462.
234. Shu, W., Ji H. and Lu M. (1999) Trimerization specificity in HIV-1 gp41: analysis with a GCN4 leucine zipper model. Biochemistry 38:5378-5385.
235. Shugars, D.C., Wild C.T., Greenwell T.K. and Matthews T.J. (1996) Biophysical characterization of recombinant proteins expressing the leucine zipper-like domain of the human immunodeficiency virus type 1 transmembrane protein gp41. J. Virol. 70:2982-2991.
236. Singh, J. and Compton T. (2000) Characterization of a panel of insertion mutants in human cytomegalovirus glycoprotein B. J. Virol.72:1383-1392.
237. Sjolander, A., van't Land B. and Lovgren Bengtsson K. (1997). Iscoms containing purified Quillaja saponins upregulate both Th1-like and Th2-like immune responses. Cell. Immunol. 177(1):69-76.
238. Spies, C.P. and Compans R.W. (1994) Effects of cytoplasmic domai length on cell surface expression and syncytium-forming capacity of the simian immunodeficiency virus envelope glycoprotein. Virology 203:8-19.
239. Srivastava, I.K., Stamatatos L., Legg H., Kan E., Coates S., Leung L., Fong A., Wininger M., Tipton A.R., Donnelly J., Ulmer J.B. and Barnett S.W. (2002) Purification and characterization of oligomeric glycoprotein from a primary R5 subtype B human immunodeficiency virus for vaccine applications. J. Virol. 76:2835-2847.
240. St. Clair, N., Shenoy B., Jacob L.D. and Margolin A.L. (1999) Cross-linked protein crystals for vaccine delivery. Proc. Nat. Acad. Sci. U.S.A. 96:9469-9474.
241. Stadler, K., Allison S.L., Schalich J. and Heinz F.X. (1997) Proteolytic activation of tick-borne encephalitis virus by furin. J. Virol.71:8475-8481.
242. Stamatatos, L., Lim M. and Cheng-Mayer C. (2000) Generation and structural analysis of soluble oligomeric gp140 envelope proteins derived from neutralization-resistant and neutralization-susceptible primary HIV type 1 isolates. AIDS Res. Hum. Retrovir. 16:981-994.
243. Staropoli, I., Chanel C., Girard M. and Altmeyer R. (2000) Processing, stability and receptor binding properties of oligomeric envelope glycoprotein from a primary HIV-1 isolate. J. Biol. Chem. 275:35137-35145.
244. Stein, B.S., and Engleman E.G. (1990) Intracellular processing of the gp160 HIV-1 envelope precursor. Endoproteolytic cleavage occurs in a cis or medial compartment of the Golgi complex. J. Biol. Chem. 265:2640-2649.
245. Stiegler, G., Kunert R., Purtscher M., Wolbank S., Voglauer R., Steindl F. and Katinger H. (2001) A potent cross-clade neutralizing human monoclonal antibody against a novel epitope on gp41 of human immunodeficiency virus type 1. AIDS Res. Hum. Retrovir. 17:1757-1765.
246. Sugrue, R.J., Brown C., Brown G., Aitken J. and McL Rixon H.W. (2001) Furin cleavage of the respiratory syncytial virus fusion protein is not a requirement for its transport to the surface of virus-infected cells. J. Gen. Virol. 82:1375-1386.
247. Sullivan, N., Sun Y., Sattentau Q., Thali M., Wu D., Denisova G., Gershoni J., Robinson J., Moore J. and Sodroski J. (1998) CD4-Induced conformational changes in the human immunodeficiency virus type 1 gp120 glycoprotein: consequences for virus entry and neutralization. J. Virol. 72:4694-4703.
248. Syu, W.J., Lee W.R., Du B., Yu Q.C., Essex M., and Lee T.H. (1991) Role of conserved gp41 cysteine residues in the processing of human immunodeficiency virus envelope precursor and viral infectivity. J. Virol. 65:6349-6352.
249. Takeuchi, Y., Inagaki M., Kobayashi N. and Hoshino H. (1987) Isolation of human immunodeficiency virus from a Japanese hemophilia B patient with AIDS. Jpn. J. Cancer Res. 78:11-15.
250. Tan, K., Liu J., Wang J., Shen S. and Lu M. (1997) Atomic structure of a thermostable subdomain of HIV-1 gp41. Proc. Natl. Acad. Sci. U.S.A 94:12303-12308.
251. Taniguchi, Y., Zolla-Pazner S., Xu Y., Zhang X., Takeda S. and Hattori T. (2000) Human monoclonal antibody 98-6 reacts with the fusogenic form of gp41. Virology 273:333-340.
252. Thali, M., Moore J.P., Furman C., Charles M., Ho D.D., Robinson J. and Sodroski J. (1993) Characterization of conserved human immunodeficiency virus type 1 gp120 neutralization epitopes exposed upon gp120-CD4 binding. J. Virol. 67:3978-3988.
253. Thomas, G., Thorne B.A., Thomas L., Allen R.G., Hruby D.E., Fuller R. and Thorner J. (1988) Yeast KEX2 endopeptidase correctly cleaves a neuroendocrine prohormone in mammalian cells. Science 241:226-230.
254. Travis, B.M., Dykers T.I., Hewgill D., Ledbetter J., Tsu T.T., Hu S.L. and Lewis J.B. (1992) Functional roles of the V3 hypervariable region of HIV-1 gp160 in the processing of gp160 and in the formation of syncytia in CD4+ cells. Virology 186:313-317.
255. Trkola, A., Dragic T., Arthos J., Binley J.M., Olson W.C., Allaway G.P., Cheng-Mayer C., Robinson J., Maddon P.J. and Moore J.P. (1996a) CD4-dependent, antibody-sensitive interactions between HIV-1 and its co-receptor CCR-5. Nature 384:184-187.
256. Trkola, A., Matthews J., Gordon C., Ketas T. and Moore J.P. (1999) A cell line-based assay for primary human immunodeficiency virus type 1 isolates that use either the CCR5 or the CXCR4 coreceptor. J. Virol. 73:8966-8974.
257. Trkola, A., Ketas T., Kewalramani V.N., Endorf F., Binley J.M., Katinger H., Robinson J., Littman D.R. and Moore J.P. (1998) Neutralization sensitivity of human immunodeficiency virus type 1 primary isolates to antibodies and CD4-based reagents is independent of coreceptor usage. J. Virol. 72:1876-1885.
258. Trkola, A., Pomales A.B., Yuan H., Korber B., Maddon P.J., Allaway G.P., Katinger H., Barbas C.F. 3rd, Burton D.R., Ho D.D., et al. (1995) Cross-clade neutralization of primary isolates of human immunodeficiency virus type 1 by human monoclonal antibodies and tetrameric CD4-IgG. J. Virol. 69:6609-6617.
259. Trkola, A., Purtscher M., Muster T., Ballaun C., Buchacher A., Sullivan N., Srinivasan K., Sodroski J., Moore J.P. and Katinger H. (1996b) Human monoclonal antibody 2G12 defines a distinctive neutralization epitope on the gp120 glycoprotein of human immunodeficiency virus type 1. J. Virol. 70:1100-1108.
260. Tschachler, E., Buchow H., Gallo R.C. and Reitz M.S. Jr., (1990) Functional contribution of cysteine residues to the human immunodeficiency virus type 1 envelope. J. Virol. 64:2250-2259.
261. VanCott, T.C., Mascola J.R., Kaminski R.W., Kalyanaraman V., Hallberg P.L., Burnett P.R., Ulrich J.T., Rechtman D.J. and Birx D.L. (1997) Antibodies with specificity to native gp120 and neutralization activity against primary human immunodeficiency virus type 1 isolates elicited by immunization with oligomeric gp160. J. Virol. 71:4319-4330.
262. Venkatesh, S.G. and Deshpande V. (1999) A comparative review of the structure and biosynthesis of thyroglobulin. Comp. Biochem. Physiol. C. Pharmacol. Toxicol. Endocrinol. 122:13-20.
263. Vidard, L., Kovacsovics-Bankowski M., Kraeft S.K., Chen L.B., Benacerraf B., and Rock K.L. (1996) Analysis of MHC class II presentation of particulate antigens of B lymphocytes. J. Immunol. 156:2809-2818.
264. Volchkov V., Feldmann H., Volchkova V.A. and Klenk H.D. (1998) Processing of the Ebola virus glycoprotein by the proprotein convertase furin. Proc. Natl. Acad. Sci. U.S.A. 95:5762-5767.
265. Vollenweider, F., Benjannet S., Decroly E., Savaria D., Lazure C., Thomas G., Chretien M. and Seidah N.G. (1996) Comparative cellular processing of the human immunodeficiency virus (HIV-1) envelope glycoprotein gp160 by the mammalian subtilisin/kexin-like convertases. Biochem. J. 314:521-532.
266. Weidmann A., Maisner A., Garten W., Seufert M., ter Meulen V. and Schneider-Schaulies S. (2000) Proteolytic cleavage of the fusion protein but not membrane fusion is required for measles virus-induced immunosuppression in vitro. J. Virol. 74:1895-1893.
267. Weissenhorn, W., Dessen A., Harrison S.C., Skehel J.J. and Wiley D.C. (1997) Atomic structure of the ectodomain from HIV-1 gp41. Nature 387:426-430.
268. Weng, Y. and Weiss C.D. (1998) Mutational analysis of residues in the coiled-coil domain of human immunodeficiency virus type 1 transmembrane protein gp41. J. Virol. 72:9676-9682.
269. Weng, Y., Z. Yang, and Weiss C.D. (2000) Structure-function studies of the self-assembly domain of the human immunodeficiency virus type 1 transmembrane protein gp41. J. Virol. 74:5368-5372.
270. White-Scharf, M.E., Potts B.J., Smith L.M., Sokolowski K.A., Rusche J.R. and Silver S. (1993) Broadly neutralizing monoclonal antibodies to the V3 region of HIV-1 can be elicited by peptide immunization. Virology 192:197-206.
271. Willey, R.L., Bonifacino J.S., Potts B.J., Martin M.A. and Klausner R.D. (1988) Biosynthesis, cleavage, and degradation of the human immunodeficiency virus 1 envelope glycoprotein gp160. Proc. Natl. Acad. Sci. U.S.A. 85:9580-9584.
272. Willey, R.L., Klimkait T., Frucht D.M., Bonifacino J.S. and Martin M.A. (1991) Mutations within the human immunodeficiency virus type 1 gp160 envelope glycoprotein alter its intracellular transport and processing. Virology 184:313-321.
273. Wu, L., Gerard N.P., Wyatt R., Choe H., Parolin C., Ruffing N., Borsetti A., Cardoso A.A., Desjardin E., Newman W., Gerard C. and Sodroski J. (1996) CD4-induced interaction of primary HIV-1 gp120 glycoproteins with the chemokine receptor CCR-5. Nature 384:179-183.
274. Wyatt, R. and Sodroski J. (1998b) The HIV-1 envelope glycoproteins: fusogens, antigens, and immunogens. Science 280:1884-1888.
275. Wyatt, R., Desjardin E., Olshevsky U., Nixon C., Binley J., Olshevsky V. and Sodroski J. (1997) Analysis of the interaction of the human immunodeficiency virus type 1 gp120 envelope glycoprotein with the gp41 transmembrane glycoprotein. J. Virol. 71:9722-9731.
276. Wyatt, R., Kwong P.D., Desjardins E., Sweet R.W., Robinson J., Hendrickson W.A. and Sodroski J.G. (1998a) The antigenic structure of the HIV gp120 envelope glycoprotein. Nature 393:705-711.
277. Wyatt, R., Moore J., Accola M., Desjardin E., Robinson J. and Sodroski J. (1995) Involvement of the V1/V2 variable loop structure in the exposure of human immunodeficiency virus type 1 gp120 epitopes induced by receptor binding. J. Virol. 69:5723-5733.
278. Xu, J.Y., Gorny M.K., Palker T., Karwowska S. and Zolla-Pazner S. (1991) Epitope mapping of two immunodominant domains of gp41, the transmembrane protein of human immunodeficiency virus type 1, using ten human monoclonal antibodies. J. Virol. 65:4832-4838.
279. Yamshchikov, G.V., Ritter G.D., Vey M. and Compans R.W. (1995) Assembly of SIV virus-like particles containing envelope proteins using a baculovirus expression system. Virology 214:50-58.
280. Yang, X., Farzan M., Wyatt R. and Sodroski J. (2000b) Characterization of stable, soluble trimers containing complete ectodomains of human immunodeficiency virus type 1 envelope glycoproteins. J. Virol. 74:5716-5725.
281. Yang, X., Florin L., Farzan M., Kolchinsky P., Kwong P.D., Sodroski J. and Wyatt R. (2000a) Modifications that stabilize human immunodefiiency virus envelope glycoprotein trimers in solution. J. Virol. 74:4746-4754.
282. Yang, X., Wyatt R. and Sodroski J. (2001) Improved elicitation of neutralizing antibodies against primary human immunodeficiency viruses by soluble stabilized envelope glycoprotein trimers. J. Virol. 75:1165-1171.
283. York, J., Follis K.E., Trahey M., Nyambi P.N., Zolla-Pazner S. and Nunberg J.H. (2001) Antibody binding and neutralization of primary and T-cell line-adapted isolates of human immunodeficiency virus type 1. J. Virol. 75:2741-2752.
284. Zhang, C.W., Chishti Y., Hussey R.E., and Reinherz E.L. (2001) Expression, purification, and characterization of recombinant HIV gp140. The gp41 ectodomain of HIV or simian immunodeficiency virus is sufficient to maintain the retroviral envelope glycoprotein as a trimer. J. Biol. Chem. 276:39577-39585.
285. Zhang, W., Canziani G., Plugariu C., Wyatt R., Sodroski J., Sweet R., Kwong P., Hendrickson W. and Chaiken I. (1999) Conformational changes of gp120 in epitopes near the CCR5 binding site are induced by CD4 and a CD4 miniprotein mimetic. Biochemistry 38:9405-9416.
286. Zhu, P., Olson W.C. and Roux K.H. (2001) Structural flexibility and functional valence of CD4-IgG2 (PRO 542): potential for cross-linking human immunodeficiency virus type 1 envelope spikes. J. Virol. 75:6682-6686.
287. Zwick, M.B., Labrijn A.F., Wang M., Spenlehauer C., Saphire E.O., Binley J.M., Moore J.P., Stiegler G., Katinger H., Burton D.R. and Parren P.W. (2001) Broadly neutralizing antibodies targeted to the membrane-proximal external region of human immunodeficiency virus type 1 glycoprotein gp41. J. Virol. 75:10892-10905.

## Claims

1. A stable HIV-1 pre-fusion envelope glycoprotein trimeric complex comprising, as a monomeric unit, (i) a first polypeptide comprising a modified gp120 of a HIV-1 isolate in which the amino acid Ala at position 492, said position being numbered by reference to the HIV-1_{JRFL} isolate, is substituted by a Cys; and (ii) a second polypeptide comprising a modified gp41 ectodomain of the same HIV-1 isolate, in which the amino acid Thr at position 596, said position being numbered by reference to the HIV-I_{JRFL} isolate, is substituted by a Cys, wherein the first and second polypeptides are bound to each other by a disulfide bond between the cysteine at position 492 of the first polypeptide and the cysteine at position 596 of the second polypeptide; and further **characterized in that** the amino acid Ile at position 559 of the second polypeptide, said position being numbered by reference to the HIV-1 HXB2 isolate, is substituted by any of the amino acids Phe, Asn, Pro, Gly or Arg, so as to increase oligomerization of the monomeric unit and stability of the trimeric complex.

2. The trimeric complex of claim 1, wherein the HIV-1 isolate comprises a subtype selected from the group consisting of clades A, B, C, D, E, F, G, H, and O.

3. The trimeric complex of claim 1 4, wherein the HIV-1 isolate is HIV-1_{JR-FL}, HIV-1_{DH123}, HIV-1_{GUN-1}, HIV-1_{89.6}, and HIV-1_{HX82}.

4. The trimeric complex of claim 2, wherein the first polypeptide further comprises a mutated furin recognition sequence.

5. The trimeric complex of claim 4, wherein the mutated furin recognition sequence has the sequence R-X-(R/K)-R.

6. The trimeric complex of claim 5, wherein the mutated furin recognition sequence has the sequence RRRKKR, RRRRKR, OR RRRRRR.

7. The trimeric complex of claim 2, wherein the first polypeptide is further **characterized by** (i) the absence of one or more canonical glycosylation sites present in wild-type HIV-1 gp120, (ii) the presence of one or more canonical glycosylation sites absent in wild-type HIV-1 gp120, or (iii) both (i) and (ii).

8. The trimeric complex of claim 2, wherein the mutation of amino acid I at position 559 is an I559P mutation.

9. A composition comprising the stable HIV-1 pre-fusion envelope glycoprotein trimeric complex of claim 1 and a pharmaceutically acceptable carrier.

10. The composition of claim 9, further comprising a cytokine and/or a chemokine.

11. The composition of claim 10, wherein the cytokine is interleukin-2, interleukin-4, interleukin-5, interleukin-12, interleukin-15, interleukin-18, GM-CSF, or a combination thereof.

12. The composition of claim 10, wherein the chemokine is SLC, ELC, MIP3α, MIP3β, IP-10, MIG, or a combination thereof.

13. The composition of claim 9, further comprising an adjuvant.

14. The composition of claim 13, wherein the adjuvant is alum, Freund's incomplete adjuvant, saponin, Quil A, Ribi Detox, monophosphoryl lipid A, a CpG oligonucleotide, CRL-1005, L-121, or a combination thereof.

15. Use of a prophylactically or therapeutically effective amount of the trimeric complex of claim 1 or the composition of claim 9 for the preparation of a medicament to be administered to a subject for eliciting an immune response in the subject against HIV-1 or an HIV-1 infected cell.

16. The use of claim 15, wherein the trimeric complex or the composition is to be administered in a single dose.

17. The use of claim 15, wherein the trimeric complex or the composition is to be administered in multiple doses.

18. The use of claim 15, wherein the trimeric complex or the composition is to be administered as part of a heterologous prime-boost regimen.

## Patentansprüche

1. Stabiler trimerer HIV-1 Präfusions-Hüllglykoprotein Komplex umfassend, als monomere Einheit,
(i) ein erstes Polypeptid umfassend ein modifiziertes gp120 eines HIV-1 Isolats in welchem die Aminosäure Ala in Position 492, die Position ist durch Bezug auf das HIV-1_{JRFL} Isolat nummeriert, durch ein Cys substituiert ist: und
(ii) ein zweites Polypeptid umfassend eine modifizierte gp41 Ektadomäne von dem gleichen HIV-1 Isolat, in welchem die Aminosäure Thr in Position 596, die Position ist durch Bezug auf das HIV-1_{JRFL} Isolat nummeriert, durch ein Cys substituiert ist,
wobei das erste und zweite Polypeptid durch eine Disulfidbindung zwischen dem Cystein in Position 492 des ersten Polypeptids und dem Cystein in Position 596 des zweiten Polypeptids aneinander gebunden sind; und weiterhin **dadurch gekennzeichnet, dass** die Aminosäure Ile in Position 559 des zweiten Polypeptids, die Position ist durch Bezug auf das HIV-1 HXB2 Isolat nummeriert, durch eine der Aminosäuren Phe, Asn, Pro, Gly oder Arg substituiert ist um die Oligomerisierung von der monomeren Einheit und die Stabilität des trimeren Komplexes zu erhöhen.

2. Trimerer Komplex nach Anspruch 1, wobei das HIV-1 Isolat einen Subtyp ausgewählt aus einer Gruppe bestehend aus den Kladen A, B, C, D, E, F, G, H, und O umfasst.

3. Trimerer Komplex nach Anspruch 1, wobei das HIV-1 Isolat ein HIV-1_{JR-FL}, HIV-1_{DH123}, HIV-1_{GUN-I}, HIV-1_{89.6}, und HIV-1_{HXB2} ist.

4. Trimerer Komplex nach Anspruch 2, wobei das erste Polypeptid weiterhin eine mutierte Furin-Erkennungssequenz umfasst,

5. Trimerer Komplex nach Anspruch 4, wobei die mutierte Furin-Erkennungssequenz die Sequenz R-X-(R/K)-R aufweist.

6. Trimerer Komplex nach Anspruch 5, wobei die mutierte Furin-Erkennungssequenz die Sequenz RRRKKR, RRRRKR, oder RRRRRR aufweist.

7. Trimerer Komplex nach Anspruch 2, wobei das erste Polypeptid weiterhin **gekennzeichnet ist durch**
(i) das Fehlen einer oder mehrerer kanonischer im HIV-1 gp120 Wildtyp vorhandenen Glykosylierungsstellen,
(ii) das Vorhandensein einer oder mehrerer kanonischer Glykosylierungsstellen, nicht vorhanden im HIV-1 gp120 Wildtyp, oder
(iii) Beides (i) und (ii).

8. Trimerer Komplex nach Anspruch 2, wobei die Mutation der Aminosäure I in Position 559 eine I559P Mutation ist.

9. Zusammensetzung umfassend den stabilen trimeren HIV-1 Präfusions-Hüllglykoprotein Komplex nach Anspruch 1 und einen pharmazeutisch geeigneten Träger.

10. Zusammensetzung nach Anspruch 9, weiterhin umfassend ein Zytokin und/oder ein Chemokin.

11. Zusammensetzung nach Anspruch 10, wobei das Zytokin ein lnterleukin-2, Interleukin-4, Interleukin-5, Interleukin-12, Interleukin-15, Interleukin-18, GM-CSF, oder eine Kombination davon ist.

12. Zusammensetzung nach Anspruch 10, wobei das Chemokin ein SLC, ELC, MIP3α, MIP3β, IP-10, MIG, oder eine Kombination davon ist.

13. Zusammensetzung nach Anspruch 9, weiterhin umfassend ein Adjuvans.

14. Zusammensetzung nach Anspruch 13, wobei das Adjuvans Alaun, unvollständiges Freund'sches Adjuvans, Saponin, Quil A, Ribi Detox, Monophosphoryl-Lipid A, CpG Oligonukleotid, CRL-1005, L-121, oder eine Kombination davon ist.

15. Verwendung einer prophylaktisch oder therapeutisch wirksamen Menge des trimeren Komplexes nach Anspruch 1 oder der Zusammensetzung nach Anspruch 9 zur Herstellung eines Medikaments, die einem Subjekt zu verabreichen ist um eine Immunantwort gegen HIV-1 oder eine HIV-1 infizierte Zelle in dem Subjekt hervorzurufen.

16. Verwendung nach Anspruch 15, wobei der trimere Komplex oder die Zusammensetzung in einer Einzeldosis zu verabreichen ist.

17. Verwendung nach Anspruch 15, wobei der trimere Komplex oder die Zusammensetzung in mehrfacher Dosis zu verabreichen ist.

18. Verwendung nach Anspruch 15, wobei der trimere Komplex oder die Zusammensetzung als Teil einer heterologen Prime-Boost-Behandlung zu verabreichen ist.

## Revendications

1. Complexe trimérique de glycoprotéine d'enveloppe de pré-fusion du VIH-1, stable, comprenant, comme motif monomère,
(i) un premier polypeptide comprenant une gp120 modifiés d'un isolat de VIH-1 dans laquelle l'acide aminé Ala en position 492, ladite position étant numérotée par référence à l'isolat de VIH-1_{JRFL}, est remplacé par une Cys; et
(ii) un deuxième polypeptide comprenant un ectodomaine de gp41 modifié du même isolat de VIH-1, dans lequel l'acide aminé Thr en position 596, ladite position étant numérotée par référence à l'isolat de VIH-1_{JRFL}, est remplacé par une Cys,
dans lequel les premier et deuxième polypeptides sont liés l'un à l'autre par un pont disulfure entre la cystéine en position 492 du premier polypeptide et la cystéine en position 596 du deuxième polypeptide; et **caractérisé en outre en ce que** l'acide aminé Ile en position 559 du deuxième polypeptide, ladite position étant numérotée par référence à l'isolat HXB2 du VIH-1, est remplacé par l'un quelconque des acides aminés Phe, Asn, Pro, Gly ou Arg, de manière à augmenter l'oligomérisation du motif monomère et la stabilité du complexe trimérique.

2. Complexe trimérique selon la revendication 1, dans lequel l'isolat du VIH-1 comprend un sous-type choisi dans le groupe constitué par les variantes A, B, C, D, E, F, G, H, et O.

3. Complexe trimérique selon la revendication 1, dans lequel l'isolat du VIH-1 est le VIH-1_{JR-FL}, le VIH-1_{DH123}, le VIH-1_{GUN-1}, le VIH-1_{89.6}, et le VIH-1_{HXB2}.

4. Complexe trimérique selon la revendication 2, dans lequel le premier polypeptide comprend en outre une séquence de reconnaissance de la furine mutée.

5. Complexe trimérique selon la revendication 4, dans lequel la séquence de reconnaissance de la furine mutée a la séquence R-X-(R/K)-R.

6. Complexe trimérique selon la revendication 5, dans lequel la séquence de reconnaissance de la furine mutée a la séquence RRRKKR, RRRRKR, ou RRRRRR.

7. Complexe trimérique selon la revendication 2, dans lequel le premier polypeptide est en outre **caractérisé par**
(i) l'absence d'un ou plusieurs sites de glycosylation canonique présents dans gp120 du VIH-1 de type sauvage,
(ii) la présence d'un ou plusieurs sites de glycosylation canonique absents dans gp120 du VIH-1 de type sauvage, ou
(iii) à la fois (i) et (ii).

8. Complexe trimérique selon la revendication 2, dans lequel la mutation de l'acide aminé I en position 559 est une mutation 1559P.

9. Composition comprenant le complexe trimérique de glycoprotéine d'enveloppe de pré-fusion du VIH-1 stable selon la revendication 1 et un support pharmaceutiquement acceptable.

10. Composition selon la revendication 9, comprenant en outre une cytokine et/ou une chimiokine.

11. Composition selon la revendication 10, dans laquelle la cytokine est l'interleukine-2, l'interleukine-4, l'interleukine-5, l'interleukine-12, l'interleukine-15, l'interleukine-18, le GM-CSF, ou une combinaison de ceux-ci.

12. Composition selon la revendication 10, dans laquelle la chimiokine est SLC, ELC, MIP3α, MIP3β, IP-10, MIG, ou une combinaison de celles-ci.

13. Composition selon la revendication 9, comprenant en outre un adjuvant.

14. Composition selon la revendication 13, dans laquelle l'adjuvant est l'alun, l'adjuvant incomplet de Freund, la saponine, le Quil A, le Ribi Detox, le monophosphoryle lipide A, un oligonucléotide CpG, le CRL-1005, le L-121, ou une combinaison de ceux-ci.

15. Utilisation d'une quantité efficace au plan prophylactique ou thérapeutique du complexe trimérique selon la revendication 1 ou de la composition selon la revendication 9 pour la préparation d'un médicament à administrer à un sujet pour susciter une réponse immunitaire chez le sujet contre le VIH-1 ou une cellule infectée par le VIH-1.

16. Utilisation selon la revendication 15, dans laquelle le complexe trimérique ou la composition doit être administré(e) en une dose unique.

17. Utilisation selon la revendication 15, dans laquelle le complexe trimérique ou la composition doit être administré(e) en doses multiples.

18. Utilisation selon la revendication 15, dans laquelle le complexe trimérique ou la composition doit être administré(e) dans le cadre d'un schéma de primo-immunisation/rappel hétérologue.
